# EUROPEAN PATENT APPLICATION

(11) **EP 4 332 098 A1**
(43) Date of publication of application: **06.03.2024**
(21) Application number: 22193139.7
(22) Date of filing: 31.08.2022
(51) Int. Cl.: C07D 401/04, C07D 401/06, C07D 403/06

(54) **CHIRAL SYNTHESIS OF NORNICOTINE AND NICOTINE**

(71) Applicant: Siegfried AG, 4800 Zofingen (CH); Contraf-Nicotex-Tobacco GmbH, 74072 Heilbronn (DE)
(72) Inventor: Straková, Karolína, 4800 Zofingen (CH); Müller, Dominik Simon, 4402 Frenkendorf (CH); van Vegten, Cornelis Hendrik, 5200 Brugg (CH); Zeller, Florian Daniel, 4632 Trimbach (CH); Debnar, Thomas, 6010 Kriens (CH)
(74) Representative: Isarpatent

(57) **Abstract**

The present invention relates to a method of producing nornicotine through enantioselective hydrosilylation of myosmine and a composition produced thereby, as well as a composition comprising nornicotine and a catalyst, wherein the catalyst is an organic chiral Lewis base, and a catalyst.

The present invention also relates to a method of producing nicotine or a salt thereof from myosmine through enantioselective hydrosilylation of myosmine to nornicotine and further reacting the nornicotine to nicotine or a salt thereof, and a composition produced thereby, as well as a composition comprising nicotine, or a salt thereof, and a catalyst, wherein the catalyst is an organic chiral Lewis base.

## Description

### Field of the invention

The present invention relates to a method of producing nornicotine through enantioselective hydrosilylation of myosmine and a composition produced thereby, as well as a composition comprising nornicotine and a catalyst, wherein the catalyst is an organic chiral Lewis base, and a catalyst.

The present invention also relates to a method of producing nicotine or a salt thereof from myosmine through enantioselective hydrosilylation of myosmine to nornicotine and further reacting the nornicotine to nicotine or a salt thereof, and a composition produced thereby, as well as a composition comprising nicotine, or a salt thereof, and a catalyst, wherein the catalyst is an organic chiral Lewis base.

The present invention relates particularly to the enantioselective preparation of nicotine from myosmine without the need of extensive purification steps and metal catalysts.

### Background of the Invention

Nicotine is a naturally occurring alkaloid used in various applications. Especially (S)-nicotine is used as active pharmaceutical ingredient to treat nicotine abuse and nicotine dependency. Success has further been reported when treating Tourette's syndrome, Alzheimer's disease, schizophrenia and other diseases related to disorders of the nervous system. Common ways of administration are gums, creams, transdermal patches, tablets, nasal sprays, and electric cigarettes.

Considerable quantities of nicotine are also used in agriculture as plant protectant or pesticide against aphids.

Natural nicotine is extracted from tobacco plants, a process that requires efficient purification steps to remove undesired, harmful impurities. The increasing demand of nicotine creates a need to offer ecological and economical ways to prepare synthetic nicotine in very pure form.

### State of the art

Nicotine ((S)-3-(1-methylpyrrolidin-2-yl)pyridine) and its enantiomers have been prepared for many years by various methods. Known syntheses are usually expensive and use agents that are problematic or even toxic for the environment. In order to obtain the desired (S) enantiomer of nicotine, it is often the case that first, the racemic mixture of (R) and (S) nicotine is produced and in a second step, the enantiomers are separated.

Pictet A. reported in 1904 already a synthesis for nicotine, including the use of tartaric acid to separate the enantiomers (Berichte der deutschen chemischen Gesellschaft, vol. 37, 1904, pages 1225-1235). Tartaric acid has then been used afterwards for decades (see for example: Aceto M. D., et al. (J. Med. Chem., 1979, vol. 22, 17 4-177)).

Katsuyama A. et al. reported a way of synthesizing nicotine using potassium tert-butanolate for the racemization of nicotine to prepare the starting material for further separation of the enantiomers (Bull. Spec. CORESTA Symposium, Winston-Salem, 1982, p. 15, S05, ISSN 0525-6240).

Further, EP 4 487 172 discloses a synthesis route over 5 different steps, offering a net yield of 37.7%.

WO 2017/117575 discloses a synthesis of nicotine using potassium hydride (KH) or sodium hydride (NaH) as strong base in tetrahydrofuran (THF) as solvent to obtain niciotinoyl-1-vinylpyrrolidin-2-one. Yield of (R/S)-nicotine is about 31%. Similar procedures as well as procedures for enantiomeric separation of the nicotine have been disclosed by Wang J. et al. (Wang J. et al., E. J Med. Chem., 2017, vol. 130, 15-25), by Desai D. et al. (Desai D. et al., J. Labeled Compd. Radiopharm, 2008, vol. 51, 226-230), Aceto M.D. et al., (Aceto M.D. et al., J. Med. Chem., 1979, vol. 2, 174-177) or by Bowman E. R. et al. (Bowman E.R. et al., Synthetic Comm., 1982, vol. 12, 11, 871-879).

In US 2016/0326134 a synthesis comprising the condensation of 1-methylpyrrolidin-2-one and methyl nicotinate in the presence of a strong base (as K tert-butoxide) at reflux to the intermediate potassium 1-methyl-3-nicotinoyl-4,5-dihydro-1H-pyrrol-2-olate is described, which then can be converted into the racemic mixture of R/S nicotine. Di-para-toluoyl-L-tartaric acid serves as resolution agent.

EP 2 484 673 (US 8,378,111) relies on well-known routes of synthesis and discloses D-DBTA (D-dibenzoyl ester of tartaric acid) as agent to separate the enantiomers.

WO 2016/065209 (EP 3 209 653, US 9,556,142) discloses a preparative way comprising three steps to the intermediate myosmine, including the condensation of N-vinylegous-pyrrolidinone and nicotinate ester in the presence of a metal hydride.

WO 2019/121644 (EP 3 728 215) teaches a one-pot synthesis of nicotine starting from ethyl nicotinate and N-vinylpyrrolidone. The method further comprises the steps of the reduction of myosmine to nornicotine and the methylation of the nornicotine to nicotine.

However, it is desirable to establish a synthesis route which opens the possibility of the enantioselective synthesis of (S)-nicotine or (R)-nicotine. This would render the resolution step redundant and would lead to a more efficient and ecological method for the production of enantiopure nicotine. In this regard, a possible strategy is the stereoselective reduction of 3-myosmine to (S)- or (R)-nornicotine, wherein the nornicotine is then further reacted to nicotine.

WO 2020/098978 (EP 3 653 617, US 2020/0157589) discloses a process for the production of (S)-nicotine from myosmine by enzymatic reduction and reductive methylation.

Buchwald S. et al. described the enantioselective catalytic hydrosilylation of myosmine to nornicotine using the titanium catalyst (S,S)-(EBTHI)TiF₂ (Buchwald S. et al., J. Am. Chem. Soc., 1996, vol. 118, 6784-6785). Through this method, it is possible to synthesize (S)- and (R)-nornicotine in high yields and enantiomeric excess (ee) with low catalyst loadings.

The enantioselective synthesis of (S)-nicotine from myosmine derivates through an Ir-catalyzed hydrogenation was reported by Zhou et al. (Zhou et al., J. Am. Chem. Soc., 2015, vol. 137, 90-93).

Fujieda et al. discloses the Ir-catalyzed assymetric hydrogenation of a pyrroline derivate (Fujieda et al., Synth. Commun., 2019, vol. 49, 814-822).

The disadvantage of the routes described above is the use of expensive metal-organic catalysts or enzymes. In the case of metal catalysts, the metal has to be separated from the nicotine (or nornicotine), especially in view of pharmaceutical and agritechnical applications of nicotine.

Lundrigan et al. shows the enantioselective imine reduction with boranes catalyzed by a diazaphosphenium triflate (Lundrigan et al., J. Am. Chem. Soc., 2019, vol. 141, 14083-14088). However, boranes are expensive and produce boric acid upon hydration, which is a substance on the Candidate List of Substances of Very High Concern of ECHA (European Chemicals Agency).

The stereoselective hydrosilylation of acylic imines in the presence of an organocatalyst is reported by Benaglia et al. (Benaglia et al., ChemCatChem, 2017, vol. 9, 941-945). Through this method, a variety of chiral amines were produced with moderate to high yields and enantiomeric excess (ee) at mild reaction conditions.

Y.-C. Xiao et al, Angew. Chem. Int. Ed., 2011, vol. 50, 10661-10664 discloses the asymmetric hydrosilylation of indoles using combined Lewis Base and Brønsted acid activation. X. Li et al. Org. Biomol. Chem., 2017, Vol. 15, 2422-2435 provides mechanistic investigations of the asymmetric hydrosilylation of ketimines with trichlorosilane.

Thus, an object of the present invention is the provision of a method for the enantioselective production of nornicotine and nicotine from myosime, particularly at low temperatures, without the application of metal catalysts, biocatalysts, boranes and/or extensive racemate splitting, wherein the catalyst applied is easy to produce with readily accessible starting materials.

### Summary of the invention

The present invention offers a novel method for the enantioselective synthesis of nornicotine and nicotine. Inventors found a method of such syntheses without the application of a metal catalyst. This reduces the amount of possible metal impurities in the nornicotine as well as in the end product nicotine, which is particularly useful when it is to be used as active pharmaceutical ingredient, being subject to strict purity regulations. Furthermore, metals used as or in catalysts for hydrogenation reactions are often expensive precious metals such as iridium, platinum or palladium. Superseding the need of a metal catalyst thus leads to cost reduction and to a more ecological way of synthesizing nicotine. Also, the present invention particularly offers an enantioselective one-pot synthesis of nicotine from myosmine, which renders the resolution step redundant.

In a first aspect, the present invention relates to a method of producing nornicotine from myosmine, comprising reacting myosmine with at least one hydrosilane using a catalyst, wherein the catalyst is an organic chiral Lewis base. The invention also relates in another aspect to a composition which is produced by said method, particularly wherein the catalyst is not removed from the reaction mixture.

In a further aspect, the present invention relates to a composition comprising nornicotine, optionally a mixture of (R)- and (S)-nornicotine, and a catalyst, wherein the catalyst is an organic chiral Lewis base.

In an even further aspect, the present invention relates to a method of producing nicotine or a salt thereof from myosmine, comprising reacting myosmine with at least one hydrosilane in the presence of a catalyst to produce nornicotine, wherein the catalyst is an organic chiral Lewis base; reacting the nornicotine to nicotine; and optionally reacting the nicotine to a salt thereof. The invention also relates to a composition which is produced by said method, wherein the catalyst is not separated from the reaction mixture.

Additionally disclosed is a composition, comprising nicotine, optionally a mixture of (R)- and (S)-nicotine, or a salt thereof, and a catalyst, wherein the catalyst is an organic chiral Lewis base.

Also disclosed is a catalyst as defined in patent claim 25, and the use thereof in a method of producing nornicotine and/or nicotine.

Further aspects and embodiments of the invention are disclosed in the dependent claims and can be taken from the following description, figures and examples, without being limited thereto.

### Figures

The enclosed drawings should illustrate embodiments of the present invention and convey a further understanding thereof. In connection with the description they serve as explanation of concepts and principles of the invention. Other embodiments and many of the stated advantages can be derived in relation to the drawings. The elements of the drawings are not necessarily to scale towards each other. Identical, functionally equivalent and acting equal features and components are denoted in the figures of the drawings with the same reference numbers, unless noted otherwise.
Figure 1 shows the effect of a variation of reaction temperature in the reduction of myosmine on the enantiomeric excess (ee) with regard to (R)-nornicotine according to Example 1A.
Figure 2 shows the effect of a variation of catalyst concentration used in the reduction of myosmine on the enantiomeric excess (ee) with regard to (R)-nornicotine according to Example 1A.
Figure 3 shows schematically exemplary process steps of the production of nicotine according to an aspect of the present invention.

### Detailed description of the present invention

### Definitions

Unless defined otherwise, technical and scientific terms used herein have the same meaning as commonly understood by one of ordinary skill in the art to which this invention belongs.

Amounts within the present invention are given in wt.%, unless stated otherwise or clear from context.

Room temperature is 20 to 25°C, e.g. 22°C.

In structural formulae, Bn is a benzyl group, Bu is a n-butyl group, Et is an ethyl group, Me is a methyl group, Ph is a phenyl group, Piv is a pivalyl or pivaloyl group (t-BuC(O)), Py is a pyridine group, tBu is a tert-butyl group.

Pseudohalogen groups in the context of the present invention are not particularly limited. As is generally understood, pseudohalogen groups are polyatomic analogues of halogens.

According to certain embodiments, pseudohalogen groups are -SCN, -NCS, -CN, -N₃, -OCN, - NCO, or -SeCN.

An alkenyl group within the scope of the present invention comprises at least one double bond between two carbon atoms in a residue that is not aromatic and/or heteroaromatic, i.e. can comprise also more than one double bond. The position of the double bond is not particularly restricted and can be e.g. within an alkyl chain and/or at a terminal end.

An alkynyl group within the scope of the present invention comprises at least one triple bond between two carbon atoms in a residue that is not aromatic and/or heteroaromatic, i.e. can comprise also more than one triple bond. The position of the triple bond is not particularly restricted and can be e.g. within an alkyl chain and/or at a terminal end.

An aralkyl group within the scope of the present invention is a group wherein an alkyl residue is substituted with at least one aromatic residue. Within the scope of the invention, it is not excluded that the at least one aromatic residue is substituted with at least one further alkyl residue.

An alkylaryl group within the scope of the present invention is a group wherein an aromatic residue is substituted with at least one alkyl residue. Within the scope of the invention, it is not excluded that the at least one alkyl residue is substituted with at least one further aromatic residue.

A heterocycloalkyl group within the scope of the present invention is a cyclic alkyl group with at least one heteroatom in the alkyl chain, e.g. comprises O, N and/or S.

An alkyl heteroaromatic group within the scope of the present is a group wherein an alkyl residue is substituted with at least one heteroaromatic residue. Within the scope of the invention, it is not excluded that the at least one heteroaromatic residue is substituted with at least one further alkyl residue.

A heteroaromatic group with at least one alkyl residue within the scope of the present invention is a group wherein a heteroaromatic residue is substituted with at least one alkyl residue. Within the scope of the invention, it is not excluded that the at least one alkyl residue is substituted with at least one further heteroaromatic and/or aromatic residue.

An alkyl (hetero)cycloalkyl group within the scope of the present invention is a group wherein a (hetero)cycloalkyl residue is substituted with at least one alkyl residue. Within the scope of the invention, it is not excluded that the at least one alkyl residue is substituted with at least one further (hetero)cycloalkyl residue.

A (hetero)cycloalkyl alkyl group within the scope of the present invention wherein an alkyl residue is substituted with at least one (hetero)cycloalkyl residue. Within the scope of the invention, it is not excluded that the at least one (hetero)cycloalkyl residue is substituted with at least one further alkyl residue.

A carboxylic acid ester group within the scope of the present invention is a group wherein a carbon containing residue R', like a linear, branched and/or cyclic alkyl residue and/or a (hetero)aromatic residue, is bonded via the functional group (CO)-O to form R'(CO)-O-, e.g. Me-(CO)-O or Et-(CO)-O.

An alkyl and/or (hetero)aryl oxycarbonyl group within the scope of the present invention is a residue wherein a linear, branched and/or cyclic alkyl and/or an aryl and/or an aryl residue R' is bonded via the functional group O-(CO) to form R'O-(CO)-.

A carboxamide group within the scope of the present invention is either a group having the formula R'(CO)(NR"), wherein a carbon containing residue R' like a linear, branched and/or cyclic alkyl residue and/or a (hetero)aromatic residue is bonded via the functional group (CO)NR"-, wherein R" can be H, a carbon containing group, or a different element or group, or a group having the formula (NR"R‴)(CO)-, wherein R" and R'" can be H, a carbon containing group, or a different element or group, where an amine derived group (NR"R‴) is bonded via (CO).

The term "organic" within the scope of the present invention is meant in a strict sense, i.e. excluding metal-organic compounds. Further, an organic chiral Lewis base, within the scope of the invention, also does not include biological material such as enzymes.

If in the context of the present invention a residue or compound part is a part of a polymer and/or is connected to the polymer, the polymer is not particularly restricted. Suitable polymers include e.g. polyethylene (PE)-based polymers, e.g. PE itself, polypropylene (PP)-based poylmers, e.g. PP itself, polystyrene(PS)-based polymers, e.g. PS itself, polyethylene-glycol (PEG)-based polymers like modified PEG, poly(meth)actrylate-based polymers, etc., and particularly water-soluble polymers and/or dendrimers. A polymer can also be used to adhere a compound, e.g. a catalyst in line with the present disclosure, to a specific surface, e.g. in a micro-reactor and/or a microfluidic cell for achieving sufficient catalytic activity, etc. Thus, it is also encompassed that if a polymer is connected or part of a molecule, e.g. a catalyst, that the polymer is connected to a particle which can be functionalized but is not particularly restricted, e.g. functionalized gold nanoparticles, magnetic particles like magnetic nano-Fe₃O₄, silica or modified silica particles, e.g. silica or modified silica nanoparticles, that the polymer is connected to a further structure, e.g. a reactor and/or flow cell, e.g. a micro-reactor and/or a microfluidic cell, which all are not particularly restricted. In this regard, it should be understood that the catalyst used in the present invention does not neee to be attached to a (nano)particle only through a polymer in case it is attached.

In the context of the present invention, a Lewis base is not particularly limited. As is generally understood, a Lewis base is an electron donating compound which has a filled orbital containing an electron pair which is not involved in bonding.

In the context of the present invention, alkoxy groups with a linear, branched, cyclic and/or aromatic residue having 1 to 20 C atoms that can comprise one or more heteroatoms selected from O, N, S and P are not particularly restricted, as well as also carboxylic ester groups with a linear, branched, cyclic and/or aromatic residue having 1 to 20 C atoms that can comprise one or more heteroatoms selected from O, N, S and P, etc. In the residues, it is thus not excluded that more than one of a linear alkyl residue, a branched alkyl residue, a cyclic alkyl residue and an aromatic residue, all of which can comprise one or more heteroatoms selected from O, N, S and P. are combined, e.g. an aromatic residue, an alkyl residue, and a heterocyclic residue, or an alkyl residue and a heteroaromatic residue, etc. Particularly, also the alkyl residue can also comprise one or more heteroatoms selected from O, N, S, P, thus also forming e.g. polyethers.

Hydrosilanes are not particularly limited in the context of this invention, a hydrosilane being a compound with at least one Si-H bond. Hydrosilanes are tetravalent silicon compounds containing one or more Si-H bond. Other bonds can comprise organic residues as well as inorganic residues, particularly halogens like F, Cl, Br, I, and/or pseudohalogens.

As used herein, the term "chiral" refers to compounds which have at least one stereocenter.

Residue Q₁ herein is O or S, particularly O. Residue X₀ is N or CR_{A1}, particularly N. Y₀ is chosen from a carbonyl group, a thiocarbonyl group, and CR₁₆R₁₇, particularly from a carbonyl group and CR₁₆R₁₇. Z₁ is chosen from a carbonyl group, a thiocarbonyl group, and CR₂₀ₐR_{20b}, particularly from a carbonyl group and CR₂₀ₐR_{20b}.

For Q₁, it has been particularly found that the residue can play a particular role in pre-arranging the hydrosilane in the catalytic reaction. X₀ has been found to play a role in the steric pre-arranging of myosmine. Likewise, Y₀ and Z₁ have been found to be able to play a role in the pre-arranging of the myosmine, thus enabling attack of the hydrogen from the hydrosilane to a specific side of the myosmine, thus enabling stereospecific synthesis of (R)- or (S)-nornicotine. Furthermore, the nitrogen in ring B adjacent to the carbon atom connecting to the bridge also has been found to be able to play a role for pre-arranging the hydrosilane, so that the hydrogen of the hydrosilane can be brought in close proximity of myosmine in case it is pre-arranged. A particular role in the pre-arrangement of the myosmine has been also found for residue R₁ₐ, particularly if the residue is bulkier. Particularly, a good stereospecific pre-arrangement of myosmine has been found for Y₀, respectively Z₁, being C=O, and/or for R₁ₐ being CR₁₀R₁₁(OR₁₂ₐ).

R₁ₐ is a saturated and/or unsaturated linear, branched, cyclic and/or aromatic and/or heteroaromatic hydrocarbon residue with 1 to 100 C atoms that can contain heteroatoms selected from the group of O, N, S and P and that can optionally be substituted with at least one of
- F;
- Cl;
- Br;
- I;
- pseudohalogen groups;
- OSiR₂₃R₂₄R₂₅, wherein R₂₃, R₂₄, and R₂₅ can be the same or different and are selected from H, linear alkyl groups with 1 to 4 C atoms that can optionally be substituted with at least one of F, Cl, Br, I, pseudohalogen groups, alkoxy groups with 1 to 20 C atoms, OH, NO₂ and NH₂, branched alkyl group with 3 to 6 C atoms that can optionally be substituted with at least one of F, Cl, Br, I, pseudohalogen groups, alkoxy groups with 1 to 20 C atoms, OH, NO₂ and NH₂, aromatic groups with 6 to 12 C atoms that can optionally be substituted with at least one of F, Cl, Br, I, pseudohalogen groups, alkoxy groups with 1 to 20 C atoms, OH, NO₂ and NH₂, and aralkyl groups with 7 to 10 C atoms that can optionally be substituted with at least one of F, Cl, Br, I, pseudohalogen groups, alkoxy groups with 1 to 20 C atoms, OH, NO₂ and NH₂, preferably wherein R₂₃, R₂₄, and R₂₅ are not all hydrogen at the same time and/or wherein R₂₃, R₂₄, and R₂₅ are not all methyl at the same time, further preferably wherein all of R₂₃, R₂₄, and R₂₅ are ethyl;
- carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂;
- alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂;
- carboxamide groups (including (CO)NH₂) with 1 to 20 C atoms that can optionally be substituted with at least one of F, Cl, Br, I, pseudohalogen groups, alkoxy groups with 1 to 20 C atoms, OH, SH, NO₂ and NH₂;
- optionally substituted alkoxy (linear, branched and/or cyclic), aryloxy, heteroaryloxy, alkenyloxy and/or alkynyloxy groups with 1 to 20 C atoms and/or heteroatoms;
- OH;
- SH;
- NO₂;
- NH₂; and
- amines of formula NR₁₈R₁₉.

Thus, residue R₁ₐ e.g. also encompasses ether functional groups in the backbone that may be substituted with carboxylic ester functions, carboxamide functions, amine functions, etc.

In the optionally substituted alkoxy - which can be linear, branched and/or cyclic, aryloxy, heteroaryloxy, alkenyloxy and/or alkynyloxy group, the substituents are not particularly restricted and can comprise linear, branched, cyclic and/or aromatic and/or heteroaromatic hydrocarbon residue with 1 to 20 C atoms that can contain heteroatoms selected from the group of O, N, S and P and that can optionally be substituted with at least one of F; Cl; Br; I; pseudohalogen groups; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; carboxamide groups with 1 to 20 C atoms that can optionally be substituted with at least one of F, Cl, Br, I, pseudohalogen groups, alkoxy groups with 1 to 20 C atoms, OH, NO₂ and NH₂; OH; NO₂; NH₂; and amines of formula NR₁₈R₁₉. Thus, it is also possible that ether functional groups are present even if the backbone of R₁ₐ contains an ether function.

R₁₈ and R₁₉ are the same or different and can be chosen from H, linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂, with the provisio that not both of R₁₈ and R₁₉ are H.

According to certain embodiments, R₁ₐ is chosen from CR₁₀R₁₁R₁₂ and (CH₂)R₁ for all compounds given, which will be defined also herein below.

According to certain embodiments, particularly in the compound of Formula III and compounds derived thereof, R₁ₐ is selected from the group consisting of
linear alkyl groups with 1 to 10 C atoms that can optionally be substituted with at least one of F, Cl, Br, I, pseudohalogen groups, alkoxy groups with 1 to 20 C atoms, carboxylic acid ester groups with 1 to 20 C atoms, alkyl and/or (hetero)aryl oxycarbonyl groups with 1 to 20 C atoms carboxamide groups with 1 to 20 C atoms (including (CO)NH₂), OH, SH, NO₂, NH₂, and amines of formula NR₁₈R₁₉;
branched alkyl groups with 3 to 10 C atoms that can optionally be substituted with at least one of F, Cl, Br, I, pseudohalogen groups, alkoxy groups with 1 to 20 C atoms, carboxylic acid ester groups with 1 to 20 C atoms, alkyl and/or (hetero)aryl oxycarbonyl groups with 1 to 20 C atoms carboxamide groups with 1 to 20 C atoms (including (CO)NH₂), OH, SH, NO₂, NH₂, and amines of formula NR₁₈R₁₉;
aralkyl groups with 7 to 24 C atoms that can optionally be substituted with at least one of F, Cl, Br, I, pseudohalogen groups, alkoxy groups with a linear, branched, cyclic and/or aromatic residue having 1 to 20 C atoms that can comprise one or more heteroatoms selected from O, N, S and P, carboxylic ester groups with a linear, branched, cyclic and/or aromatic residue having 1 to 20 C atoms that can comprise one or more heteroatoms selected from O, N, S and P, oxycarbonyl groups with a linear, branched, cyclic and/or (hetero)aromatic residue having 1 to 20 C atoms that can comprise one or more heteroatoms selected from O, N, S and P, carboxamide groups with a linear, branched, cyclic and/or aromatic residue having 1 to 20 C atoms that can comprise one or more heteroatoms selected from O, N, S and P, OH, SH, NO₂ and NH₂;
alkylaryl groups with 7 to 24 C atoms that can optionally be substituted with at least one of F, Cl, Br, I, pseudohalogen groups, alkoxy groups with a linear, branched, cyclic and/or aromatic residue having 1 to 20 C atoms that can comprise one or more heteroatoms selected from O, N, S and P, carboxylic ester groups with a linear, branched, cyclic and/or aromatic residue having 1 to 20 C atoms that can comprise one or more heteroatoms selected from O, N, S and P, oxycarbonyl groups with a linear, branched, cyclic and/or (hetero)aromatic residue having 1 to 20 C atoms that can comprise one or more heteroatoms selected from O, N, S and P, carboxamide groups with a linear, branched, cyclic and/or aromatic residue having 1 to 20 C atoms that can comprise one or more heteroatoms selected from O, N, S and P, OH, SH, NO₂ and NH₂;
cycloalkyl groups with 3 to 20 C atoms that can optionally be substituted with at least one of F, Cl, Br, I, pseudohalogen groups, alkoxy groups with 1 to 20 C atoms, carboxylic acid ester groups with 1 to 20 C atoms, alkyl and/or (hetero)aryl oxycarbonyl groups with 1 to 20 C atoms carboxamide groups with 1 to 20 C atoms (including (CO)NH₂), OH, SH, NO₂, NH₂, and amines of formula NR₁₈R₁₉;
heterocycloalkyl groups with 1 to 15 C atoms that can optionally be substituted with at least one of F, Cl, Br, I, pseudohalogen groups, alkoxy groups with 1 to 20 C atoms, carboxylic acid ester groups with 1 to 20 C atoms, alkyl and/or (hetero)aryl oxycarbonyl groups with 1 to 20 C atoms carboxamide groups with 1 to 20 C atoms (including (CO)NH₂), OH, SH, NO₂, NH₂, and amines of formula NR₁₈R₁₉;
aromatic and/or heteroaromatic groups with 2 to 40 C atoms that can optionally be substituted with at least one of F, Cl, Br, I, pseudohalogen groups, alkoxy groups with a linear, branched, cyclic and/or aromatic residue having 1 to 20 C atoms that can comprise one or more heteroatoms selected from O, N, S and P, carboxylic ester groups with a linear, branched, cyclic and/or aromatic residue having 1 to 20 C atoms that can comprise one or more heteroatoms selected from O, N, S and P, oxycarbonyl groups with a linear, branched, cyclic and/or (hetero)aromatic residue having 1 to 20 C atoms that can comprise one or more heteroatoms selected from O, N, S and P, carboxamide groups with a linear, branched, cyclic and/or aromatic residue having 1 to 20 C atoms that can comprise one or more heteroatoms selected from O, N, S and P, OH, SH, NO₂ and NH₂;
alkyl heteroaromatic groups with 3 to 50 C atoms that can optionally be substituted with at least one of F, Cl, Br, I, pseudohalogen groups, alkoxy groups with a linear, branched, cyclic and/or aromatic residue having 1 to 20 C atoms that can comprise one or more heteroatoms selected from O, N, S and P, carboxylic ester groups with a linear, branched, cyclic and/or aromatic residue having 1 to 20 C atoms that can comprise one or more heteroatoms selected from O, N, S and P, oxycarbonyl groups with a linear, branched, cyclic and/or (hetero)aromatic residue having 1 to 20 C atoms that can comprise one or more heteroatoms selected from O, N, S and P, carboxamide groups with a linear, branched, cyclic and/or aromatic residue having 1 to 20 C atoms that can comprise one or more heteroatoms selected from O, N, S and P, OH, SH, NO₂ and NH₂;
heteroaromatic groups with at least one alkyl residue with 3 to 50 C atoms that can optionally be substituted with at least one of F, Cl, Br, I, pseudohalogen groups, alkoxy groups with a linear, branched, cyclic and/or aromatic residue having 1 to 20 C atoms that can comprise one or more heteroatoms selected from O, N, S and P, carboxylic ester groups with a linear, branched, cyclic and/or aromatic residue having 1 to 20 C atoms that can comprise one or more heteroatoms selected from O, N, S and P, oxycarbonyl groups with a linear, branched, cyclic and/or (hetero)aromatic residue having 1 to 20 C atoms that can comprise one or more heteroatoms selected from O, N, S and P, carboxamide groups with a linear, branched, cyclic and/or aromatic residue having 1 to 20 C atoms that can comprise one or more heteroatoms selected from O, N, S and P, OH, SH, NO₂ and NH₂;
alkyl cycloalkyl groups with 4 to 24 C atoms that can optionally be substituted with at least one of F, Cl, Br, I, pseudohalogen groups, alkoxy groups with 1 to 20 C atoms, carboxylic acid ester groups with 1 to 20 C atoms, alkyl and/or (hetero)aryl oxycarbonyl groups with 1 to 20 C atoms carboxamide groups with 1 to 20 C atoms (including (CO)NH₂), OH, SH, NO₂, NH₂, and amines of formula NR₁₈R₁₉;
cycloalkyl alkyl groups with 4 to 24 C atoms that can optionally be substituted with at least one of F, Cl, Br, I, pseudohalogen groups, alkoxy groups with 1 to 20 C atoms, carboxylic acid ester groups with 1 to 20 C atoms, alkyl and/or (hetero)aryl oxycarbonyl groups with 1 to 20 C atoms carboxamide groups with 1 to 20 C atoms (including (CO)NH₂), OH, SH, NO₂, NH₂, and amines of formula NR₁₈R₁₉;
carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂;
alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂; and
carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂. Therein, R₁₈ and R₁₉ are as defined above.

Before the invention is described in exemplary detail, it is to be understood that this invention is not limited to the particular component parts of the process steps of the methods described herein as such methods may vary. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. For example, the term "a" as used herein can be understood as one single entity or in the meaning of "one or more" entities. It is also to be understood that plural forms include singular and/or plural referents unless the context clearly dictates otherwise. It is moreover to be understood that, in case parameter ranges are given which are delimited by numeric values, the ranges are deemed to include these limitation values.

In a first aspect, the invention relates to a method of producing nornicotine from myosmine. The method comprises reacting myosmine with at least one hydrosilane in the presence of, respectively using, a catalyst, wherein the catalyst is an organic chiral Lewis base. The organic chiral Lewis base is therein not particularly restricted, as long as it is organic and chiral, i.e. has a stereocenter.. The inventors found that it is surprisingly possible to react myosmine to nornicotine with high efficiency with an organic chiral Lewis base, not requiring metal-containing catalysts.

The reaction conditions are not particularly limited. The conversion of mysomine to nornicotine generally can be described with the following reaction scheme:

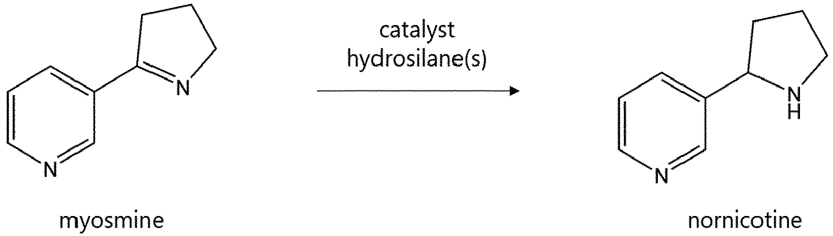

According to certain embodiments, the catalyst is a catalyst is a catalyst of the following formula I:

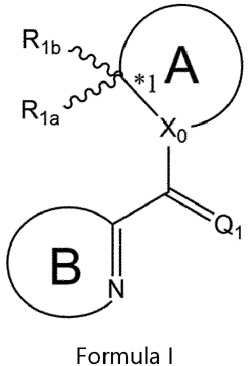

In the compound of Formula I, as well as any of the following formulas directly or indirectly derived therefrom, ring A may comprise, besides X₀ and the chiral C with residues R₁ₐ and R_{1b} atom (with chiral center *1, residues R₁ₐ and R_{1b} being different), a carbonyl group, a thiocarbonyl group, substituted or unsubstituted C atoms and/or substituted or unsubstituted heteroatoms, the heteroatoms not being particularly restricted, e.g. being O, N and/or S, particularly N, thus including ring members like NH (=NH) and NR (=NR), wherein R is a substituent as described in the following. The number of substituted or unsubstituted C-atoms and/or substituted or unsubstituted heteroatoms is not particularly limited, and can be e.g. between 0 and 10, i.e. there can also be no substituted or unsubstituted heteroatoms and/or no substituted or unsubstituted C-atoms besides the chiral C atom with chiral center *1. If more than one substituent is on C-atoms and or heteroatoms in ring A, the more than one substituent can be the same or different. According to preferred embodiments, ring A only comprises single bonds within the ring structure.

The compound of Formula I thus represents a molecule with a bridge (C=Q₁) connecting ring A and ring B, the bridge (C=Q₁) being connected to atom X₀ in ring A, atom X₀ being adjacent to the chiral carbon atom at center *1 with the two different residues R₁ₐ and R_{1b}, and the bridge (C=Q₁) further being connected to a carbon atom in ring B, the carbon atom adjacent to a nitrogen atom N and connected via a double bond to the nitrogen atom. Within this invention, ring B represents a ring comprising conjugated double bonds that is particularly aromatic, i.e. is fully conjugated, planar and follows the Hueckel's rule.

According to certain embodiments, the substituents comprise any of, particularly at least one of or two or more of F, Cl, Br, I, pseudohalogen groups, alkoxy groups with 1 to 20 C atoms, OH, SR₂₁ (i.e. thioethers), S(CO)R₂₂ (i.e.g thioesters), NO₂, NH₂, amines of formula NR₁₈R₁₉, (NR₁₈'R₁₉'R₂₀')⁺, linear alkyl groups with 1 to 10 C atoms that can optionally be substituted with at least one of F, Cl, Br, I, pseudohalogen groups, alkoxy groups with 1 to 20 C atoms, OH, NO₂ and NH₂; linear alkenyl groups and/or alkynyl groups with 2 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; branched alkenyl and/or alkynyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂; with the provisio that two or more of the substituents can form one ring or more rings, optionally wherein at least one substituent is part of a polymer; wherein R₁₈, R₁₉, R₁₈', R₁₉', R₂₀', R₂₁, and R₂₂ are the same or different and can be chosen from H; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂, with the provisio that not both of R₁₈ and R₁₉ are H.

This means that, according to certain embodiments, the substituents may comprise any of, particularly at least one of or two or more of F, Cl, Br, I, pseudohalogen groups, alkoxy groups with 1 to 20 C atoms, OH, SR₂₁, S(CO)R₂₂, NO₂, NH₂, amines of formula NR₁₈R₁₉, (NR₁₈'R₁₉'R₂₀')⁺, wherein R₁₈, R₁₉ R₁₈', R₁₉', R₂₀', R₂₁, and R₂₂ are the same or different and can be chosen from H; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂, with the provisio that not both of R₁₈ and R₁₉ are H.

Alternatively or in addition, the substituents may comprise any of, particularly at least one of or two or more of,
- linear alkyl groups with 1 to 10 C atoms that can optionally be substituted with at least one of F, Cl, Br, I, pseudohalogen groups, alkoxy groups with 1 to 20 C atoms, OH, NO₂ and NH₂;
- linear alkenyl groups and/or alkynyl groups with 2 to 10 C atoms that can optionally be substituted with at least one of F, Cl, Br, I, pseudohalogen groups, alkoxy groups with 1 to 20 C atoms, OH, NO₂ and NH₂;
- branched alkyl groups with 3 to 10 C atoms that can optionally be substituted with at least one of F, Cl, Br, I, pseudohalogen groups, alkoxy groups with 1 to 20 C atoms, OH, NO₂ and NH₂;
- branched alkenyl and/or alkynyl groups with 3 to 10 C atoms that can optionally be substituted with at least one of F, Cl, Br, I, pseudohalogen groups, alkoxy groups with 1 to 20 C atoms, OH, NO₂ and NH₂;
- aralkyl groups with 7 to 24 C atoms that can optionally be substituted with at least one of F, Cl, Br, I, pseudohalogen groups, alkoxy groups with 1 to 20 C atoms, OH, NO₂ and NH₂;
- alkylaryl groups with 7 to 24 C atoms that can optionally be substituted with at least one of F, Cl, Br, I, pseudohalogen groups, alkoxy groups with 1 to 20 C atoms, OH, NO₂ and NH₂;
- cycloalkyl groups with 3 to 20 C atoms that can optionally be substituted with at least one of F, Cl, Br, I, pseudohalogen groups, alkoxy groups with 1 to 20 C atoms, OH, NO₂ and NH₂;
- heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂;
- aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂;
- alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂;
- heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂;
- alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂;
- cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂;
- alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂;
- heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂;
- carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂;
- alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and
- carboxamide groups with 1 to 20 C atoms that can optionally be substituted with at least one of F, Cl, Br, I, pseudohalogen groups, alkoxy groups with 1 to 20 C atoms, OH, NO₂ and NH₂;
with the provisio that two or more of the substituents can form one ring or more rings, optionally wherein at least one substituent is part of a polymer.

Furthermore, according to preferred embodiments, Ring A can be a 4- to 6-membered ring, particularly a 5-membered ring. According to certain embodiments, Ring A can comprise a lactame unit.

The respective interpretation of residues also applies for the following groups in ring B as well as for all other residues, particularly residues R₄₁, R₁ₐ, R_{1b}, R₁, R₂, R₃, R₄, R₄ₐ, R_{4b}, R₅, R₅ₐ, R₆, R₆ₐ, R_{6b}, R_{6c}, R_{6d}, R₇ₐ, R_{7b}, R_{7c}, R_{7d}, R₇ₑ, R₈, R₉, R₁₀, R₁₁, R₁₂, R₁₂ₐ, R₁₃, R₁₃ₐ, R_{13b}, R₁₄, R₁₅, R₁₆, R₁₇, R₁₈, R₁₉, R₂₀ₐ, R_{20b}, Y₁, Y₂.

In the compound of Formula I, as well as any of the following formulas directly or indirectly derived therefrom, ring B is a five- or six-membered ring comprising conjugated double bonds that is particularly aromatic, i.e. is fully conjugated, planar and follows the Hueckel's rule, and that comprises, besides the carbon atom connected to the bridge and the adjacent nitrogen atom, substituted or unsubstituted C atoms and/or substituted or unsubstituted heteroatoms, wherein the substituents can comprise any one of F; Cl; Br; I; pseudohalogen groups; alkoxy groups with 1 to 20 C atoms; OH; NO₂; NH₂; amines of formula NR₁₈R₁₉; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂; with the provisio that two or more of the substituents can form one ring or more rings.

In the compound of Formula I, as well as any of the following formulas directly or indirectly derived therefrom, X₀ is selected from N and CR_{A1}, wherein R_{A1} is selected from the group consisting of H, linear alkyl groups with 1 to 6 C atoms that can optionally be substituted with at least one of F, Cl, Br, I, pseudohalogen groups, alkoxy groups with 1 to 6 C atoms, OH, NO₂ and NH₂; branched alkyl groups with 3 to 6 C atoms that can optionally be substituted with at least one of F, Cl, Br, I, pseudohalogen groups, alkoxy groups with 1 to 6 C atoms, OH, NO₂ and NH₂; cycloalkyl groups with 3 to 7 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 6 C atoms that can optionally be substituted with at least one of F, Cl, Br, I, pseudohalogen groups, alkoxy groups with 1 to 6 C atoms, OH, NO₂ and NH₂; aromatic and/or heteroaromatic groups with 2 to 6 carbon atoms that can optionally be substituted with at least one of F, Cl, Br, I, pseudohalogen groups, alkoxy groups with 1 to 6 C atoms, OH, NO₂ and NH₂; carboxylic acid ester groups with 1 to 6 C atoms that can optionally be substituted with at least one of F, Cl, Br, I, pseudohalogen groups, alkoxy groups with 1 to 6 C atoms, OH, NO₂ and NH₂; alkyl and/or (hetero)aryl oxycarbonyl groups with 1 to 6 C atoms that can optionally be substituted with at least one of F, Cl, Br, I, pseudohalogen groups, alkoxy groups with 1 to 6 C atoms, OH, NO₂ and NH₂; and carboxamide groups with 1 to 6 C atoms that can optionally be substituted with at least one of F, Cl, Br, I, pseudohalogen groups, alkoxy groups with 1 to 6 C atoms, OH, NO₂ and NH₂.

In the compound of Formula I, as well as any of the following formulas directly or indirectly derived therefrom, Q₁ is O or S.

In the compound of Formula I, as well as any of the following formulas directly or indirectly derived therefrom, R₁ₐ and R_{1b} are different.

In the compound of Formula I, as well as any of the following formulas directly or indirectly derived therefrom, R₁ₐ is a saturated and/or unsaturated linear, branched, cyclic and/or aromatic and/or heteroaromatic hydrocarbon residue with 1 to 100 C atoms that can contain heteroatoms selected from the group of O, N, S and P and that can optionally be substituted with at least one of F; Cl; Br; I; pseudohalogen groups; carboxylic acid ester groups with 1 to 20 C atoms that can optionally be substituted with at least one of F, Cl, Br, I, pseudohalogen groups, OSiR₂₃R₂₄R₂₅,alkoxy groups with 1 to 20 C atoms, OH, SH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂; carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂; optionally substituted alkoxy (linear, branched and/or cyclic), aryloxy, heteroaryloxy, alkenyloxy and/or alkynyloxy groups with 1 to 20 C atoms and/or heteroatoms; OH; SH; NO₂; NH₂; and amines of formula NR₁₈R₁₉.

In the compound of Formula I, as well as any of the following formulas directly or indirectly derived therefrom, R₁₈ and R₁₉ are the same or different and can be chosen from H, linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂, with the provisio that not both of R₁₈ and R₁₉ are H.

In the compound of Formula I, as well as any of the following formulas directly or indirectly derived therefrom, R₂₃, R₂₄, and R₂₅ can be the same or different and are selected from H, linear alkyl groups with 1 to 4 C atoms that can optionally be substituted with at least one of F, Cl, Br, I, pseudohalogen groups, alkoxy groups with 1 to 20 C atoms, OH, NO₂ and NH₂, branched alkyl group with 3 to 6 C atoms that can optionally be substituted with at least one of F, Cl, Br, I, pseudohalogen groups, alkoxy groups with 1 to 20 C atoms, OH, NO₂ and NH₂, aromatic groups with 6 to 12 C atoms that can optionally be substituted with at least one of F, Cl, Br, I, pseudohalogen groups, alkoxy groups with 1 to 20 C atoms, OH, NO₂ and NH₂, and aralkyl groups with 7 to 10 C atoms that can optionally be substituted with at least one of F, Cl, Br, I, pseudohalogen groups, alkoxy groups with 1 to 20 C atoms, OH, NO₂ and NH₂, preferably wherein R23, R24, and R25 are not all hydrogen at the same time and/or wherein R23, R24, and R25 are not all methyl at the same time, further preferably wherein all of R₂₃, R₂₄, and R₂₅ are ethyl.

In the compound of Formula I, as well as any of the following formulas directly or indirectly derived therefrom, R_{1b} is selected from the group consisting of H, linear alkyl groups with 1 to 6 C atoms that can optionally be substituted with at least one of F, Cl, Br, I, pseudohalogen groups, alkoxy groups with 1 to 6 C atoms, OH, NO₂ and NH₂; branched alkyl groups with 3 to 6 C atoms that can optionally be substituted with at least one of F, Cl, Br, I, pseudohalogen groups, alkoxy groups with 1 to 6 C atoms, OH, NO₂ and NH₂; heterocycloalkyl groups with 1 to 6 C atoms that can optionally be substituted with at least one of F, Cl, Br, I, pseudohalogen groups, alkoxy groups with 1 to 6 C atoms, OH, NO₂ and NH₂; aromatic and/or heteroaromatic groups with 2 to 6 carbon atoms that can optionally be substituted with at least one of F, Cl, Br, I, pseudohalogen groups, alkoxy groups with 1 to 6 C atoms, OH, NO₂ and NH₂; carboxylic acid ester groups with 1 to 6 C atoms that can optionally be substituted with at least one of F, Cl, Br, I, pseudohalogen groups, alkoxy groups with 1 to 6 C atoms, OH, NO₂ and NH₂; alkyl and/or (hetero)aryl oxycarbonyl groups with 1 to 6 C atoms that can optionally be substituted with at least one of F, Cl, Br, I, pseudohalogen groups, alkoxy groups with 1 to 6 C atoms, OH, NO₂ and NH₂; and carboxamide groups with 1 to 6 C atoms that can optionally be substituted with at least one of F, Cl, Br, I, pseudohalogen groups, alkoxy groups with 1 to 6 C atoms, OH, NO₂ and NH₂. Preferably R_{1b} is H or methyl.

The stereocenter *1 of the catalyst according to Formula I, as well as any of the following formulas directly or indirectly derived therefrom, is in (R) and/or (S) configuration. According to certain embodiments, when the stereocenter *1 of the catalyst of Formula I or any of the following formulas in the following is in (R) configuration, (S)-nornicotine is produced in excess. Furthermore, according to certain embodiments, when the stereocenter *1 of the catalyst is in (S) configuration, (R)-nornicotine is produced in excess.According to certain embodiments, when the stereocenter *1 of the catalyst of Formula I or any of the following formulas in the following is in (R) configuration, (R)-nornicotine is produced in excess. Furthermore, according to certain embodiments, when the stereocenter *1 of the catalyst is in (S) configuration, (S)-nornicotine is produced in excess.

With the catalytic system described above, the inventors have surprisingly found that it is possible to reduce myosmine, which is a cyclic imine, to nornicotine. It is assumed that the bridge C=Q₁, particularly a (C=O) bridge, of the catalyst according to Formula I together with the N in ring B are responsible for the coordination of the silane and chemical activation. This allows the hydride transfer from the silane to myosmine, which can be coordinated by ring A. The stereocenter in ortho position with the residues R₁ₐ and R_{1b} is thought to direct the coordination of myosmine, which enables its enantioselective hydrosilylation.

According to certain embodiments, the catalyst, particularly the catalyst of Formula I, is a catalyst of Formula I-A:

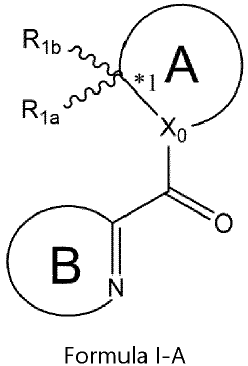

In the compound of Formula I-A, ring A, ring B, R₁ₐ and R_{1b}, and X₀ are as defined for the compound of Formula I. In the compound of Formula I-A, R₁ₐ and R_{1b} are different. The stereocenter *1 of the catalyst according to Formula I-A is in (R) and/or (S) configuration.

According to certain embodiments, the catalyst, particularly the catalyst of Formula I-A, is a catalyst of Formula I-B:

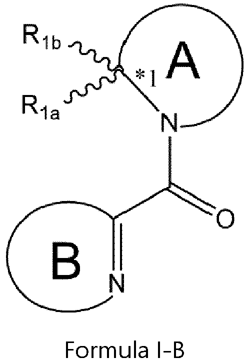

In the compound of Formula I-B, ring A, ring B, R₁ₐ and R_{1b} are as defined for the compound of Formula I. In the compound of Formula I-B, R₁ₐ and R_{1b} are different. The stereocenter *1 of the catalyst according to Formula I-B is in (R) and/or (S) configuration.

According to certain embodiments, the catalyst, particularly the catalyst of Formula I, is a catalyst of Formula Ia:

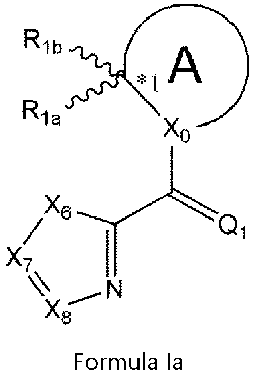

In the compound of Formula Ia, ring A, R₁ₐ and R_{1b}, Q₁ and X₀ are as defined for the compound of Formula I. R₁ₐ and R_{1b} are different.

In the compound of Formula Ia, as well as any of the following formulas directly or indirectly derived therefrom, X₆ is selected from O, S, NR₁₃ and CR₁₃ₐR_{13b}.

In the compound of Formula Ia, as well as any of the following formulas directly or indirectly derived therefrom, R₁₃ is chosen from H, linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂. According to certain embodiments, R₁₃ is H, a linear alkyl group with 1 to 10, e.g. 1 to 6, C atoms, e.g. a methyl or ethyl group, an aromatic group with 6 to 20 C atoms, e.g.6 to 10 or 6 C atoms, e.g. a phenyl group, or an aralkyl group with 7 to 20 C atoms, e.g. 7 to 11 C atoms, e.g. 7 C atoms, e.g. a benzyl group, in the formulas comprising R₁₃.

In the compound of Formula Ia, as well as any of the following formulas directly or indirectly derived therefrom, R₁₃ₐ and R_{13b} are the same or different and can be chosen from H, linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂.

In the compound of Formula Ia, as well as any of the following formulas directly or indirectly derived therefrom, X₇ and X₈ are independently selected from N and CR₆; with the provisio that when both X₇ and X₈ are represented by CR₆, the two R₆ can be the same or different and/or the two R₆ can form a ring, and with the provisio that if at least one of X₇ and X₈ is represented by CR₆, the at least one R₆ can form a ring with R₁₃, R₁₃ₐ and/or R_{13b}, preferably with the provisio that at least one of X₇ and X₈ is CR₆ and/or X₆ is CR₁₃ₐR_{13b}. According to preferred embodiments, X₇ and X₈ are independently selected from N and CR₆; with the provisio that when both X₇ and X₈ are represented by CR₆, the two R₆ can be the same or different, preferably with the provisio that at least one of X₇ and X₈ is CR₆ and/or X₆ is CR₁₃ₐR_{13b}. A formation of a ring is not preferable.

In the compound of Formula Ia, as well as any of the following formulas directly or indirectly derived therefrom, R₆ is chosen from H; F; Cl; Br; I; alkoxy groups with 1 to 20 C atoms; pseudohalogen groups; OH; NO₂; NH₂; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂.

The stereocenter *1 of the catalyst according to Formula la is in (R) and/or (S) configuration.

According to certain embodiments, the catalyst, particularly the catalyst of Formula la, is a catalyst of Formula la-1:

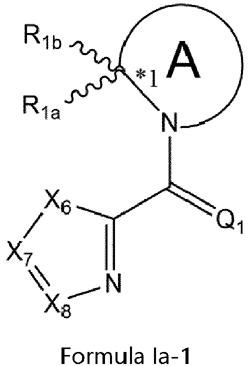

In the compound of Formula la-1, ring A, R₁ₐ, R_{1b}, X₆, X₇ and X₈ are as defined in the compound of Formula la. In the compound of Formula la-1, R₁ₐ and R_{1b} are different. The stereocenter *1 of the catalyst according to Formula la-1 is in (R) and/or (S) configuration.

According to certain embodiments, the catalyst of Formula la is used as catalyst and is a catalyst of the following Formula II:

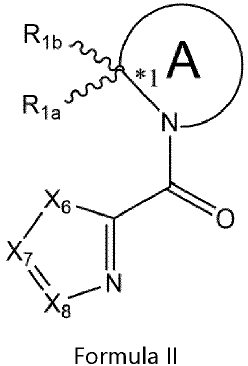

In the compound of Formula II, ring A, R₁ₐ, R_{1b}, X₆, X₇ and X₈ are as defined in the compound of Formula la. In the compound of Formula II, R₁ₐ and R_{1b} are different.

The stereocenter *1 of the catalyst according to Formula II is in (R) and/or (S) configuration. According to certain embodiments, when the stereocenter *1 of the catalyst is in (R) configuration, (S)-nornicotine is produced in excess. Furthermore, when the stereocenter *1 of the catalyst is in (S) configuration, (R)-nornicotine is produced in excess.

With the catalytic system described above, the inventors have surprisingly found that it is possible to reduce myosmine, which is a cyclic imine, to nornicotine. It is assumed that the amide unit of the catalyst according to Formula II and the N in ring B, respectively the lower ring with X₆, X₇, and X₈, are responsible for the coordination of the silane and chemical activation. This allows the hydride transfer from the silane to myosmine, which is coordinated by ring A and the nitrogen therein, as well as possibly particular residues R₁ₐ. The stereocenter in ortho position with the residues R₁ₐ and R_{1b} is thought to direct the coordination of myosmine which enables its enantioselective hydrosilylation.

According to certain embodiments, the catalyst, particularly the catalyst of Formula II, is a catalyst of the following Formula IIa:

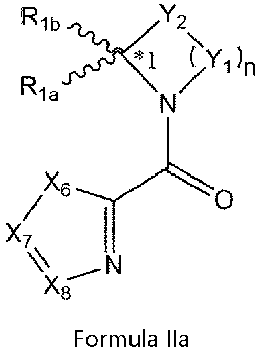

In the compound of Formula IIa, R₁ₐ, R_{1b}, X₆, X₇ and X₈ are as defined in the compound of Formula II. In the compound of Formula IIa, R₁ₐ and R_{1b} are different.

In the compound of Formula IIa, as well as any of the following formulas directly or indirectly derived therefrom, Y₁ is chosen from substituted or unsubstituted C atoms and/or substituted or unsubstituted heteroatoms, wherein the substituents can comprise any one of F; Cl; Br; I; pseudohalogen groups; alkoxy groups with 1 to 20 C atoms; OH; SR₂₁; S(CO)R₂₂; NO₂; NH₂; amines of formula NR₁₈R₁₉; (NR₁₈'R₁₉'R₂₀')⁺; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂; with the provisio that two or more of the substituents can form a ring or more rings, optionally wherein at least one substituent is part of a polymer, and with the provisio that if more than one Y₁ is present, these can be the same or different, wherein
R₁₈, R₁₉, R₁₈', R₁₉', R₂₀', R₂₁, and R₂₂ are the same or different and can be chosen from H; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂, with the provisio that not both of R₁₈ and R₁₉ are H;

In the compound of Formula IIa, as well as any of the following formulas directly or indirectly derived therefrom, Y₂ is chosen from a carbonyl group, a thiocarbonyl group, and substituted or unsubstituted C atoms and/or substituted or unsubstituted heteroatoms, wherein the substituents can comprise any one of F; Cl; Br; I; pseudohalogen groups; alkoxy groups with 1 to 20 C atoms; OH; NO₂; NH₂; amines of formula NR₁₈R₁₉; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂.

In the compound of Formula IIa, as well as any of the following formulas directly or indirectly derived therefrom, n is an integer from 0 to 8, preferably from 1 to 5, further preferably from 1 to 3, even further preferably 2 or 3. The stereocenter *1 of the catalyst according to Formula IIa is in (R) and/or (S) configuration;

According to certain embodiments, the catalyst, particularly the catalyst of Formula IIa, is a catalyst of the following Formula IIa-1:

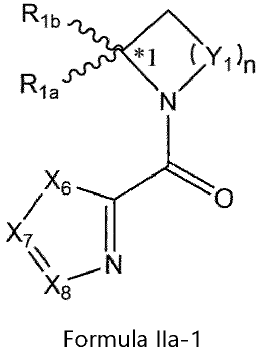

In the compound of Formula IIa-1, R₁ₐ, R_{1b}, n, Y₁, X₆, X₇ and X₈ are as defined in the compound of Formula IIa. In the compound of Formula IIa-1, R₁ₐ and R_{1b} are different. The stereocenter *1 of the catalyst according to Formula IIa-1 is in (R) and/or (S) configuration.

According to certain embodiments, the catalyst, particularly the catalyst of Formula IIa-1, is a catalyst of the following Formula IIa-2:

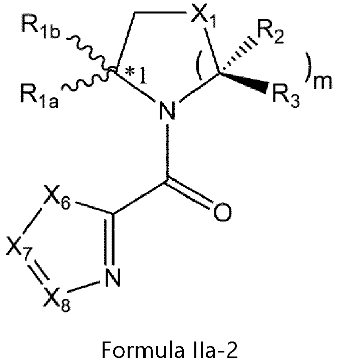

In the compound of Formula IIa-2, R₁ₐ, R_{1b}, X₆, X₇ and X₈ are as defined in the compound of Formula IIa-1.

In the compound of Formula IIa-2, as well as any of the following formulas directly or indirectly derived therefrom, m is an integer from 0 to 7, preferably from0 to 4, further preferably 1 or 2, even further preferably 1.

In the compound of Formula IIa-2, as well as any of the following formulas directly or indirectly derived therefrom, R₂ and R₃ are the same or different and are independently selected from H; F; Cl; Br; I; pseudohalogen groups; alkoxy groups with 1 to 20 C atoms; OH; NO₂; NH₂; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂.

In the compound of Formula IIa-2, as well as any of the following formulas directly or indirectly derived therefrom, X₁ is selected from the group of O, NR₄, and CR₄ₐR_{4b}, wherein R₄ₐ and R_{4b} can be the same or different.

In the compound of Formula IIa-2, as well as any of the following formulas directly or indirectly derived therefrom, R₄, R₄ₐ, and R_{4b} are independently selected from H; F; Cl; Br; I; pseudohalogen groups; alkoxy groups with 1 to 20 C atoms; OH; SR₂₁; S(CO)R₂₂; NO₂; NH₂; amines of formula NR₁₈R₁₉; (NR₁₈'R₁₉'R₂₀')⁺; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester (carboxylate) groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂; wherein R₄ₐ and R_{4b} can be the same or different; with the provisio that two or more of R₂, R₃, R₄, R₄ₐ, and R_{4b} can form a ring or more rings, optionally wherein at least one of R₂, R₃, R₄, R₄ₐ, and R_{4b} is part of a polymer, wherein
R₁₈, R₁₉, R₁₈', R₁₉', R₂₀', R₂₁, and R₂₂ are the same or different and can be chosen from H; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂, with the provisio that not both of R₁₈ and R₁₉ are H.

In the compound of Formula IIa-2, R₁ₐ and R_{1b} are different. The stereocenter *1 of the catalyst according to Formula IIa-2 is in (R) and/or (S) configuration.

According to certain embodiments, the catalyst, particularly the catalyst of Formula II, is a catalyst of the following Formula IIb:

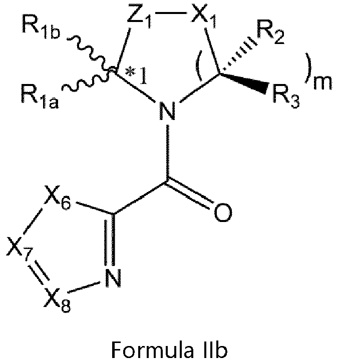

In the compound of Formula IIb, R₁ₐ, R_{1b}, X₆, X₇ and X₈ are as defined in the compound of Formula II. In the compound of Formula IIb, R₁ₐ and R_{1b} are different. In the compound of Formula IIb, m is an integer from 0 to 7, preferably from0 to 4, further preferably 1 or 2, even further preferably 1.

In the compound of Formula IIb, as well as any of the following formulas directly or indirectly derived therefrom, R₂ and R₃ are the same or different and are independently selected from H; F; Cl; Br; I; pseudohalogen groups; alkoxy groups with 1 to 20 C atoms; OH; NO₂; NH₂; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂;

In the compound of Formula IIb, as well as any of the following formulas directly or indirectly derived therefrom, X₁ is selected from the group of O, NR₄, and CR₄ₐR_{4b}, wherein R₄ₐ and R_{4b} can be the same or different.

In the compound of Formula IIb, as well as any of the following formulas directly or indirectly derived therefrom, R₄, R₄ₐ, and R_{4b} are independently selected from H; F; Cl; Br; I; pseudohalogen groups; alkoxy groups with 1 to 20 C atoms; OH; SR₂₁; S(CO)R₂₂; NO₂; NH₂; amines of formula NR₁₈R₁₉; (NR₁₈'R₁₉'R₂₀')⁺; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester (carboxylate) groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂; wherein R₄ₐ and R_{4b} can be the same or different; with the provisio that two or more of R₂, R₃, R₄, R₄ₐ, and R_{4b} can form a ring or more rings, optionally wherein at least one of R₂, R₃, R₄, R₄ₐ, and R_{4b} is part of a polymer, wherein
R₁₈, R₁₉, R₁₈', R₁₉', R₂₀', R₂₁, and R₂₂ are the same or different and can be chosen from H; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂, with the provisio that not both of R₁₈ and R₁₉ are H.

In the compound of Formula IIb, as well as any of the following formulas directly or indirectly derived therefrom, Z₁ is chosen from a carbonyl group, a thiocarbonyl group, and CR₂₀ₐR_{20b}.

In the compound of Formula IIb, as well as any of the following formulas directly or indirectly derived therefrom, R₂₀ₐ and R_{20b} are the same or different and can be chosen from H, linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂.

The stereocenter *1 of the catalyst according to Formula IIb is in (R) and/or (S) configuration.

According to certain embodiments, the catalyst, particularly the catalyst of Formula IIb, is a catalyst of the following Formula IIb1:

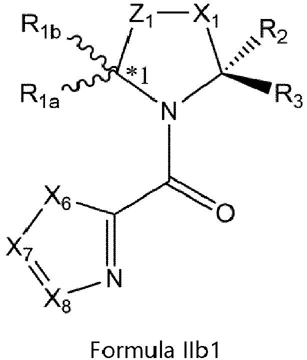

In the compound of Formula IIb1, R₁ₐ, R_{1b}, R₂, R₃, X₁, X₆, X₇, X₈ and Z₁ are as defined in the compound of Formula IIb. In the compound of Formula IIb1, R₁ₐ and R_{1b} are different. The stereocenter *1 of the catalyst according to Formula IIb1 is in (R) and/or (S) configuration.

According to certain embodiments, the catalyst, particularly the catalyst of Formula IIb, is a catalyst of the following Formula IIb2

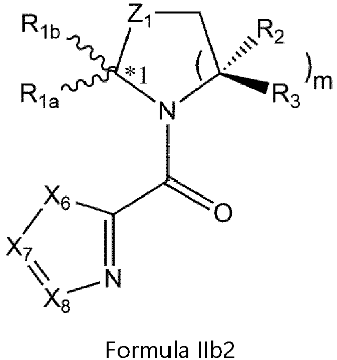

In the compound of Formula IIb2, m, R₁ₐ, R_{1b}, R₂, R₃, X₆, X₇, X₈ and Z₁ are as defined in the compound of Formula IIb. In the compound of Formula IIb2, R₁ₐ and R_{1b} are different. The stereocenter *1 of the catalyst according to Formula IIb2 is in (R) and/or (S) configuration.

According to certain embodiments, in the catalyst of Formula II, respectively the catalyst of Formula IIa or the catalyst of Formula IIb, preferably the catalyst of Formula IIa-1, the catalyst of Formula IIb1, or the catalyst of Formula IIb2, as well as any of the following formulas directly or indirectly derived therefrom, R₁ₐ is represented by a tertiary residue CR₁₀R₁₁R₁₂.

In the tertiary residue CR₁₀R₁₁R₁₂ in the above formulas and any formulas derived thereof, R₁₀ and R₁₁ are the same or different, e.g. can be the same, and are chosen from H; F; Cl; Br; I; pseudohalogen groups; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂. According to preferred embodiments, in the tertiary residue CR₁₀R₁₁R₁₂ in the above formulas and any formulas derived thereof,R₁₀ and R₁₁ are the same or different, e.g. can be the same, and are chosen from H; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 18 C atoms that can optionally be substituted with at least one of F, Cl, Br, I, pseudohalogen groups, alkoxy groups with 1 to 20 C atoms, OH, NO₂ and NH₂; alkylaryl groups with 7 to 18 C atoms that can optionally be substituted with at least one of F, Cl, Br, I, pseudohalogen groups, alkoxy groups with 1 to 20 C atoms, OH, NO₂ and NH₂; and aromatic and/or heteroaromatic groups with 2 to 20 C atoms that can optionally be substituted with at least one of F, Cl, Br, I, pseudohalogen groups, alkoxy groups with 1 to 20 C atoms, OH, NO₂ and NH₂. According to more preferred embodiments, in the tertiary residue CR₁₀R₁₁R₁₂ in the above formulas and any formulas derived thereof,R₁₀ and R₁₁ are the same or different, e.g. can be the same, and are chosen from H; linear alkyl groups with 1 to 10 C atoms, preferably 1 to 6 C atoms, e.g. a methyl and ethyl group; aralkyl groups with 7 to 13 C atoms, preferably 7 to 10 C atoms, e.g. a (C₆H₅)-CH₂- group; alkylaryl groups with 7 to 15 C atoms, preferably 7 to 13 C atoms, e.g. a CH₃-C₆H₄-group; and aromatic groups with 6 to 14 C atoms, preferably 6 to 10 C atoms, e.g. a phenyl (Ph) and naphthy group (Naph).

In the tertiary residue CR₁₀R₁₁R₁₂, R₁₂ in the above formulas and any formulas derived thereof, is chosen from H, F; Cl; Br; I; pseudohalogen groups; OH; OR₁₂ₐ; SH; SR₁₂ₐ; COOH; COOR₁₂ₐ; NO₂; NH₂; (NR₁₈'R₁₉'R₂₀')⁺; and amine groups NR₁₄R₁₅, with the provisio that not both of R₁₄ and R₁₅ are H, and preferably R₁₂ is H; OH; OR₁₂ₐ, NH₂; or NR₁₄R₁₅, particularly preferably R₁₂ is H; OH; or OR₁₂ₐ, wherein R₁₈', R₁₉', and R₂₀' are the same or different and can be chosen from H; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂.
R₁₂ₐ is chosen from H;
linear alkyl groups with 1 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups (including (CO)NH₂) with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
linear alkenyl and/or alkynyl groups with 2 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
branched alkyl, alkenyl and/or alkynyl groups with 3 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
aralkyl groups with 7 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkylaryl groups with 7 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
cycloalkyl groups with 3 to 20 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heterocycloalkyl groups with 1 to 15 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
aromatic and/or heteroaromatic groups with 2 to 40 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl heteroaromatic groups with 2 to 50 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heteroaromatic groups with at least one alkyl residue with 3 to 50 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl cycloalkyl groups with 4 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
cycloalkyl alkyl groups with 4 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl heterocycloalkyl groups with 2 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heterocycloalkyl alkyl groups with 2 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
SiR₂₃R₂₄R₂₅;
R₂₃, R₂₄, and R₂₅ are the same or different and are selected from H, linear alkyl groups with 1 to 4 C atoms that can optionally be substituted with at least one of F, Cl, Br, I, pseudohalogen groups, alkoxy groups with 1 to 20 C atoms, OH, NO₂ and NH₂, branched alkyl group with 3 to 6 C atoms that can optionally be substituted with at least one of F, Cl, Br, I, pseudohalogen groups, alkoxy groups with 1 to 20 C atoms, OH, NO₂ and NH₂, aromatic groups with 6 to 12 C atoms that can optionally be substituted with at least one of F, Cl, Br, I, pseudohalogen groups, alkoxy groups with 1 to 20 C atoms, OH, NO₂ and NH₂, and aralkyl groups with 7 to 10 C atoms that can optionally be substituted with at least one of F, Cl, Br, I, pseudohalogen groups, alkoxy groups with 1 to 20 C atoms, OH, NO₂ and NH₂, preferably wherein R₂₃, R₂₄, and R₂₅ are not all hydrogen at the same time and/or wherein R₂₃, R₂₄, and R₂₅ are not all methyl at the same time, further preferably wherein all of R₂₃, R₂₄, and R₂₅ are ethyl;
R₁₄ and R₁₅ are the same or different and can be chosen from H;
linear alkyl groups with 1 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups (including (CO)NH₂) with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
linear alkenyl and/or alkynyl groups with 2 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
branched alkyl, alkenyl and/or alkynyl groups with 3 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
aralkyl groups with 7 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkylaryl groups with 7 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
cycloalkyl groups with 3 to 20 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heterocycloalkyl groups with 1 to 15 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
aromatic and/or heteroaromatic groups with 2 to 40 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl heteroaromatic groups with 2 to 50 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heteroaromatic groups with at least one alkyl residue with 3 to 50 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl cycloalkyl groups with 4 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
cycloalkyl alkyl groups with 4 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl heterocycloalkyl groups with 2 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heterocycloalkyl alkyl groups with 2 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂.

According to certain embodiments, alternatively or in addition, in the catalyst of Formula II, respectively the catalyst of Formula IIa or the catalyst of Formula IIb, preferably the catalyst of Formula IIa-1, the catalyst of Formula IIb1, or the catalyst of Formula IIb2, as well as any of the following formulas directly or indirectly derived therefrom, X₆ is NR₁₃. In reside NR₁₃, R₁₃ is chosen from H, linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂.

According to certain embodiments, the catalyst, particularly the catalyst of Formula IIb1, respectively the catalyst of Formula IIa-2, is a catalyst of the following Formula IIa-3:

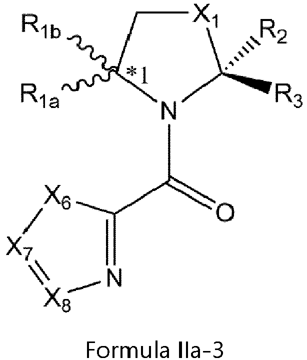

In the compound of Formula IIa-3, R₁ₐ, R_{1b}, R₂, R₃, X₁, X₆, X₇ and X₈ are as defined in the compound of Formula IIa-2, respectively the compound of Formula IIb1. In the compound of Formula IIa-3, R₁ₐ and R_{1b} are different. The stereocenter *1 of the catalyst according to Formula IIa-3 is in (R) and/or (S) configuration.

According to certain embodiments, the catalyst, particularly the catalyst of Formula IIb1, is a catalyst of the following Formula IIb1-a, a catalyst of the following formula IIb1-b, or a catalyst of the following formula IIb1-c:

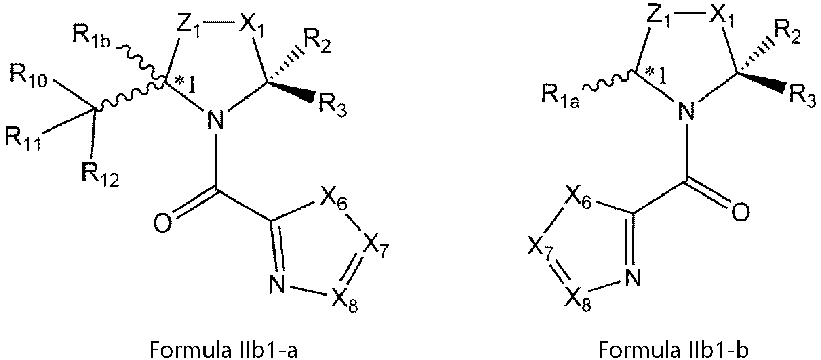

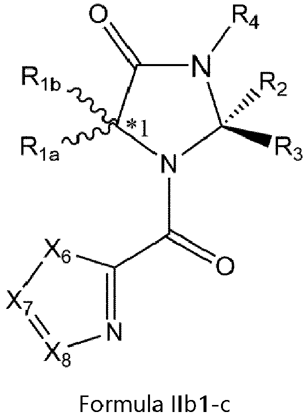

In the compound of Formula IIb1-a, R_{1b}, R₂, R₃, X₁, X₆, X₇, X₈ and Z₁ are as defined in the compound of Formula IIb1. The stereocenter *1 of the catalyst according to Formula IIb1-a is in (R) and/or (S) configuration.

In the compound of Formula IIb1-a, as well as any of the following formulas directly or indirectly derived therefrom, R₁₀ and R₁₁ are the same or different and are chosen from H; F; Cl; Br; I; pseudohalogen groups; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂; and

In the compound of Formula IIb1-a, as well as any of the following formulas directly or indirectly derived therefrom, R₁₂ is chosen from H, F; Cl; Br; I; pseudohalogen groups; OH; OR₁₂ₐ; SH; SR₁₂ₐ, COOH; COOR₁₂ₐ; NO₂; NH₂; (NR₁₈'R₁₉'R₂₀')⁺; and amine groups NR₁₄R₁₅, with the provisio that not both of R₁₄ and R₁₅ are H; wherein R₁₈', R₁₉', and R₂₀' are the same or different and can be chosen from H; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂;
R₁₂ₐ is chosen from H;
linear alkyl groups with 1 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups (including (CO)NH₂) with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
linear alkenyl and/or alkynyl groups with 2 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
branched alkyl, alkenyl and/or alkynyl groups with 3 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
aralkyl groups with 7 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkylaryl groups with 7 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
cycloalkyl groups with 3 to 20 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heterocycloalkyl groups with 1 to 15 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
aromatic and/or heteroaromatic groups with 2 to 40 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl heteroaromatic groups with 2 to 50 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heteroaromatic groups with at least one alkyl residue with 3 to 50 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl cycloalkyl groups with 4 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
cycloalkyl alkyl groups with 4 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl heterocycloalkyl groups with 2 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heterocycloalkyl alkyl groups with 2 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
SiR₂₃R₂₄R₂₅;
R₂₃, R₂₄, and R₂₅ are the same or different and are selected from H, linear alkyl groups with 1 to 4 C atoms that can optionally be substituted with at least one of F, Cl, Br, I, pseudohalogen groups, alkoxy groups with 1 to 20 C atoms, OH, NO₂ and NH₂, branched alkyl group with 3 to 6 C atoms that can optionally be substituted with at least one of F, Cl, Br, I, pseudohalogen groups, alkoxy groups with 1 to 20 C atoms, OH, NO₂ and NH₂, aromatic groups with 6 to 12 C atoms that can optionally be substituted with at least one of F, Cl, Br, I, pseudohalogen groups, alkoxy groups with 1 to 20 C atoms, OH, NO₂ and NH₂, and aralkyl groups with 7 to 10 C atoms that can optionally be substituted with at least one of F, Cl, Br, I, pseudohalogen groups, alkoxy groups with 1 to 20 C atoms, OH, NO₂ and NH₂, preferably wherein R₂₃, R₂₄, and R₂₅ are not all hydrogen at the same time and/or wherein R₂₃, R₂₄, and R₂₅ are not all methyl at the same time, further preferably wherein all of R₂₃, R₂₄, and R₂₅ are ethyl;
R₁₄ and R₁₅ are the same or different and can be chosen from H,
linear alkyl groups with 1 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups (including (CO)NH₂) with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
linear alkenyl and/or alkynyl groups with 2 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
branched alkyl, alkenyl and/or alkynyl groups with 3 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
aralkyl groups with 7 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkylaryl groups with 7 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
cycloalkyl groups with 3 to 20 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heterocycloalkyl groups with 1 to 15 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
aromatic and/or heteroaromatic groups with 2 to 40 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl heteroaromatic groups with 2 to 50 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heteroaromatic groups with at least one alkyl residue with 3 to 50 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl cycloalkyl groups with 4 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
cycloalkyl alkyl groups with 4 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl heterocycloalkyl groups with 2 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heterocycloalkyl alkyl groups with 2 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂.

In the compound of Formula IIb1-b, R₁ₐ, R₂, R₃, X₁, X₆, X₇, X₈ and Z₁ are as defined in the compound of Formula IIb1. The stereocenter *1 of the catalyst according to Formula IIb1-b is in (R) and/or (S) configuration.

In the compound of Formula IIb1-c, R₁ₐ, R_{1b}, R₂, R₃, X₆, X₇ and X₈ are as defined in the compound of Formula IIb1. In the compound of Formula IIb1-c, R₁ₐ and R_{1b} are different. The stereocenter *1 of the catalyst according to Formula IIb1-c is in (R) and/or (S) configuration.

In the compound of Formula IIb1-c, as well as any of the following formulas directly or indirectly derived therefrom, R₄ is selected from H; F; Cl; Br; I; pseudohalogen groups; alkoxy groups with 1 to 20 C atoms; OH; NO₂; NH₂; amines of formula NR₁₈R₁₉; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester (carboxylate) groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂; with the provisio that two or more of R₂, R₃, and R₄ can form a ring or more rings, optionally wherein at least one of R₂, R₃, and R₄ is part of a polymer.

R₁₈ and R₁₉ are the same or different and can be chosen from H, linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂, with the provisio that not both of R₁₈ and R₁₉ are H.

According to certain embodiments, the catalyst, particularly the catalyst of Formula IIb2, is a catalyst of the following Formula IIb2-1:

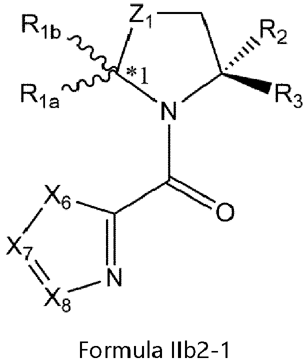

In the compound of Formula IIb2-1, R₁ₐ, R_{1b}, R₂, R₃, X₆, X₇, X₈ and Z₁ are as defined in the compound of Formula IIb2. In the compound of Formula IIb2-1, R₁ₐ and R_{1b} are different. The stereocenter *1 of the catalyst according to Formula IIb2-1 is in (R) and/or (S) configuration.

According to certain embodiments, the catalyst, particularly the catalyst of Formula IIb2-1, is a catalyst of the following Formula IIb2-1a or a catalyst of the following formula IIb2-1b, and particularly a catalyst of the following formula IIb2-2:

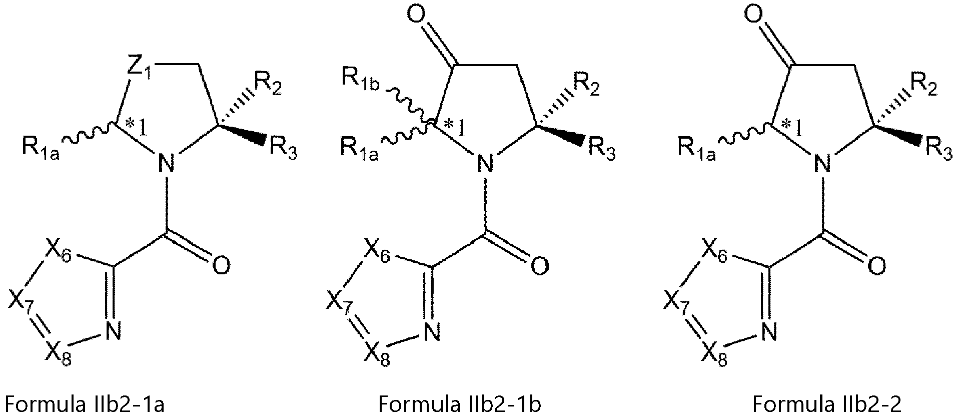

In the compound of Formula IIb2-1a, R₁ₐ, R₂, R₃, X₆, X₇, X₈ and Z₁ are as defined in the compound of Formula IIb2-1. The stereocenter *1 of the catalyst according to Formula IIb2-1a is in (R) and/or (S) configuration.

In the compound of Formula IIb2-1b, R₁ₐ, R_{1b}, R₂, R₃, X₆, X₇ and X₈ are as defined in the compound of Formula IIb2-1. In the compound of Formula IIb2-1b, R₁ₐ and R_{1b} are different. The stereocenter *1 of the catalyst according to Formula IIb2-1b is in (R) and/or (S) configuration.

In the compound of Formula IIb2-2, R₁ₐ, R₂, R₃, X₆, X₇ and X₈ are as defined in the compound of Formula IIb2-1a and/or the compound of Formula IIb2-1b. The stereocenter *1 of the catalyst according to Formula IIb2-2 is in (R) and/or (S) configuration.

According to certain embodiments, the catalyst, particularly the catalyst of Formula IIb1-a, is a catalyst of the following Formula IIb1-a1, and/or the catalyst of Formula IIb1-b is a catalyst of the following Formula IIb1-a1 or a catalyst of the following formula IIb1-b1, and/or the catalyst of Formula IIb1-c is a catalyst of the following Formula IIb1-b1:

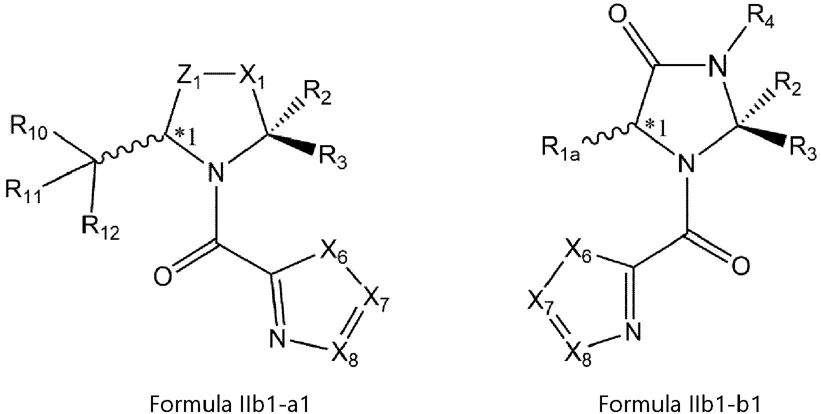

In the compound of Formula IIb1-a1, R₂, R₃, R₁₀, R₁₁, R₁₂, X₁, X₆, X₇, X₈ and Z₁ are as defined in the compound of Formula IIb1-a. The stereocenter *1 of the catalyst according to Formula IIb1-a1 is in (R) and/or (S) configuration.

In the compound of Formula IIb1-b1, R₁ₐ, R₂, R₃, R₄, X₆, X₇ and X₈ are as defined in the compound of Formula IIb1-c. In the compound of Formula IIb1-b1, R₁ₐ and R_{1b} are different. The stereocenter *1 of the catalyst according to Formula Ilb1-b1 is in (R) and/or (S) configuration.

According to certain embodiments, the catalyst, particularly the catalyst of Formula IIa-3, is a catalyst of the following Formula IIa-4, preferably a catalyst of the following Formula IIa-5, further preferably a catalyst of the following Formula IIa-6, even further preferably a catalyst of the following Formula IIa-7, even further preferably a catalyst of the following Formula IIa-8, even further preferably of the following Formula IIa-9:

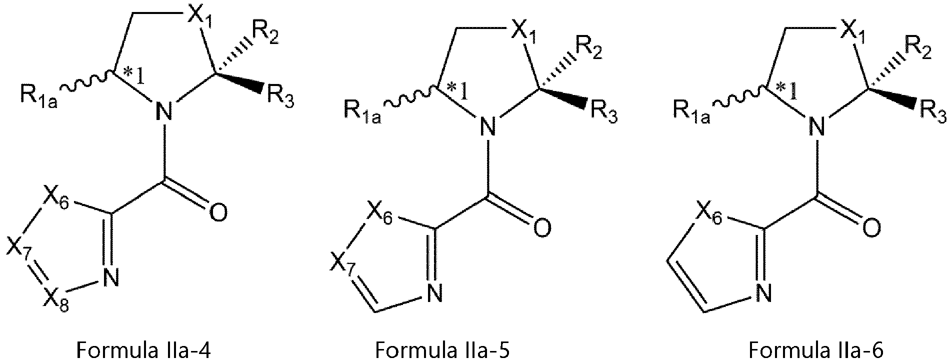

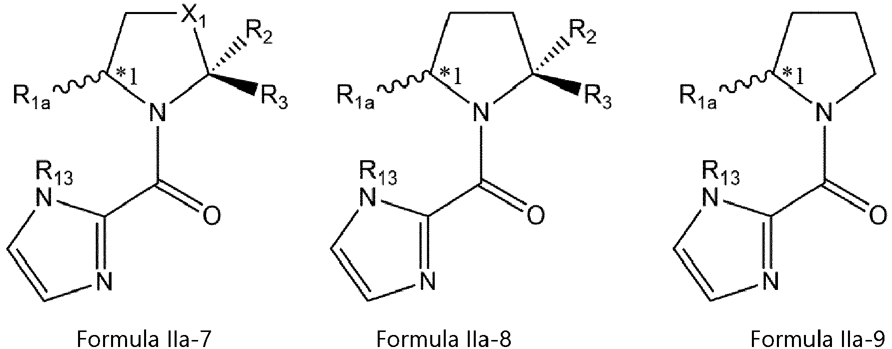

In the compound of Formula IIa-4, R₁ₐ, R₂, R₃, X₁, X₆, X₇ and X₈ are as defined in the compound of Formula IIa-3. The stereocenter *1 of the catalyst according to Formula IIa-4 is in (R) and/or (S) configuration.

In the compound of Formula IIa-5, R₁ₐ, R₂, R₃, X₁, X₆, and X₇ are as defined in the compound of Formula IIa-4. The stereocenter *1 of the catalyst according to Formula IIa-5 is in (R) and/or (S) configuration.

In the compound of Formula IIa-6, R₁ₐ, R₂, R₃, X₁ and X₆ are as defined in the compound of Formula IIa-5. The stereocenter *1 of the catalyst according to Formula IIa-6 is in (R) and/or (S) configuration.

In the compound of Formula IIa-7, R₁ₐ, R₂, R₃ and X₁ are as defined in the compound of Formula IIa-6. The stereocenter *1 of the catalyst according to Formula IIa-7 is in (R) and/or (S) configuration.

In the compound of Formula IIa-7, as well as any of the following formulas directly or indirectly derived therefrom, R₁₃ is chosen from H, linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂.

In the compound of Formula IIa-8, R₁ₐ, R₂, R₃ and R₁₃ are as defined in the compound of Formula IIa-7. The stereocenter *1 of the catalyst according to Formula IIa-8 is in (R) and/or (S) configuration.

In the compound of Formula IIa-9, R₁ₐ and R₁₃ are as defined in the compound of Formula IIa-8. The stereocenter *1 of the catalyst according to Formula IIa-9 is in (R) and/or (S) configuration.

According to certain embodiments, the catalyst, particularly the catalyst of Formula IIb1-a1, is a catalyst of the following Formula IIb1-a2, preferably a catalyst of the following Formula IIb1 - a3, further preferably a catalyst of the following Formula IIb1-a4 or a catalyst of the following Formula IIb1-a4'. The catalyst of Formula IIb1-a4 is, according to certain embodiments, a catalyst of the following Formula IIb1-a5, preferably of the following Formula IIb1-a6 or a catalyst of the following Formula IIb1-a6':

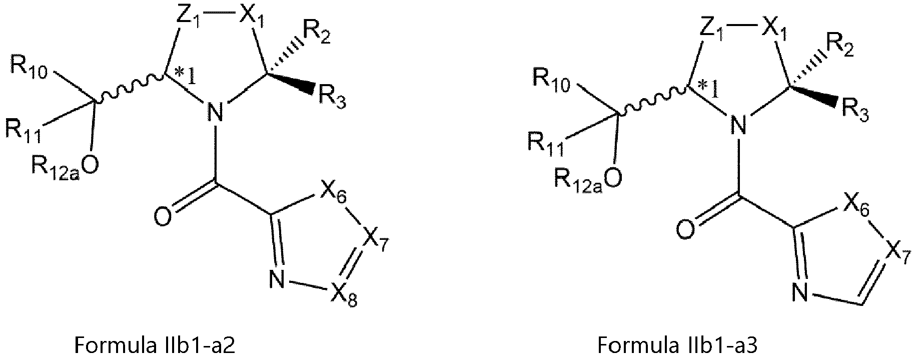

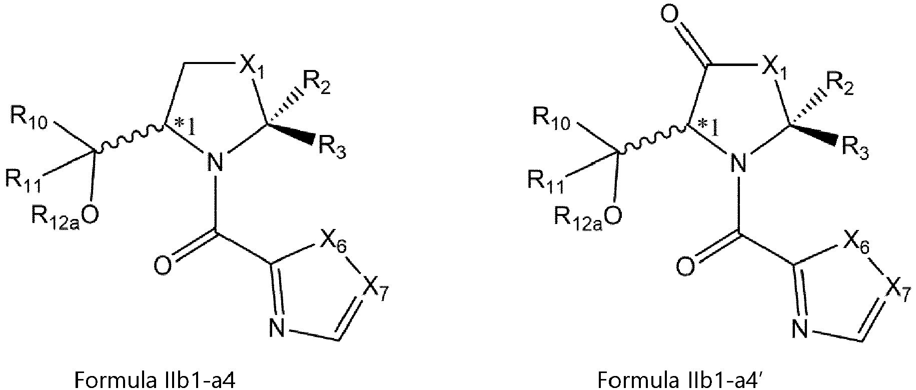

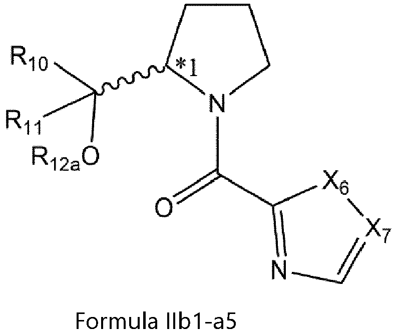

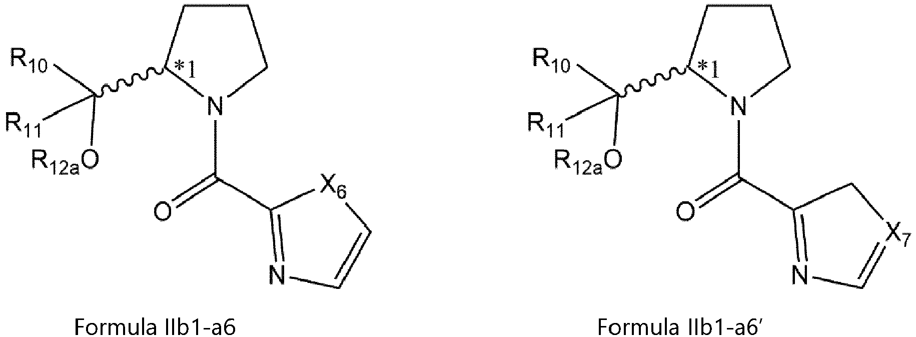

In the compound of Formula IIb1-a2, R₂, R₃, R₁₀, R₁₁, R₁₂ₐ, X₁, X₆, X₇, X₈ and Z₁ are as defined in the compound of Formula IIb1-a1. The stereocenter *1 of the catalyst according to Formula IIb1-a2 is in (R) and/or (S) configuration.

In the compound of Formula IIb1-a3, R₂, R₃, R₁₀, R₁₁, R₁₂ₐ, X₁, X₆, X₇ and Z₁ are as defined in the compound of Formula IIb1-a2. The stereocenter *1 of the catalyst according to Formula IIb1-a3 is in (R) and/or (S) configuration.

In the compound of Formula IIb1-a4 and the compound of Formula IIb1-a4', R₂, R₃, R₁₀, R₁₁, R₁₂ₐ, X₁, X₆ and X₇ are as defined in the compound of Formula IIb1-a3. The stereocenter *1 of the catalyst according to Formula IIb1-a4 and in the catalyst according to Formula IIb1-a4' is in (R) and/or (S) configuration.

In the compound of Formula IIb1-a5, R₁₀, R₁₁, R₁₂ₐ, X₆ and X₇ are as defined in the compound of Formula IIb1-a4. The stereocenter *1 of the catalyst according to Formula IIb1-a5 is in (R) and/or (S) configuration.

In the compound of Formula IIb1-a6, R₁₀, R₁₁, R₁₂ₐ and X₆ are as defined in the compound of Formula IIb1-a5, and in the compound of Formula IIb1-a6', R₁₀, R₁₁, R₁₂ₐ and X₇ are as defined in the compound of Formula IIb1-a5. The stereocenter *1 of the catalyst according to Formula IIb1-a6 and in the catalyst according to Formula IIb1-a6' is in (R) and/or (S) configuration.

According to certain embodiments, the catalyst, particularly the catalyst of Formula IIb1-a6, or the catalyst of Formula IIa-9, is a catalyst of the following Formula IIa-10:

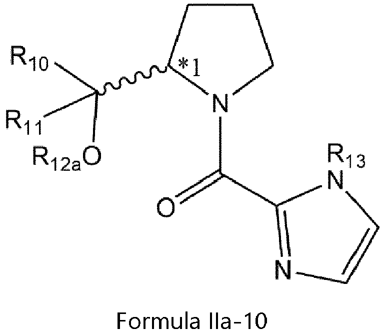

In the compound of Formula IIa-10, R₁₀, R₁₁, R₁₂ₐ and R₁₃ are as defined in the compound of Formula IIb1-a6. The stereocenter *1 of the catalyst according to Formula IIa-10 is in (R) and/or (S) configuration.

According to certain embodiments, the catalyst, particularly the catalyst of Formula I, is a catalyst of Formula la': 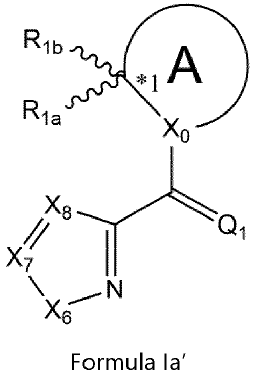
wherein in Formula la' ring A, R₁ₐ and R_{1b}, Q₁, X₀, X₆, X₇ and X₈ are as defined for the catalyst of Formula la;
R₁ₐ and R_{1b} are different; and
wherein the stereocenter *1 of the catalyst according to Formula la' is in (R) and/or (S) configuration.

According to certain embodiments, the catalyst, particularly the catalyst of Formula la', is a catalyst of Formula la'-1:

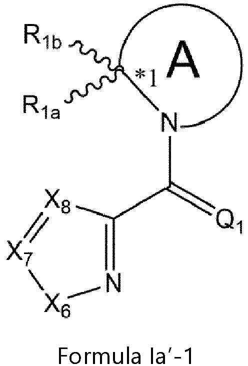

In the compound of Formula la'-1, ring A, R₁ₐ, R_{1b}, X₆, X₇ and X₈ are as defined in the compound of Formula la'. In the compound of Formula la'-1, R₁ₐ and R_{1b} are different. The stereocenter *1 of the catalyst according to Formula la'-1 is in (R) and/or (S) configuration.

According to certain embodiments, the catalyst of Formula la' is used as catalyst and the catalyst is particularly a catalyst of the following Formula II':

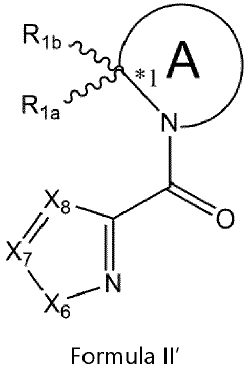

In the compound of Formula II', ring A, R₁ₐ, R_{1b}, X₆, X₇ and X₈ are as defined in the compound of Formula la'. In the compound of Formula II', R₁ₐ and R_{1b} are different.

The stereocenter *1 of the catalyst according to Formula II' is in (R) and/or (S) configuration. According to certain embodiments, when the stereocenter *1 of the catalyst is in (R) configuration, (S)-nornicotine is produced in excess. Furthermore, when the stereocenter *1 of the catalyst is in (S) configuration, (R)-nornicotine is produced in excess.

With the catalytic system described above, the inventors have surprisingly found that it is possible to reduce myosmine, which is a cyclic imine, to nornicotine. It is assumed that the amide unit of the catalyst according to Formula II' and the N in ring B, respectively the lower ring with X₆, X₇, and X₈, are responsible for the coordination of the silane and chemical activation. This allows the hydride transfer from the silane to myosmine, which is coordinated by ring A and the nitrogen therein, as well as possibly particular residues R₁ₐ. The stereocenter in ortho position with the residues R₁ₐ and R_{1b} is thought to direct the coordination of myosmine which enables its enantioselective hydrosilylation.

According to certain embodiments, the catalyst, particularly the catalyst of Formula II', is a catalyst of the following Formula IIa':

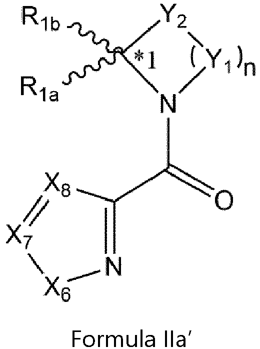

In the compound of Formula IIa', n, R₁ₐ, R_{1b}, R₁₈, R₁₉, X₆, X₇, X₈, Y₁ and Y₂ are as defined in the compound of Formula IIa. In the compound of Formula IIa', R₁ₐ and R_{1b} are different. The stereocenter *1 of the catalyst according to Formula IIa' is in (R) and/or (S) configuration;

According to certain embodiments, the catalyst, particularly the catalyst of Formula IIa', is a catalyst of the following Formula IIa'-1:

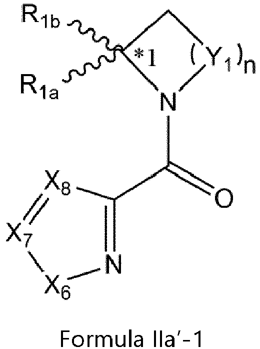

In the compound of Formula IIa'-1, R₁ₐ, R_{1b}, n, Y₁, X₆, X₇ and X₈ are as defined in the compound of Formula IIa'. In the compound of Formula IIa'-1, R₁ₐ and R_{1b} are different. The stereocenter *1 of the catalyst according to Formula IIa'-1 is in (R) and/or (S) configuration.

According to certain embodiments, the catalyst, particularly the catalyst of Formula IIa'-1, is a catalyst of the following Formula IIa'-2:

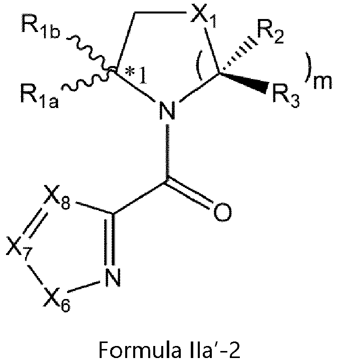

In the compound of Formula IIa'-2, m, R₁ₐ, R_{1b}, R₂, R₃, X₁, X₆, X₇ and X₈ are as defined in the compound of Formula IIa-2. In the compound of Formula IIa'-2, R₁ₐ and R_{1b} are different. The stereocenter *1 of the catalyst according to Formula IIa'-2 is in (R) and/or (S) configuration.

According to certain embodiments, the catalyst, particularly the catalyst of Formula II', is a catalyst of the following Formula IIb':

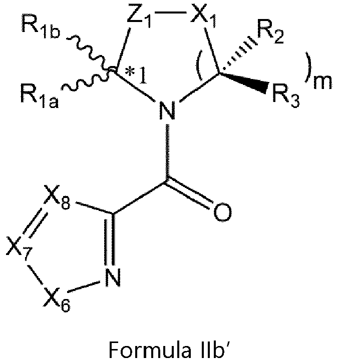

In the compound of Formula IIb', m, R₁ₐ, R_{1b}, R₂, R₃, R₄, R₄ₐ, R_{4b}, R₂₀ₐ, R_{20b}, X₁, X₆, X₇, X₈ and Z₁ are as defined in the compound of Formula IIb. In the compound of Formula IIb', R₁ₐ and R_{1b} are different. The stereocenter *1 of the catalyst according to Formula IIb' is in (R) and/or (S) configuration.

According to certain embodiments, the catalyst, particularly the catalyst of Formula IIb', is a catalyst of the following Formula IIb'1:

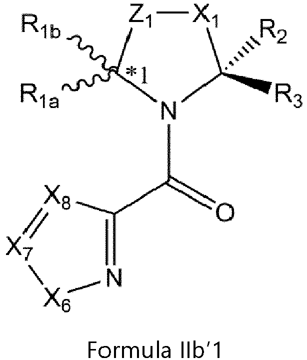

In the compound of Formula IIb'1, R₁ₐ, R_{1b}, R₂, R₃, X₁, X₆, X₇, X₈ and Z₁ are as defined in the compound of Formula IIb'. In the compound of Formula IIb'1, R₁ₐ and R_{1b} are different. The stereocenter *1 of the catalyst according to Formula IIb'1 is in (R) and/or (S) configuration.

According to certain embodiments, the catalyst, particularly the catalyst of Formula IIb', is a catalyst of the following Formula IIb'2:

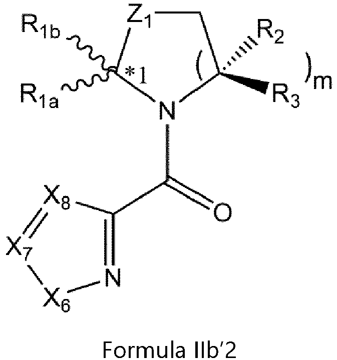

In the compound of Formula IIb'2, m, R₁ₐ, R_{1b}, R₂, R₃, X₆, X₇, X₈ and Z₁ are as defined in the compound of Formula IIb'. In the compound of Formula IIb'2, R₁ₐ and R_{1b} are different. The stereocenter *1 of the catalyst according to Formula IIb'2 is in (R) and/or (S) configuration.

According to certain embodiments, in the catalyst, particularly the catalyst of Formula II', respectively the catalyst of Formula IIa' or the catalyst of Formula IIb', preferably the catalyst of Formula IIa'-1, the catalyst of Formula IIb'1, or the catalyst of Formula IIb'2, as well as any of the following formulas directly or indirectly derived therefrom, R₁ₐ is represented by a tertiary residue CR₁₀R₁₁R₁₂.

In the tertiary residue CR₁₀R₁₁R₁₂ in the above formulas and any formulas derived thereof, R₁₀ and R₁₁ are the same or different, e.g. can be the same, and are chosen from H; F; Cl; Br; I; pseudohalogen groups; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂. According to preferred embodiments, in the tertiary residue CR₁₀R₁₁R₁₂ in the above formulas and any formulas derived thereof,R₁₀ and R₁₁ are the same or different, e.g. can be the same, and are chosen from H; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 18 C atoms that can optionally be substituted with at least one of F, Cl, Br, I, pseudohalogen groups, alkoxy groups with 1 to 20 C atoms, OH, NO₂ and NH₂; alkylaryl groups with 7 to 18 C atoms that can optionally be substituted with at least one of F, Cl, Br, I, pseudohalogen groups, alkoxy groups with 1 to 20 C atoms, OH, NO₂ and NH₂; and aromatic and/or heteroaromatic groups with 2 to 20 C atoms that can optionally be substituted with at least one of F, Cl, Br, I, pseudohalogen groups, alkoxy groups with 1 to 20 C atoms, OH, NO₂ and NH₂. According to more preferred embodiments, in the tertiary residue CR₁₀R₁₁R₁₂ in the above formulas and any formulas derived thereof,R₁₀ and R₁₁ are the same or different, e.g. can be the same, and are chosen from H; linear alkyl groups with 1 to 10 C atoms, preferably 1 to 6 C atoms, e.g. a methyl and ethyl group; aralkyl groups with 7 to 13 C atoms, preferably 7 to 10 C atoms, e.g. a (C₆H₅)-CH₂- group; alkylaryl groups with 7 to 15 C atoms, preferably 7 to 13 C atoms, e.g. a CH₃-C₆H₄-group; and aromatic groups with 6 to 14 C atoms, preferably 6 to 10 C atoms, e.g. a phenyl (Ph) and naphthy group (Naph).

In the tertiary residue CR₁₀R₁₁R₁₂, R₁₂ in the above formulas and any formulas derived thereof, is chosen from H, F; Cl; Br; I; pseudohalogen groups; OH; OR₁₂ₐ; SH; SR₁₂ₐ; COOH; COOR₁₂ₐ; NO₂; NH₂; (NR₁₈'R₁₉'R₂₀')⁺; and amine groups NR₁₄R₁₅, with the provisio that not both of R₁₄ and R₁₅ are H, and preferably R₁₂ is H; OH; OR₁₂ₐ, NH₂; or NR₁₄R₁₅, particularly preferably R₁₂ is H; OH; or OR₁₂ₐ, wherein R₁₈', R₁₉', and R₂₀' are the same or different and can be chosen from H; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂.
R₁₂ₐ is chosen from H;
linear alkyl groups with 1 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups (including (CO)NH₂) with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
linear alkenyl and/or alkynyl groups with 2 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
branched alkyl, alkenyl and/or alkynyl groups with 3 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
aralkyl groups with 7 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkylaryl groups with 7 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
cycloalkyl groups with 3 to 20 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heterocycloalkyl groups with 1 to 15 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
aromatic and/or heteroaromatic groups with 2 to 40 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl heteroaromatic groups with 2 to 50 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heteroaromatic groups with at least one alkyl residue with 3 to 50 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl cycloalkyl groups with 4 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
cycloalkyl alkyl groups with 4 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl heterocycloalkyl groups with 2 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heterocycloalkyl alkyl groups with 2 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂.
SiR₂₃R₂₄R₂₅;
R₂₃, R₂₄, and R₂₅ are the same or different and are selected from H, linear alkyl groups with 1 to 4 C atoms that can optionally be substituted with at least one of F, Cl, Br, I, pseudohalogen groups, alkoxy groups with 1 to 20 C atoms, OH, NO₂ and NH₂, branched alkyl group with 3 to 6 C atoms that can optionally be substituted with at least one of F, Cl, Br, I, pseudohalogen groups, alkoxy groups with 1 to 20 C atoms, OH, NO₂ and NH₂, aromatic groups with 6 to 12 C atoms that can optionally be substituted with at least one of F, Cl, Br, I, pseudohalogen groups, alkoxy groups with 1 to 20 C atoms, OH, NO₂ and NH₂, and aralkyl groups with 7 to 10 C atoms that can optionally be substituted with at least one of F, Cl, Br, I, pseudohalogen groups, alkoxy groups with 1 to 20 C atoms, OH, NO₂ and NH₂, preferably wherein R₂₃, R₂₄, and R₂₅ are not all hydrogen at the same time and/or wherein R₂₃, R₂₄, and R₂₅ are not all methyl at the same time, further preferably wherein all of R₂₃, R₂₄, and R₂₅ are ethyl;
R₁₄ and R₁₅ are the same or different and can be chosen from H,
linear alkyl groups with 1 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups (including (CO)NH₂) with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
linear alkenyl and/or alkynyl groups with 2 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
branched alkyl, alkenyl and/or alkynyl groups with 3 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
aralkyl groups with 7 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkylaryl groups with 7 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
cycloalkyl groups with 3 to 20 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heterocycloalkyl groups with 1 to 15 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
aromatic and/or heteroaromatic groups with 2 to 40 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl heteroaromatic groups with 2 to 50 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heteroaromatic groups with at least one alkyl residue with 3 to 50 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl cycloalkyl groups with 4 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
cycloalkyl alkyl groups with 4 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl heterocycloalkyl groups with 2 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heterocycloalkyl alkyl groups with 2 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂.

According to certain embodiments, alternatively or in addition, in the catalyst of Formula II', respectively the catalyst of Formula IIa' or the catalyst of Formula IIb', preferably the catalyst of Formula IIa'-1, the catalyst of Formula IIb'1, or the catalyst of Formula IIb'2, as well as any of the following formulas directly or indirectly derived therefrom, X₆ is NR₁₃. In reside NR₁₃, R₁₃ is chosen from H, linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂.

According to certain embodiments, the catalyst, particularly the catalyst of Formula IIb'1, respectively the catalyst of Formula IIa'-2, is a catalyst of the following Formula IIa'-3:

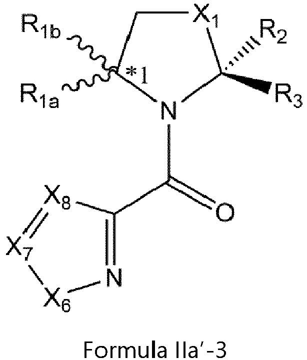

In the compound of Formula IIa'-3, R₁ₐ, R_{1b}, R₂, R₃, X₁, X₆, X₇ and X₈ are as defined in the compound of Formula IIa'-2, respectively the compound of Formula IIb'1. In the compound of Formula IIa'-3, R₁ₐ and R_{1b} are different. The stereocenter *1 of the catalyst according to Formula IIa'-3 is in (R) and/or (S) configuration.

According to certain embodiments, the catalyst, particularly the catalyst of Formula IIb'1, is a catalyst of the following Formula IIb'1-a, a catalyst of the following formula IIb'1-b, or a catalyst of the following formula IIb'1-c:

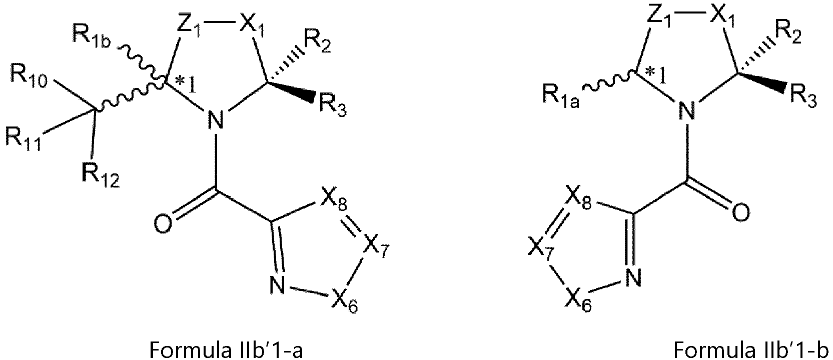

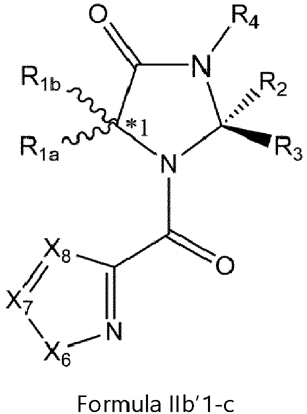

In the compound of Formula IIb'1-a R_{1b}, R₂, R₃, R₁₀, R₁₁, R₁₂, X₁, X₆, X₇, X₈ and Z₁ are as defined in the compound of Formula IIb1-a. The stereocenter *1 of the catalyst according to Formula IIb'1-a is in (R) and/or (S) configuration.

In the compound of Formula IIb'1-b, R₁ₐ, R₂, R₃, X₁, X₆, X₇, X₈ and Z₁ are as defined in the compound of Formula IIb'1. The stereocenter *1 of the catalyst according to Formula IIb'1-b is in (R) and/or (S) configuration.

In the compound of Formula IIb'1-c, R₁ₐ, R_{1b}, R₂, R₃, R₄, X₆, X₇ and X₈ are as defined in the compound of Formula IIb1-c. In the compound of Formula IIb'1-c, R₁ₐ and R_{1b} are different. The stereocenter *1 of the catalyst according to Formula IIb'1-c is in (R) and/or (S) configuration.

According to certain embodiments, the catalyst, particularly the catalyst of Formula IIb'2, is a catalyst of the following Formula IIb'2-1:

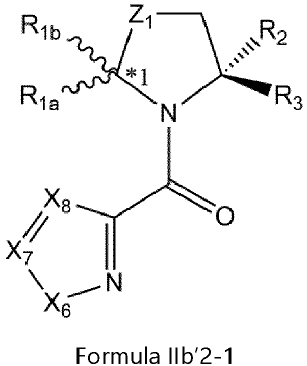

In the compound of Formula IIb'2-1, R₁ₐ, R_{1b}, R₂, R₃, X₆, X₇, X₈ and Z₁ are as defined in the compound of Formula IIb'2. In the compound of Formula IIb'2-1, R₁ₐ and R_{1b} are different. The stereocenter *1 of the catalyst according to Formula IIb'2-1 is in (R) and/or (S) configuration.

According to certain embodiments, the catalyst, particularly the catalyst of Formula IIb'2-1, is a catalyst of the following Formula IIb'2-1a or a catalyst of the following formula IIb'2-1b, and particularly a catalyst of the following formula IIb'2-2:

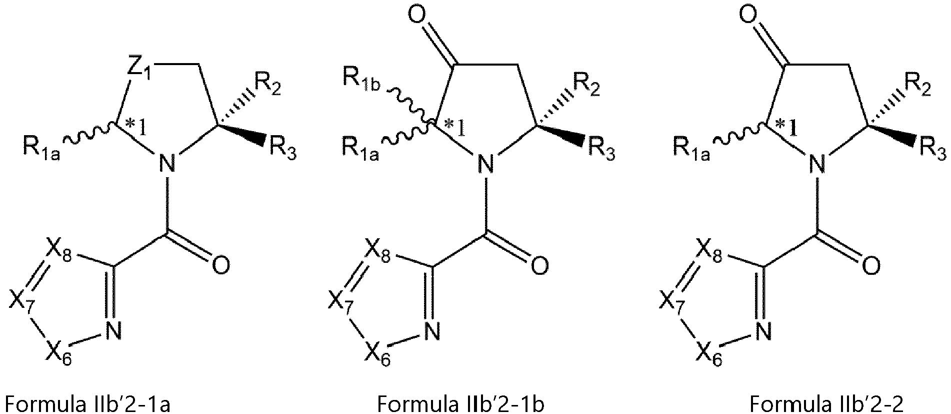

In the compound of Formula IIb2'-1a, R₁ₐ, R₂, R₃, X₆, X₇, X₈ and Z₁ are as defined in the compound of Formula IIb'2-1. The stereocenter *1 of the catalyst according to Formula IIb'2-1a is in (R) and/or (S) configuration.

In the compound of Formula IIb'2-1b, R₁ₐ, R_{1b}, R₂, R₃, X₆, X₇ and X₈ are as defined in the compound of Formula IIb'2-1. In the compound of Formula IIb'2-1 b, R₁ₐ and R_{1b} are different. The stereocenter *1 of the catalyst according to Formula IIb'2-1b is in (R) and/or (S) configuration.

In the compound of Formula IIb'2-2, R₁ₐ, R₂, R₃, X₆, X₇ and X₈ are as defined in the compound of Formula IIb'2-1a and/or the compound of Formula IIb'2-1b. The stereocenter *1 of the catalyst according to Formula IIb'2-2 is in (R) and/or (S) configuration.

According to certain embodiments, the catalyst, particularly the catalyst of Formula IIb'1-a, is a catalyst of the following Formula IIb'1-a1, and/or the catalyst of Formula IIb'1-b is a catalyst of the following Formula IIb'1-a1 or a catalyst of the following formula IIb'1-b1, and/or the catalyst of Formula IIb'1-c is a catalyst of the following Formula IIb'1-b1:

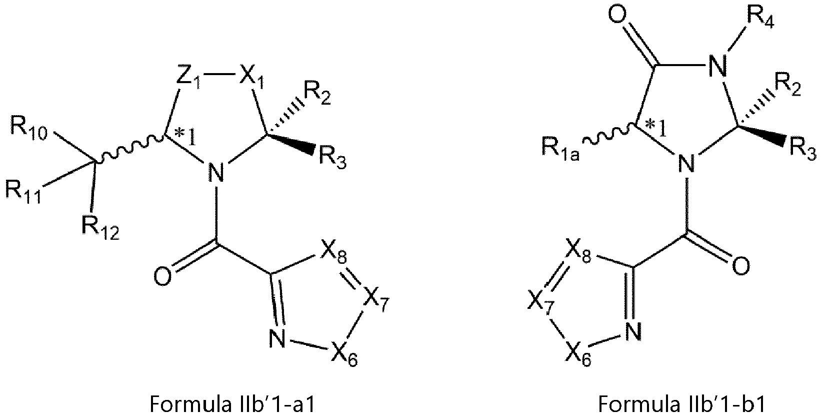

In the compound of Formula IIb'1-a1, R₂, R₃, R₁₀, R₁₁, R₁₂, X₁, X₆, X₇, X₈ and Z₁ are as defined in the compound of Formula IIb'1-a. The stereocenter *1 of the catalyst according to Formula IIb'1-a1 is in (R) and/or (S) configuration.

In the compound of Formula IIb'1-b1, R₁ₐ, R₂, R₃, R₄, X₆, X₇ and X₈ are as defined in the compound of Formula IIb'1-c. In the compound of Formula IIb'1-b1, R₁ₐ and R_{1b} are different. The stereocenter *1 of the catalyst according to Formula IIb'1-b1 is in (R) and/or (S) configuration.

According to certain embodiments, the catalyst, particularly the catalyst of Formula IIa'-3, is a catalyst of the following Formula IIa'-4, preferably a catalyst of the following Formula IIa'-5, further preferably a catalyst of the following Formula IIa'-6, even further preferably a catalyst of the following Formula IIa'-7, even further preferably a catalyst of the following Formula IIa'-8, even further preferably of the following Formula IIa'-9:

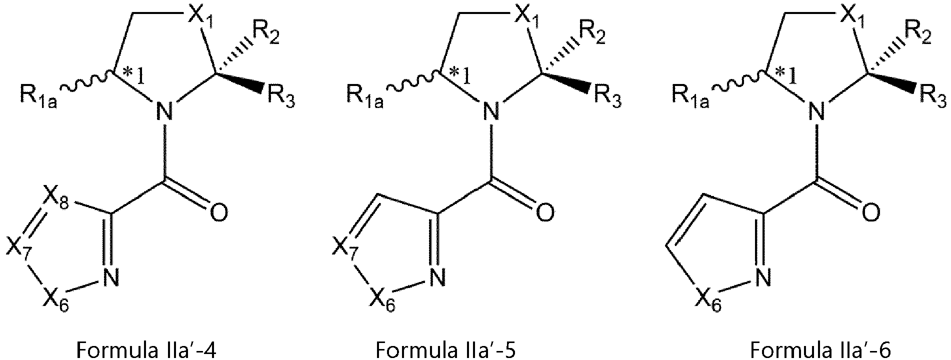

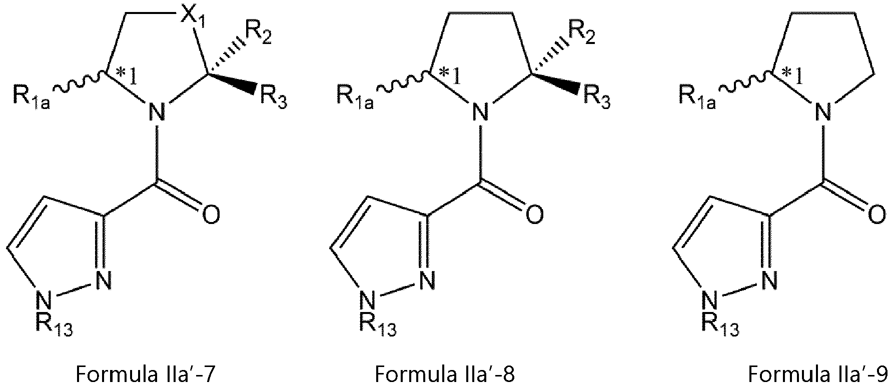

In the compound of Formula IIa'-4, R₁ₐ, R₂, R₃, X₁, X₆, X₇ and X₈ are as defined in the compound of Formula IIa'-3. The stereocenter *1 of the catalyst according to Formula IIa'-4 is in (R) and/or (S) configuration.

In the compound of Formula IIa'-5, R₁ₐ, R₂, R₃, X₁, X₆, and X₇ are as defined in the compound of Formula IIa'-4. The stereocenter *1 of the catalyst according to Formula IIa'-5 is in (R) and/or (S) configuration.

In the compound of Formula IIa'-6, R₁ₐ, R₂, R₃, X₁ and X₆ are as defined in the compound of Formula IIa-5. The stereocenter *1 of the catalyst according to Formula IIa'-6 is in (R) and/or (S) configuration.

In the compound of Formula IIa'-7, R₁ₐ, R₂, R₃, R₁₃ and X₁ are as defined in the compound of Formula IIa-7. The stereocenter *1 of the catalyst according to Formula IIa'-7 is in (R) and/or (S) configuration.

In the compound of Formula IIa'-8, R₁ₐ, R₂, R₃ and R₁₃ are as defined in the compound of Formula IIa'-7. The stereocenter *1 of the catalyst according to Formula IIa'-8 is in (R) and/or (S) configuration.

In the compound of Formula IIa'-9, R₁ₐ and R₁₃ are as defined in the compound of Formula IIa'-8. The stereocenter *1 of the catalyst according to Formula IIa'-9 is in (R) and/or (S) configuration.

According to certain embodiments, the catalyst, particularly the catalyst of Formula IIb'1-a1, is a catalyst of the following Formula IIb'1-a2, preferably a catalyst of the following Formula IIb'1-a3, further preferably a catalyst of the following Formula IIb'1-a4 or a catalyst of the following Formula IIb'1-a4'. The catalyst of Formula IIb'1-a4 is, according to certain embodiments, a catalyst of the following Formula IIb'1-a5, preferably of the following Formula IIb'1-a6 or a catalyst of the following Formula IIb'1-a6':

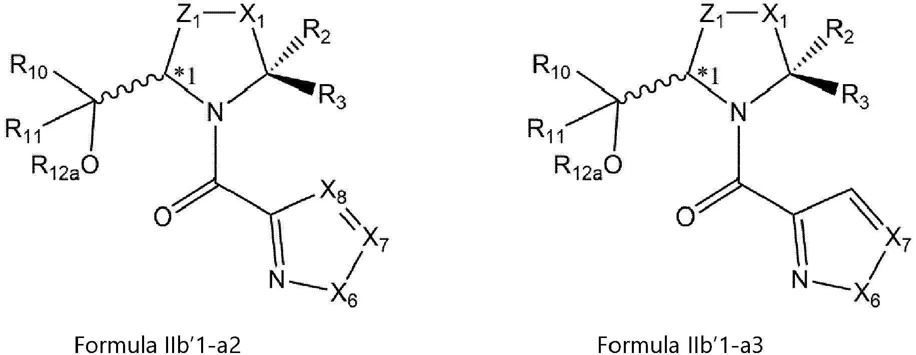

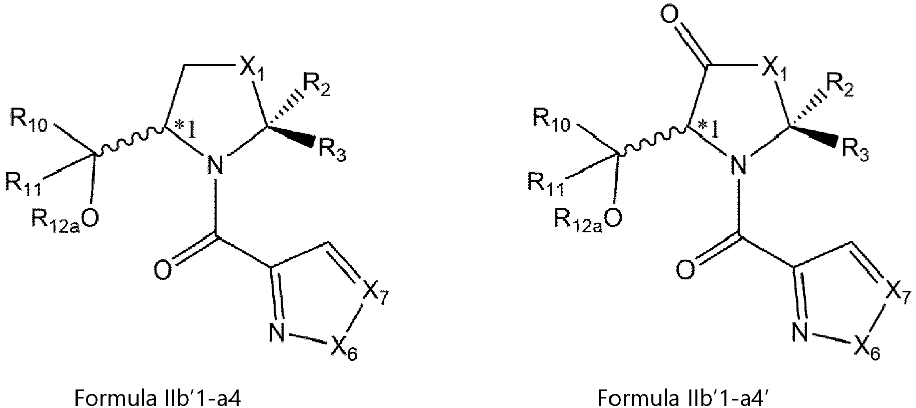

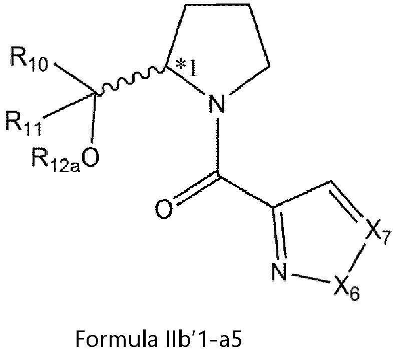

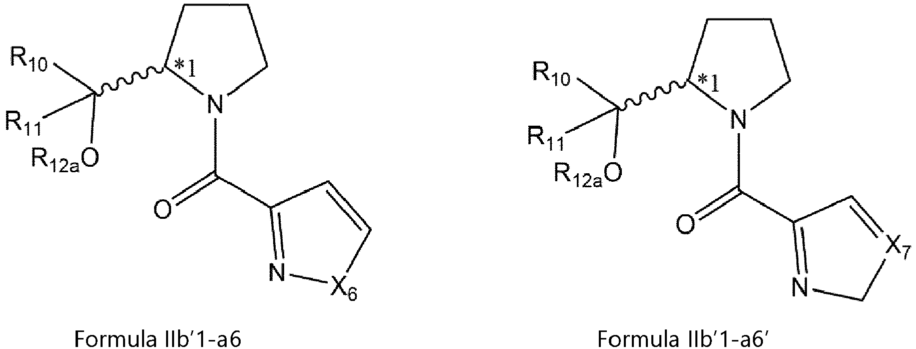

In the compound of Formula IIb'1-a2, R₂, R₃, R₁₀, R₁₁, R₁₂ₐ, X₁, X₆, X₇, X₈ and Z₁ are as defined in the compound of Formula IIb'1-a1. The stereocenter *1 of the catalyst according to Formula IIb'1-a2 is in (R) and/or (S) configuration.

In the compound of Formula IIb'1-a3, R₂, R₃, R₁₀, R₁₁, R₁₂ₐ, X₁, X₆, X₇ and Z₁ are as defined in the compound of Formula IIb'1-a2. The stereocenter *1 of the catalyst according to Formula IIb'1-a3 is in (R) and/or (S) configuration.

In the compound of Formula IIb'1-a4 and the compound of Formula IIb'1-a4', R₂, R₃, R₁₀, R₁₁, R₁₂ₐ, X₁, X₆ and X₇ are as defined in the compound of Formula IIb'1-a3. The stereocenter *1 of the catalyst according to Formula IIb'1-a4 and in the catalyst according to Formula IIb'1-a4' is in (R) and/or (S) configuration.

In the compound of Formula IIb'1-a5, R₁₀, R₁₁, R₁₂ₐ, X₆ and X₇ are as defined in the compound of Formula IIb'1-a4. The stereocenter *1 of the catalyst according to Formula IIb'1-a5 is in (R) and/or (S) configuration.

In the compound of Formula IIb'1-a6, R₁₀, R₁₁, R₁₂ₐ and X₆ are as defined in the compound of Formula IIb'1-a5, and in the compound of Formula IIb'1-a6', R₁₀, R₁₁, R₁₂ₐ and X₇ are as defined in the compound of Formula IIb'1-a5. The stereocenter *1 of the catalyst according to Formula IIb'1-a6 and in the catalyst according to Formula IIb'1-a6' is in (R) and/or (S) configuration.

According to certain embodiments, the catalyst, particularly the catalyst of Formula IIb'1-a6, or the catalyst of Formula IIa'-9, is a catalyst of the following Formula IIa'-10:

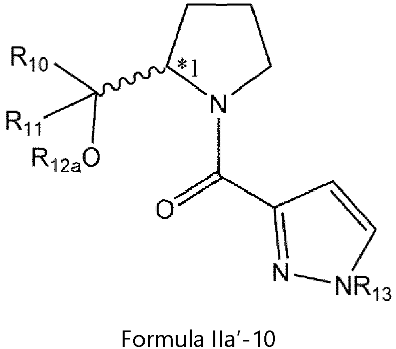

In the compound of Formula IIa'-10, R₁₀, R₁₁, R₁₂ₐ and R₁₃ are as defined in the compound of Formula IIb'1-a6. The stereocenter *1 of the catalyst according to Formula IIa'-10 is in (R) and/or (S) configuration.

According to certain embodiments, the catalyst, particularly the catalyst of Formula I, is a catalyst of Formula Ib:

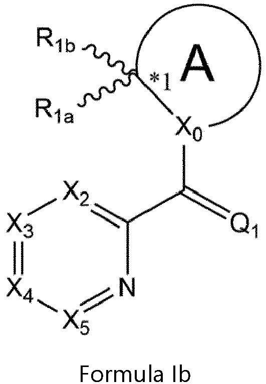

In Formula Ib, as well as any of the following formulas directly or indirectly derived therefrom, ring A, R₁ₐ and R_{1b}, Q₁ and X₀ are as defined above;
R₁ₐ and R_{1b} are different; and
X₂, X₃, X₄, and X₅ are independently selected from N and CR₆, with the provisio that when more than one of X₂, X₃, X₄, and X₅ is represented by CR₆, the more than one R₆ can be the same or different and/or more than one R₆ can form at least one ring, and with the provisio that at least one of X₂, X₃, X₄, and X₅ is CR₆. According to preferred embodiments, X₂, X₃, X₄, and X₅ are independently selected from N and CR₆, with the provisio that when more than one of X₂, X₃, X₄, and X₅ is represented by CR₆, the more than one R₆ can be the same or different, and with the provisio that at least one of X₂, X₃, X₄, and X₅ is CR₆. A formation of a ring is not preferable.

In the compound of Formula Ib, as well as any of the following formulas directly or indirectly derived therefrom, R₆ is chosen from H; F; Cl; Br; I; alkoxy groups with 1 to 20 C atoms; pseudohalogen groups; OH; NO₂; NH₂; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂.

The stereocenter *1 of the catalyst according to Formula Ib is in (R) and/or (S) configuration.

According to certain embodiments, the catalyst, particularly the catalyst of Formula Ib, is a catalyst of Formula Ib-1:

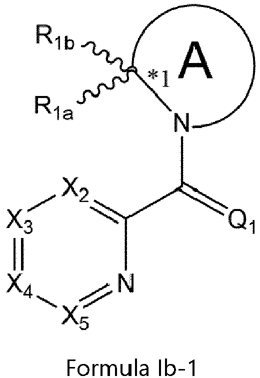

In the compound of Formula Ib-1, ring A, R₁ₐ, R_{1b}, X₂, X₃, X₄ and X₅ are as defined in the compound of Formula lb. In the compound of Formula Ib-1, R₁ₐ and R_{1b} are different. The stereocenter *1 of the catalyst according to Formula Ib-1 is in (R) and/or (S) configuration.

According to certain embodiments, the catalyst, particularly the catalyst of Formula I-b, is a catalyst of the following Formula III:

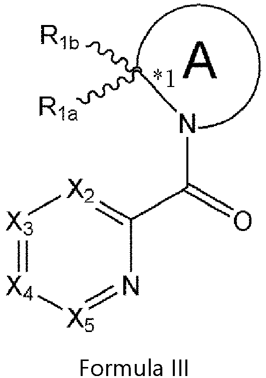

In the compound of Formula III, ring A, R₁ₐ, R_{1b}, X₂, X₃, X₄ and X₅ are as defined in the compound of Formula lb. In the compound of Formula III, R₁ₐ and R_{1b} are different. The stereocenter *1 of the catalyst according to Formula III is in (R) and/or (S) configuration.

In the compound of Formula III, as well as any of the following formulas directly or indirectly derived therefrom, R₁ₐ can be, according to certain embodiments, a linear alkyl group with 1 to 10 C atoms that can optionally be substituted with at least one of F, Cl, Br, I, pseudohalogen groups, alkoxy groups with 1 to 20 C atoms, carboxylic acid ester groups with 1 to 20 C atoms, alkyl and/or (hetero)aryl oxycarbonyl groups with 1 to 20 C atoms carboxamide groups with 1 to 20 C atoms (including (CO)NH₂), OH, SH, NO₂, NH₂, and amines of formula NR₁₈R₁₉, with R₁₈ and R₁₉ as defined above. Preferably, the linear alkyl group has 1 to 6 C atoms and can be substituted with at least one of or two or more of F, Cl, OH, an amide group with 1 to 6 C atoms (including (CO)NH₂), and/or alkoxy groups with 1 to 4 C atoms.

In certain embodiments, R₁ₐ can be a branched alkyl group with 3 to 10 C atoms that can optionally be substituted with at least one of F, Cl, Br, I, pseudohalogen groups, alkoxy groups with 1 to 20 C atoms, carboxylic acid ester groups with 1 to 20 C atoms, alkyl and/or (hetero)aryl oxycarbonyl groups with 1 to 20 C atoms carboxamide groups with 1 to 20 C atoms (including (CO)NH₂), OH, SH, NO₂, NH₂, and amines of formula NR₁₈R₁₉, with R₁₈ and R₁₉ as defined above. According to preferred embodiments, the branched alkyl group has 3 to 6 C atoms and can be substituted with at least one of or two or more of F, Cl, OH, an amide group with 1 to 6 C atoms (including (CO)NH₂), and/or alkoxy groups with 1 to 4 C atoms.

According to certain embodiments, R₁ₐ is an aralkyl group with 7 to 24 C atoms, particularly with 7 to 14 C atoms. The aralkyl group may be substituted with at least one of or two or more of F, Cl, Br, I, pseudohalogen groups, OH, SH, NO₂ and/or NH₂. Alternatively or in addition, the aralkyl group can be substituted with at least one of or two or more of alkoxy groups with a linear, branched, cyclic and/or aromatic residue having 1 to 20 C atoms that can comprise one or more heteroatoms selected from O, N, S and P. Specifically, the residue of the alkoxy group can be a linear, branched and/or cyclic alkyl residue with 1 to 20 C atoms that can have one or more heteroatoms selected from O, N, S and P at any position and can be linked at any position, thus including e.g. ethers, amides, thioethers, etc., can be an aromatic residue with 3 to 20 C atoms that can have one or more heteroatoms selected from O, N, S and P at any position and can be linked at any position, thus including heteroaromatic residues with furane, pyridine, pyrrole, indole, thiophene, etc. units, or can be a linear, branched and/or cyclic alkyl residue and/or an aromatic residue with 3 to 20 C atoms. Alternatively or in addition, the aralkyl group can be substituted with at least one of or two or more of carboxylic ester groups with a linear, branched, cyclic and/or aromatic residue having 1 to 20 C atoms that can comprise one or more heteroatoms selected from O, N, S and P. Specifically, the residue of the carboxylic ester group can be a linear, branched and/or cyclic alkyl residue with 1 to 20 C atoms that can have one or more heteroatoms selected from O, N, S and P at any position and can be linked at any position, thus including ethers, amides, thioethers, etc., can also be an aromatic residue with 3 to 20 C atoms that can have one or more heteroatoms selected from O, N, S and P at any position and can be linked at any position, thus including heteroaromatic residues with furane, pyridine, pyrrole, indole, thiophene, etc. units, or can be a linear, branched and/or cyclic alkyl residue and/or an aromatic residue with 3 to 20 C atoms. Alternatively or in addition, the aralkyl group can be substituted with at least one of or two or more of alkyl and/or (hetero)aryl oxycarbonyl groups with a linear, branched, cyclic and/or aromatic residue having 1 to 20 C atoms that can comprise one or more heteroatoms selected from O, N, S and P. Specifically, the residue of the oxycarbonyl group can be a linear, branched and/or cyclic alkyl residue with 1 to 20 C atoms that can have one or more heteroatoms selected from O, N, S and P at any position and can be linked at any position, thus including ethers, amides, thioethers, etc., can also be an aromatic residue with 3 to 20 C atoms that can have one or more heteroatoms selected from O, N, S and P at any position and can be linked at any position, thus including heteroaromatic residues with furane, pyridine, pyrrole, indole, thiophene, etc. units, or can be a linear, branched and/or cyclic alkyl residue and/or an aromatic residue with 3 to 20 C atoms. Alternatively or in addition, the aralkyl group can be substituted with at least one of or two or more of carboxamide groups with a linear, branched, cyclic and/or aromatic residue having 1 to 20 C atoms that can comprise one or more heteroatoms selected from O, N, S and P. Specifically, the residue of the carboxamide group can be a linear, branched and/or cyclic alkyl residue with 1 to 20 C atoms that can have one or more heteroatoms selected from O, N, S and P at any position and can be linked at any position, thus including ethers, amides, thioethers, etc., can also be an aromatic residue with 3 to 20 C atoms, that can have one or more heteroatoms selected from O, N, S and P at any position and can be linked at any position, thus including heteroaromatic residues with furane, pyridine, pyrrole, indole, thiophene, etc. units, or can be a linear, branched and/or cyclic alkyl residue and/or an aromatic residue with 3 to 20 C atoms. According to preferred embodiments, R₁ₐ is a benzyl residue which may be substituted with a hydroxyl group, an alkoxy group as defined above or a carboxylic ester group as defined above. Preferably, the substituent is in para position.

Furthermore, R₁ₐ can in some embodiments be an alkylaryl group with 7 to 24 C atoms that may be substituted with at least one of F, Cl, Br, I, pseudohalogen groups, alkoxy groups with a linear, branched, cyclic and/or aromatic residue having 1 to 20 C atoms that can comprise one or more heteroatoms selected from O, N, S and P, carboxylic ester groups with a linear, branched, cyclic and/or aromatic residue having 1 to 20 C atoms that can comprise one or more heteroatoms selected from O, N, S and P, oxycarbonyl groups with a linear, branched, cyclic and/or aromatic residue having 1 to 20 C atoms that can comprise one or more heteroatoms selected from O, N, S and P, carboxamide groups with a linear, branched, cyclic and/or aromatic residue having 1 to 20 C atoms that can comprise one or more heteroatoms selected from O, N, S and P, OH, NO₂ and NH₂. According to preferred embodiments, the alkylaryl group has 7 to 10 C atoms and can be substituted with at least one of or two or more of F, Cl, OH and/or alkoxy groups with 1 to 4 C atoms.

In certain embodiments, R₁ₐ can be a cycloalkyl group with 3 to 20 C atoms that can be substituted with at least one of or two or more of F, Cl, Br, I, pseudohalogen groups, alkoxy groups with 1 to 20 C atoms, carboxylic acid ester groups with 1 to 20 C atoms, alkyl and/or (hetero)aryl oxycarbonyl groups with 1 to 20 C atoms carboxamide groups with 1 to 20 C atoms (including (CO)NH₂), OH, SH, NO₂, NH₂, and amines of formula NR₁₈R₁₉, with R₁₈ and R₁₉ as defined above. It is preferred that the cycloalkyl group has 3 to 6 C atoms and can be substituted with at least one of or two or more of F, Cl, OH and/or alkoxy groups with 1 to 4 C atoms.

R₁ₐ can further be a heterocycloalkyl group with 1 to 15 C atoms that can be substituted with at least one of F, Cl, Br, I, pseudohalogen groups, alkoxy groups with 1 to 20 C atoms, carboxylic acid ester groups with 1 to 20 C atoms, alkyl and/or (hetero)aryl oxycarbonyl groups with 1 to 20 C atoms carboxamide groups with 1 to 20 C atoms (including (CO)NH₂), OH, SH, NO₂, NH₂, and amines of formula NR₁₈R₁₉, with R₁₈ and R₁₉ as defined above. In preferred embodiments, the heterocycloalkyl group has 1 to 6 C atoms and can be substituted with at least one of or two or more of F, Cl, OH and/or alkoxy groups with 1 to 4 C atoms.

In certain embodiments, R₁ₐ can be an aromatic and/or heteroaromatic group with 2 to 40 C atoms, e.g. an aromatic group with 6 to 40 C atoms and/or a heteroaromatic group with 2 to 40 C atoms, that can be substituted with at least one of F, Cl, Br, I, pseudohalogen groups, alkoxy groups with a linear, branched, cyclic and/or aromatic residue having 1 to 20 C atoms that can comprise one or more heteroatoms selected from O, N, S and P, carboxylic ester groups with a linear, branched, cyclic and/or aromatic residue having 1 to 20 C atoms that can comprise one or more heteroatoms selected from O, N, S and P, oxycarbonyl groups with a linear, branched, cyclic and/or aromatic residue having 1 to 20 C atoms that can comprise one or more heteroatoms selected from O, N, S and P, carboxamide groups with a linear, branched, cyclic and/or aromatic residue having 1 to 20 C atoms that can comprise one or more heteroatoms selected from O, N, S and P, OH, SH, NO₂ and NH₂. Preferably, the aromatic or heteroaromatic group has 2 to 6 C atoms and can be substituted with at least one of or two or more of F, Cl, OH and/or alkoxy groups with 1 to 4 C atoms.

Further, R₁ₐ can be an alkyl heteroaromatic group with 3 to 50 C atoms that can be substituted with at least one of F, Cl, Br, I, pseudohalogen groups, alkoxy groups with a linear, branched, cyclic and/or aromatic residue having 1 to 20 C atoms that can comprise one or more heteroatoms selected from O, N, S and P, carboxylic ester groups with a linear, branched, cyclic and/or aromatic residue having 1 to 20 C atoms that can comprise one or more heteroatoms selected from O, N, S and P, oxycarbonyl groups with a linear, branched, cyclic and/or aromatic residue having 1 to 20 C atoms that can comprise one or more heteroatoms selected from O, N, S and P, carboxamide groups with a linear, branched, cyclic and/or aromatic residue having 1 to 20 C atoms that can comprise one or more heteroatoms selected from O, N, S and P, OH, SH, NO₂ and NH₂. In preferred embodiments, the alkyl heteroaromatic group has 3 to 10 C atoms and can be substituted with at least one of or two or more of F, Cl, OH and/or alkoxy groups with 1 to 4 C atoms.

R₁ₐ can in some embodiments be a heteroaromatic group with at least one alkyl residue with 3 to 50 C atoms that can be substituted with at least one of F, Cl, Br, I, pseudohalogen groups, alkoxy groups with a linear, branched, cyclic and/or aromatic residue having 1 to 20 C atoms that can comprise one or more heteroatoms selected from O, N, S and P, carboxylic ester groups with a linear, branched, cyclic and/or aromatic residue having 1 to 20 C atoms that can comprise one or more heteroatoms selected from O, N, S and P, oxycarbonyl groups with a linear, branched, cyclic and/or aromatic residue having 1 to 20 C atoms that can comprise one or more heteroatoms selected from O, N, S and P, carboxamide groups with a linear, branched, cyclic and/or aromatic residue having 1 to 20 C atoms that can comprise one or more heteroatoms selected from O, N, S and P, OH, SH, NO₂ and NH₂. Preferably, the heteroaromatic group with at least one alkyl residue has 3 to 10 C atoms and can be substituted at least one of or two or more of F, Cl, OH and/or alkoxy groups with 1 to 4 C atoms.

According to certain embodiments, R₁ₐ can be an alkyl cycloalkyl group with 4 to 24 C atoms that can be substituted with at least one of F, Cl, Br, I, pseudohalogen groups, alkoxy groups with 1 to 20 C atoms, carboxylic acid ester groups with 1 to 20 C atoms, alkyl and/or (hetero)aryl oxycarbonyl groups with 1 to 20 C atoms carboxamide groups with 1 to 20 C atoms (including (CO)NH₂), OH, SH, NO₂, NH₂, and amines of formula NR₁₈R₁₉, with R₁₈ and R₁₉ as defined above. It is preferred that the alkyl cycloalkyl group has 4 to 10 C atoms and can be substituted with at least one of or two or more of F, Cl, OH and/or alkoxy groups with 1 to 4 C atoms. Furthermore, R₁ₐ can in certain embodiments be a cycloalkyl alkyl group with 4 to 24 C atoms that can be substituted with at least one of F, Cl, Br, I, pseudohalogen groups, alkoxy groups with 1 to 20 C atoms, carboxylic acid ester groups with 1 to 20 C atoms, alkyl and/or (hetero)aryl oxycarbonyl groups with 1 to 20 C atoms carboxamide groups with 1 to 20 C atoms (including (CO)NH₂), OH, SH, NO₂, NH₂, and amines of formula NR₁₈R₁₉, with R₁₈ and R₁₉ as defined above. Preferably, the cycloalkyl alkyl group has 4 to 10 C atoms and can be substituted with at least one of or two or more of F, Cl, OH and/or alkoxy groups with 1 to 4 C atoms.

In some embodiments, R₁ₐ can be a carboxylic acid ester group with 1 to 20 C atoms that can be substituted with at least one of or two or more of F, Cl, Br, I, pseudohalogen groups, alkoxy groups with 1 to 20 C atoms, OH, SH, NO₂ and/or NH₂. According to preferred embodiments, the carboxylic acid ester group has 1 to 8 C atoms and can be substituted with at least one of or two or more of F, Cl, OH and/or alkoxy groups with 1 to 4 C atoms.

According to certain embodiments, R₁ₐ can be an alkyl and/or (hetero)aryl oxycarbonyl group with 1 to 20 C atoms that can be substituted with at least one of or two or more of F, Cl, Br, I, pseudohalogen groups, alkoxy groups with 1 to 20 C atoms, OH, SH, NO₂ and/or NH₂. Preferably, the linear, branched, cyclic alkyl and/or (hetero)aromatic oxycarbonyl group has 1 to 8 C atoms and can be substituted with at least one of or two or more of F, Cl, OH and/or alkoxy groups with 1 to 4 C atoms.

Further, R₁ₐ can in some embodiments be a carboxamide group with 1 to 20 C atoms that can be substituted with at least one of or two or more of F, Cl, Br, I, pseudohalogen groups, alkoxy groups with 1 to 20 C atoms, OH, SH, NO₂ and/or NH₂. It is preferred that the carboxamide group has 1 to 8 C atoms and can be substituted with at least one of or two or more of F, Cl, OH and/or alkoxy groups with 1 to 4 C atoms.

According to preferred embodiments, R₁ₐ is an aralkyl group with 7 to 24 C atoms, particularly with 7 to 13 C atoms, as defined above, that can be substituted, as above.

In the compound of Formula III, R₁ₐ and R_{1b} are different. This means that there is a chiral center at the atom marked with *1 in the compound of Formula III, which is a carbon atom.

According to some embodiments, in the compound of Formula III, as well as any of the following formulas directly or indirectly derived therefrom, R_{1b} is H. R_{1b} can in certain embodiments be a linear alkyl group with 1 to 6 C atoms that can be substituted with at least one of or two or more of F, Cl, Br, I, pseudohalogen groups, alkoxy groups with 1 to 6 C atoms, OH, NO₂ and/or NH₂. Preferably, the linear alkyl group has 1 to 4 C atoms and can be substituted with at least one of or two or more of F, Cl and/or OH.

According to some embodiments, R_{1b} can be a branched alkyl group with 3 to 6 C atoms that can be substituted with at least one of or two or more of F, Cl, Br, I, pseudohalogen groups, alkoxy groups with 1 to 6 C atoms, OH, NO₂ and/or NH₂. It is preferred that the branched alkyl group has 3 to 4 C atoms and can be can be substituted with at least one of or two or more of F, CI and/or OH.

Further, R_{1b} can in certain embodiments be a heterocycloalkyl group with 1 to 6 C atoms that can be substituted with at least one of or two or more of F, Cl, Br, I, pseudohalogen groups, alkoxy groups with 1 to 6 C atoms, OH, NO₂ and/or NH₂. According to preferred embodiments, the heterocycloalkyl group has 1 to 5 C atoms and can be substituted with at least one of or two or more of F, Cl and/or OH.

According to some embodiments, R_{1b} can be an aromatic and/or heteroaromatic group with 2 to 6 carbon atoms that can be substituted with at least one of or two or more of F, Cl, Br, I, pseudohalogen groups, alkoxy groups with 1 to 6 C atoms, OH, NO₂ and/or NH₂, preferably with F, CI and/or OH.

Furthermore, R_{1b} can in certain embodiments be a carboxylic acid ester group with 1 to 6 C atoms that can be substituted with at least one of or two or more of F, Cl, Br, I, pseudohalogen groups, alkoxy groups with 1 to 6 C atoms, OH, NO₂ and/or NH₂. It is preferred that the carboxylic acid ester group has 1 to 4 C atoms and can be substituted with at least one of or two or more of F, Cl and/or OH.

According to certain embodiments, R_{1b} can be a linear, branched and/or cyclic alkyl and/or (hetero)aryl oxycarbonyl group with 1 to 6 C atoms that can be substituted with at least one of or two or more of F, Cl, Br, I, pseudohalogen groups, alkoxy groups with 1 to 6 C atoms, OH, NO₂ and/or NH₂. Preferably, the carboxylic acid ester group has 1 to 4 C atoms and can be substituted with at least one of or two or more of F, Cl and/or OH.

Also, R_{1b} can in some embodiments be a carboxamide groups with 1 to 6 C atoms that can be substituted with at least one of or two or more of F, Cl, Br, I, pseudohalogen groups, alkoxy groups with 1 to 6 C atoms, OH, NO₂ and/or NH₂. According to preferred embodiments, the carboxylic acid ester group has 1 to 4 C atoms and can be substituted with at least one of or two or more of F, Cl and/or OH.

According to preferred embodiments, R_{1b} is H or methyl, particularly H.

In the compound of Formula III, as well as any of the following formulas directly or indirectly derived therefrom, X₂, X₃, X₄, and X₅ are independently selected from N and CR₆, with the provisio that when more than one of X₂, X₃, X₄, and X₅ is represented by CR₆, the more than one R₆ can be the same or different and/or more than one R₆ can form at least one ring, and with the provisio that at least one of X₂, X₃, X₄, and X₅ is CR₆. According to preferred embodiments, X₂, X₃, X₄, and X₅ are independently selected from N and CR₆, with the provisio that when more than one of X₂, X₃, X₄, and X₅ is represented by CR₆, the more than one R₆ can be the same or different, and with the provisio that at least one of X₂, X₃, X₄, and X₅ is CR₆. A formation of a ring is not preferable.

According to certain embodiments, R₆ can be H, F, Cl, Br, I, a pseudohalogen group, OH, NO₂ and/or NH₂. R₆ can alternatively or in addition, in some embodiments, be an alkoxy group with 1 to 20 C atoms, preferably with 1 to 4 C atoms.

According to certain embodiments, R₆ can be, alternatively or in addition, a linear alkyl group with 1 to 10 C atoms that can be substituted with at least one of or two or more of F, Cl, Br, I, pseudohalogen groups, alkoxy groups with 1 to 20 C atoms, OH, NO₂ and/or NH₂. Preferably, the linear alkyl group has 1 to 6 C atoms and can be substituted with at least one of or two or more of F, Cl, OH and/or alkoxy groups with 1 to 4 C atoms.

Alternatively or in addition, R₆ can in some embodiments be a branched alkyl group with 3 to 10 C atoms that can be substituted with at least one of or two or more of F, Cl, Br, I, pseudohalogen groups, alkoxy groups with 1 to 20 C atoms, OH, NO₂ and/or NH₂. In preferred embodiments, the branched alkyl group has 3 to 6 C atoms and can be substituted with at least one of or two or more of F, Cl, OH and/or alkoxy groups with 1 to 4 C atoms.

In certain embodiments, R₆ can be, alternatively or in addition, an aralkyl group with 7 to 24 C atoms that can be substituted with at least one of or two or more of F, Cl, Br, I, pseudohalogen groups, alkoxy groups with 1 to 20 C atoms, OH, NO₂ and/or NH₂. In preferred embodiments, the aralkyl group has 7 to 10 C atoms and can be substituted with at least one of or two or more of F, Cl, OH and/or alkoxy groups with 1 to 4 C atoms.

R₆ can according to certain embodiments be, alternatively or in addition, an alkylaryl group with 7 to 24 C atoms that can be substituted with at least one of or two or more of F, Cl, Br, I, pseudohalogen groups, alkoxy groups with 1 to 20 C atoms, OH, NO₂ and/or NH₂. According to preferred embodiments, the alkylaryl group has 7 to 10 C atoms and can be substituted with at least one of or two or more of F, Cl, OH and/or alkoxy groups with 1 to 4 C atoms.

In some embodiments, R₆ can be, alternatively or in addition, a cycloalkyl group with 3 to 20 C atoms that can be substituted with at least one of or two or more of F, Cl, Br, I, pseudohalogen groups, alkoxy groups with 1 to 20 C atoms, OH, NO₂ and/or NH₂. It is preferred that the cycloalkyl group has 3 to 6 C atoms and can be substituted with at least one of or two or more of F, Cl, OH and/or alkoxy groups with 1 to 4 C atoms.

Alternatively or in addition, according to certain embodiments, R₆ can be a heterocycloalkyl group with 1 to 15 C atoms that can be substituted with at least one of or two or more of F, Cl, Br, I, pseudohalogen groups, alkoxy groups with 1 to 20 C atoms, OH, NO₂ and/or NH₂. In preferred embodiments, the heterocycloalkyl group has 1 to 6 C atoms and can be substituted with at least one of or two or more of F, Cl, OH and/or alkoxy groups with 1 to 4 C atoms. Furthermore, R₆ can in some embodiments be, alternatively or in addition, an aromatic and/or heteroaromatic group with 2 to 40 C atoms, e.g. aromatic group with 6 to 40 C atoms and/or heteroaromatic group with 2 to 40 C atoms, that can be substituted with at least one of or two or more of F, Cl, Br, I, pseudohalogen groups, alkoxy groups with 1 to 20 C atoms, OH, NO₂ and/or NH₂. Preferably, the aromatic or heteroaromatic group has 2 to 6 C atoms and can be substituted with at least one of or two or more of F, Cl, OH and/or alkoxy groups with 1 to 4 C atoms.

In some embodiments, R₆ can be, alternatively or in addition, an alkyl heteroaromatic group with 3 to 50 C atoms that can be substituted with at least one of or two or more of F, Cl, Br, I, pseudohalogen groups, alkoxy groups with 1 to 20 C atoms, OH, NO₂ and/or NH₂. In preferred embodiments, the alkyl heteroaromatic group has 3 to 10 C atoms and can be substituted with at least one of or two or more of F, Cl, OH and/or alkoxy groups with 1 to 4 C atoms. Alternatively or in addition, according to certain embodiments, R₆ can be a heteroaromatic group with at least one alkyl residue with 3 to 50 C atoms that can be substituted with at least one of or two or more of F, Cl, Br, I, pseudohalogen groups, alkoxy groups with 1 to 20 C atoms, OH, NO₂ and/or NH₂. Preferably, the heteroaromatic group with at least one alkyl residue has 3 to 10 C atoms and can be substituted at least one of or two or more of F, Cl, OH and/or alkoxy groups with 1 to 4 C atoms.

R₆ can in some embodiments be, alternatively or in addition, an alkyl cycloalkyl group with 4 to 24 C atoms that can be substituted with at least one of or two or more of F, Cl, Br, I, pseudohalogen groups, alkoxy groups with 1 to 20 C atoms, OH, NO₂ and/or NH₂. It is preferred that the alkyl cycloalkyl group has 4 to 10 C atoms and can be substituted with at least one of or two or more of F, Cl, OH and/or alkoxy groups with 1 to 4 C atoms.

According to some embodiments, R₆ can be, alternatively or in addition, a cycloalkyl alkyl group with 4 to 24 C atoms that can be substituted with at least one of or two or more of F, Cl, Br, I, pseudohalogen groups, alkoxy groups with 1 to 20 C atoms, OH, NO₂ and/or NH₂. Preferably, the cycloalkyl alkyl group has 4 to 10 C atoms and can be substituted with at least one of or two or more of F, Cl, OH and/or alkoxy groups with 1 to 4 C atoms.

R₆ can in some embodiments be, alternatively or in addition, an alkyl heterocycloalkyl group with 2 to 24 C atoms that can optionally be substituted with at least one of F, Cl, Br, I, pseudohalogen groups, alkoxy groups with 1 to 20 C atoms, OH, NO₂ and NH₂. Preferably, the alkyl heterocycloalkyl group has 2 to 10 C atoms and can be substituted with at least one of or two or more of F, Cl, OH and/or alkoxy groups with 1 to 4 C atoms.

R₆ can in some embodiments be, alternatively or in addition, a heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂. Preferably, the heterocycloalkyl alkyl group has 4 to 10 C atoms and can be substituted with at least one of or two or more of F, Cl, OH and/or alkoxy groups with 1 to 4 C atoms.

Further, alternatively or in addition, R₆ can according to certain embodiments be a carboxylic acid ester group with 1 to 20 C atoms that can be substituted with at least one of or two or more of F, Cl, Br, I, pseudohalogen groups, alkoxy groups with 1 to 20 C atoms, OH, NO₂ and/or NH₂. According to preferred embodiments, the carboxylic acid ester group has 1 to 8 C atoms and can be substituted with at least one of or two or more of F, Cl, OH and/or alkoxy groups with 1 to 4 C atoms.

Also, R₆ can in some embodiments be, alternatively or in addition, an alkyl and/or (hetero)aryl oxycarbonyl group with 1 to 20 C atoms that can be substituted with at least one of or two or more of F, Cl, Br, I, pseudohalogen groups, alkoxy groups with 1 to 20 C atoms, OH, NO₂ and/or NH₂. Preferably, the alkyl and/or (hetero)aryl oxycarbonyl group has 1 to 8 C atoms and can be substituted with at least one of or two or more of F, Cl, OH and/or alkoxy groups with 1 to 4 C atoms.

In certain embodiments, R₆ can be, alternatively or in addition, a carboxamide group with 1 to 20 C atoms that can be substituted with at least one of or two or more of F, Cl, Br, I, pseudohalogen groups, alkoxy groups with 1 to 20 C atoms, OH, NO₂ and/or NH₂. It is preferred that the carboxamide group has 1 to 8 C atoms and can be substituted with at least one of or two or more of F, Cl, OH and/or alkoxy groups with 1 to 4 C atoms.

When more than one of X₂, X₃, X₄, and X₅ is represented by CR₆, the more than one R₆ can be the same or different, and/or the more than one R₆ can form a ring. In the compound of Formula III, at least one of X₂, X₃, X₄, and X₅ is CR₆.

According to preferred embodiments, R₆ is H or an electron withdrawing group such as an alkoxy group, like methoxy or ethoxy, F, Cl, Br, I, or NO₂, particularly preferably H. Such an electron withdrawing group can be at any position of the pyridine-derived ring, but may be, according to certain, preferred, embodiments, in meta-position to the amide-bonded ring A. Preferably, X₂, X₃, X₄ and X₅ are all represented by CR₆. Preferably, all of R₆ are H.

The stereocenter *1 of the catalyst according to Formula III is in (R) and/or (S) configuration. According to certain embodiments, when the stereocenter *1 of the catalyst is in (R) configuration, (S)-nornicotine is produced in excess. Furthermore, when the stereocenter *1 of the catalyst is in (S) configuration, (R)-nornicotine is produced in excess.

With the catalytic system described above, the inventors have surprisingly found that it is possible to reduce myosmine, which is a cyclic imine, to nornicotine. It is assumed that the amide unit of the catalyst according to Formula III is responsible for the coordination of the silane and chemical activation. This allows the hydride transfer from the silane to myosmine, which is coordinated by the carbonyl group and the nitrogen of the adjacent heteroaromatic unit. The stereocenter in ortho position with the residues R₁ₐ and R_{1b} is thought to direct the coordination of myosmine which enables its enantioselective hydrosilylation.

According to certain embodiments, the catalyst, particularly the catalyst of Formula III, is represented by the following Formula IIIa:

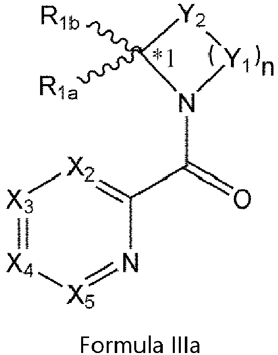

In the compound of Formula IIIa, R₁ₐ, R_{1b}, X₂, X₃, X₄, and X₅ are as defined in the compound of Formula III.

In the compound of Formula IIIa, R₁ₐ and R_{1b} are different. The stereocenter *1 of the catalyst according to Formula IIIa is in (R) and/or (S) configuration.

In the compound of Formula IIIa, as well as any of the following formulas directly or indirectly derived therefrom, Y₁ and Y₂ are substituted or unsubstituted C and/or substituted or unsubstituted heteroatoms. If more than one Y₁ is present, these can be the same or different The substituents can comprise at least one of or two or more of F, Cl, Br, I, pseudohalogen groups, alkoxy groups with 1 to 20 C atoms, preferably 1 to 4 C atoms, OH, SR₂₁, S(CO)R₂₂, NO₂, NH₂, (NR₁₈'R₁₉'R₂₀')⁺, and /or amines of formula NR₁₈R₁₉, with R₁₈, R₁₉, R₁₈', R₁₉', R₂₀', R₂₁, and R₂₂ as defined above. Alternatively or in addition, the substituents can comprise linear alkyl groups with 1 to 10 C atoms, preferably 1 to 6 C atoms, that can be substituted with at least one of or two or more of F, Cl, Br, I, pseudohalogen groups, alkoxy groups with 1 to 20 C atoms, preferably 1 to 4 C atoms, OH, NO₂ and/or NH₂. Alternatively or in addition, the substituents can comprise branched alkyl groups with 3 to 10 C atoms, preferably 3 to 6 C atoms, that can be substituted with at least one of or two or more of F, Cl, Br, I, pseudohalogen groups, alkoxy groups with 1 to 20 C atoms, preferably 1 to 4 atoms, OH, NO₂ and/or NH₂. Alternatively or in addition, the substituents can comprise aralkyl groups with 7 to 24 C atoms, preferably 7 to 10 C atoms, that can be substituted with at least one of or two or more of F, Cl, Br, I, pseudohalogen groups, alkoxy groups with 1 to 20 C atoms, preferably 1 to 4 atoms, OH, NO₂ and/or NH₂. Alternatively or in addition, the substituents can comprise alkylaryl groups with 7 to 24 C atoms, preferably 7 to 10 C atoms, that can be substituted with at least one of or two or more of F, Cl, Br, I, pseudohalogen groups, alkoxy groups with 1 to 20 C atoms, preferably 1 to 4 C atoms, OH, NO₂ and/or NH₂. Alternatively or in addition, the substituents can comprise cycloalkyl groups with 3 to 20 C atoms, preferably 3 to 10 C atoms, that can be substituted with at least one of or two or more of F, Cl, Br, I, pseudohalogen groups, alkoxy groups with 1 to 20 C atoms, preferably 1 to 4 atoms, OH, NO₂ and/or NH₂. Alternatively or in addition, the substituents can comprise heterocycloalkyl groups with 1 to 15 C atoms, preferably 1 to 6 C atoms, that can be substituted with at least one of or two or more of F, Cl, Br, I, pseudohalogen groups, alkoxy groups with 1 to 20 C atoms, preferably 1 to 4 C atoms, OH, NO₂ and/or NH₂. Alternatively or in addition, the substituents can comprise aromatic and/or heteroaromatic groups with 2 to 40 C atoms, e.g. aromatic groups with 6 to 40 C atoms and/or heteroaromatic groups with 2 to 40 C atoms, preferably aromatic and/or heteroaromatic groups 2 to 6 C atoms, that can be substituted with at least one of or two or more of F, Cl, Br, I, pseudohalogen groups, alkoxy groups with 1 to 20 C atoms, preferably 1 to 4 C atoms, OH, NO₂ and/or NH₂. Alternatively or in addition, the substituents can comprise alkyl heteroaromatic groups with 3 to 50 C atoms, preferably 3 to 10 C atoms, that can be substituted with at least one of or two or more of F, Cl, Br, I, pseudohalogen groups, alkoxy groups with 1 to 20 C atoms, preferably 1 to 4 C atoms, OH, NO₂ and/or NH₂. Alternatively or in addition, the substituents can comprise heteroaromatic groups with at least one alkyl residue with 3 to 50 C atoms, preferably 3 to 10 C atoms, that can be substituted with at least one of or two or more of F, Cl, Br, I, pseudohalogen groups, alkoxy groups with 1 to 20 C atoms, preferably 1 to 4 C atoms, OH, NO₂ and/or NH₂. Alternatively or in addition, the substituents can comprise alkyl cycloalkyl groups with 4 to 24 C atoms, preferably 4 to 10 C atoms, that can be substituted with at least one of or two or more of F, Cl, Br, I, pseudohalogen groups, alkoxy groups with 1 to 20 C atoms, preferably 1 to 4 C atoms, OH, NO₂ and/or NH₂. Alternatively or in addition, the substituents can comprise cycloalkyl alkyl groups with 4 to 24 C atoms, preferably 4 to 10 C atoms, that can be substituted with at least one of or two or more of F, Cl, Br, I, pseudohalogen groups, alkoxy groups with 1 to 20 C atoms, preferably 1 to 4 C atoms, OH, NO₂ and/or NH₂. Alternatively or in addition, the substituents can comprise alkyl heterocycloalkyl groups with 2 to 24 C atoms, preferably 2 to 10 C atoms, that can optionally be substituted with at least one of F, Cl, Br, I, pseudohalogen groups, alkoxy groups with 1 to 20 C atoms, preferably 1 to 4 C atoms, OH, NO₂ and NH₂. Alternatively or in addition, the substituents can comprise heterocycloalkyl alkyl groups with 2 to 24 C atoms, preferably 2 to 10 C atoms, that can optionally be substituted with at least one of F, Cl, Br, I, pseudohalogen groups, alkoxy groups with 1 to 20 C atoms, preferably 1 to 4 C atoms, OH, NO₂ and NH₂ Alternatively or in addition, the substituents can comprise carboxylic acid ester groups with 1 to 20 C atoms, preferably 1 to 6 C atoms, that can be substituted with at least one of or two or more of F, Cl, Br, I, pseudohalogen groups, alkoxy groups with 1 to 20 C atoms, preferably 1 to 4 C atoms, OH, NO₂ and/or NH₂. Alternatively or in addition, the substituents can comprise alkyl and/or (hetero)aryl oxycarbonyl groups with 1 to 20 C atoms, preferably 1 to 10 C atoms, that can be substituted with at least one of or two or more of F, Cl, Br, I, pseudohalogen groups, alkoxy groups with 1 to 20 C atoms, preferably 1 to 4 C atoms, OH, NO₂ and/or NH₂. Alternatively or in addition, the substituents can comprise carboxamide groups with 1 to 20 C atoms, preferably 1 to 10 C atoms, that can be substituted with at least one of or two or more of F, Cl, Br, I, pseudohalogen groups, alkoxy groups with 1 to 20 C atoms, preferably 1 to 4 atoms, OH, NO₂ and/or NH₂.

In certain embodiments, two or more of the substituents of Y₁ and/or Y₂ can form a ring or more rings. According to some embodiments, at least one substituent can be part of a polymer. In the compound of Formula IIIa, n is an integer from 0 to 8, preferably from 1 to 5, further preferably from 1 to 3, even further preferably 2 or 3. R₁ₐ, R_{1b}, X₂, X₃, X₄, and X₅ are as defined above in the compound of Formula III.

According to certain embodiments, the catalyst, particularly the catalyst of Formula IIIa, can be represented by the following Formula IIIa1:

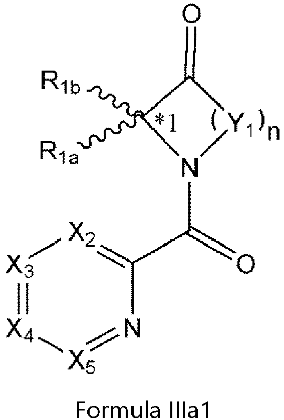

In the compound of Formula IIIa1, Y₁, R₁ₐ, R_{1b}, X₂, X₃, X₄, and X₅ are as defined in the compound of Formula IIIa. In the compound of Formula IIIa1, R₁ₐ and R_{1b} are different. The stereocenter *1 of the catalyst according to Formula IIIa1 is in (R) and/or (S) configuration. In the compound of Formula IIIa1, as well as any of the following formulas directly or indirectly derived therefrom, n can be an integer from 0 to 8, preferably from 1 to 5, further preferably from 1 to 3, even further preferably 2 or 3. According to preferred embodiments, Y₁ is a substituted or unsubstituted C.

According to certain embodiments, the catalyst, particularly the catalyst of Formula IIIa can be represented by the following Formula IIIa2:

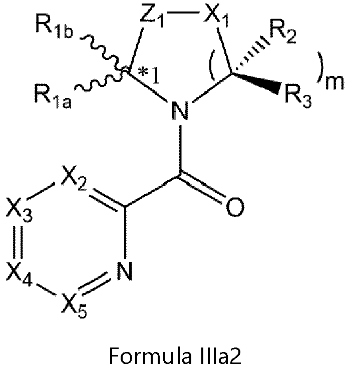

In the compound of Formula IIIa2, R₁ₐ, R_{1b}, X₂, X₃, X₄, and X₅ are as defined in the compound of Formula IIIa. In the compound of Formula IIIa, R₂ and R₃ can be the same or different.

In the compound of Formula IIIa2, as well as any of the following formulas directly or indirectly derived therefrom, according to certain embodiments, R₂ and/or R₃ can be H, F, Cl, Br, I, pseudohalogen groups, alkoxy groups with 1 to 20 C atoms, preferably 1 to 4 C atoms, OH, NO₂ and/or NH₂.

Alternatively or in addition, R₂ and/or R₃ can be linear alkyl groups with 1 to 10 C atoms that can be substituted with at least one of or two or more of F, Cl, Br, I, pseudohalogen groups, alkoxy groups with 1 to 20 C atoms, OH, NO₂ and/or NH₂. Preferably, the linear alkyl groups have 1 to 6 C atoms and can be substituted with can be substituted with at least one of or two or more of F, Cl, OH and/or alkoxy groups with 1 to 4 C atoms.

Alternatively or in addition, R₂ and/or R₃ can be branched alkyl groups with 3 to 10 C atoms that can be substituted with at least one of or two or more of F, Cl, Br, I, pseudohalogen groups, alkoxy groups with 1 to 20 C atoms, OH, NO₂ and/or NH₂. According to preferred embodiments, the branched alkyl groups have 3 to 6 C atoms and can be substituted with at least one of or two or more of F, Cl, OH and/or alkoxy groups with 1 to 4 C atoms. Alternatively or in addition, R₂ and/or R₃ can in some embodiments be aralkyl groups with 7 to 24 C atoms that can be substituted with at least one of or two or more of F, Cl, Br, I, pseudohalogen groups, alkoxy groups with 1 to 20 C atoms, OH, NO₂ and/or NH₂. Preferably, the aralkyl groups have 7 to 10 C atoms and can be can be substituted with at least one of or two or more of F, Cl, OH and/or alkoxy groups with 1 to 4 C atoms.

Alternatively or in addition, R₂ and/or R₃ can be alkylaryl groups with 7 to 24 C atoms that can be substituted with at least one of or two or more of F, Cl, Br, I, pseudohalogen groups, alkoxy groups with 1 to 20 C atoms, OH, NO₂ and/or NH₂. In preferred embodiments, the alkylaryl groups have 7 to 10 C atoms and can be substituted with at least one of or two or more of F, Cl, OH and/or alkoxy groups with 1 to 4 C atoms.

Alternatively or in addition, R₂ and/or R₃ can in some embodiments be cycloalkyl groups with 3 to 20 C atoms that can be substituted with at least one of or two or more of F, Cl, Br, I, pseudohalogen groups, alkoxy groups with 1 to 20 C atoms, OH, NO₂ and/or NH₂. Preferably, the cycloalkyl groups have 3 to 6 C atoms and can be substituted with at least one of or two or more of F, Cl, OH and/or alkoxy groups with 1 to 4 C atoms.

Alternatively or in addition, R₂ and/or R₃ can according to certain embodiments be heterocycloalkyl groups with 1 to 15 C atoms that can be substituted with at least one of or two or more of F, Cl, Br, I, pseudohalogen groups, alkoxy groups with 1 to 20 C atoms, OH, NO₂ and/or NH₂. It is preferred that the heterocycloalkyl groups have 1 to 6 C atoms and can be substituted with at least one of or two or more of F, Cl, OH and/or alkoxy groups with 1 to 4 C atoms.

In some embodiments, R₂ and/or R₃ can, alternatively or in addition, further be aromatic and/or heteroaromatic groups with 2 to 40 C atoms, e.g. aromatic groups with 6 to 40 C atoms and/or heteroaromatic groups with 2 to 40 C atoms, that can be substituted with at least one of or two or more of F, Cl, Br, I, pseudohalogen groups, alkoxy groups with 1 to 20 C atoms, OH, NO₂ and/or NH₂. According to preferred embodiments, the aromatic or heteroaromatic groups have 2 to 6 C atoms and can be substituted with at least one of or two or more of F, Cl, OH and/or alkoxy groups with 1 to 4 C atoms.

Alternatively or in addition, R₂ and/or R₃ can further be alkyl heteroaromatic groups with 3 to 50 C atoms that can be substituted with at least one of or two or more of F, Cl, Br, I, pseudohalogen groups, alkoxy groups with 1 to 20 C atoms, OH, NO₂ and/or NH₂. Preferably, the alkyl heteroaromatic groups have 3 to 10 C atoms and can be substituted with at least one of or two or more of F, Cl, OH and/or alkoxy groups with 1 to 4 C atoms.

Furthermore, alternatively or in addition, R₂ and/or R₃ can in some embodiments be heteroaromatic groups with at least one alkyl residue with 3 to 50 C atoms that can be substituted with at least one of or two or more of F, Cl, Br, I, pseudohalogen groups, alkoxy groups with 1 to 20 C atoms, OH, NO₂ and/or NH₂. It is preferred that the heteroaromatic groups with at least one alkyl residue have 3 to 10 C atoms and can be substituted at least one of or two or more of F, Cl, OH and/or alkoxy groups with 1 to 4 C atoms.

In certain embodiments, R₂ and/or R₃ can be, alternatively or in addition, alkyl cycloalkyl groups with 4 to 24 C atoms that can be substituted with at least one of or two or more of F, Cl, Br, I, pseudohalogen groups, alkoxy groups with 1 to 20 C atoms, OH, NO₂ and/or NH₂. Preferably, the alkyl cycloalkyl groups have 4 to 10 C atoms and can be substituted with at least one of or two or more of F, Cl, OH and/or alkoxy groups with 1 to 4 C atoms.

Alternatively or in addition, R₂ and/or R₃ can be cycloalkyl alkyl groups with 4 to 24 C atoms that can be substituted with at least one of or two or more of F, Cl, Br, I, pseudohalogen groups, alkoxy groups with 1 to 20 C atoms, OH, NO₂ and/or NH₂. In preferred embodiments, the cycloalkyl alkyl groups have 4 to 10 C atoms and can be substituted with at least one of or two or more of F, Cl, OH and/or alkoxy groups with 1 to 4 C atoms.

Alternatively or in addition, R₂ and/or R₃ can be alkyl heterocycloalkyl groups with 2 to 24 C atoms, preferably 4 to 10 C atoms, that can optionally be substituted with at least one of F, Cl, Br, I, pseudohalogen groups, alkoxy groups with 1 to 20 C atoms, OH, NO₂ and NH₂. Alternatively or in addition, R₂ and/or R₃ can be heterocycloalkyl alkyl groups with 2 to 24 C atoms, preferably 4 to 10 C atoms, that can optionally be substituted with at least one of F, Cl, Br, I, pseudohalogen groups, alkoxy groups with 1 to 20 C atoms, OH, NO₂ and NH₂ Alternatively or in addition, R₂ and/or R₃ can in some embodiments be carboxylic acid ester groups with 1 to 20 C atoms that can be substituted with at least one of or two or more of F, Cl, Br, I, pseudohalogen groups, alkoxy groups with 1 to 20 C atoms, OH, NO₂ and/or NH₂. According to preferred embodiments, the carboxylic acid ester groups has 1 to 8 C atoms and can be substituted with at least one of or two or more of F, Cl, OH and/or alkoxy groups with 1 to 4 C atoms.

Alternatively or in addition, R₂ and/or R₃ can be alkyl and/or (hetero)aryl oxycarbonyl groups with 1 to 20 C atoms that can be substituted with at least one of or two or more of F, Cl, Br, I, pseudohalogen groups, alkoxy groups with 1 to 20 C atoms, OH, NO₂ and/or NH₂. Preferably, the alkyl and/or (hetero)aryl oxycarbonyl groups have 1 to 8 C atoms and can be substituted with at least one of or two or more of F, Cl, OH and/or alkoxy groups with 1 to 4 C atoms. Furthermore, R₂ and/or R₃ can alternatively or in addition be carboxamide groups with 1 to 20 C atoms that can be substituted with at least one of or two or more of F, Cl, Br, I, pseudohalogen groups, alkoxy groups with 1 to 20 C atoms, OH, NO₂ and/or NH₂. It is preferred that the carboxamide groups have 1 to 8 C atoms and can be substituted with at least one of or two or more of F, Cl, OH and/or alkoxy groups with 1 to 4 C atoms.

According to certain embodiments, one or two or more of R₂ and R₃ can form a ring or more than one ring, e.g. a cyclohexyl ring.

In preferred embodiments, R₂ and/or R₃ are independently selected from H, methyl, ethyl, n-propyl, n-butyl, *tert-butyl, iso*-butyl, *iso*-propyl and/or cyclopropyl. According to preferred embodiments, at least one of R₂ and R₃ is H or methyl. According to further preferred embodiments, at least one of R₂ and R₃ is H or methyl, and the other of R₂ and R₃ is a linear or branched alkyl residue with 1 to 4 C atoms.

In the compound of Formula IIIa2, as well as any of the following formulas directly or indirectly derived therefrom, X₁ is selected from the group of O, NR₄, and CR₄ₐR_{4b}.

R₄ₐ, and R_{4b} can be the same or different. According to certain embodiments, R₄, R₄ₐ, and/or R_{4b} can be H, F, Cl, Br, I, pseudohalogen groups, alkoxy groups with 1 to 20 C atoms, preferably 1 to 4 C atoms, OH, SR₂₁, S(CO)R₂₂, NO₂, NH₂, (NR₁₈'R₁₉'R₂₀')⁺, and/or amines of formula NR₁₈R₁₉, wherein R₁₈, R₁₉, R₁₈', R₁₉', R₂₀', R₂₁, and R₂₂ are as defined above.

Alternatively or in addition, R₄, R₄ₐ, and/or R_{4b} can be linear alkyl groups with 1 to 10 C atoms that can be substituted with at least one of or two or more of F, Cl, Br, I, pseudohalogen groups, alkoxy groups with 1 to 20 C atoms, OH, NO₂ and/or NH₂. Preferably, the linear alkyl groups have 1 to 6 C atoms and can be substituted with at least one of or two or more of F, Cl, OH and/or alkoxy groups with 1 to 4 C atoms.

Alternatively or in addition, R₄, R₄ₐ, and/or R_{4b} can be branched alkyl groups with 3 to 10 C atoms that can be substituted with at least one of or two or more of F, Cl, Br, I, pseudohalogen groups, alkoxy groups with 1 to 20 C atoms, OH, NO₂ and/or NH₂. According to preferred embodiments, the branched alkyl groups have 3 to 6 C atoms and can be substituted with at least one of or two or more of F, Cl, OH and/or alkoxy groups with 1 to 4 C atoms.

Further, alternatively or in addition, R₄, R₄ₐ, and/or R_{4b} can in some embodiments be aralkyl groups with 7 to 24 C atoms that can be substituted with at least one of or two or more of F, Cl, Br, I, pseudohalogen groups, alkoxy groups with 1 to 20 C atoms, OH, NO₂ and/or NH₂. Preferably, the aralkyl groups have 7 to 10 C atoms and can be can be substituted with at least one of or two or more of F, Cl, OH and/or alkoxy groups with 1 to 4 C atoms.

Alternatively or in addition, R₄, R₄ₐ, and/or R_{4b} can be alkylaryl groups with 7 to 24 C atoms that can be substituted with at least one of or two or more of F, Cl, Br, I, pseudohalogen groups, alkoxy groups with 1 to 20 C atoms, OH, NO₂ and/or NH₂. In preferred embodiments, the alkylaryl groups have 7 to 10 C atoms and can be substituted with at least one of or two or more of F, Cl, OH and/or alkoxy groups with 1 to 4 C atoms.

Alternatively or in addition, R₄, R₄ₐ, and/or R_{4b} can in some embodiments be cycloalkyl groups with 3 to 20 C atoms that can be substituted with at least one of or two or more of F, Cl, Br, I, pseudohalogen groups, alkoxy groups with 1 to 20 C atoms, OH, NO₂ and/or NH₂. Preferably, the cycloalkyl groups have 3 to 6 C atoms and can be substituted with at least one of or two or more of F, Cl, OH and/or alkoxy groups with 1 to 4 C atoms.

Alternatively or in addition, R₄, R₄ₐ, and/or R_{4b} can according to certain embodiments be heterocycloalkyl groups with 1 to 15 C atoms that can be substituted with at least one of or two or more of F, Cl, Br, I, pseudohalogen groups, alkoxy groups with 1 to 20 C atoms, OH, NO₂ and/or NH₂. It is preferred that the heterocycloalkyl groups have 1 to 6 C atoms and can be substituted with at least one of or two or more of F, Cl, OH and/or alkoxy groups with 1 to 4 C atoms.

In some embodiments, R₄, R₄ₐ, and/or R_{4b} can, alternatively or in addition, further be aromatic and/or heteroaromatic groups with 2 to 40 C atoms, e.g. aromatic groups with 6 to 40 C atoms and/or heteroaromatic groups with 2 to 40 C atoms, that can be substituted with at least one of or two or more of F, Cl, Br, I, pseudohalogen groups, alkoxy groups with 1 to 20 C atoms, OH, NO₂ and/or NH₂. According to preferred embodiments, the aromatic or heteroaromatic groups have 2 to 6 C atoms and can be substituted with at least one of or two or more of F, Cl, OH and/or alkoxy groups with 1 to 4 C atoms.

Alternatively or in addition, R₄, R₄ₐ, and/or R_{4b} can further be alkyl heteroaromatic groups with 3 to 50 C atoms that can be substituted with at least one of or two or more of F, Cl, Br, I, pseudohalogen groups, alkoxy groups with 1 to 20 C atoms, OH, NO₂ and/or NH₂. Preferably, the alkyl heteroaromatic groups have 3 to 10 C atoms and can be substituted with at least one of or two or more of F, Cl, OH and/or alkoxy groups with 1 to 4 C atoms.

Furthermore, R₄, R₄ₐ, and/or R_{4b} can alternatively or in addition be heteroaromatic groups with at least one alkyl residue with 3 to 50 C atoms that can be substituted with at least one of or two or more of F, Cl, Br, I, pseudohalogen groups, alkoxy groups with 1 to 20 C atoms, OH, NO₂ and/or NH₂. It is preferred that the heteroaromatic groups with at least one alkyl residue have 3 to 10 C atoms and can be substituted with at least one of or two or more of F, Cl, OH and/or alkoxy groups with 1 to 4 C atoms.

In certain embodiments, R₄, R₄ₐ, and/or R_{4b} can alternatively or in addition be alkyl cycloalkyl groups with 4 to 24 C atoms that can be substituted with at least one of or two or more of F, Cl, Br, I, pseudohalogen groups, alkoxy groups with 1 to 20 C atoms, OH, NO₂ and/or NH₂. Preferably, the alkyl cycloalkyl groups have 4 to 10 C atoms and can be substituted with at least one of or two or more of F, Cl, OH and/or alkoxy groups with 1 to 4 C atoms.

Alternatively or in addition, R₄, R₄ₐ, and/or R4_{b} can be cycloalkyl alkyl groups with 4 to 24 C atoms that can be substituted with at least one of or two or more of F, Cl, Br, I, pseudohalogen groups, alkoxy groups with 1 to 20 C atoms, OH, NO₂ and/or NH₂. In preferred embodiments, the cycloalkyl alkyl groups have 4 to 10 C atoms and can be substituted with at least one of or two or more of F, Cl, OH and/or alkoxy groups with 1 to 4 C atoms.

Alternatively or in addition, R₄, R₄ₐ, and/or R_{4b} can be alkyl heterocycloalkyl groups with 2 to 24 C atoms, preferably 2 to 10 C atoms, that can optionally be substituted with at least one of F, Cl, Br, I, pseudohalogen groups, alkoxy groups with 1 to 20 C atoms, OH, NO₂ and NH₂. Alternatively or in addition, R₄, R₄ₐ, and/or R_{4b} can be heterocycloalkyl alkyl groups with 2 to 24 C atoms, preferably 2 to 10 C atoms, that can optionally be substituted with at least one of F, Cl, Br, I, pseudohalogen groups, alkoxy groups with 1 to 20 C atoms, OH, NO₂ and NH₂ Alternatively or in addition, R₄, R₄ₐ, and/or R_{4b} can in some embodiments be carboxylic acid ester groups with 1 to 20 C atoms that can be substituted with at least one of or two or more of F, Cl, Br, I, pseudohalogen groups, alkoxy groups with 1 to 20 C atoms, OH, NO₂ and/or NH₂. According to preferred embodiments, the carboxylic acid ester groups have 1 to 8 C atoms and can be substituted with at least one of or two or more of F, Cl, OH and/or alkoxy groups with 1 to 4 C atoms.

In certain embodiments, R₄, R₄ₐ, and/or R_{4b} can, alternatively or in addition, be alkyl and/or (hetero)aryl oxycarbonyl groups with 1 to 20 C atoms that can be substituted with at least one of or two or more of F, Cl, Br, I, pseudohalogen groups, alkoxy groups with 1 to 20 C atoms, OH, NO₂ and/or NH₂. Preferably, the alkyl and/or (hetero)aryl oxycarbonyl groups have 1 to 8 C atoms and can be substituted with at least one of or two or more of F, Cl, OH and/or alkoxy groups with 1 to 4 C atoms.

Furthermore, alternatively or in addition, R₄, R₄ₐ, and/or R_{4b} can according to some embodiments be carboxamide groups with 1 to 20 C atoms that can be substituted with at least one of or two or more of F, Cl, Br, I, pseudohalogen groups, alkoxy groups with 1 to 20 C atoms, OH, NO₂ and/or NH₂. It is preferred that the carboxamide groups have 1 to 8 C atoms and can be substituted with at least one of or two or more of F, Cl, OH and/or alkoxy groups with 1 to 4 C atoms.

According to certain embodiments, two or more of R₂, R₃, R₄, R₄ₐ, and R_{4b} can form a ring or more rings. In some embodiments, at least one of R₂, R₃, R₄, R₄ₐ, and R_{4b} is part of a polymer.

In the compound of Formula IIIa2, as well as any of the following formulas directly or indirectly derived therefrom, m can be, according to some embodiments an integer from 0 to 7, preferably from 0 to 4, further preferably 1 or 2, particularly preferably 1.

In the compound of Formula IIIa2, as well as any of the following formulas directly or indirectly derived therefrom, Z₁ is chosen from a carbonyl group, a thiocarbonyl group, and CR₂₀ₐR_{20b}. R₂₀ₐ and R_{20b} are the same or different and can be chosen from H, linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂.

In the compound of Formula IIIa2, R₁ₐ and R_{1b} are different. The stereocenter *1 of the catalyst according to Formula IIIa2 is in (R) and/or (S) configuration.

According to certain embodiments, the catalyst, particularly the catalyst of Formula IIIa or the catalyst of Formula IIIa2, can be represented by the following Formula IIIa3:

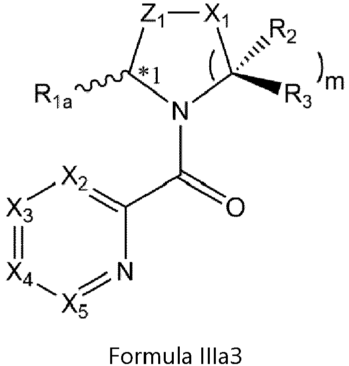

In the compound of Formula IIIa3, m, R₁ₐ, R₂, R₃, X₁, X₂, X₃, X₄, X₅, and Z₁ are as defined in the compound of Formula IIIa2.

The stereocenter *1 of the catalyst according to Formula IIIa3 is in (R) and/or (S) configuration.

According to certain embodiments, the catalyst, particularly the catalyst of Formula IIIa1 or the catalyst of Formula IIIa2, can be represented by the following Formula IIIa2-a:

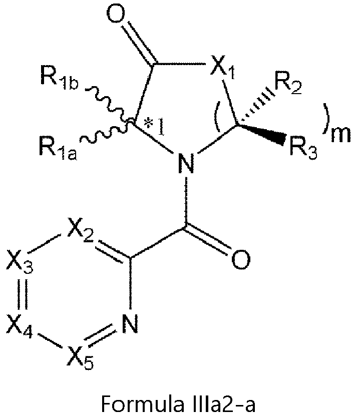

In the compound of Formula IIIa2-a, m, R₁ₐ, R_{1b}, R₂, R₃, X₁, X₂, X₃, X₄, and X₅, are as defined in the compound of Formula IIIa2. In the compound of Formula IIIa2-a, R₁ₐ and R_{1b} are different. The stereocenter *1 of the catalyst according to Formula IIIa2-a is in (R) and/or (S) configuration.

According to certain embodiments, the catalyst, particularly the catalyst of Formula IIIa2, can be represented by the following Formula IIIa2-b:

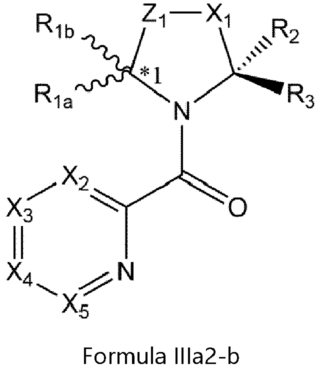

In the compound of Formula IIIa2-b, R₁ₐ, R_{1b}, R₂, R₃, X₁, X₂, X₃, X₄, X₅, and Z₁ are as defined in the compound of Formula IIIa2. In the compound of Formula IIIa2-b, R₁ₐ and R_{1b} are different. The stereocenter *1 of the catalyst according to Formula IIIa2-b is in (R) and/or (S) configuration.

According to certain embodiments, the catalyst, particularly the catalyst of Formula IIIa2, can be represented by the following Formula IIIa2-c:

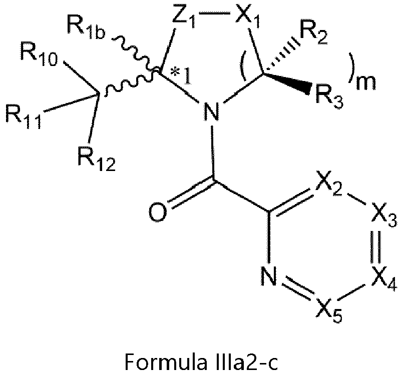

In the compound of Formula IIIa2-c, m, R_{1b}, R₂, R₃, X₁, X₂, X₃, X₄, X₅, and Z₁ are as defined in the compound of Formula IIIa2 In the compound of Formula IIIa2-c, R₁₀, R₁₁, and R₁₂ are as defined in the compound of Formula IIb1-a. The stereocenter *1 of the catalyst according to Formula IIIa2-c is in (R) and/or (S) configuration.

According to certain embodiments, the catalyst, particularly the catalyst of Formula IIIa2-c or the catalyst of Formula IIIa3, can be represented by the following Formula IIIa3-a:

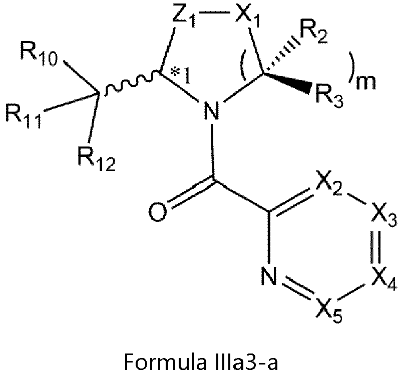

In the compound of Formula IIIa3-a, m, R₂, R₃, R₁₀, R₁₁, R₁₂, X₁, X₂, X₃, X₄, X₅, and Z₁ are as defined in the compound of Formula IIIa2-c. The stereocenter *1 of the catalyst according to Formula IIIa3-a is in (R) and/or (S) configuration.

According to certain embodiments, the catalyst, particularly the catalyst of Formula IIIa2-b or the catalyst of Formula IIIa3, can be represented by the following Formula IIIa3-b:

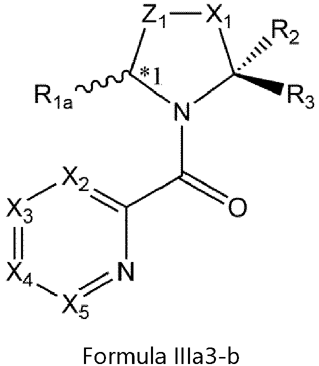

In the compound of Formula IIIa3-b, R₁ₐ, R₂, R₃, X₁, X₂, X₃, X₄, X₅, and Z₁ are as defined in the compound of Formula IIIa3. The stereocenter *1 of the catalyst according to Formula IIIa3-b is in (R) and/or (S) configuration.

According to certain embodiments, the catalyst, particularly the catalyst of Formula IIIa2-a, can be represented by the following Formula IIIa2-a1 or the following Formula IIIa2-a2, the catalyst of Formula IIIa2-a2 also being a particular representation of the catalyst of Formula IIIa2-a1:

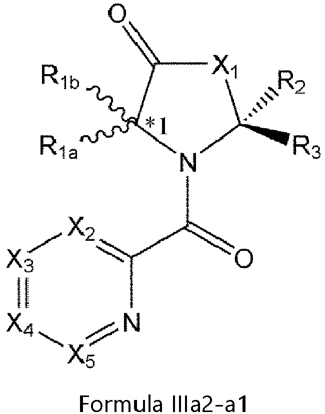

In the compound of Formula IIIa2-a1, R₁ₐ, R_{1b}, R₂, R₃,X₁,X₂,X₃,X₄ and X₅ are as defined in the compound of Formula IIIa2-a. In the compound of Formula IIIa2-a1, R₁ₐ and R_{1b} are different. The stereocenter *1 of the catalyst according to Formula IIIa2-a1 is in (R) and/or (S) configuration.

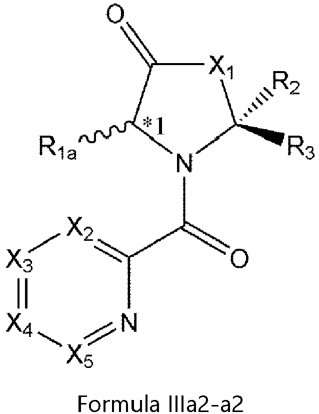

In the compound of Formula IIIa2-a2, R₁ₐ, R₂, R₃, X₁, X₂, X₃, X₄ and X₅ are as defined in the compound of Formula IIIa2-a. The stereocenter *1 of the catalyst according to Formula IIIa2-a2 is in (R) and/or (S) configuration.

According to certain embodiments, the catalyst, particularly the catalyst of Formula IIIa2-a1, can be represented by the following Formula IIIa2-a1a or the following Formula IIIa2-a1b, and the catalyst of Formula IIIa2-a2 can be represented by the following Formula IIIa2-a2a or the following Formula IIIa2-a2b, the catalyst of Formula IIIa2-a2b also being a particular representation of the catalyst of Formula IIIa2-a1a and the catalyst of Formula IIIa2-a2a also being a particular representation of the catalyst of Formula IIIa2-a1b:

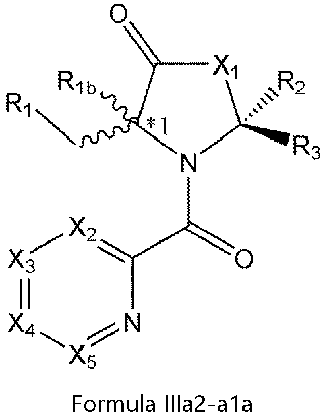

In the compound of Formula IIIa2-a1a, R_{1b}, R₂, R₃, X₁, X₂, X₃, X₄ and X₅ are as defined in the compound of Formula IIIa2-a1. The stereocenter *1 of the catalyst according to Formula IIIa2-a1a is in (R) and/or (S) configuration.

In the compound of Formula IIIa2-a1a, as well as any of the following formulas directly or indirectly derived therefrom, according to some embodiments, R₁ can be a linear alkyl group with 1 to 9 C atoms that can be substituted with at least one of or two or more of F, Cl, Br, I, pseudohalogen groups, alkoxy groups with 1 to 20 C atoms, OH, SH, NO₂ and/or NH₂. Preferably, the linear alkyl groups have 1 to 5 C atoms and can be substituted with at least one of or two or more of F, Cl, OH and/or alkoxy groups with 1 to 4 C atoms.

In certain embodiments, R₁ can be a branched alkyl group with 3 to 9 C atoms that can be substituted with at least one of or two or more of F, Cl, Br, I, pseudohalogen groups, alkoxy groups with 1 to 20 C atoms, OH, SH, NO₂ and/or NH₂. According to preferred embodiments, the branched alkyl group has 3 to 5 C atoms and can be substituted with at least one of or two or more of F, Cl, OH and/or alkoxy groups with 1 to 4 C atoms.

According to certain embodiments, R₁ is an aralkyl group with 7 to 23 C atoms that can be substituted with at least one of or two or more of F, Cl, Br, I, pseudohalogen groups, alkoxy groups with 1 to 20 C atoms, OH, SH, NO₂ and/or NH₂. According to preferred embodiments, the aralkyl group has 7 to 9 C atoms and can be substituted with at least one of or two or more of F, Cl, OH and/or alkoxy groups with 1 to 4 C atoms.

Furthermore, R₁ can in some embodiments be an alkylaryl group with 7 to 23 C atoms that may be substituted with at least one of or two or more of F, Cl, Br, I, pseudohalogen groups, alkoxy groups with 1 to 20 C atoms, OH, SH, NO₂ and/or NH₂. According to preferred embodiments, the alkyl aryl group has 7 to 9 C atoms and can be substituted with at least one of or two or more of F, Cl, OH and/or alkoxy groups with 1 to 4 C atoms.

In certain embodiments, R₁ can be a cycloalkyl group with 3 to 19 C atoms that can be substituted with at least one of or two or more of F, Cl, Br, I, pseudohalogen groups, alkoxy groups with 1 to 20 C atoms, OH, SH, NO₂ and/or NH₂. It is preferred that the cycloalkyl group has 3 to 6 C atoms and can be substituted with at least one of or two or more of F, Cl, OH and/or alkoxy groups with 1 to 4 C atoms.

R₁ can further be a heterocycloalkyl group with 1 to 14 C atoms that can be substituted with at least one of or two or more of F, Cl, Br, I, pseudohalogen groups, alkoxy groups with 1 to 20 C atoms, OH, SH, NO₂ and/or NH₂. In preferred embodiments, the heterocycloalkyl group has 1 to 6 C atoms and can be substituted with at least one of or two or more of F, Cl, OH and/or alkoxy groups with 1 to 4 C atoms.

In certain, preferred, embodiments, R₁ can be an aromatic group with 6 to 19 C atoms that can be substituted with at least one of or two or more of F, Cl, Br, I, pseudohalogen groups, OH, SH, NO₂ and/or NH₂. Preferably, the aromatic or heteroaromatic group has 2 to 6 C atoms and can be substituted with F, Cl, OH and/or alkoxy groups with 1 to 4 C atoms. Alternatively or in addition, the aromatic group can be substituted with at least one of or two or more of alkoxy groups with a linear, branched, cyclic and/or aromatic residue having 1 to 20 C atoms that can comprise one or more heteroatoms selected from O, N, S and P. Specifically, the residue of the alkoxy group can be a linear, branched and/or cyclic alkyl residue with 1 to 20 C atoms that can have one or more heteroatoms selected from O, N, S and P at any position and can be linked at any position, thus including ethers, amides, thioethers, etc., can also be an aromatic residue with 3 to 20 C atoms that can have one or more heteroatoms selected from O, N, S and P at any position and can be linked at any position, thus including heteroaromatic residues with furane, pyridine, pyrrole, indole, thiophene, etc. units, or can be a linear, branched and/or cyclic alkyl residue and/or an aromatic residue with 3 to 20 C atoms. Alternatively or in addition, the aromatic group can be substituted with at least one of or two or more of carboxylic ester groups with a linear, branched, cyclic and/or aromatic residue having 1 to 20 C atoms that can comprise one or more heteroatoms selected from O, N, S and P. Specifically, the residue of the carboxylic ester group can be a linear, branched and/or cyclic alkyl residue with 1 to 20 C atoms that can have one or more heteroatoms selected from O, N, S and P at any position and can be linked at any position, thus including ethers, amides, thioethers, etc., can also be an aromatic residue with 3 to 20 C atoms, that can have one or more heteroatoms selected from O, N, S and P at any position and can be linked at any position, thus including heteroaromatic residues with furane, pyridine, pyrrole, indole, thiophene, etc. units, or can be a linear, branched and/or cyclic alkyl residue and/or an aromatic residue with 3 to 20 C atoms. Alternatively or in addition, the aromatic group can be substituted with at least one of or two or more of alkyl and/or (hetero)aryl oxycarbonyl groups with a linear, branched, cyclic and/or aromatic residue having 1 to 20 C atoms that can comprise one or more heteroatoms selected from O, N, S and P. Specifically, the residue of the alkyl and/or (hetero)aryl oxycarbonyl group can be a linear, branched and/or cyclic alkyl residue with 1 to 20 C atoms that can have one or more heteroatoms selected from O, N, S and P at any position and can be linked at any position, thus including ethers, amides, thioethers, etc., can also be an aromatic residue with 3 to 20 C atoms, that can have one or more heteroatoms selected from O, N, S and P at any position and can be linked at any position, thus including heteroaromatic residues with furane, pyridine, pyrrole, indole, thiophene, etc. units, or can be a linear, branched and/or cyclic alkyl residue and/or an aromatic residue with 3 to 20 C atoms. Alternatively or in addition, the aromatic group can be substituted with at least one of or two or more of carboxamide groups with a linear, branched, cyclic and/or aromatic residue having 1 to 20 C atoms that can comprise one or more heteroatoms selected from O, N, S and P. Specifically, the residue of the carboxamide group can be a linear, branched and/or cyclic alkyl residue with 1 to 20 C atoms that can have one or more heteroatoms selected from O, N, S and P at any position and can be linked at any position, thus including ethers, amides, thioethers, etc., can also be an aromatic residue with 3 to 20 C atoms that can have one or more heteroatoms selected from O, N, S and P at any position and can be linked at any position, thus including heteroaromatic residues with furane, pyridine, pyrrole, indole, thiophene, etc. units, or can be a linear, branched and/or cyclic alkyl residue and/or an aromatic residue with 3 to 20 C atoms. Preferably, R₁ is selected from aromatic groups with 6 to 39 C atoms, and further preferably is a phenyl or naphtyl group, that may be substituted with at least one of or two or more of F, Cl, Br, I, pseudohalogen groups, alkoxy groups as defined above, particularly alkoxy groups with 1 to 20 C atoms, carboxylic ester groups as defined above, particularly carboxylic ester groups with 1 to 20 C atoms, alkyl and/or (hetero)aryl oxycarbonyl groups as defined above, particularly oxycarbonyl groups with 1 to 20 C atoms, carboxamide groups as defined above, particularly carboxamide groups with 1 to 20 C atoms, OH, NO₂ and/or NH₂. According to preferred embodiments, R₁ is a phenyl group which may be substituted with a hydroxyl group, an alkoxy group as defined above or a carboxylic ester group as defined above. Preferably, the substituent is in para position.

In certain embodiments, R₁ can be a heteroaromatic group with 2 to 39 C atoms that can be substituted with at least one of or two or more of F, Cl, Br, I, pseudohalogen groups, alkoxy groups with 1 to 20 C atoms, OH, SH, NO₂ and/or NH₂. Preferably, the heteroaromatic group has 2 to 6 C atoms and can be substituted with at least one of or two or more of F, Cl, OH and/or alkoxy groups with 1 to 4 C atoms.

Further, R₁ can be an alkyl heteroaromatic group with 3 to 49 C atoms that can be substituted with at least one of or two or more of F, Cl, Br, I, pseudohalogen groups, alkoxy groups with 1 to 20 C atoms, OH, SH, NO₂ and/or NH₂. In preferred embodiments, the alkyl heteroaromatic group has 3 to 10 C atoms and can be substituted with at least one of or two or more of F, Cl, OH and/or alkoxy groups with 1 to 4 C atoms.

R₁ can in some embodiments be a heteroaromatic group with at least one alkyl and/or aromatic residue with 3 to 49 C atoms that can be substituted with at least one of or two or more of F, Cl, Br, I, pseudohalogen groups, alkoxy groups with 1 to 20 C atoms, OH, SH, NO₂ and/or NH₂. Preferably, the heteroaromatic group with at least one alkyl residue has 3 to 10 C atoms and can be substituted with at least one of or two or more of F, Cl, OH and/or alkoxy groups with 1 to 4 C atoms.

According to certain embodiments, R₁ can be an alkyl cycloalkyl group with 4 to 23 C atoms that can be substituted with at least one of or two or more of F, Cl, Br, I, pseudohalogen groups, alkoxy groups with 1 to 20 C atoms, OH, SH, NO₂ and/or NH₂. It is preferred that the alkyl cycloalkyl group has 4 to 10 C atoms and can be substituted with at least one of or two or more of F, Cl, OH and/or alkoxy groups with 1 to 4 C atoms.

Furthermore, R₁ can in certain embodiments be a cycloalkyl alkyl group with 4 to 23 C atoms that can be substituted with at least one of or two or more of F, Cl, Br, I, pseudohalogen groups, alkoxy groups with 1 to 20 C atoms, OH, SH, NO₂ and/or NH₂. Preferably, the cycloalkyl alkyl group has 4 to 10 C atoms and can be substituted with at least one of or two or more of F, Cl, OH and/or alkoxy groups with 1 to 4 C atoms.

In some embodiments, R₁ can be a carboxylic acid ester group with 1 to 19 C atoms that can be substituted with at least one of or two or more of F, Cl, Br, I, pseudohalogen groups, alkoxy groups with 1 to 20 C atoms, OH, SH, NO₂ and/or NH₂. According to preferred embodiments, the carboxylic acid ester group has 1 to 8 C atoms and can be substituted with at least one of or two or more of F, Cl, OH and/or alkoxy groups with 1 to 4 C atoms.

According to certain embodiments, R₁ can be an alkyl and/or (hetero)aryl oxycarbonyl group with 1 to 19 C atoms that can be substituted with at least one of or two or more of F, Cl, Br, I, pseudohalogen groups, alkoxy groups with 1 to 20 C atoms, OH, SH, NO₂ and/or NH₂. Preferably, the alkyl and/or (hetero)aryl oxycarbonyl group has 1 to 8 C atoms and can be substituted with at least one of or two or more of F, Cl, OH and/or alkoxy groups with 1 to 4 C atoms.

Further, R₁ can in some embodiments be a carboxamide group with 1 to 19 C atoms (a carboxamide group with 1 C atom being (CO)NH₂) that can be substituted with at least one of or two or more of F, Cl, Br, I, pseudohalogen groups, alkoxy groups with 1 to 20 C atoms, OH, SH, NO₂ and/or NH₂. It is preferred that the carboxamide group has 1 to 8 C atoms and can be substituted with at least one of or two or more of F, Cl, OH and/or alkoxy groups with 1 to 4 C atoms. According to certain embodiments, R₁ is selected from aromatic groups with 6 to 39 C atoms, particularly phenyl groups and naphtyl groups, that can optionally be substituted with at least one of F, Cl, Br, I, pseudohalogen groups, alkoxy groups with 1 to 20 C atoms, carboxylic ester groups with 1 to 20 C atoms, alkyl and/or (hetero)aryl oxycarbonyl groups with 1 to 20 C atoms, carboxamide groups with 1 to 20 C atoms, OH, NO₂ and NH₂.

According to certain embodiments, in the catalyst of Formula IIla2-a1a, and any of the following formulas directly or indirectly derived therefrom, R_{1b} is H or methyl, preferably H.

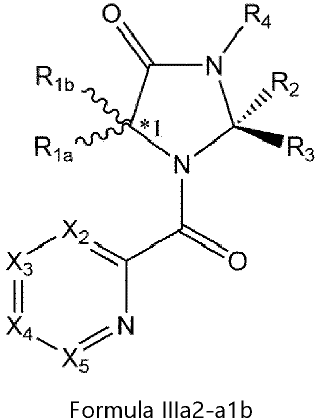

In the compound of Formula IIla2-a1b, R₁ₐ, R_{1b}, R₂, R₃, X₂, X₃, X₄ and X₅ are as defined in the compound of Formula IIIa2-a1. In the compound of Formula IIIa2-a1b, R₁ₐ and R_{1b} are different. The stereocenter *1 of the catalyst according to Formula IIIa2-a1b is in (R) and/or (S) configuration.

In the compound of Formula IIIa2-a1b, as well as any of the following formulas directly or indirectly derived therefrom, R₄ can be H, F, Cl, Br, I, a pseudohalogen group, an alkoxy group with 1 to 20 C atoms, preferably 1 to 4 C atoms, OH, NO₂, NH₂, or an amine of formula NR₁₈R₁₉, with R₁₈ and R₁₉ being as defined above.

According to certain, preferred, embodiments, R₄ alternatively can be a linear alkyl group with 1 to 10 C atoms that can be substituted with at least one of or two or more of F, Cl, Br, I, pseudohalogen groups, alkoxy groups with 1 to 20 C atoms, OH, NO₂ and/or NH₂. Preferably, the linear alkyl group has 1 to 6 C atoms and can be substituted with at least one of or two or more of F, Cl, OH and/or alkoxy groups with 1 to 4 C atoms. Particularly preferably R₄ is an unsubstituted linear alkyl group with 1 to 6 C atoms, particularly 1 to 4 C atoms.

In certain embodiments, alternatively R₄ can be a branched alkyl group with 3 to 10 C atoms that can be substituted with at least one of or two or more of F, Cl, Br, I, pseudohalogen groups, alkoxy groups with 1 to 20 C atoms, OH, NO₂ and/or NH₂. According to preferred embodiments, the branched alkyl group has 3 to 6 C atoms and can be substituted with at least one of or two or more of F, Cl, OH and/or alkoxy groups with 1 to 4 C atoms.

Further alternatively, R₄, can in some embodiments be an aralkyl group with 7 to 24 C atoms that can be substituted with at least one of or two or more of F, Cl, Br, I, pseudohalogen groups, alkoxy groups with 1 to 20 C atoms, OH, NO₂ and/or NH₂. Preferably, the aralkyl group has 7 to 10 C atoms and can be can be substituted with at least one of or two or more of F, Cl, OH and/or alkoxy groups with 1 to 4 C atoms.

According to certain embodiments, R₄ alternatively can be an alkylaryl group with 7 to 24 C atoms that can be substituted with at least one of or two or more of F, Cl, Br, I, pseudohalogen groups, alkoxy groups with 1 to 20 C atoms, OH, NO₂ and/or NH₂. In preferred embodiments, the alkylaryl group has 7 to 10 C atoms and can be substituted with at least one of or two or more of F, Cl, OH and/or alkoxy groups with 1 to 4 C atoms.

R₄ alternatively can in some embodiments be a cycloalkyl group with 3 to 20 C atoms that can be substituted with at least one of or two or more of F, Cl, Br, I, pseudohalogen groups, alkoxy groups with 1 to 20 C atoms, OH, NO₂ and/or NH₂. Preferably, the cycloalkyl group has 3 to 6 C atoms and can be substituted with at least one of or two or more of F, Cl, OH and/or alkoxy groups with 1 to 4 C atoms.

R₄ can according to certain embodiments alternatively be a heterocycloalkyl group with 1 to 15 C atoms that can be substituted with at least one of or two or more of F, Cl, Br, I, pseudohalogen groups, alkoxy groups with 1 to 20 C atoms, OH, NO₂ and/or NH₂. It is preferred that the heterocycloalkyl group has 1 to 6 C atoms and can be substituted with at least one of or two or more of F, Cl, OH and/or alkoxy groups with 1 to 4 C atoms.

In some embodiments, R₄ can further alternatively be an aromatic and/or heteroaromatic group with 2 to 40 C atoms, e.g. an aromatic group with 6 to 40 C atoms and/or a heteroaromatic group with 2 to 40 C atoms, that can be substituted with at least one of or two or more of F, Cl, Br, I, pseudohalogen groups, alkoxy groups with 1 to 20 C atoms, OH, NO₂ and/or NH₂. According to preferred embodiments, the aromatic or heteroaromatic group has 2 to 6 C atoms and can be substituted with at least one of or two or more of F, Cl, OH and/or alkoxy groups with 1 to 4 C atoms.

According to certain embodiments, R₄ can further alternatively be an alkyl heteroaromatic group with 3 to 50 C atoms that can be substituted with at least one of or two or more of F, Cl, Br, I, pseudohalogen groups, alkoxy groups with 1 to 20 C atoms, OH, NO₂ and/or NH₂. Preferably, the alkyl heteroaromatic group has 3 to 10 C atoms and can be substituted with at least one of or two or more of F, Cl, OH and/or alkoxy groups with 1 to 4 C atoms. Furthermore, R₄ can in some embodiments alternatively be a heteroaromatic group with at least one alkyl residue with 3 to 50 C atoms that can be substituted with at least one of or two or more of F, Cl, Br, I, pseudohalogen groups, alkoxy groups with 1 to 20 C atoms, OH, NO₂ and/or NH₂. It is preferred that the heteroaromatic group with at least one alkyl residue has 3 to 10 C atoms and can be substituted at least one of or two or more of F, Cl, OH and/or alkoxy groups with 1 to 4 C atoms.

In certain embodiments, R₄ can alternatively be an alkyl cycloalkyl group with 4 to 24 C atoms that can be substituted with at least one of or two or more of F, Cl, Br, I, pseudohalogen groups, alkoxy groups with 1 to 20 C atoms, OH, NO₂ and/or NH₂. Preferably, the alkyl cycloalkyl group has 4 to 10 C atoms and can be substituted with at least one of or two or more of F, Cl, OH and/or alkoxy groups with 1 to 4 C atoms.

According to some embodiments, R₄ alternatively can be a cycloalkyl alkyl group with 4 to 24 C atoms that can be substituted with at least one of or two or more of F, Cl, Br, I, pseudohalogen groups, alkoxy groups with 1 to 20 C atoms, OH, NO₂ and/or NH₂. In preferred embodiments, the cycloalkyl alkyl group has 4 to 10 C atoms and can be substituted with at least one of or two or more of F, Cl, OH and/or alkoxy groups with 1 to 4 C atoms.

In certain embodiments, R₄ can alternatively be an alkyl heterocycloalkyl group with 2 to 24 C atoms, preferably 4 to 10 C atoms, that can optionally be substituted with at least one of F, Cl, Br, I, pseudohalogen groups, alkoxy groups with 1 to 20 C atoms, OH, NO₂ and NH₂.

In certain embodiments, R₄ can alternatively be a heterocycloalkyl alkyl group with 2 to 24 C atoms, preferably 4 to 10 C atoms, that can optionally be substituted with at least one of F, Cl, Br, I, pseudohalogen groups, alkoxy groups with 1 to 20 C atoms, OH, NO₂ and NH₂.

R₄ can alternatively in some embodiments be a carboxylic acid ester group with 1 to 20 C atoms that can be substituted with at least one of or two or more of F, Cl, Br, I, pseudohalogen groups, alkoxy groups with 1 to 20 C atoms, OH, NO₂ and/or NH₂. According to preferred embodiments, the carboxylic acid ester group has 1 to 8 C atoms and can be substituted with at least one of or two or more of F, Cl, OH and/or alkoxy groups with 1 to 4 C atoms.

In certain embodiments, R₄ can alternatively be an alkyl and/or (hetero)aryl oxycarbonyl group with 1 to 20 C atoms that can be substituted with at least one of or two or more of F, Cl, Br, I, pseudohalogen groups, alkoxy groups with 1 to 20 C atoms, OH, NO₂ and/or NH₂. Preferably, the alkyl and/or (hetero)aryl oxycarbonyl group has 1 to 8 C atoms and can be substituted with at least one of or two or more of F, Cl, OH and/or alkoxy groups with 1 to 4 C atoms. Furthermore, R₄ can according to some embodiments alternatively be a carboxamide group with 1 to 20 C atoms that can be substituted with at least one of or two or more of F, Cl, Br, I, pseudohalogen groups, alkoxy groups with 1 to 20 C atoms, OH, NO₂ and/or NH₂. It is preferred that the carboxamide group has 1 to 8 C atoms and can be substituted with at least one of or two or more of F, Cl, OH and/or alkoxy groups with 1 to 4 C atoms.

According to certain embodiments, R₄ can be part of a polymer.

In preferred embodiments, R₄ is selected from H, linear alkyl groups with 1 to 4 C atoms and branched alkyl groups with 3 to 6 C atoms. According to further preferred embodiments, R₄ is methyl, ethyl, propyl, or butyl, particularly methyl or iso-butyl.

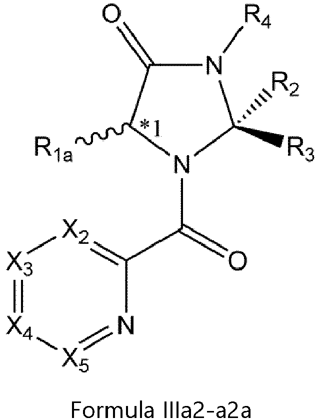

In the compound of Formula IIIa2-a2a, R₁ₐ, R₂, R₃, R₄, X₂, X₃, X₄ and X₅ are as defined in the compound of Formula IIIa2-a1b. The stereocenter *1 of the catalyst according to Formula IIIa2-a2a is in (R) and/or (S) configuration.

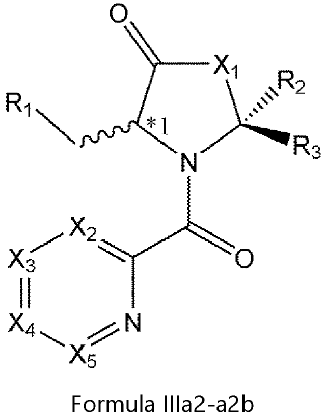

In the compounds of Formula IIIa2-a2b, R₁, R₂, R₃, X₁, X₂, X₃, X₄ and X₅ are as defined in the compound of Formula IIIa2-a1a. The stereocenter *1 of the catalyst according to Formula IIIa2-a2b is in (R) and/or (S) configuration.

In certain embodiments in the compound of Formula IIIa2-a1a or the compound of Formula IIIa2-a2b, as well as any compounds derived therefrom, R₁ can be a group according to the following Formula III-R1:

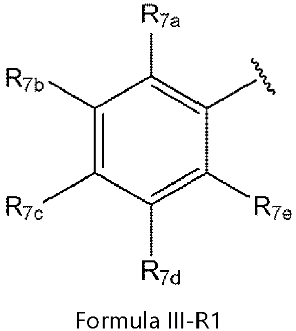

In Formula III-R1, R₇ₐ, R_{7b}, R_{7c}, R_{7d}, R₇ₑ can be the same or different. According to certain embodiments, R₇ₐ, R_{7b}, R_{7c}, R_{7d} and/or R₇ₑ can be H, F, Cl, Br, I, pseudohalogen groups, OH, NO₂ and/or NH₂. Alternatively or in addition,, R₇ₐ, R_{7b}, R_{7c}, R_{7d} and/or R₇ₑ can be alkoxy groups with a linear, branched, cyclic and/or aromatic residue having 1 to 20 C atoms that can comprise one or more heteroatoms selected from O, N, S and P. Specifically, the residue of the alkoxy group can be a linear, branched and/or cyclic alkyl residue with 1 to 20 C atoms that can have one or more heteroatoms selected from O, N, S and P at any position and can be linked at any position, which includes ethers, amides, thioethers, etc. The residue can also be an aromatic or heteroaromatic residue with 3 to 20 C atoms that can have one or more heteroatoms selected from O, N, S and P at any position and can be linked at any position, which includes heteroaromatic residues with furane, pyridine, pyrrole, indole, thiophene, etc. units The residue can further be a linear, branched and/or cyclic alkyl residue and/or an aromatic residue with 3 to 20 C atoms.

Alternatively or in addition, R₇ₐ, R_{7b}, R_{7c}, R_{7d} and/or R₇ₑ can be carboxylic ester groups with a linear, branched, cyclic and/or aromatic residue having 1 to 20 C atoms that can comprise one or more heteroatoms selected from O, N, S and P. Specifically, the residue of the carboxylic ester group can be a linear, branched or cyclic alkyl residue with 1 to 20 C atoms that can have one or more heteroatoms selected from O, N, S and P at any position and can be linked at any position, which includes ethers, amides, thioethers, etc. The residue can also be an aromatic residue with 3 to 20 C atoms that can have one or more heteroatoms selected from O, N, S and P at any position and can be linked at any position, which includes heteroaromatic residues with furane, pyridine, pyrrole, indole, thiophene, etc. units. The residue can further be a linear, branched and/or cyclic alkyl residue and/or an aromatic residue with 3 to 20 C atoms

In preferred embodiments, R₇ₐ, R_{7b}, R_{7d} and R₇ₑ are H. According to further preferred embodiments, R_{7c} is an alkoxy group with a linear, branched, cyclic and/or aromatic residue having 1 to 20 C atoms that can comprise one or more heteroatoms selected from O, N, S and P, preferably wherein the residue of the alkoxy group is a linear alkyl residue with 1 to 4 C atoms, e.g. a methyl group, an ethyl group, a n-propyl group, an isopropyl group; a benzyl group and/or a group comprising a heterocycle, e.g. a 1-benzyl-1H-1,2,3-triazol-4-methylene group. According to other preferred embodiments, R_{7c} is a carboxylic ester group with a linear, branched, cyclic and/or aromatic residue having 1 to 20 C atoms that can comprise one or more heteroatoms selected from O, N, S and P, preferably wherein the residue of the carboxylic ester group is a linear alkyl residue with 1 to 4 C atoms, a benzyl group and/or a group comprising a heterocycle, particularly a pyridyl group. In further preferred embodiments, R_{7c} is OH.

According to certain embodiments, the catalyst, particularly the catalyst of Formula IIIa2-a1a or the catalyst of Formula IIIa2-a1b, can be represented by the following Formula IIIa2-a1a1:

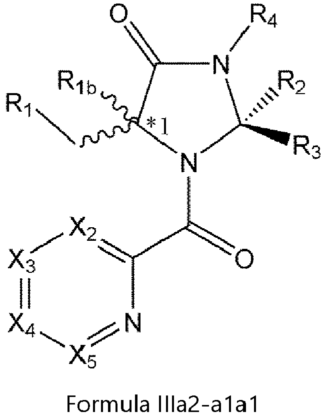

In the compound of Formula IIIa1a1, R₁, R_{1b}, R₂, R₃, X₂, X₃, X₄ and X₅ are as defined in the compound of Formula IIIa2-a1a, and R₄ is as defined in the compound of Formula IIIa2-a1b. R₁ can particularly have the Formula III-R1. The stereocenter *1 of the catalyst according to Formula IIIa2-a1a1 is in (R) and/or (S) configuration.

According to certain embodiments, the catalyst, particularly the catalyst of Formula IIIa2-a2a or the catalyst of Formula IIIa2-a2b or the catalyst of Formula IIIa2-a1a1, can be represented by the following Formula IIIa2-a2a1:

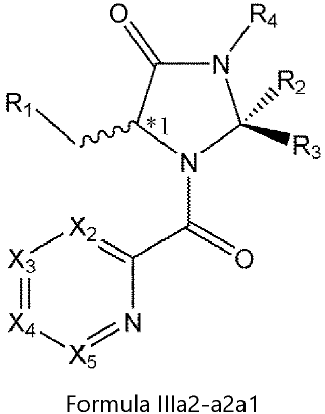

In the compound of Formula IIIa2-a2a1, R₁, R₂, R₃, R₄, X₂, X₃, X₄ and X₅ are as defined in the compound of Formula IIIa2-a1a1. R₁ can particularly have the Formula III-R1. The stereocenter *1 of the catalyst according to Formula IIIa2-a2a1 is in (R) and/or (S) configuration.

According to certain embodiments, the catalyst, particularly the catalyst of Formula IIIa2-a1a1, can be represented by the following Formula IIIa2-a1a2:

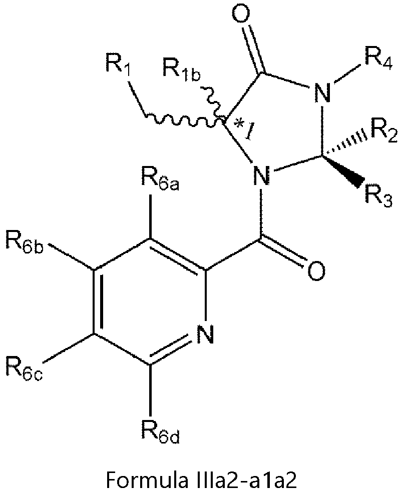

In the compound of Formula IIIa2-a1a2, R₁, R_{1b}, R₂, R₃, and R₄, are as defined in the compound of Formula IIIa2-a1a1. R₁ can particularly have the Formula III-R1. The stereocenter *1 of the catalyst according to Formula IIIa2-a1a2 is in (R) and/or (S) configuration.

In the compound of Formula IIa2-a1a2, as well as any of the following formulas directly or indirectly derived therefrom, R₆ₐ, R_{6b}, R_{6c}, R_{6d} can be the same or different. According to certain embodiments, R₆ₐ, R_{6b}, R_{6c} and/or R_{6d} can be H, F, Cl, Br, I, pseudohalogen groups, OH, NO₂ and/or NH₂. Alternatively or in addition, R₆ₐ, R_{6b}, R_{6c} and/or R_{6d} can in some embodiments be alkoxy groups with 1 to 20 C atoms, preferably with 1 to 4 C atoms.

According to certain embodiments, R₆ₐ, R_{6b}, R_{6c} and/or R_{6d} can be, alternatively or in addition, linear alkyl groups with 1 to 10 C atoms that can be substituted with at least one of or two or more of F, Cl, Br, I, pseudohalogen groups, alkoxy groups with 1 to 20 C atoms, OH, NO₂ and/or NH₂. Preferably, the linear alkyl groups have 1 to 6 C atoms and can be substituted with at least one of or two or more of F, Cl, OH and/or alkoxy groups with 1 to 4 C atoms.

Further, R₆ₐ, R_{6b}, R_{6c} and/or R_{6d} can, alternatively or in addition, in some embodiments be branched alkyl groups with 3 to 10 C atoms that can be substituted with at least one of or two or more of F, Cl, Br, I, pseudohalogen groups, alkoxy groups with 1 to 20 C atoms, OH, NO₂ and/or NH₂. In preferred embodiments, the branched alkyl groups have 3 to 6 C atoms and can be substituted with at least one of or two or more of F, Cl, OH and/or alkoxy groups with 1 to 4 C atoms.

Alternatively or in addition, R₆ₐ, R_{6b}, R_{6c} and/or R_{6d} can be aralkyl groups with 7 to 24 C atoms that can be substituted with at least one of or two or more of F, Cl, Br, I, pseudohalogen groups, alkoxy groups with 1 to 20 C atoms, OH, NO₂ and/or NH₂. In preferred embodiments, the aralkyl groups have 7 to 10 C atoms and can be substituted with at least one of or two or more of F, Cl, OH and/or alkoxy groups with 1 to 4 C atoms.

Alternatively or in addition, R₆ₐ, R_{6b}, R_{6c} and/or R_{6d} can according to certain embodiments be alkylaryl groups with 7 to 24 C atoms that can be substituted with at least one of or two or more of F, Cl, Br, I, pseudohalogen groups, alkoxy groups with 1 to 20 C atoms, OH, NO₂ and/or NH₂. According to preferred embodiments, the alkyl aryl groups have 7 to 10 C atoms and can be substituted with at least one of or two or more of F, Cl, OH and/or alkoxy groups with 1 to 4 C atoms.

In some embodiments, R₆ₐ, R_{6b}, R_{6c} and/or R_{6d} can be, alternatively or in addition, cycloalkyl groups with 3 to 20 C atoms that can be substituted with at least one of or two or more of F, Cl, Br, I, pseudohalogen groups, alkoxy groups with 1 to 20 C atoms, OH, NO₂ and/or NH₂. It is preferred that the cycloalkyl groups have 3 to 6 C atoms and can be substituted with at least one of or two or more of F, Cl, OH and/or alkoxy groups with 1 to 4 C atoms.

Alternatively or in addition, R₆ₐ, R_{6b}, R_{6c} and/or R_{6d} can be heterocycloalkyl groups with 1 to 15 C atoms that can be substituted with at least one of or two or more of F, Cl, Br, I, pseudohalogen groups, alkoxy groups with 1 to 20 C atoms, OH, NO₂ and/or NH₂. In preferred embodiments, the heterocycloalkyl groups have 1 to 6 C atoms and can be substituted with at least one of or two or more of F, Cl, OH and/or alkoxy groups with 1 to 4 C atoms.

Furthermore, R₆ₐ, R_{6b}, R_{6c} and/or R_{6d} can in some embodiments be, alternatively or in addition, aromatic and/or heteroaromatic groups with 2 to 40 C atoms, e.g. aromatic groups with 6 to 40 C atoms and/or heteroaromatic groups with 2 to 40 C atoms, that can be substituted with at least one of or two or more of F, Cl, Br, I, pseudohalogen groups, alkoxy groups with 1 to 20 C atoms, OH, NO₂ and/or NH₂. Preferably, the aromatic or heteroaromatic groups have 2 to 6 C atoms and can be substituted with at least one of or two or more of F, Cl, OH and/or alkoxy groups with 1 to 4 C atoms.

In some embodiments, R₆ₐ, R_{6b}, R_{6c} and/or R_{6d} can be, alternatively or in addition, alkyl heteroaromatic groups with 3 to 50 C atoms that can be substituted with at least one of or two or more of F, Cl, Br, I, pseudohalogen groups, alkoxy groups with 1 to 20 C atoms, OH, NO₂ and/or NH₂. In preferred embodiments, the alkyl heteroaromatic groups have 3 to 10 C atoms and can be substituted with at least one of or two or more of F, Cl, OH and/or alkoxy groups with 1 to 4 C atoms.

Alternatively or in addition, R₆ₐ, R_{6b}, R_{6c} and/or R_{6d} can be heteroaromatic groups with at least one alkyl residue with 3 to 50 C atoms that can be substituted with at least one of or two or more of F, Cl, Br, I, pseudohalogen groups, alkoxy groups with 1 to 20 C atoms, OH, NO₂ and/or NH₂. Preferably, the heteroaromatic groups with at least one alkyl residue have 3 to 10 C atoms and can be substituted with at least one of or two or more of F, Cl, OH and/or alkoxy groups with 1 to 4 C atoms.

R₆ₐ, R_{6b}, R_{6c} and/or R_{6d} can in some embodiments be, alternatively or in addition, alkyl cycloalkyl groups with 4 to 24 C atoms that can be substituted with at least one of or two or more of F, Cl, Br, I, pseudohalogen groups, alkoxy groups with 1 to 20 C atoms, OH, NO₂ and/or NH₂. It is preferred that the alkyl cycloalkyl groups have 4 to 10 C atoms and can be substituted with at least one of or two or more of F, Cl, OH and/or alkoxy groups with 1 to 4 C atoms. Alternatively or in addition, R₆ₐ, R_{6b}, R_{6c} and/or R_{6d} can be cycloalkyl alkyl groups with 4 to 24 C atoms that can be substituted with at least one of or two or more of F, Cl, Br, I, pseudohalogen groups, alkoxy groups with 1 to 20 C atoms, OH, NO₂ and/or NH₂. Preferably, the cycloalkyl alkyl groups have 4 to 10 C atoms and can be substituted with at least one of or two or more of F, Cl, OH and/or alkoxy groups with 1 to 4 C atoms.

Alternatively or in addition, R₆ₐ, R_{6b}, R_{6c} and/or R_{6d} can be alkyl heterocycloalkyl groups with 2 to 24 C atoms, preferably 2 to 10 C atoms, that can optionally be substituted with at least one of F, Cl, Br, I, pseudohalogen groups, alkoxy groups with 1 to 20 C atoms, OH, NO₂ and NH₂. Alternatively or in addition, R₆ₐ, R_{6b}, R_{6c} and/or R_{6d} can be heterocycloalkyl alkyl groups with 2 to 24 C atoms, preferably 2 to 10 C atoms, that can optionally be substituted with at least one of F, Cl, Br, I, pseudohalogen groups, alkoxy groups with 1 to 20 C atoms, OH, NO₂ and NH₂ Further, R₆ₐ, R_{6b}, R_{6c} and/or R_{6d} can, alternatively or in addition, according to certain embodiments be carboxylic acid ester groups with 1 to 20 C atoms that can be substituted with at least one of or two or more of F, Cl, Br, I, pseudohalogen groups, alkoxy groups with 1 to 20 C atoms, OH, NO₂ and/or NH₂. According to preferred embodiments, the carboxylic acid ester groups have 1 to 8 C atoms and can be substituted with at least one of or two or more of F, Cl, OH and/or alkoxy groups with 1 to 4 C atoms.

Also, R₆ₐ, R_{6b}, R_{6c} and/or R_{6d} can, alternatively or in addition, in some embodiments be alkyl and/or (hetero)aryl oxycarbonyl groups with 1 to 20 C atoms that can be substituted with at least one of or two or more of F, Cl, Br, I, pseudohalogen groups, alkoxy groups with 1 to 20 C atoms, OH, NO₂ and/or NH₂. Preferably, the alkyl and/or (hetero)aryl oxycarbonyl groups have 1 to 8 C atoms and can be substituted with at least one of or two or more of F, Cl, OH and/or alkoxy groups with 1 to 4 C atoms.

Alternatively or in addition, R₆ₐ, R_{6b}, R_{6c} and/or R_{6d} can be carboxamide groups with 1 to 20 C atoms that can be substituted with at least one of or two or more of F, Cl, Br, I, pseudohalogen groups, alkoxy groups with 1 to 20 C atoms, OH, NO₂ and/or NH₂. It is preferred that the carboxamide groups have 1 to 8 C atoms and can be substituted with at least one of or two or more of F, Cl, OH and/or alkoxy groups with 1 to 4 C atoms.

In preferred embodiments, R₆ₐ, R_{6b}, R_{6c} and/or R_{6d} are independently selected from H, an alkoxy group, like methoxy or ethoxy, F, Cl, Br, I, and/or NO₂, linear alkyl groups with 1 to 4 C atoms and branched alkyl groups with 3 to 4 C atoms. According to further preferred embodiments, at least one of R₆ₐ, R_{6b}, R_{6c} and/or R_{6d} is an alkoxy group like methoxy or ethoxy, a carboxylic acid ester group like methylcarbonyloxy or ethylcarbonyloxy, F, Cl, Br, I, and/or NO₂. In further preferred embodiments, R₆ₐ, R_{6c} and R_{6d} are H and R_{6b} is H, an alkoxy group like methoxy or ethoxy, a carboxylic acid ester group like methylcarbonyloxy or ethylcarbonyloxy, F, Cl, Br, I, or NO₂. According to certain embodiments, all of R₆ₐ, R_{6b}, R_{6c} and R_{6d} are H.

According to certain embodiments, the catalyst, particularly the catalyst of Formula IIIa2-a2a1 or the catalyst of Formula IIIa2-a1a2, can be represented by the following Formula IIIa2-a2a2:

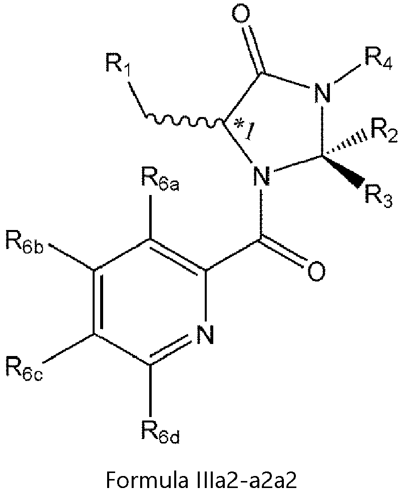

In the compound of Formula IIIa2-a2a2, R₁, R₂, R₃, R₄, R₆ₐ, R_{6b}, R_{6c}, and R_{6d} are as defined in the compound of Formula IIIa2-a1a2. R₁ can particularly have the Formula III-R1. As is clear from the formula, the other atom bound to the ring at the stereocenter *1 is H. The stereocenter *1 of the catalyst according to Formula IIIa2-a2a2 is in (R) and/or (S) configuration.

According to certain embodiments, the catalyst, particularly the catalyst of Formula IIIa2-a1a2, can be represented by the following Formula IIIa2-a1a3a, the following Formula IIIa2-a1a3b, or the following Formula IIIa2-a1a3c, and the latter three can particularly be represented by the following Formula IIIa2-a1a4:

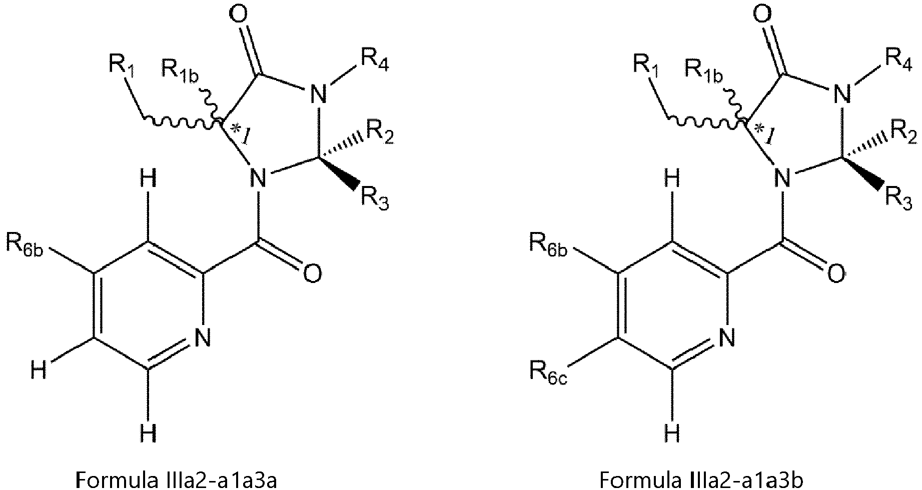

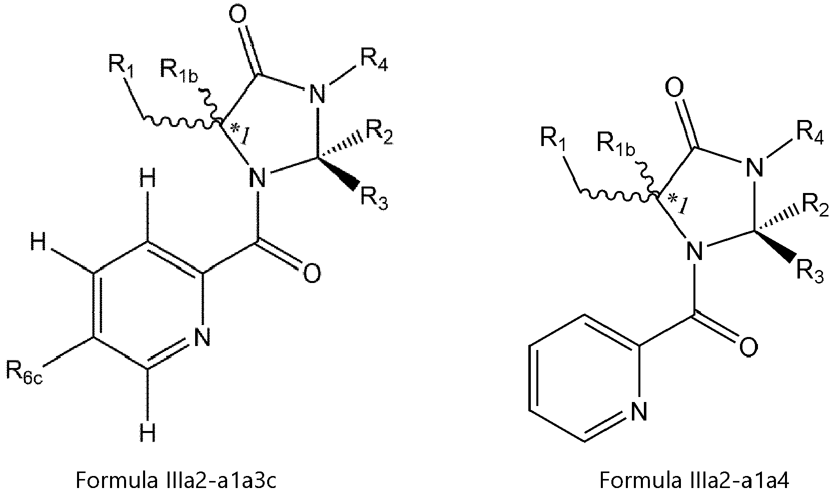

In the compound of Formula IIIa2-a1a3a, R₁, R_{1b}, R₂, R₃, R₄, and R_{6b} are as defined in the compound of Formula IIIa2-a1a2. R₁ can particularly have the Formula III-R1. The stereocenter *1 of the catalyst according to Formula IIIa2-a1a3a is in (R) and/or (S) configuration.

In the compound of Formula IIIa2-a1a3b, R₁, R_{1b}, R₂, R₃, R₄, R_{6b} and R_{c} are as defined in the compound of Formula IIIa2-a1a2. R₁ can particularly have the Formula III-R1. The stereocenter *1 of the catalyst according to Formula IIIa2-a1a3b is in (R) and/or (S) configuration.

In the compound of Formula IIIa2-a1a3c, R₁, R_{1b}, R₂, R₃, R₄, and R_{6c} are as defined in the compound of Formula IIIa2-a1a2. R₁ can particularly have the Formula III-R1. The stereocenter *1 of the catalyst according to Formula IIIa2-a1a3c is in (R) and/or (S) configuration.

In the compound of Formula IIIa2-a1a4, R₁, R_{1b}, R₂, R₃, and R₄ are as defined in the compound of Formula IIIa2-a1a2. R₁ can particularly have the Formula III-R1. The stereocenter *1 of the catalyst according to Formula IIIa2-a1a4 is in (R) and/or (S) configuration.

According to certain embodiments, the catalyst, particularly the catalyst of Formula IIIa2-a2a2, can be represented by the following Formula IIIa2-a2a3a, the following Formula IIIa2-a2a3b, or the following Formula IIIa2-a2a3c, and the latter three can particularly be represented by the following Formula IIIa2-a2a4, the latter four also being representations of the catalysts of Formula IIIa2-a1a3a, Formula IIIa2-a1a3b, Formula IIIa2-a1a3c, and Formula IIIa2-a1a4, respectively:

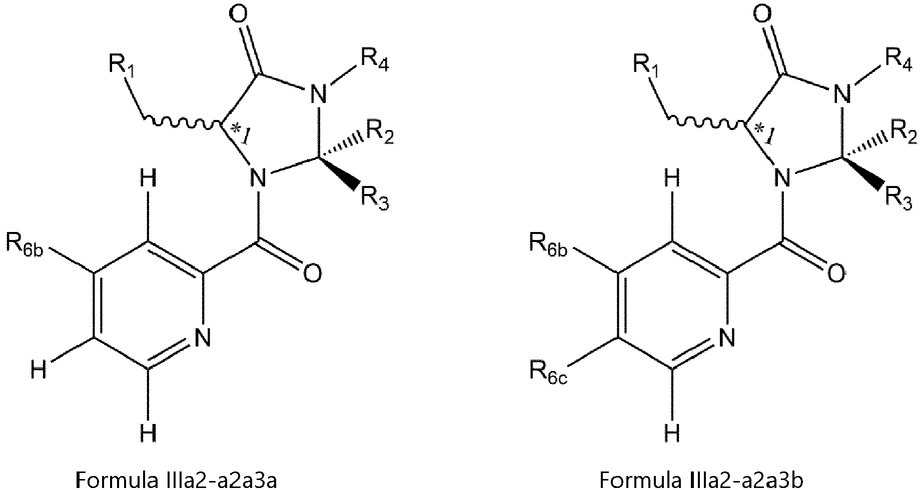

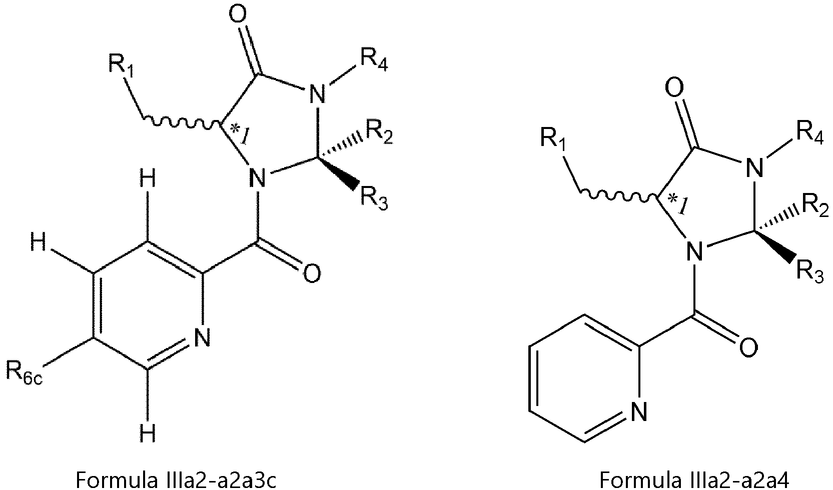

In the compound of Formula IIIa2-a2a3a, R₁, R₂, R₃, R₄, and R_{6b} are as defined in the compound of Formula IIIa2-a2a2. R₁ can particularly have the Formula III-R1. The stereocenter *1 of the catalyst according to Formula IIIa2-a2a3a is in (R) and/or (S) configuration.

In the compound of Formula IIIa2-a2a3b, R₁, R₂, R₃, R₄, R_{6b} and R_{c} are as defined in the compound of Formula IIIa2-a2a2. R₁ can particularly have the Formula III-R1. The stereocenter *1 of the catalyst according to Formula IIIa2-a2a3b is in (R) and/or (S) configuration.

In the compound of Formula IIIa2-a2a3c, R₁, R₂, R₃, R₄, and R_{6c} are as defined in the compound of Formula IIIa2-a2a2. R₁ can particularly have the Formula III-R1. The stereocenter *1 of the catalyst according to Formula IIIa2-a2a3c is in (R) and/or (S) configuration.

In the compound of Formula IIIa2-a2a4, R₁, R₂, R₃, and R₄ are as defined in the compound of Formula IIIa2-a2a2. R₁ can particularly have the Formula III-R1. The stereocenter *1 of the catalyst according to Formula IIIa2-a2a4 is in (R) and/or (S) configuration.

According to certain embodiments, the catalyst, particularly the catalyst of Formula IIIa2-a1a4, can be represented by the following Formula IIIa2-a1a5:

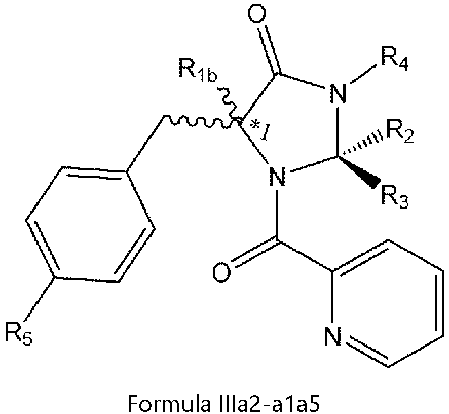

In the compound of Formula IIIa2-a1a5, R_{1b}, R₂, R₃, and R₄ are as defined in the compound of Formula IIIa2-a1a4. The stereocenter *1 of the catalyst according to Formula IIIa2-a1a5 is in (R) and/or (S) configuration.

In the compound of Formula IIIa2-a1a5, as well as any of the following formulas directly or indirectly derived therefrom, according to certain embodiments, R₅ can be H, F, Cl, Br, I, a pseudohalogen group, OH, SH, NO₂, NH₂ and/or an amine of formula NR₁₈R₁₉, R₁₈ and R₁₉ as defined above. In some embodiments, R₅ can be an alkoxy group with a linear, branched, cyclic, heteroaromatic and/or aromatic residue having 1 to 20 C atoms that can comprise one or more heteroatoms selected from O, N, S and P and that can optionally be substituted with at least one of F, Cl, Br, I, pseudohalogen groups, alkoxy groups with 1 to 20 C atoms, OH, NO₂ and NH₂. Specifically, the residue of the alkoxy group can be a linear, branched and/or cyclic alkyl residue with 1 to 20 C atoms that can have one or more heteroatoms selected from O, N, S and P at any position and can be linked at any position, thus including ethers, amides, thioethers, etc., can also be an aromatic residue with 3 to 20 C atoms that can have one or more heteroatoms selected from O, N, S and P at any position and can be linked at any position, thus including heteroaromatic residues with furane, pyridine, pyrrole, indole, thiophene, etc. units, or can be a linear, branched and/or cyclic alkyl residue and/or an aromatic residue with 3 to 20 C atoms, and that can optionally be substituted with at least one of F, Cl, Br, I, pseudohalogen groups, alkoxy groups with 1 to 20 C atoms, OH, SH, NO₂ and NH₂.

In certain embodiments, R₅ can be a carboxylic ester group with a linear, branched, cyclic and/or aromatic residue having 1 to 20 C atoms that can comprise one or more heteroatoms selected from O, N, S and P and that can optionally be substituted with at least one of F, Cl, Br, I, pseudohalogen groups, alkoxy groups with 1 to 20 C atoms, OH, SH, NO₂ and NH₂. Specifically, the residue of the carboxylic ester group can be a linear, branched and/or cyclic alkyl residue with 1 to 20 C atoms that can have one or more heteroatoms selected from O, N, S and P at any position and can be linked at any position, thus including ethers, amides, thioethers, etc., can also be an aromatic residue with 3 to 20 C atoms that can have one or more heteroatoms selected from O, N, S and P at any position and can be linked at any position, thus including heteroaromatic residues with furane, pyridine, pyrrole, indole, thiophene, etc. units, or can be a linear, branched and/or cyclic alkyl residue and/or an aromatic residue with 3 to 20 C atoms, and that can optionally be substituted with at least one of F, Cl, Br, I, pseudohalogen groups, alkoxy groups with 1 to 20 C atoms, OH, NO₂ and NH₂.

In certain embodiments, R₅ can be an alkyl and/or (hetero)aryl oxycarbonyl group with a linear, branched, cyclic and/or aromatic residue having 1 to 20 C atoms that can comprise one or more heteroatoms selected from O, N, S and P and that can optionally be substituted with at least one of F, Cl, Br, I, pseudohalogen groups, alkoxy groups with 1 to 20 C atoms, OH, SH, NO₂ and NH₂.

In certain embodiments, R₅ can be a carboxamide groups with a linear, branched, cyclic and/or aromatic residue having 1 to 20 C atoms that can comprise one or more heteroatoms selected from O, N, S and P and that can optionally be substituted with at least one of F, Cl, Br, I, pseudohalogen groups, alkoxy groups with 1 to 20 C atoms, OH, SH, NO₂ and NH₂. According to preferred embodiments, R₅ is H, a benzyl group or OH. In further preferred embodiments, R₅ is a alkoxy group wherein the residue of the alkoxy group is an isopropyl group, a benzyl group or a 1-benzyl-1H-1,2,3-triazol-4-methylene group. Further preferably, R₅ is a carboxylic ester group wherein the residue of the carboxylic ester group is a pyridyl group.

According to certain embodiments, the catalyst, particularly the catalyst of Formula IIIa2-a1a5 or the catalyst of Formula IIIa2-a2a4, can be represented by the following Formula IIIa2-a2a5:

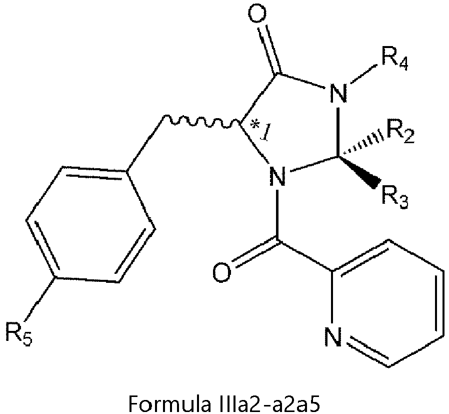

In the compound of Formula IIIa2-a2a5, R₂, R₃, R₄ and R₅ are as defined in the compound of Formula IIIa2-a1a5. The stereocenter *1 of the catalyst according to Formula IIIa2-a2a5 is in (R) and/or (S) configuration.

According to certain embodiments, the catalyst, particularly the catalyst of Formula IIIa2-a1a5, can be represented by the following Formula IIIa2-a1a6:

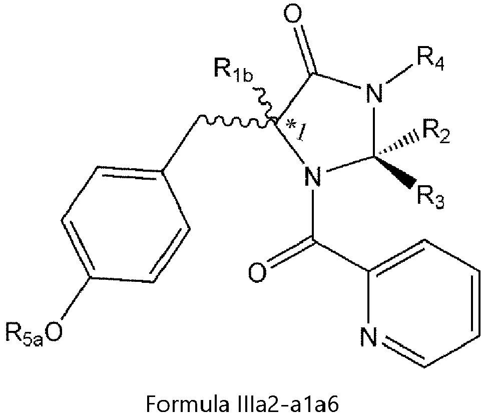

In the compound of Formula IIIa2-a1a6, R_{1b}, R₂, R₃ and R₄ are as defined in the compound of Formula IIIa2-a1a5. The stereocenter *1 of the catalyst according to Formula IIIa2-a2a5 is in (R) and/or (S) configuration.

In the compound of Formula IIIa2-a1a6, as well as any of the following formulas directly or indirectly derived therefrom, according to certain embodiments, R₅ₐ can be H, a linear alkyl group with 1 to 4 C atoms that can optionally be substituted with at least one of F, Cl, Br, I, pseudohalogen groups, alkoxy groups with 1 to 20 C atoms, OH, SH, NO₂ and NH₂, a branched alkyl group with 3 to 6 C atom that can optionally be substituted with at least one of F, Cl, Br, I, pseudohalogen groups, alkoxy groups with 1 to 20 C atoms, OH, SH, NO₂ and NH₂s, an aromatic group with 6 to 12 C atoms that can optionally be substituted with at least one of F, Cl, Br, I, pseudohalogen groups, alkoxy groups with 1 to 20 C atoms, OH, SH, NO₂ and NH₂, an aralkyl group with 7 to 10 C atoms that can optionally be substituted with at least one of F, Cl, Br, I, pseudohalogen groups, alkoxy groups with 1 to 20 C atoms, OH, SH, NO₂ and NH₂, a substituted carbonyl group with a heteroaromatic residue having 3 to 5 C atoms, or a heteroaromatic group with at least one alkyl residue with 3 to 6 C atoms that can be substituted with at least one aralkyl group with 7 to 10 C atoms. According to preferred embodiments, R₅ₐ is H, isopropyl, benzyl, a pyridinecarbonyl group or a 1-benzyl-1H-1,2,3-triazol-4-methylene group.

According to certain embodiments, the catalyst, particularly the catalyst of Formula IIIa2-a1a6 or the catalyst of Formula IIIa2-a2a5, can be represented by the following Formula IIIa2-a2a6:

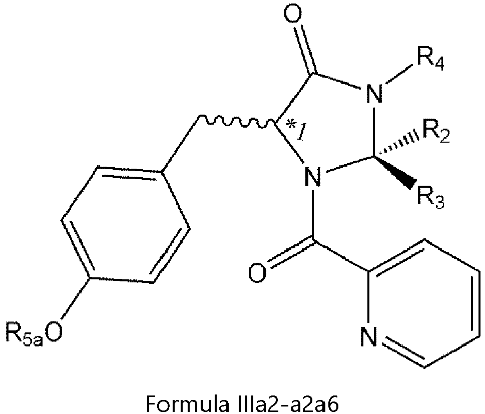

In the compound of Formula IIIa2-a2a6, R₂, R₃, R₄ and R₅ₐ are as defined in the compound of Formula IIIa2-a1a6. The stereocenter *1 of the catalyst according to Formula IIIa2-a2a6 is in (R) and/or (S) configuration.

According to certain embodiments, the catalyst of Formula I can be represented by the following Formula IIIr:

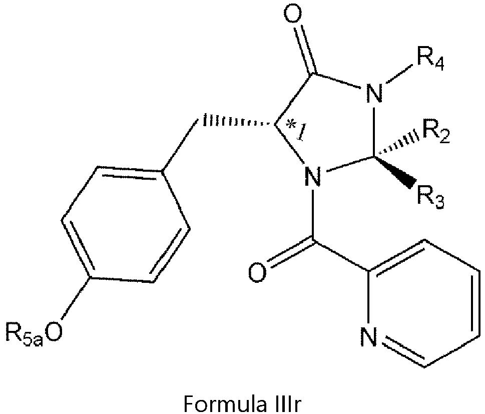

In the compound of Formula IIIr, R₂, R₃, R₄ and R₅ₐ are as defined above.

According to certain embodiments, the catalyst, particularly the catalyst of Formula IIIa2-a2a6, can be represented by the following Formula IIIa2-a2a7a, preferably the following Formula IIIa2-a2a8a, further preferably the following Formula IIIa2-a2a9a, or can be represented by the following Formula IIIa2-a2a7b, preferably the following Formula IIIa2-a2a8b, further preferably the following Formula IIIa2-a2a9b:

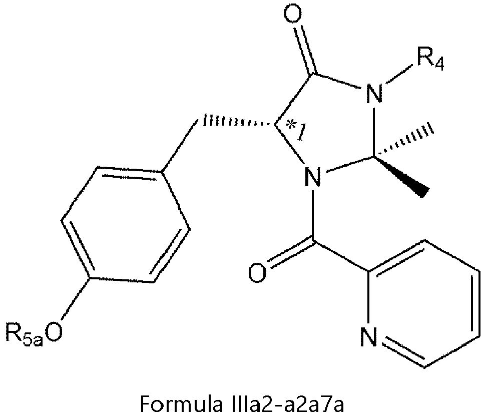

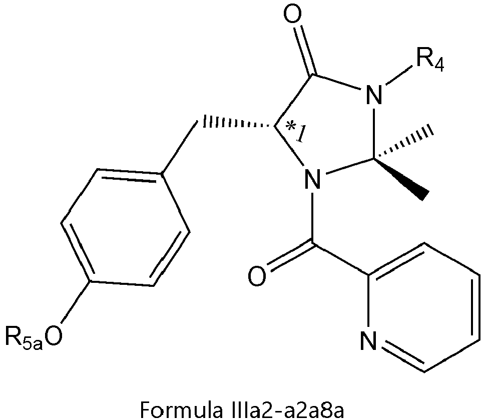

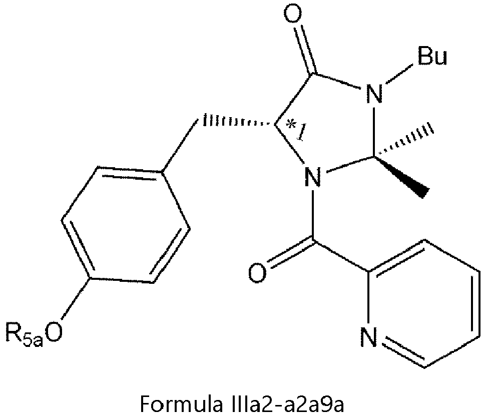

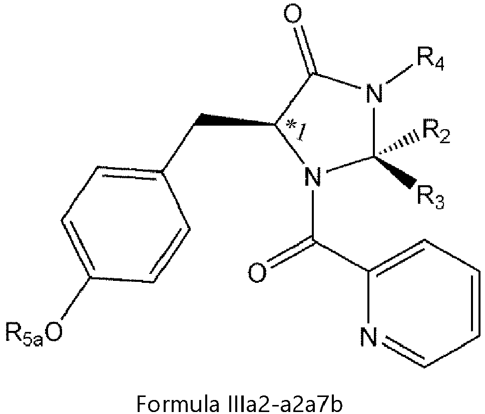

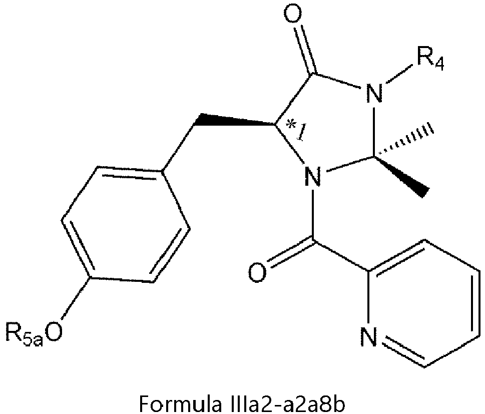

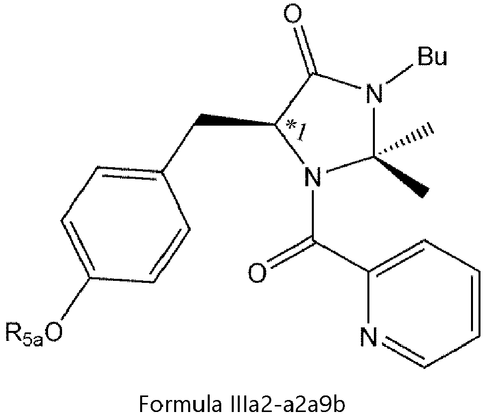

In the compounds of Formula IIIa2-a2a7a and Formula IIIa2-a2a7b, R₂, R₃, R₄ and R₅ₐ are as defined in the compound of Formula IIIa2-a2a6, in the compounds of Formula IIIa2-a2a8a and Formula IIIa2-a2a8b, R₄ and R₅ₐ are as defined in the compound of Formula IIIa2-a2a6, and in the compounds of Formula IIIa2-a2a9a and Formula IIIa2-a2a9b, R₅ₐ is as defined in the compound of Formula IIIa2-a2a6.

According to certain embodiments, the catalyst, particularly the catalyst of Formula IIIa2-c, can be represented by the following Formula IIIa2-c1, preferably the following Formula IIIa2-c2, further preferably the following Formula IIIa2-c3, even further preferably the following Formula IIIa2-c4:

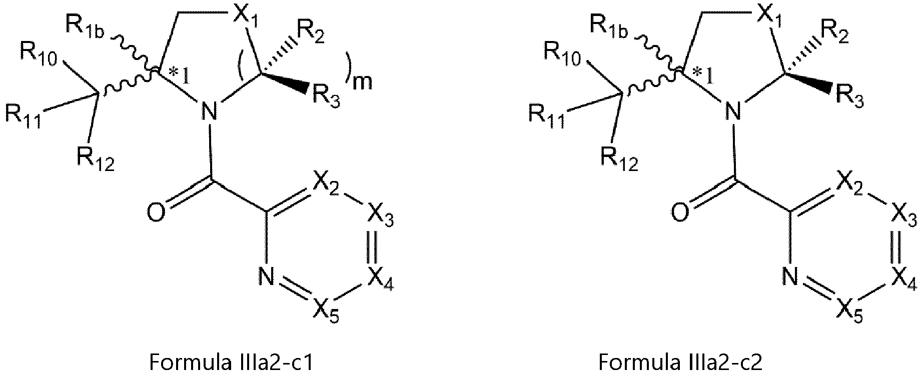

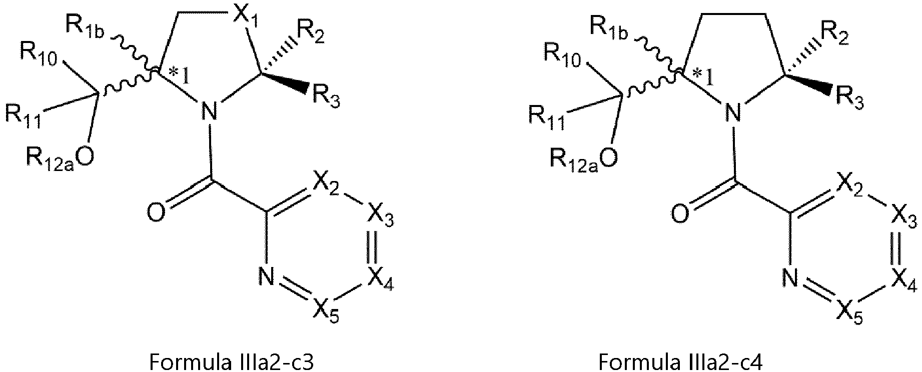

In the compound of Formula IIIa2-c1, m, R_{1b}, R₂, R₃, R₁₀. R₁₁, R₁₂, X₁, X₂, X₃, X₄, and X₅ are as defined in the compound of Formula IIIa2-c. The stereocenter *1 of the catalyst according to Formula IIIa2-c1 is in (R) and/or (S) configuration.

In the compound of Formula IIIa2-c2, R_{1b}, R₂, R₃, R₁₀. R₁₁, R₁₂, X₁, X₂, X₃, X₄, and X₅ are as defined in the compound of Formula IIIa2-c. The stereocenter *1 of the catalyst according to Formula IIIa2-c2 is in (R) and/or (S) configuration.

In the compound of Formula IIIa2-c3, R_{1b}, R₂, R₃, R₁₀. R₁₁, X₁, X₂, X₃, X₄, and X₅ are as defined in the compound of Formula IIIa2-c. The stereocenter *1 of the catalyst according to Formula IIIa2-c3 is in (R) and/or (S) configuration.
R₁₂ₐ is chosen from H;
linear alkyl groups with 1 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups (including (CO)NH₂) with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
linear alkenyl and/or alkynyl groups with 2 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
branched alkyl, alkenyl and/or alkynyl groups with 3 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
aralkyl groups with 7 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkylaryl groups with 7 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
cycloalkyl groups with 3 to 20 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heterocycloalkyl groups with 1 to 15 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
aromatic and/or heteroaromatic groups with 2 to 40 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl heteroaromatic groups with 2 to 50 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heteroaromatic groups with at least one alkyl residue with 3 to 50 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl cycloalkyl groups with 4 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
cycloalkyl alkyl groups with 4 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl heterocycloalkyl groups with 2 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heterocycloalkyl alkyl groups with 2 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂.
SiR₂₃R₂₄R₂₅;
R₂₃, R₂₄, and R₂₅ are the same or different and are selected from H, linear alkyl groups with 1 to 4 C atoms that can optionally be substituted with at least one of F, Cl, Br, I, pseudohalogen groups, alkoxy groups with 1 to 20 C atoms, OH, NO₂ and NH₂, branched alkyl group with 3 to 6 C atoms that can optionally be substituted with at least one of F, Cl, Br, I, pseudohalogen groups, alkoxy groups with 1 to 20 C atoms, OH, NO₂ and NH₂, aromatic groups with 6 to 12 C atoms that can optionally be substituted with at least one of F, Cl, Br, I, pseudohalogen groups, alkoxy groups with 1 to 20 C atoms, OH, NO₂ and NH₂, and aralkyl groups with 7 to 10 C atoms that can optionally be substituted with at least one of F, Cl, Br, I, pseudohalogen groups, alkoxy groups with 1 to 20 C atoms, OH, NO₂ and NH₂, preferably wherein R₂₃, R₂₄, and R₂₅ are not all hydrogen at the same time and/or wherein R₂₃, R₂₄, and R₂₅ are not all methyl at the same time, further preferably wherein all of R₂₃, R₂₄, and R₂₅ are ethyl;

In the compound of Formula IIIa2-c4, R_{1b}, R₂, R₃, R₁₀. R₁₁, R₁₂ₐ, X₂, X₃, X₄, and X₅ are as defined in the compound of Formula IIIa2-c3. The stereocenter *1 of the catalyst according to Formula IIIa2-c4 is in (R) and/or (S) configuration.

According to certain embodiments, the catalyst, particularly the catalyst of Formula IIIa2-c1 or the catalyst of Formula IIIa3-a, can be represented by the following Formula IIIa3-a1, preferably the following Formula IIIa3-a2 (this also being a preferable embodiment of the catalyst of Formula IIIa2-c2), further preferably the following Formula IIIa3-a3 (this also being a preferable embodiment of the catalyst of Formula IIIa2-c3), even further preferably the following Formula IIIa3-a4 (this also being a preferable embodiment of the catalyst of Formula IIIa2-c4):

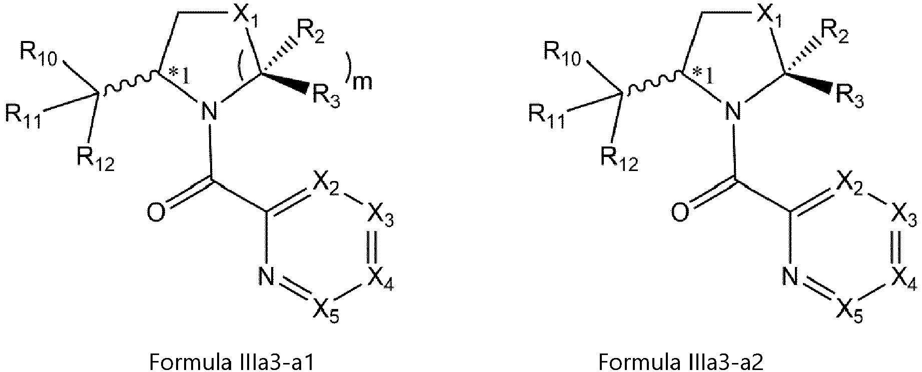

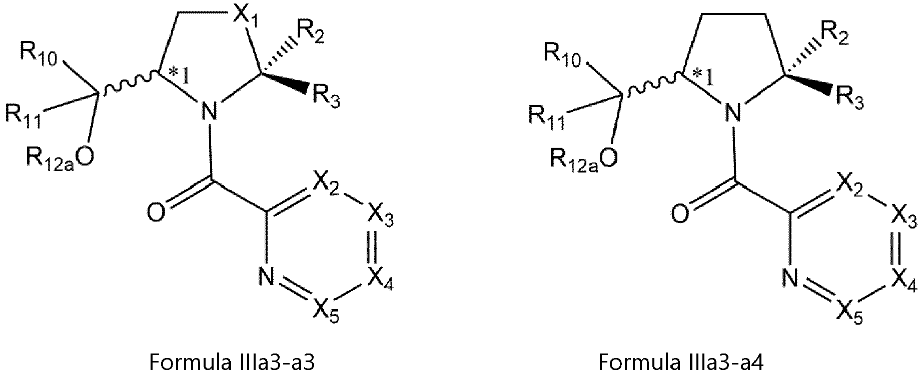

In the compound of Formula IIIa3-a1, m, R₂, R₃, R₁₀. R₁₁, R₁₂, X₁, X₂, X₃, X₄, and X₅ are as defined in the compound of Formula IIIa2-c1. The stereocenter *1 of the catalyst according to Formula IIIa3-a1 is in (R) and/or (S) configuration.

In the compound of Formula IIIa3-a2, R₂, R₃, R₁₀. R₁₁, R₁₂, X₁, X₂, X₃, X₄, and X₅ are as defined in the compound of Formula IIIa2-c2. The stereocenter *1 of the catalyst according to Formula IIIa3-a2 is in (R) and/or (S) configuration.

In the compound of Formula IIIa3-a3, R₂, R₃, R₁₀. R₁₁, R₁₂ₐ, X₁, X₂, X₃, X₄, and X₅ are as defined in the compound of Formula IIIa2-c3. The stereocenter *1 of the catalyst according to Formula IIIa3-a3 is in (R) and/or (S) configuration.

In the compound of Formula IIIa3-a4, R₂, R₃, R₁₀. R₁₁, R₁₂ₐ, X₂, X₃, X₄, and X₅ are as defined in the compound of Formula IIIa2-c4. The stereocenter *1 of the catalyst according to Formula IIIa3-a4 is in (R) and/or (S) configuration.

According to certain embodiments, the catalyst, particularly the catalyst of Formula I, is a catalyst of Formula Ic:

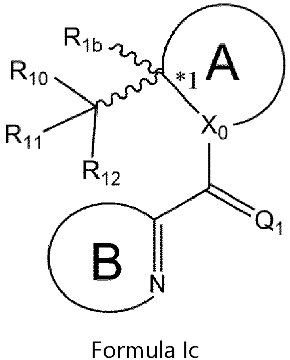

In Formula Ic, as well as any of the following formulas directly or indirectly derived therefrom, ring A, ring B, R_{1b}, Q₁ and X₀ are as defined above, particularly for the catalyst of Formula I.

In the compound of Formula Ic, as well as any of the following formulas directly or indirectly derived therefrom, R₁₀ and R₁₁ are the same or different, e.g. can be the same, and are chosen from H; F; Cl; Br; I; pseudohalogen groups; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂. According to preferred embodiments, R₁₀ and R₁₁ are the same or different, e.g. can be the same, and are chosen from H; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 18 C atoms that can optionally be substituted with at least one of F, Cl, Br, I, pseudohalogen groups, alkoxy groups with 1 to 20 C atoms, OH, NO₂ and NH₂; alkylaryl groups with 7 to 18 C atoms that can optionally be substituted with at least one of F, Cl, Br, I, pseudohalogen groups, alkoxy groups with 1 to 20 C atoms, OH, NO₂ and NH₂; and aromatic and/or heteroaromatic groups with 2 to 20 C atoms that can optionally be substituted with at least one of F, Cl, Br, I, pseudohalogen groups, alkoxy groups with 1 to 20 C atoms, OH, NO₂ and NH₂. According to more preferred embodiments, in the tertiary residue CR₁₀R₁₁R₁₂ in the above formulas and any formulas derived thereof,R₁₀ and R₁₁ are the same or different, e.g. can be the same, and are chosen from H; linear alkyl groups with 1 to 10 C atoms, preferably 1 to 6 C atoms, e.g. a methyl and ethyl group; aralkyl groups with 7 to 13 C atoms, preferably 7 to 10 C atoms, e.g. a (C₆H₅)-CH₂- group; alkylaryl groups with 7 to 15 C atoms, preferably 7 to 13 C atoms, e.g. a CH₃-C₆H₄- group; and aromatic groups with 6 to 14 C atoms, preferably 6 to 10 C atoms, e.g. a phenyl (Ph) and naphthy group (Naph).

In the tertiary residue CR₁₀R₁₁R₁₂ in Formula Ic, R₁₂ is chosen from H, F; Cl; Br; I; pseudohalogen groups; OH; OR₁₂ₐ; SH; SR₁₂ₐ; COOH; COOR₁₂ₐ; NO₂; NH₂; (NR₁₈'R₁₉'R₂₀')⁺; and amine groups NR₁₄R₁₅, with the provisio that not both of R₁₄ and R₁₅ are H, and preferably R₁₂ is H; OH; OR₁₂ₐ, NH₂; or NR₁₄R₁₅, particularly preferably R₁₂ is H; OH; or OR₁₂ₐ, wherein R₁₈', R₁₉', and R₂₀' are the same or different and can be chosen from H; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂.
R₁₂ₐ is chosen from H;
linear alkyl groups with 1 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups (including (CO)NH₂) with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
linear alkenyl and/or alkynyl groups with 2 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
branched alkyl, alkenyl and/or alkynyl groups with 3 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
aralkyl groups with 7 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkylaryl groups with 7 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
cycloalkyl groups with 3 to 20 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heterocycloalkyl groups with 1 to 15 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
aromatic and/or heteroaromatic groups with 2 to 40 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl heteroaromatic groups with 2 to 50 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heteroaromatic groups with at least one alkyl residue with 3 to 50 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl cycloalkyl groups with 4 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
cycloalkyl alkyl groups with 4 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl heterocycloalkyl groups with 2 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heterocycloalkyl alkyl groups with 2 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂.
SiR₂₃R₂₄R₂₅;
R₂₃, R₂₄, and R₂₅ are the same or different and are selected from H, linear alkyl groups with 1 to 4 C atoms that can optionally be substituted with at least one of F, Cl, Br, I, pseudohalogen groups, alkoxy groups with 1 to 20 C atoms, OH, NO₂ and NH₂, branched alkyl group with 3 to 6 C atoms that can optionally be substituted with at least one of F, Cl, Br, I, pseudohalogen groups, alkoxy groups with 1 to 20 C atoms, OH, NO₂ and NH₂, aromatic groups with 6 to 12 C atoms that can optionally be substituted with at least one of F, Cl, Br, I, pseudohalogen groups, alkoxy groups with 1 to 20 C atoms, OH, NO₂ and NH₂, and aralkyl groups with 7 to 10 C atoms that can optionally be substituted with at least one of F, Cl, Br, I, pseudohalogen groups, alkoxy groups with 1 to 20 C atoms, OH, NO₂ and NH₂, preferably wherein R₂₃, R₂₄, and R₂₅ are not all hydrogen at the same time and/or wherein R₂₃, R₂₄, and R₂₅ are not all methyl at the same time, further preferably wherein all of R₂₃, R₂₄, and R₂₅ are ethyl;
R₁₄ and R₁₅ are the same or different and can be chosen from H,
linear alkyl groups with 1 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups (including (CO)NH₂) with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
linear alkenyl and/or alkynyl groups with 2 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
branched alkyl, alkenyl and/or alkynyl groups with 3 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
aralkyl groups with 7 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkylaryl groups with 7 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
cycloalkyl groups with 3 to 20 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heterocycloalkyl groups with 1 to 15 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
aromatic and/or heteroaromatic groups with 2 to 40 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl heteroaromatic groups with 2 to 50 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heteroaromatic groups with at least one alkyl residue with 3 to 50 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl cycloalkyl groups with 4 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
cycloalkyl alkyl groups with 4 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl heterocycloalkyl groups with 2 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heterocycloalkyl alkyl groups with 2 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂.

The stereocenter *1 of the catalyst according to Formula Ic is in (R) and/or (S) configuration.

According to certain embodiments, the catalyst, particularly the catalyst of Formula Ic, is a catalyst of Formula Ic-1:

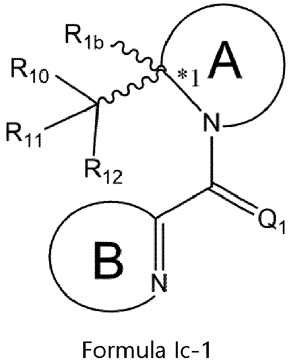

In the compound of Formula Ic-1, ring A, ring B, R_{1b}, R₁₀, R₁₁, and R₁₂ are as defined in the compound of Formula Ic. The stereocenter *1 of the catalyst according to Formula Ic-1 is in (R) and/or (S) configuration.

According to certain embodiments, the catalyst, particularly the catalyst of Formula I-c, is a catalyst of the following Formula IV:

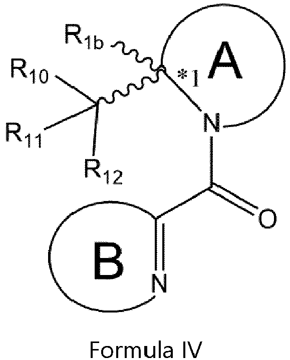

In the compound of Formula IV, ring A, ring B, R_{1b}, R₁₀, R₁₁, and R₁₂ are as defined in the compound of Formula Ic. The stereocenter *1 of the catalyst according to Formula IV is in (R) and/or (S) configuration.

According to some embodiments, in the compound of Formula IV, as well as any of the following formulas directly or indirectly derived therefrom, R_{1b} is H. R_{1b} can in certain embodiments be a linear alkyl group with 1 to 6 C atoms that can be substituted with at least one of or two or more of F, Cl, Br, I, pseudohalogen groups, alkoxy groups with 1 to 6 C atoms, OH, NO₂ and/or NH₂. Preferably, the linear alkyl group has 1 to 4 C atoms and can be substituted with at least one of or two or more of F, Cl and/or OH.

According to some embodiments, R_{1b} can be a branched alkyl group with 3 to 6 C atoms that can be substituted with at least one of or two or more of F, Cl, Br, I, pseudohalogen groups, alkoxy groups with 1 to 6 C atoms, OH, NO₂ and/or NH₂. It is preferred that the branched alkyl group has 3 to 4 C atoms and can be can be substituted with at least one of or two or more of F, CI and/or OH.

Further, R_{1b} can in certain embodiments be a heterocycloalkyl group with 1 to 6 C atoms that can be substituted with at least one of or two or more of F, Cl, Br, I, pseudohalogen groups, alkoxy groups with 1 to 6 C atoms, OH, NO₂ and/or NH₂. According to preferred embodiments, the heterocycloalkyl group has 1 to 5 C atoms and can be substituted with at least one of or two or more of F, Cl and/or OH.

According to some embodiments, R_{1b} can be an aromatic and/or heteroaromatic group with 2 to 6 carbon atoms that can be substituted with at least one of or two or more of F, Cl, Br, I, pseudohalogen groups, alkoxy groups with 1 to 6 C atoms, OH, NO₂ and/or NH₂, preferably with F, CI and/or OH.

Furthermore, R_{1b} can in certain embodiments be a carboxylic acid ester group with 1 to 6 C atoms that can be substituted with at least one of or two or more of F, Cl, Br, I, pseudohalogen groups, alkoxy groups with 1 to 6 C atoms, OH, NO₂ and/or NH₂. It is preferred that the carboxylic acid ester group has 1 to 4 C atoms and can be substituted with at least one of or two or more of F, Cl and/or OH.

According to certain embodiments, R_{1b} can be an alkyl and/or (hetero)aryl oxycarbonyl group with 1 to 6 C atoms that can be substituted with at least one of or two or more of F, Cl, Br, I, pseudohalogen groups, alkoxy groups with 1 to 6 C atoms, OH, NO₂ and/or NH₂. Preferably, the carboxylic acid ester group has 1 to 4 C atoms and can be substituted with at least one of or two or more of F, Cl and/or OH.

Also, R_{1b} can in some embodiments be a carboxamide groups with 1 to 6 C atoms that can be substituted with at least one of or two or more of F, Cl, Br, I, pseudohalogen groups, alkoxy groups with 1 to 6 C atoms, OH, NO₂ and/or NH₂. According to preferred embodiments, the carboxylic acid ester group has 1 to 4 C atoms and can be substituted with at least one of or two or more of F, Cl and/or OH.

According to preferred embodiments, R_{1b} is H or methyl, particularly H.

The stereocenter *1 of the catalyst according to Formula IV is in (R) and/or (S) configuration. According to certain embodiments, when the stereocenter *1 of the catalyst is in (R) configuration, (S)-nornicotine is produced in excess. Furthermore, when the stereocenter *1 of the catalyst is in (S) configuration, (R)-nornicotine is produced in excess.

With the catalytic system described above, the inventors have surprisingly found that it is possible to reduce myosmine, which is a cyclic imine, to nornicotine. It is assumed that the amide unit of the catalyst according to Formula IV is responsible for the coordination of the silane and chemical activation. This allows the hydride transfer from the silane to myosmine.

The stereocenter in ortho position on ring A with the residues R_{1b} and CR₁₀R₁₁R₁₂ is thought to direct the coordination of myosmine which enables its enantioselective hydrosilylation.

According to certain embodiments, the catalyst, particularly the catalyst of Formula IV, is represented by the following Formula IVa and/or the following Formula IVb:

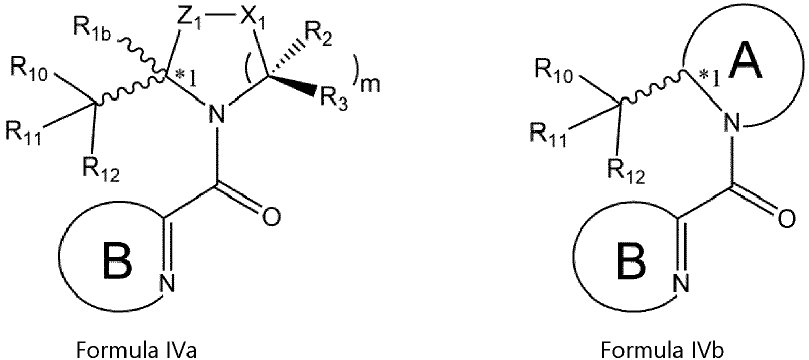

In the compound of Formula IVa, ring B, R_{1b}, R₁₀, R₁₁, and R₁₂ are as defined in the compound of Formula IV. The stereocenter *1 of the catalyst according to Formula IVa is in (R) and/or (S) configuration.

In the compound of Formula IVa, as well as any of the following formulas directly or indirectly derived therefrom, Z₁ is chosen from a carbonyl group, a thiocarbonyl group, and CR₂₀ₐR_{20b}. R₂₀ₐ and R_{20b} are the same or different and can be chosen from H, linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂.

In the compound of Formula IVa, as well as any of the following formulas directly or indirectly derived therefrom, m is an integer from 0 to 7, preferably from0 to 4, further preferably 1 or 2, even further preferably 1.

In the compound of Formula IVa, as well as any of the following formulas directly or indirectly derived therefrom, R₂ and R₃ are the same or different and are independently selected from H; F; Cl; Br; I; pseudohalogen groups; alkoxy groups with 1 to 20 C atoms; OH; NO₂; NH₂; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂.

In the compound of Formula IVa, as well as any of the following formulas directly or indirectly derived therefrom, X₁ is selected from the group of O, NR4, and CR₄ₐR_{4b}, wherein R₄ₐ and R_{4b} can be the same or different.

In the compound of Formula IVa, as well as any of the following formulas directly or indirectly derived therefrom, R₄, R₄ₐ, and R_{4b} are independently selected from H; F; Cl; Br; I; pseudohalogen groups; alkoxy groups with 1 to 20 C atoms; OH; SR₂₁; S(CO)R₂₂; NO₂; NH₂; amines of formula NR₁₈R₁₉; (NR₁₈'R₁₉'R₂₀')⁺, linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester (carboxylate) groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂; wherein R₄ₐ and R_{4b} can be the same or different; with the provisio that two or more of R₂, R₃, R₄, R₄ₐ, and R_{4b} can form a ring or more rings, optionally wherein at least one of R₂, R₃, R₄, R₄ₐ, and R_{4b} is part of a polymer, wherein
R₁₈, R₁₉, R₁₈', R₁₉', R₂₀', R₂₁, and R₂₂ are the same or different and can be chosen from H; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂, with the provisio that not both of R₁₈ and R₁₉ are H.

In the compound of Formula IVb, ring A, ring B, R₁₀, R₁₁, and R₁₂ are as defined in the compound of Formula IV. The stereocenter *1 of the catalyst according to Formula IVb is in (R) and/or (S) configuration.

According to certain embodiments, the catalyst, particularly the catalyst of Formula IVa, is represented by the following Formula IVa1a and/or Formula IVa1b, and the catalyst of Formula IVa1a is preferably represented by the following Formula IVa2, further preferably the following Formula IVa3:

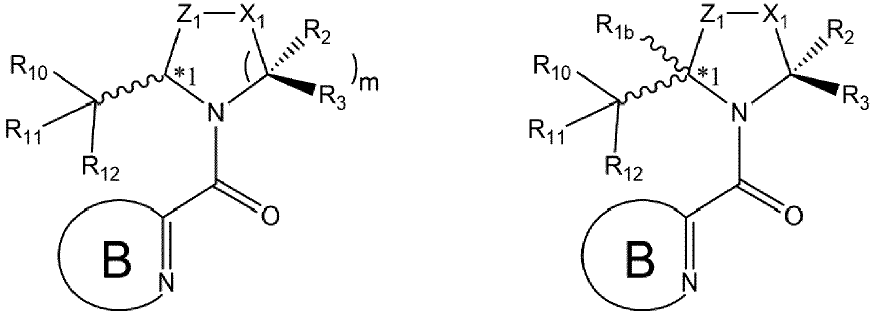

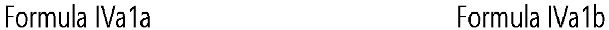

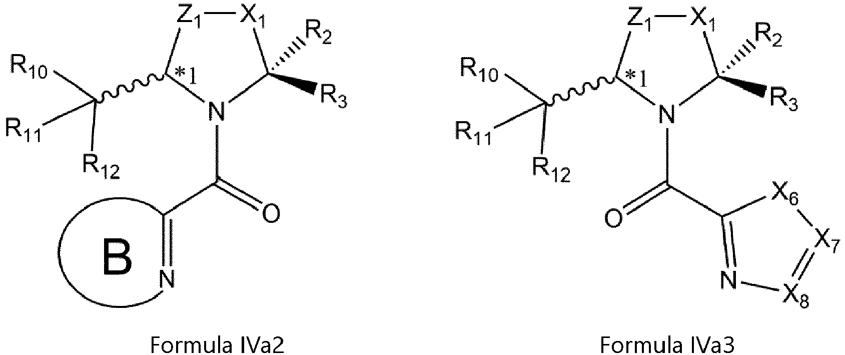

In the compound of Formula IVa1a, ring B, m, R₂, R₃, R₁₀, R₁₁, R₁₂, X₁ and Z₁ are as defined in the compound of Formula IVa. The stereocenter *1 of the catalyst according to Formula IVa1 is in (R) and/or (S) configuration.

In the compound of Formula IVa1b, ring B, R_{1b}, R₂, R₃, R₁₀, R₁₁, R₁₂, X₁ and Z₁ are as defined in the compound of Formula IVa. The stereocenter *1 of the catalyst according to Formula IVa2 is in (R) and/or (S) configuration.

In the compound of Formula IVa2, ring B,, R₂, R3, R₁₀, R₁₁, R₁₂, X₁ and Z₁ are as defined in the compound of Formula IVa. The stereocenter *1 of the catalyst according to Formula IVa3 is in (R) and/or (S) configuration.

In the compound of Formula IVa3, R₂, R₃, R₁₀, R₁₁, R₁₂, X₁ and Z₁ are as defined in the compound of Formula IVa. The stereocenter *1 of the catalyst according to Formula IVa3 is in (R) and/or (S) configuration.

In the compound of Formula IVa3, X₆ is selected from O, S, NR₁₃ and CR₁₃ₐR_{13b}.

In the compound of Formula IVa3, R₁₃ is chosen from H, linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂. According to certain embodiments, R₁₃ is H, a linear alkyl group with 1 to 10, e.g. 1 to 6, C atoms, e.g. a methyl or ethyl group, an aromatic group with 6 to 20 C atoms, e.g.6 to 10 or 6 C atoms, e.g. a phenyl group, or an aralkyl group with 7 to 20 C atoms, e.g. 7 to 11 C atoms, e.g. 7 C atoms, e.g. a benzyl group, in the formulas comprising R₁₃.

In the compound of Formula IVa3, R₁₃ₐ and R_{13b} are the same or different and can be chosen from H, linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂.

In the compound of Formula IVa3, X₇ and X₈ are independently selected from N and CR₆; with the provisio that when both X₇ and X₈ are represented by CR₆, the two R₆ can be the same or different and/or the two R₆ can form a ring, and with the provisio that if at least one of X₇ and X₈ is represented by CR₆, the at least one R₆ can form a ring with R₁₃, R₁₃ₐ and/or R_{13b}, preferably with the provisio that at least one of X₇ and X₈ is CR₆ and/or X₆ is CR₁₃ₐR_{13b}. According to preferred embodiments, X₇ and X₈ are independently selected from N and CR₆; with the provisio that when both X₇ and X₈ are represented by CR₆, the two R₆ can be the same or different, preferably with the provisio that at least one of X₇ and X₈ is CR₆ and/or X₆ is CR₁₃ₐR_{13b}. A formation of a ring is not preferable.

In the compound of Formula IVa3, R₆ is chosen from H; F; Cl; Br; I; alkoxy groups with 1 to 20 C atoms; pseudohalogen groups; OH; NO₂; NH₂; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂.

According to certain embodiments, the catalyst, particularly the catalyst of Formula I, is a catalyst of Formula Id:

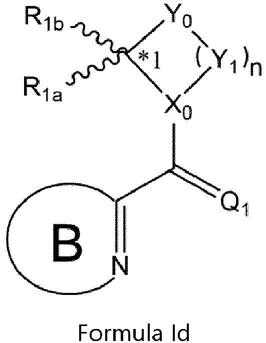

In Formula Id, as well as any of the following formulas directly or indirectly derived therefrom, ring B, R₁ₐ, R_{1b}, Q₁ and X₀ are as defined above, particularly for the catalyst of Formula I.

In Formula Id, Y₁ is chosen from substituted or unsubstituted C atoms and/or substituted or unsubstituted heteroatoms, wherein the substituents can comprise any one of F; Cl; Br; I; pseudohalogen groups; alkoxy groups with 1 to 20 C atoms; OH; SR₂₁; S(CO)R₂₂; NO₂; NH₂; amines of formula NR₁₈R₁₉; (NR₁₈'R₁₉'R₂₀')⁺; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂; with the provisio that two or more of the substituents can form a ring or more rings, optionally wherein at least one substituent is part of a polymer, and with the provisio that if more than one Y₁ is present, these can be the same or different, wherein
R₁₈, R₁₉, R₁₈', R₁₉', R₂₀', R₂₁, and R₂₂ are the same or different and can be chosen from H; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂, with the provisio that not both of R₁₈ and R₁₉ are H.

In Formula Id, n is an integer from 0 to 8, preferably from 1 to 5, further preferably from 1 to 3, even further preferably 2 or 3.

In Formula Id, Y₀ is chosen from a carbonyl group, a thiocarbonyl group, and CR₁₆R₁₇.

In Formula Id, R₁₆ is chosen from H; F; Cl; Br; I; pseudohalogen groups; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂.

In Formula Id, R₁₇ is chosen from alkoxy groups with 1 to 20 C atoms; OH; NO₂; NH₂; and amine groups NR₁₈R₁₉.

The stereocenter *1 of the catalyst according to Formula Id is in (R) and/or (S) configuration.

According to certain embodiments, the catalyst, particularly the catalyst of Formula Id, is a catalyst of Formula Id-1:

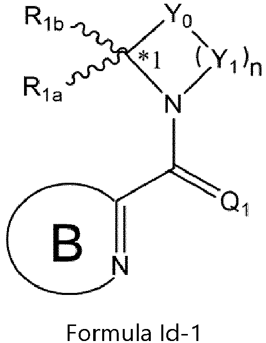

In the compound of Formula Id-1, ring B, n, R₁ₐ, R_{1b}, Q₁, Y₀ and Y₁ are as defined in the compound of Formula Id. The stereocenter *1 of the catalyst according to Formula Id-1 is in (R) and/or (S) configuration.

According to certain embodiments, the catalyst, particularly the catalyst of Formula I-d, is a catalyst of the following Formula V:

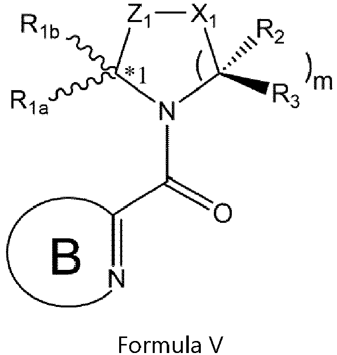

In the compound of Formula V, ring B, R₁ₐ, and R_{1b} as defined in the compound of Formula Id. The stereocenter *1 of the catalyst according to Formula V is in (R) and/or (S) configuration.

In the compound of Formula V, as well as any of the following formulas directly or indirectly derived therefrom, Z₁ is chosen from a carbonyl group, a thiocarbonyl group, and CR₂₀ₐR_{20b}. R₂₀ₐ and R_{20b} are the same or different and can be chosen from H, linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂. In the compound of Formula V, as well as any of the following formulas directly or indirectly derived therefrom, m is an integer from 0 to 7, preferably from0 to 4, further preferably 1 or 2, even further preferably 1.

In the compound of Formula V, as well as any of the following formulas directly or indirectly derived therefrom, R₂ and R₃ are the same or different and are independently selected from H; F; Cl; Br; I; pseudohalogen groups; alkoxy groups with 1 to 20 C atoms; OH; NO₂; NH₂; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂.

In the compound of Formula V, as well as any of the following formulas directly or indirectly derived therefrom, X₁ is selected from the group of O, NR₄, and CR₄ₐR_{4b}, wherein R₄ₐ and R_{4b} can be the same or different.

In the compound of Formula V, as well as any of the following formulas directly or indirectly derived therefrom, R₄, R₄ₐ, and R_{4b} are independently selected from H; F; Cl; Br; I; pseudohalogen groups; alkoxy groups with 1 to 20 C atoms; OH; SR₂₁; S(CO)R₂₂; NO₂; NH₂; amines of formula NR₁₈R₁₉; (NR₁₈'R₁₉'R₂₀')⁺; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester (carboxylate) groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂; wherein P₄ₐ and R_{4b} can be the same or different; with the provisio that two or more of R₂, R₃, R₄, R₄ₐ, and R_{4b} can form a ring or more rings, optionally wherein at least one of R₂, R₃, R₄, R₄ₐ, and R_{4b} is part of a polymer, wherein
R₁₈, R₁₉, R₁₈', R₁₉', R₂₀', R₂₁, and R₂₂ are the same or different and can be chosen from H; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂, with the provisio that not both of R₁₈ and R₁₉ are H.

According to certain embodiments, the catalyst, particularly the catalyst of Formula V, is a catalyst of the following Formula Va and/or of the following Formula Vb:

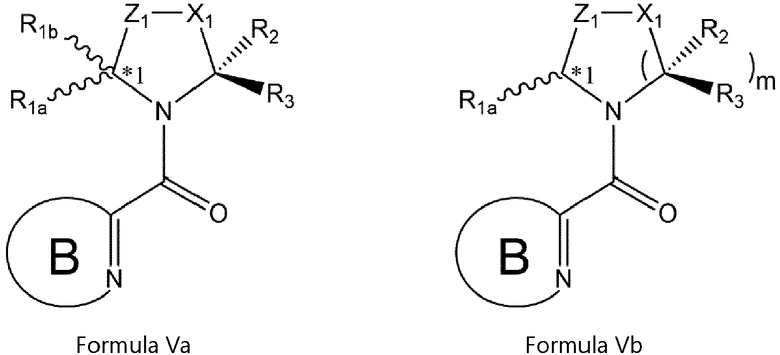

In the compound of Formula Va, ring B, R₁ₐ, R_{1b}, R₂, R₃, X₁, and Z₁ are as defined in the compound of Formula V. The stereocenter *1 of the catalyst according to Formula Va is in (R) and/or (S) configuration.

In the compound of Formula Vb, ring B, m, R₁ₐ, R₂, R₃, X₁, and Z₁ are as defined in the compound of Formula V. The stereocenter *1 of the catalyst according to Formula Vb is in (R) and/or (S) configuration.

According to certain embodiments, the catalyst, particularly the catalyst of Formula Va or the catalyst of Formula Vb, is represented by the following Formula Vb1:

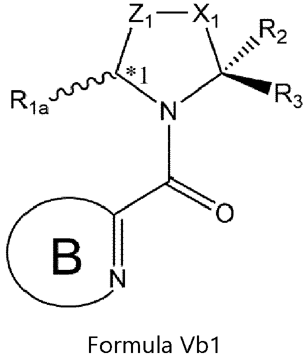

In the compound of Formula Vb1, ring B, R₁ₐ, R₂, R₃, X₁, and Z₁ are as defined in the compound of Formula V. The stereocenter *1 of the catalyst according to Formula Vb1 is in (R) and/or (S) configuration.

According to certain embodiments, the catalyst, particularly the catalyst of Formula Va is represented by the following Formula Va1, by the following Formula Va'1, or by the following Formula Va2:

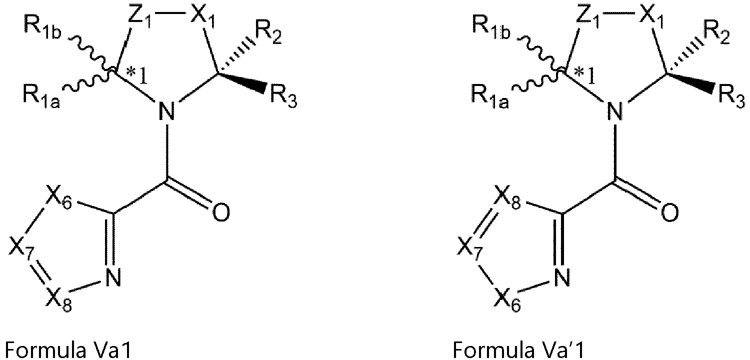

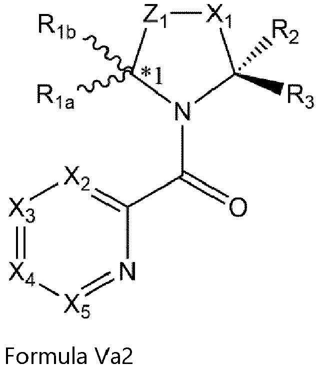

In the compound of Formula Va1, R₁ₐ, R_{1b}, R₂, R₃, X₁, and Z₁ are as defined in the compound of Formula Va. The stereocenter *1 of the catalyst according to Formula Va1 is in (R) and/or (S) configuration.

In the compound of Formula Va1, X₆ is selected from O, S, NR₁₃ and CR₁₃ₐR_{13b}.

In the compound of Formula Va1, R₁₃ is chosen from H, linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂. According to certain embodiments, R₁₃ is H, a linear alkyl group with 1 to 10, e.g. 1 to 6, C atoms, e.g. a methyl or ethyl group, an aromatic group with 6 to 20 C atoms, e.g.6 to 10 or 6 C atoms, e.g. a phenyl group, or an aralkyl group with 7 to 20 C atoms, e.g. 7 to 11 C atoms, e.g. 7 C atoms, e.g. a benzyl group, in the formulas comprising R₁₃.

In the compound of Formula Va1, R₁₃ₐ and R_{13b} are the same or different and can be chosen from H, linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂.

In the compound of Formula Va1, X₇ and Xs are independently selected from N and CR₆; with the provisio that when both X₇ and X₈ are represented by CR₆, the two R₆ can be the same or different and/or the two R₆ can form a ring, and with the provisio that if at least one of X₇ and X₈ is represented by CR₆, the at least one R₆ can form a ring with R₁₃, R₁₃ₐ and/or R_{13b}, preferably with the provisio that at least one of X₇ and X₈ is CR₆ and/or X₆ is CR₁₃ₐR_{13b}. According to preferred embodiments, X₇ and X₈ are independently selected from N and CR₆; with the provisio that when both X₇ and X₈ are represented by CR₆, the two R₆ can be the same or different, preferably with the provisio that at least one of X₇ and X₈ is CR₆ and/or X₆ is CR₁₃ₐR_{13b}. A formation of a ring is not preferable.

In the compound of Formula Va1, R₆ is chosen from H; F; Cl; Br; I; alkoxy groups with 1 to 20 C atoms; pseudohalogen groups; OH; NO₂; NH₂; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂.

In the compound of Formula Va'1, R₁ₐ, R_{1b}, R₂, R₃, X₁, X₆, X₇, X₈, and Z₁ are as defined in the compound of Formula Va1. The stereocenter *1 of the catalyst according to Formula Va'1 is in (R) and/or (S) configuration.

In the compound of Formula Va2, R₁ₐ, R_{1b}, R₂, R₃, X₁, and Z₁ are as defined in the compound of Formula Va. The stereocenter *1 of the catalyst according to Formula Va2 is in (R) and/or (S) configuration.

In the compound of Formula Va2, X₂, X₃, X₄, and X₅ are independently selected from N and CR₆, with the provisio that when more than one of X₂, X₃, X₄, and X₅ is represented by CR₆, the more than one R₆ can be the same or different and/or more than one R₆ can form at least one ring, and with the provisio that at least one of X₂, X₃, X₄, and X₅ is CR₆. According to preferred embodiments, X₂, X₃, X₄, and X₅ are independently selected from N and CR₆, with the provisio that when more than one of X₂, X₃, X₄, and X₅ is represented by CR₆, the more than one R₆ can be the same or different, and with the provisio that at least one of X₂, X₃, X₄, and X₅ is CR₆. A formation of a ring is not preferable.

In the compound of Formula Va2, as well as any of the following formulas directly or indirectly derived therefrom, R₆ is chosen from H; F; Cl; Br; I; alkoxy groups with 1 to 20 C atoms; pseudohalogen groups; OH; NO₂; NH₂; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂.

According to certain embodiments, the catalyst, particularly the catalyst of Formula Vb1 is represented by the following Formula Vb1a, by the following Formula Vb1b, or by the following Formula Vb'1b, the catalyst of Formula Vb1a being a representation of the catalyst of Formula Va2, the catalyst of Formula Vb1b being a representation of the catalyst of Formula Va1, and the catalyst of Formula Vb'1b being a representation of the catalyst of Formula Va'1.

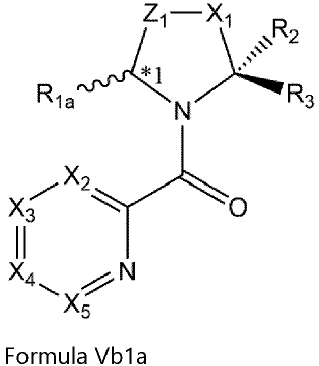

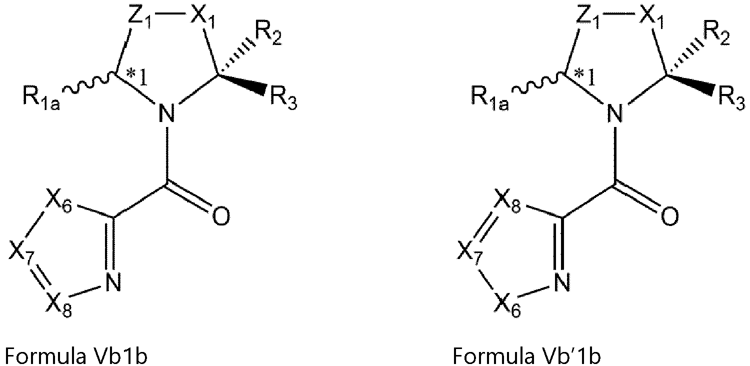

In the compound of Formula Vb1a, R₁ₐ, R₂, R₃, X₁, X₂, X₃, X₄, X₅, and Z₁ are as defined in the compound of Formula Va2. The stereocenter *1 of the catalyst according to Formula Vb1a is in (R) and/or (S) configuration.

In the compound of Formula Vb1b, R₁ₐ, R₂, R₃, X₁, X₆, X₇, X₈, and Z₁ are as defined in the compound of Formula Va1. The stereocenter *1 of the catalyst according to Formula Vb1b is in (R) and/or (S) configuration.

In the compound of Formula Vb'1b, R₁ₐ, R₂, R₃, X₁, X₆, X₇, X₈, and Z₁ are as defined in the compound of Formula Va'1. The stereocenter *1 of the catalyst according to Formula Vb'1b is in (R) and/or (S) configuration.

In the compounds of Formula IIb1-a, IIb1-a1, IIb1-a2, IIb1-a3, IIb1-a4, IIb1-a4', IIb1-a5, IIb1-a6, IIb1-a6', IIa-10, IIb'1-a, IIb'1-a1, IIb'1-a2, IIb'1-a3, IIb'1-a4, IIb'1-a4', IIb'1-a5, IIb'1-a6, IIb'1-a6', IIa'-10, IIIa2-c, IIIa3-a, IIIa2-c1, IIIa3-a1, IIIa2-c2, IIIa3-a2, IIIa2-c3, IIIa3-a3, IIIa2-c4, IIIa3-a4Ic-1, IV, IVa, IVb, IVa1a, IVa1b, IVa2, and IVa3, R₁₂ is particularly chosen from H, OH and OR₁₂ₐ, and R₁₂ₐ is preferably chosen from H;
linear alkyl groups with 1 to 10 C atoms that can optionally be substituted with at least one of F, Cl, Br, I, pseudohalogen groups, carboxylic acid ester groups with 1 to 10 C atoms that can optionally be substituted with at least one of F, Cl, Br, I, pseudohalogen groups, alkoxy groups with 1 to 10 C atoms, OH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 10 C atoms, OH, NO₂ and NH₂, carboxamide groups with 1 to 20 C atoms that can optionally be substituted with at least one of F, Cl, Br, I, pseudohalogen groups, alkoxy groups with 1 to 10 C atoms, OH, NO₂ and NH₂, alkoxy groups with 1 to 20 C atoms, OH, NO₂ and NH₂, e.g. a methyl group or ethyl group that can be carboxylated and/or partially or fully substituted with CN and/or F;
linear alkenyl and/or alkynyl groups with 2 to 10 C atoms that can optionally be substituted with at least one of F, Cl, Br, I, pseudohalogen groups, carboxylic acid ester groups with 1 to 10 C atoms that can optionally be substituted with at least one of F, Cl, Br, I, pseudohalogen groups, alkoxy groups with 1 to 10 C atoms, OH, NO₂ and NH₂, alkyl and/or (hetero)aryl oxycarbonyl groups with 1 to 10 C atoms that can optionally be substituted with at least one of F, Cl, Br, I, pseudohalogen groups, alkoxy groups with 1 to 10 C atoms, OH, NO₂ and NH₂, carboxamide groups with 1 to 10 C atoms that can optionally be substituted with at least one of F, Cl, Br, I, pseudohalogen groups, alkoxy groups with 1 to 10 C atoms, OH, NO₂ and NH₂, alkoxy groups with 1 to 10 C atoms, OH, NO₂ and NH₂, e.g. a propenyl or propinyl group;
aralkyl groups with 7 to 15 C atoms that can optionally be substituted with at least one of F, Cl, Br, I, pseudohalogen groups, carboxylic acid ester groups with 1 to 10 C atoms that can optionally be substituted with at least one of F, Cl, Br, I, pseudohalogen groups, alkoxy groups with 1 to 10 C atoms, OH, NO₂ and NH₂, alkyl and/or (hetero)aryl oxycarbonyl groups with 1 to 10 C atoms that can optionally be substituted with at least one of F, Cl, Br, I, pseudohalogen groups, alkoxy groups with 1 to 10 C atoms, OH, NO₂ and NH₂, carboxamide groups with 1 to 20 C atoms that can optionally be substituted with at least one of F, Cl, Br, I, pseudohalogen groups, alkoxy groups with 1 to 10 C atoms, OH, NO₂ and NH₂, alkoxy groups with 1 to 10 C atoms, OH, NO₂ and NH₂, e.g. a benzyl group;
alkyl heteroaromatic groups with 2 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, NO₂ and NH₂, alkoxy groups with 1 to 20 C atoms, OH, NO₂ and NH₂, e.g. a group based on an optionally substituted - e.g. alkyl substituted - tetrazole or triazole bound via an alkylene - e-g- methylene - linker;
heteroaromatic groups with at least one alkyl residue with 3 to 20 C atoms that can optionally be substituted with at least one of F, Cl, Br, I, pseudohalogen groups, carboxylic acid ester groups with 1 to 10 C atoms that can optionally be substituted with at least one of F, Cl, Br, I, pseudohalogen groups, alkoxy groups with 1 to 10 C atoms, OH, NO₂ and NH₂, alkyl and/or (hetero)aryl oxycarbonyl groups with 1 to 10 C atoms that can optionally be substituted with at least one of F, Cl, Br, I, pseudohalogen groups, alkoxy groups with 1 to 10 C atoms, OH, NO₂ and NH₂, carboxamide groups with 1 to 10 C atoms that can optionally be substituted with at least one of F, Cl, Br, I, pseudohalogen groups, alkoxy groups with 1 to 10 C atoms, OH, NO₂ and NH₂, alkoxy groups with 1 to 10 C atoms, OH, NO₂ and NH₂;
and SiR₂₃R₂₄R₂₅;
wherein R₂₃, R₂₄, and R₂₅ are the same or different and are selected from H, linear alkyl groups with 1 to 4 C atoms that can optionally be substituted with at least one of F, Cl, Br, I, pseudohalogen groups, alkoxy groups with 1 to 20 C atoms, OH, NO₂ and NH₂, branched alkyl group with 3 to 6 C atoms that can optionally be substituted with at least one of F, Cl, Br, I, pseudohalogen groups, alkoxy groups with 1 to 20 C atoms, OH, NO₂ and NH₂, aromatic groups with 6 to 12 C atoms that can optionally be substituted with at least one of F, Cl, Br, I, pseudohalogen groups, alkoxy groups with 1 to 20 C atoms, OH, NO₂ and NH₂, and aralkyl groups with 7 to 10 C atoms that can optionally be substituted with at least one of F, Cl, Br, I, pseudohalogen groups, alkoxy groups with 1 to 20 C atoms, OH, NO₂ and NH₂, preferably wherein R₂₃, R₂₄, and R₂₅ are not all hydrogen at the same time and/or wherein R₂₃, R₂₄, and R₂₅ are not all methyl at the same time, further preferably wherein all of R₂₃, R₂₄, and R₂₅ are ethyl.
R₁₂ₐ in these formulas is particularly preferably chosen from H;
linear alkyl groups with 1 to 10 C atoms that can optionally be substituted with at least one of F, Cl, Br, I, pseudohalogen groups, carboxylic acid ester groups with 1 to 10 C atoms that can optionally be substituted with at least one of F, Cl, Br, I, pseudohalogen groups, alkoxy groups with 1 to 10 C atoms, OH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 10 C atoms, OH, NO₂ and NH₂, carboxamide groups with 1 to 20 C atoms that can optionally be substituted with at least one of F, Cl, Br, I, pseudohalogen groups, alkoxy groups with 1 to 10 C atoms, OH, NO₂ and NH₂, alkoxy groups with 1 to 20 C atoms, OH, NO₂ and NH₂, e.g. a methyl group or ethyl group that can be carboxylated and/or partially or fully substituted with CN and/or F;
linear alkenyl and/or alkynyl groups with 2 to 10 C atoms that can optionally be substituted with at least one of F, Cl, Br, I, pseudohalogen groups, carboxylic acid ester groups with 1 to 10 C atoms that can optionally be substituted with at least one of F, Cl, Br, I, pseudohalogen groups, alkoxy groups with 1 to 10 C atoms, OH, NO₂ and NH₂, alkyl and/or (hetero)aryl oxycarbonyl groups with 1 to 10 C atoms that can optionally be substituted with at least one of F, Cl, Br, I, pseudohalogen groups, alkoxy groups with 1 to 10 C atoms, OH, NO₂ and NH₂, carboxamide groups with 1 to 10 C atoms that can optionally be substituted with at least one of F, Cl, Br, I, pseudohalogen groups, alkoxy groups with 1 to 10 C atoms, OH, NO₂ and NH₂, alkoxy groups with 1 to 10 C atoms, OH, NO₂ and NH₂, e.g. a propenyl or propinyl group;
aralkyl groups with 7 to 15 C atoms that can optionally be substituted with at least one of F, Cl, Br, I, pseudohalogen groups, carboxylic acid ester groups with 1 to 10 C atoms that can optionally be substituted with at least one of F, Cl, Br, I, pseudohalogen groups, alkoxy groups with 1 to 10 C atoms, OH, NO₂ and NH₂,
alkyl and/or (hetero)aryl oxycarbonyl groups with 1 to 10 C atoms that can optionally be substituted with at least one of F, Cl, Br, I, pseudohalogen groups, alkoxy groups with 1 to 10 C atoms, OH, NO₂ and NH₂, carboxamide groups with 1 to 20 C atoms that can optionally be substituted with at least one of F, Cl, Br, I, pseudohalogen groups, alkoxy groups with 1 to 10 C atoms, OH, NO₂ and NH₂, alkoxy groups with 1 to 10 C atoms, OH, NO₂ and NH₂, e.g. a benzyl group; and
alkyl heteroaromatic groups with 2 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, NO₂ and NH₂, alkoxy groups with 1 to 20 C atoms, OH, NO₂ and NH₂, e.g. a group based on an optionally substituted - e.g. alkyl substituted - tetrazole or triazole bound via an alkylene - e-g- methylene - linker; preferably aralkyl groups with 7 to 15 C atoms that can optionally be substituted with at least one of F, Cl, Br, I, pseudohalogen groups, carboxylic acid ester groups with 1 to 10 C atoms that can optionally be substituted with at least one of F, Cl, Br, I, pseudohalogen groups, alkoxy groups with 1 to 10 C atoms, OH, NO₂ and NH₂,

According certain embodiments, the catalyst, particularly the catalyst of Formula Ia, is selected from the following Catalysts A1 to A4, preferably from Catalysts A1 and A3, and is further preferably Catalyst A1:

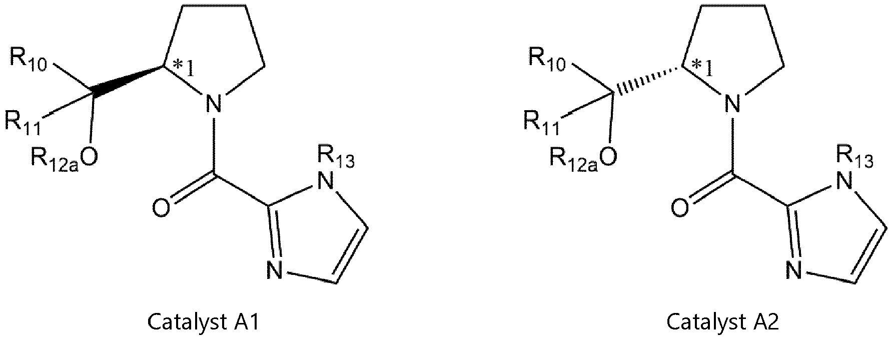

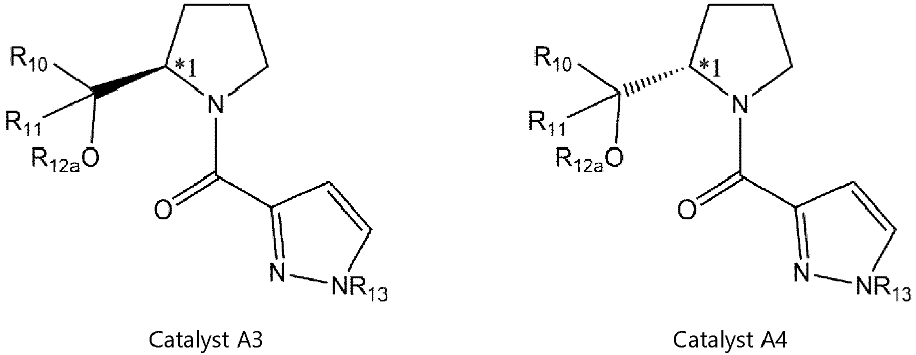

In Catalysts A1, A2, A3 and A4, R₁₀, R₁₁, R₁₂, and R₁₃ can have the meaning as above.

In Catalysts A1, A2, A3 and A4,
R₁₃ is particularly selected from H, alkyl residues with 1 to 4 C atoms, aryl residues with 6 to 12 C atoms, and aralkyl residues with 7 to 14 C atoms, preferably selected from H, methyl, ethyl, phenyl and benzyl;
R₁₀ and R₁₁ are the same or different, but preferably are the same, and are particularly selected from optionally substituted aryl residues with 6 to 12 C atoms, optionally substituted aralkyl residues with 7 to 14 C atoms, and optionally substituted alkylaryl residues with 7 to 14 20 C atoms that can comprise one or more alkyl group attached to the arlyene unit, wherein the optional substituents can be chosen from halogens like F, Cl, Br, I, and pseudohalogen groups, preferably wherein R₁₀ and R₁₁ are the same and are selected from optionally substituted phenyl, tolyl, naphthyl or xylenyl (e.g. 3,5-xylenyl) groups; and
R₁₂ₐ is selected from H, alkyl groups with 1 to 4 C atoms, alkenyl groups with 1 to 4 C atoms, alkynyl groups with 1 to 4 C atoms, optionally substituted aryl residues with 6 to 12 C atoms, and optionally substituted aralkyl residues with 7 to 14 C atoms, wherein the optional substituents can be chosen from halogens like F, Cl, Br, I, and pseudohalogen groups, preferably methyl, ethyl, 2-propynyl (= propargyl), benzyl and pentafluorobenzyl.

According certain embodiments, the catalyst, particularly the catalyst of Formula Ia, is selected from the following Catalysts A-I to A-LXVI:

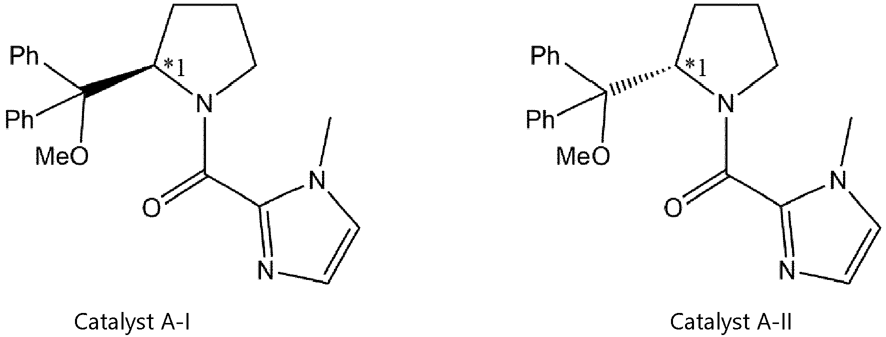

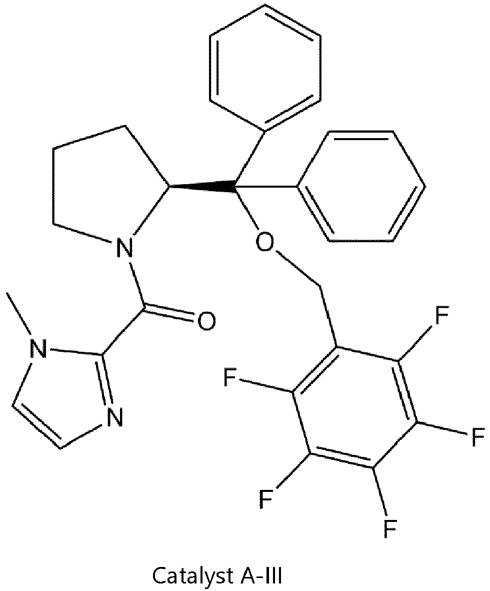

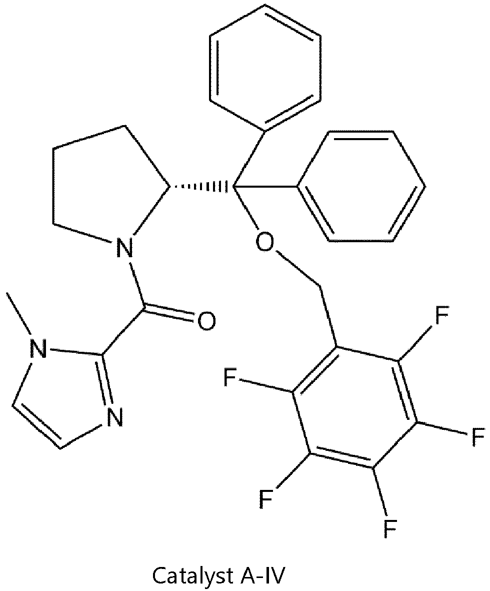

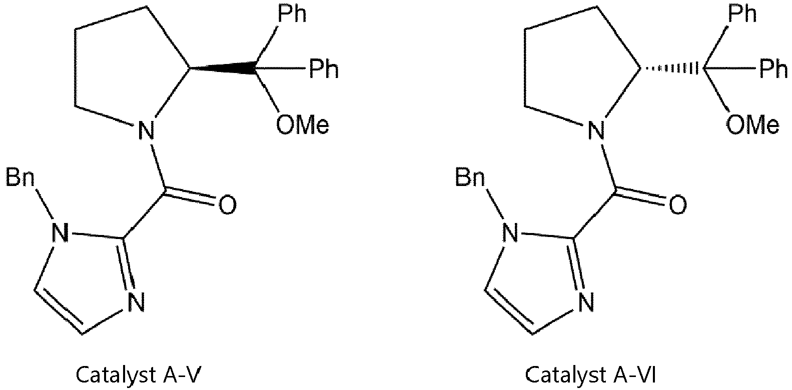

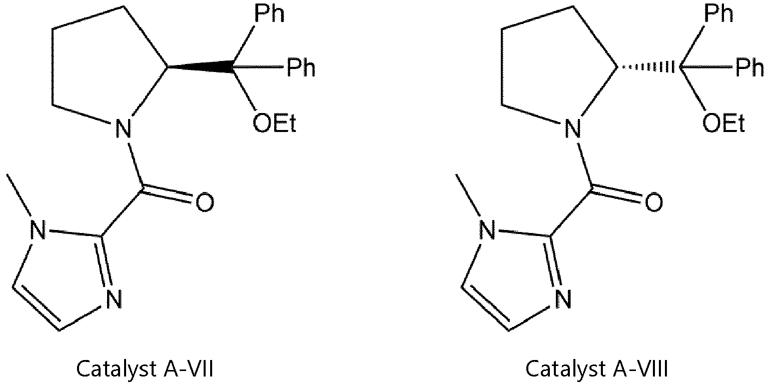

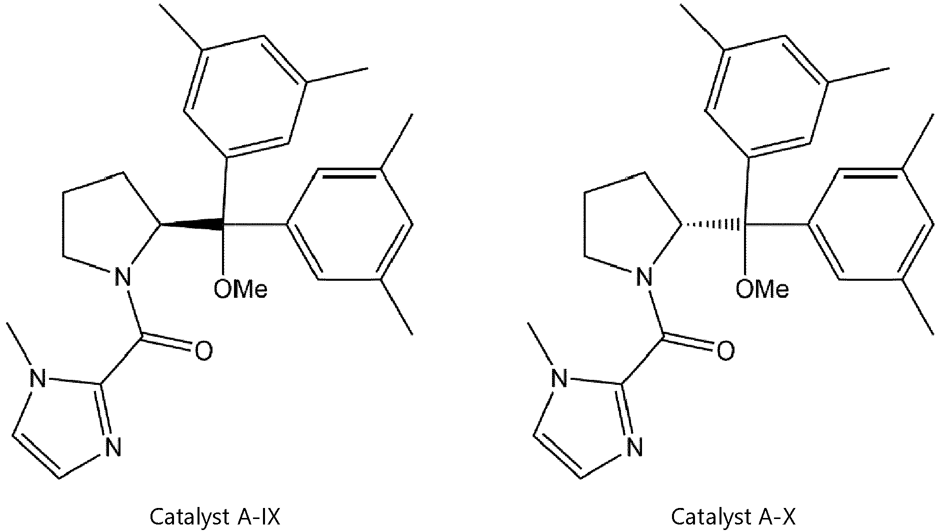

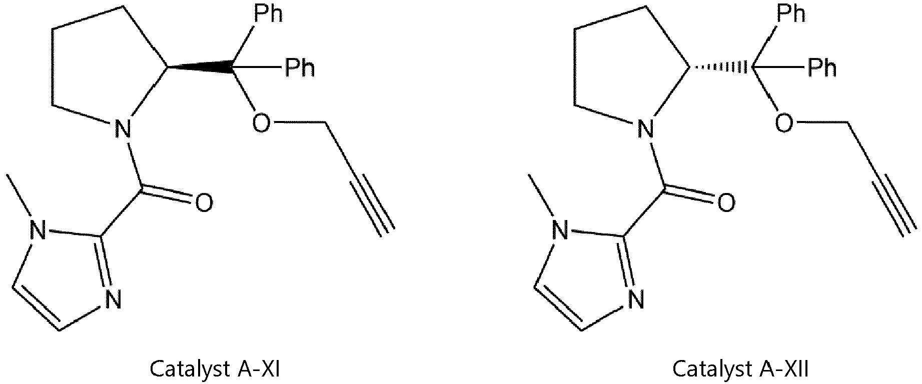

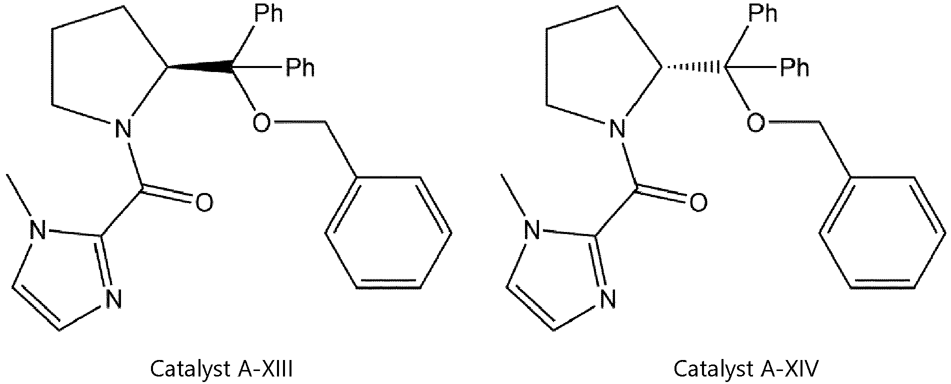

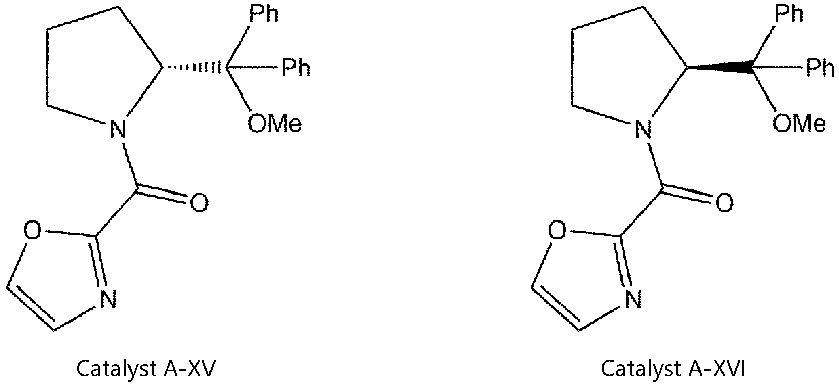

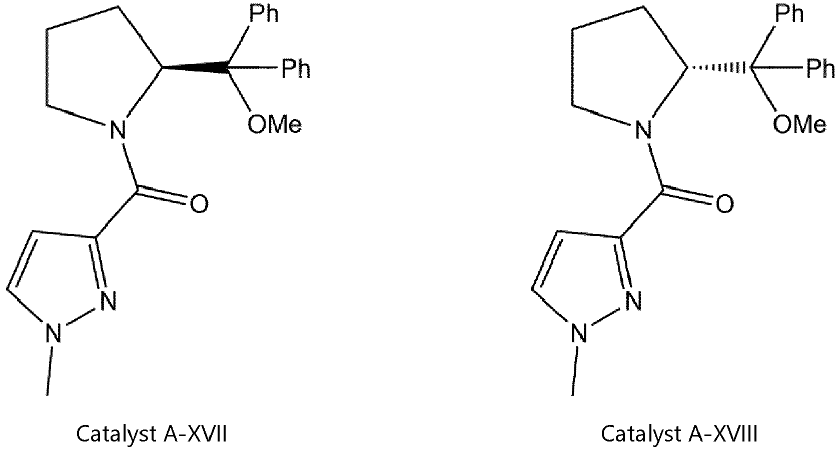

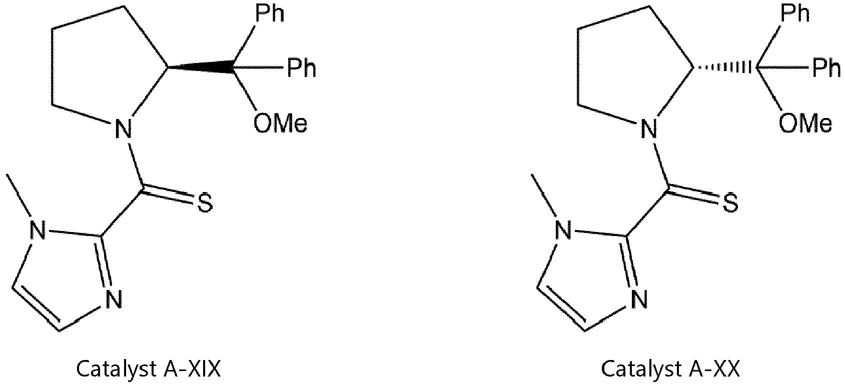

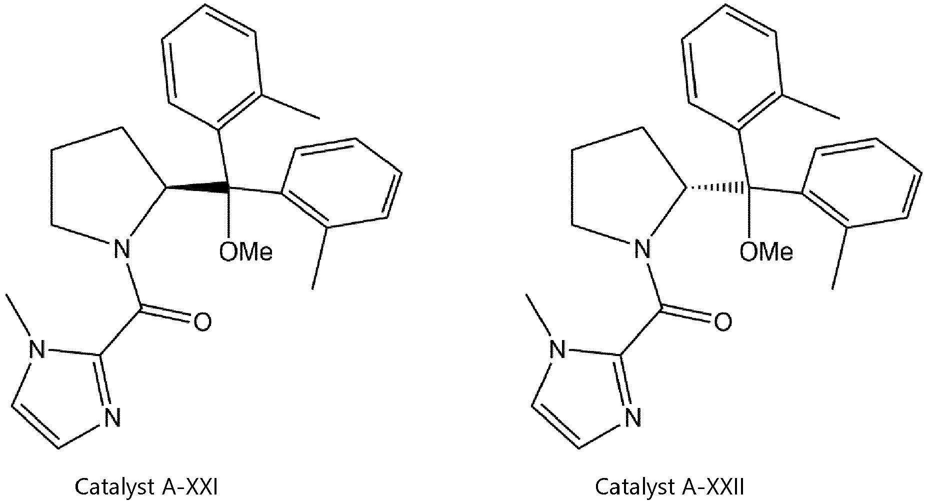

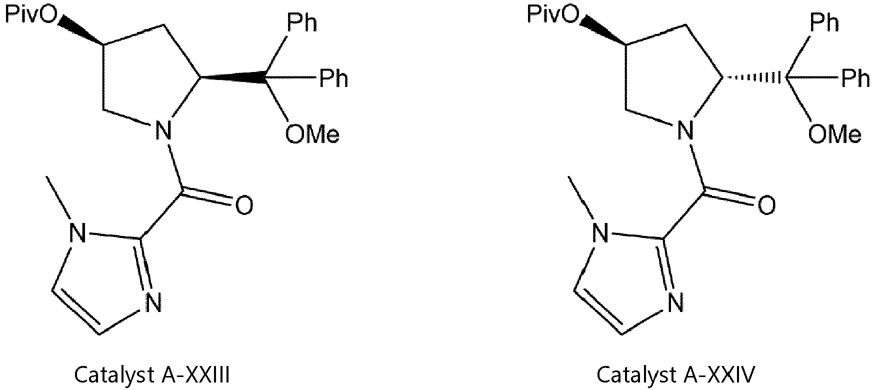

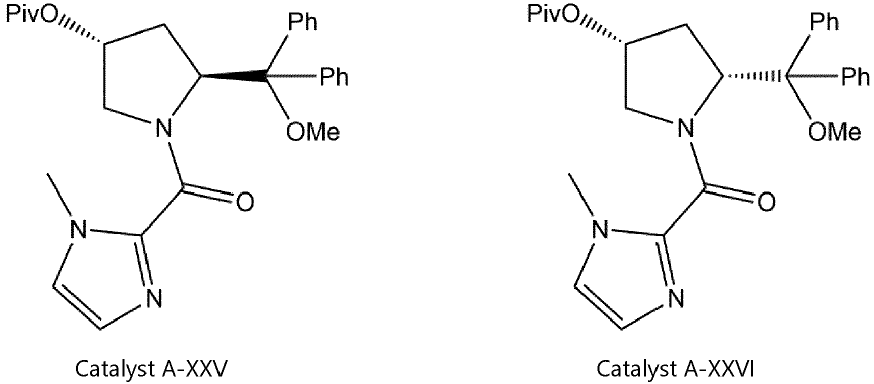

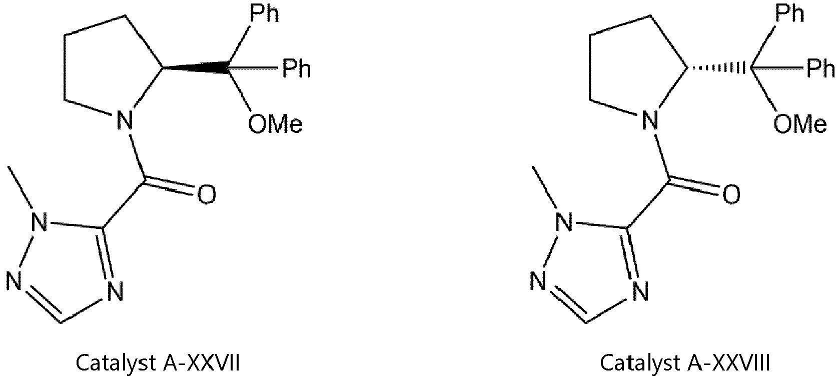

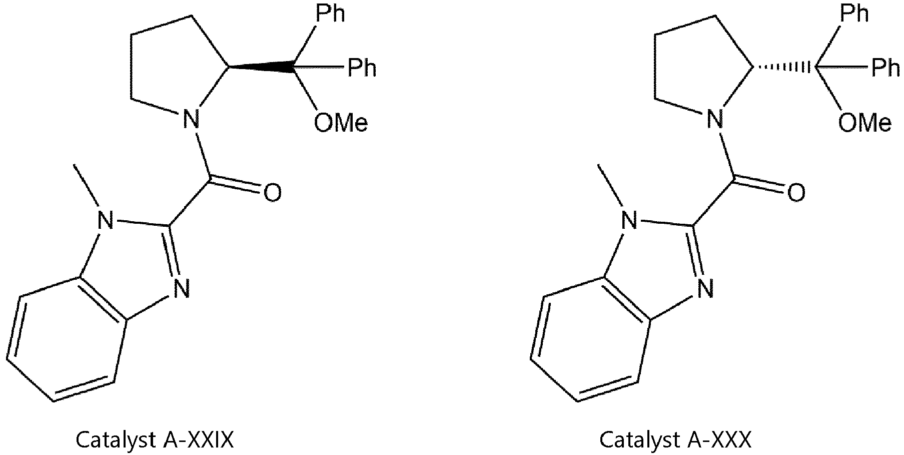

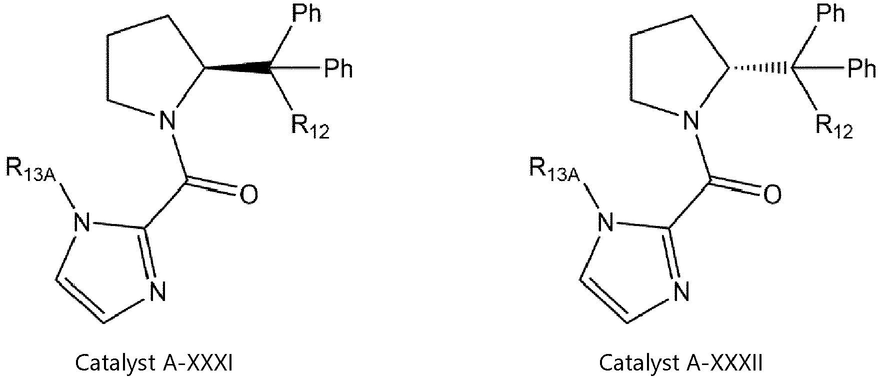

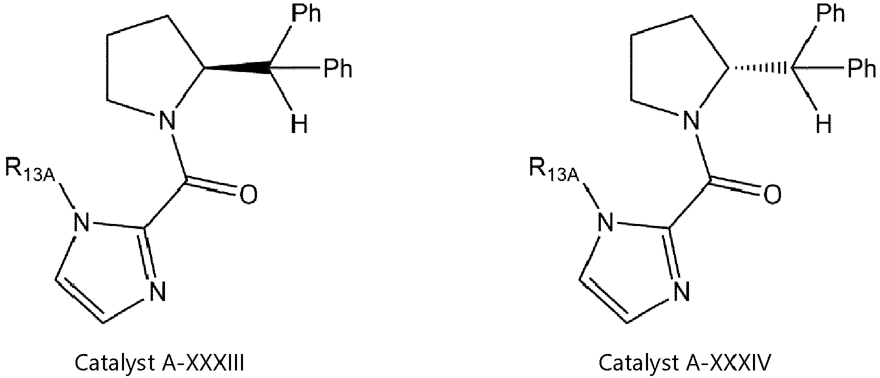

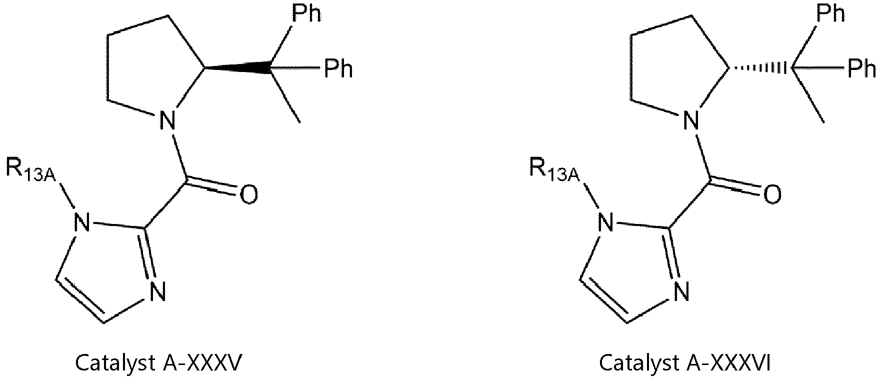

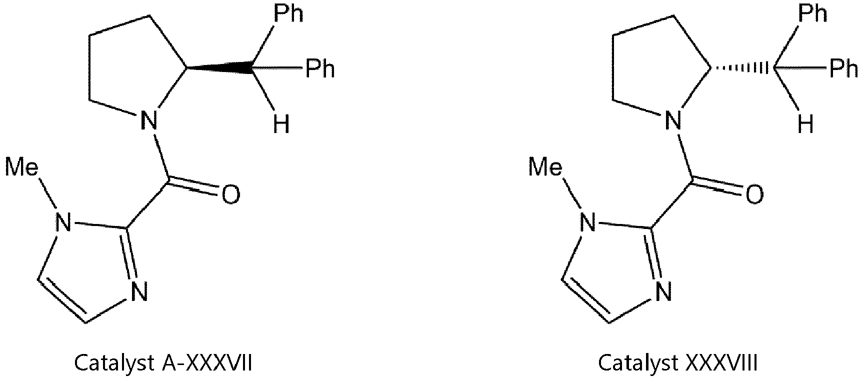

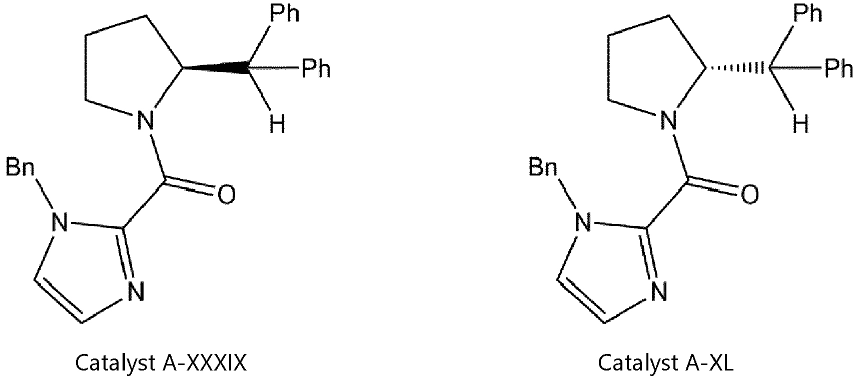

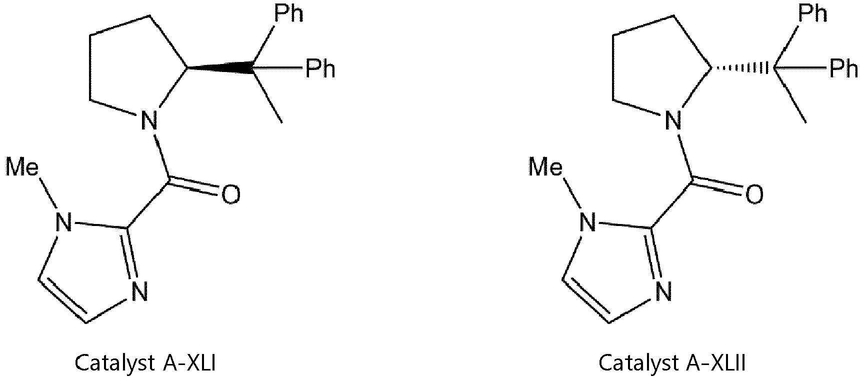

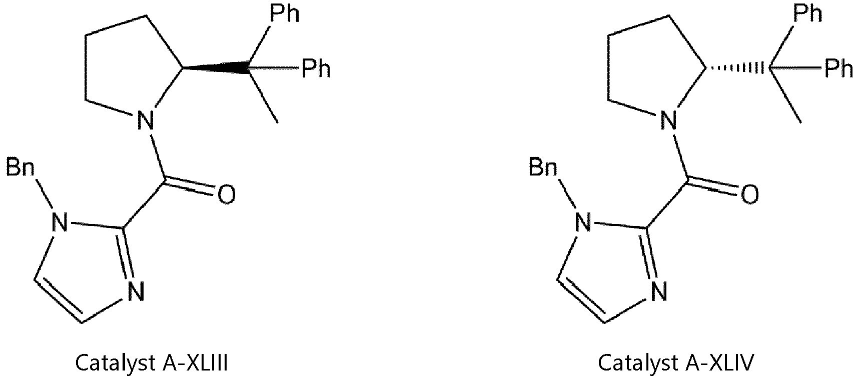

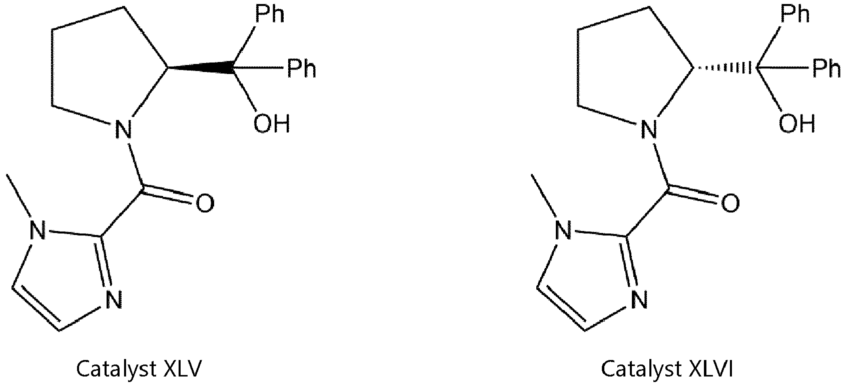

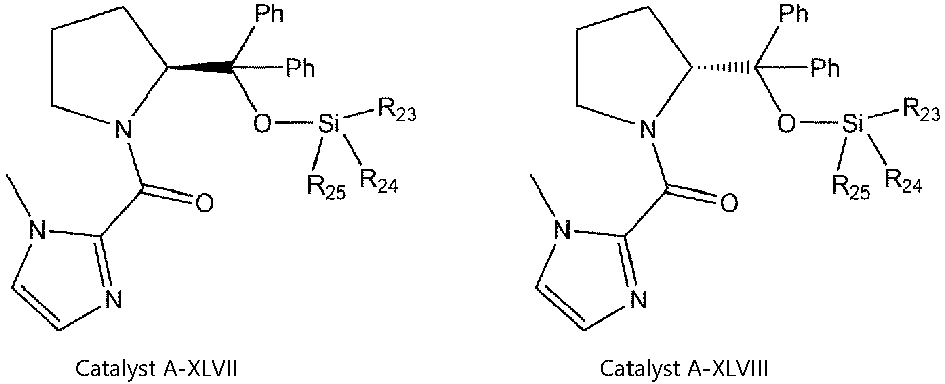

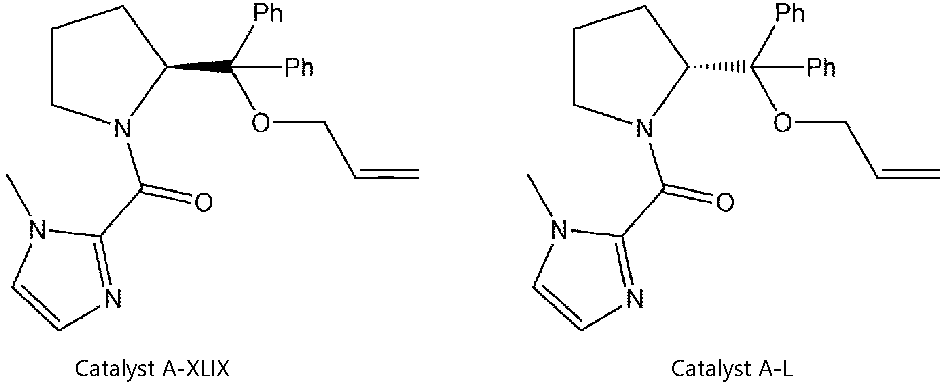

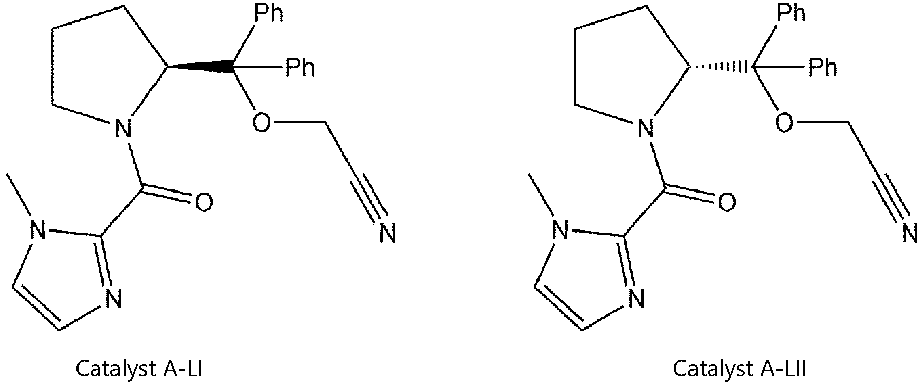

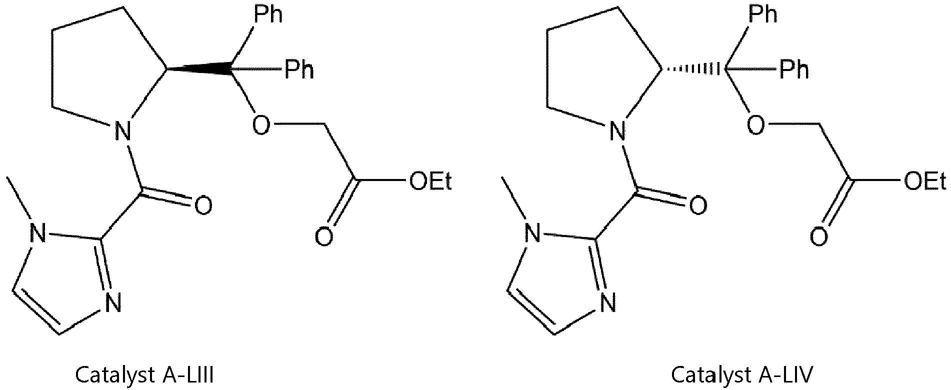

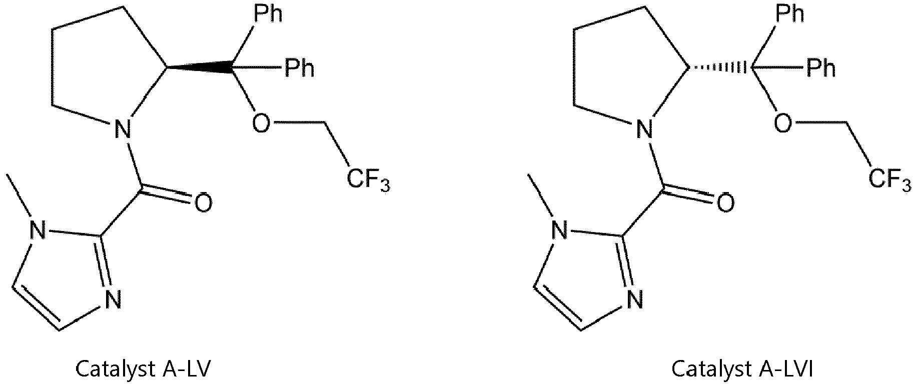

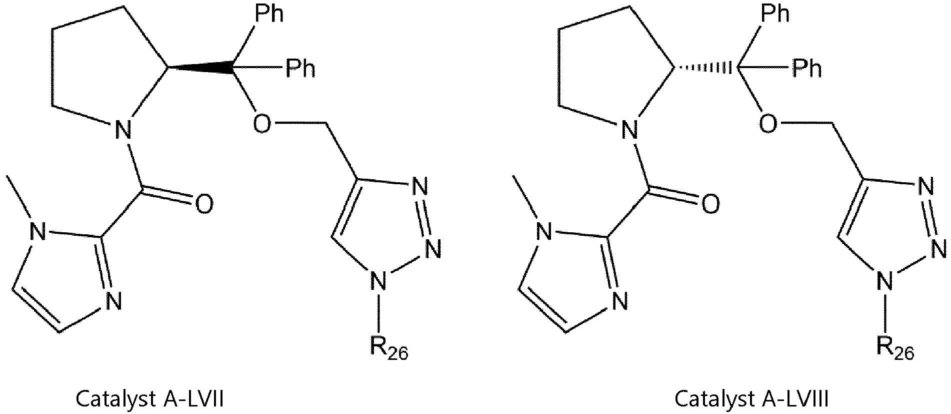

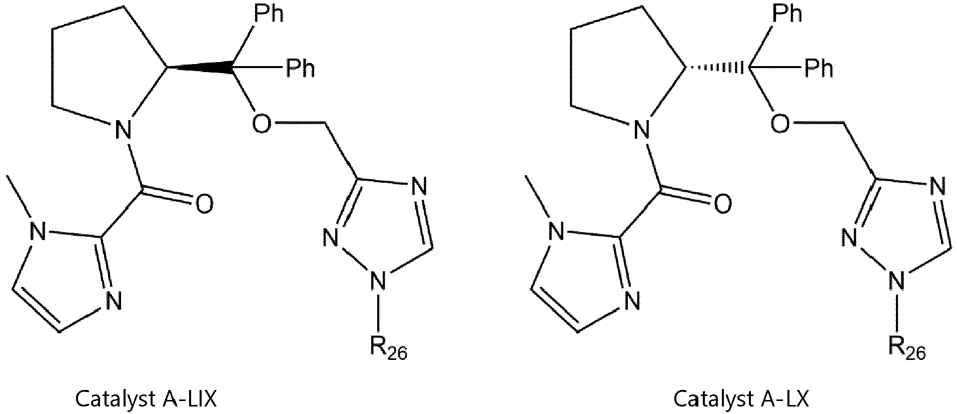

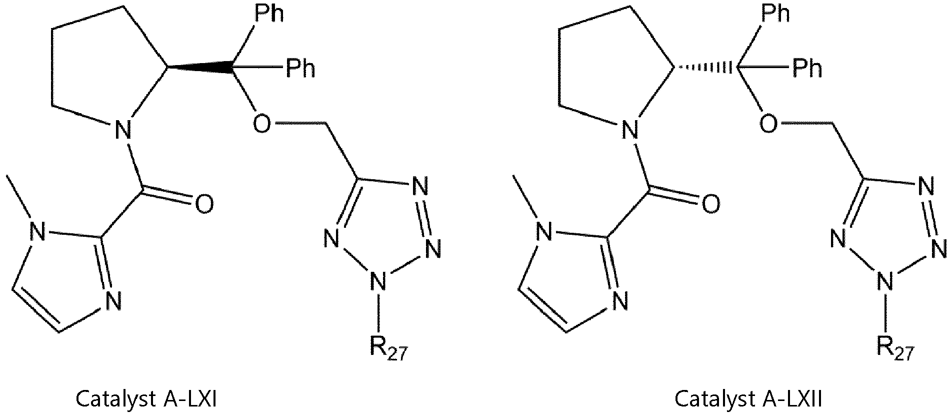

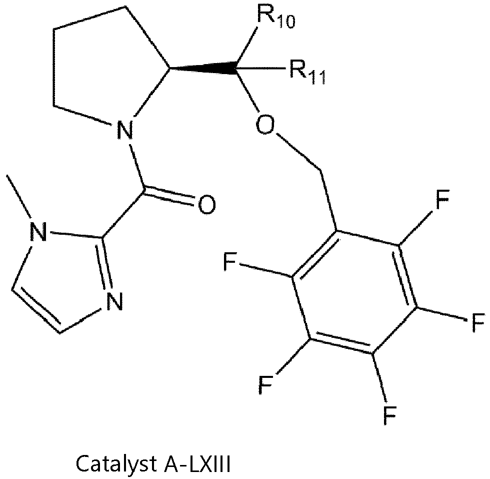

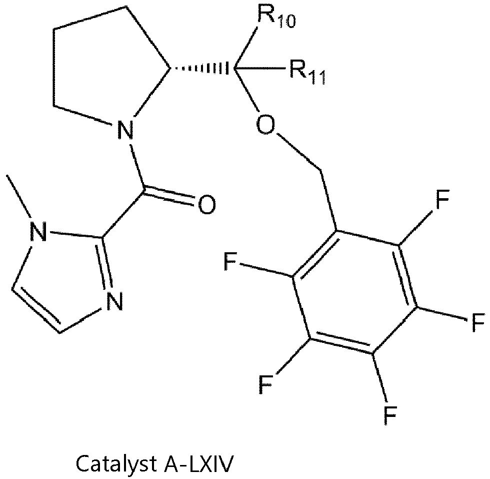

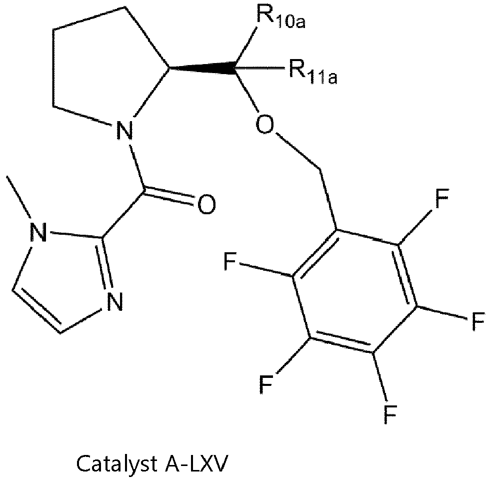

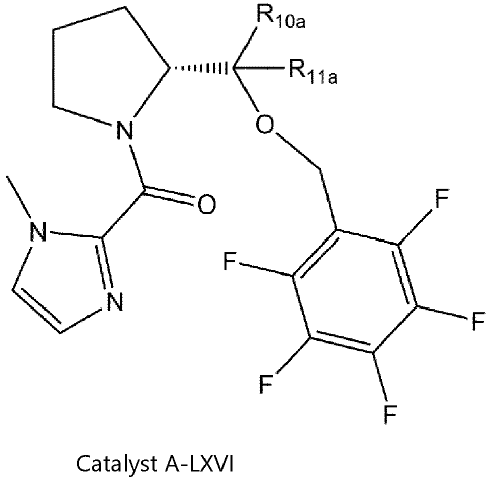

In Catalyst A-XXXI and Catalyst A-XXXII, R₁₂ is as defined above, and R_{13A} is particularly chosen from H, alkyl residues with 1 to 4 C atoms, aryl residues with 6 to 12 C atoms, and aralkyl residues with 7 to 14 C atoms, preferably selected from H, methyl, ethyl, phenyl and benzyl. In Catalysts A-XXXIII, A-XXXIV, A-XXXV and A-XXXVI, R_{13A} is particularly chosen from H, alkyl residues with 1 to 4 C atoms, aryl residues with 6 to 12 C atoms, and aralkyl residues with 7 to 14 C atoms, preferably selected from H, methyl, ethyl, phenyl and benzyl.

In Catalyst A-XLVII and Catalyst A-XLVIII, R₂₃, R₂₄, and R₂₅ are the same or different and as defined above. According to certain embodiments, R₂₃ and R₂₄ are the same and R₂₅ is different. According to certain embodiments, R₂₃, R₂₄, and R₂₅ are selected from H, linear alkyl groups with 1 to 4 C atoms that can optionally be substituted with at least one of F, Cl, Br, I, pseudohalogen groups, alkoxy groups with 1 to 20 C atoms, OH, NO₂ and NH₂, and branched alkyl group with 3 to 6 C atoms that can optionally be substituted with at least one of F, Cl, Br, I, pseudohalogen groups, alkoxy groups with 1 to 20 C atoms, OH, NO₂ and NH₂. According to certain embodiments, R₂₃ and R₂₄ are Me, Et, or tBu (tert-butyl), e.g. Me, and R₂₅ is Me, Et, or tBu, e.g. tBu. According to certain embodiments, R₂₃ and R₂₄ are Me and R₂₅ is tBu.

In Catalyst A-LVII, Catalyst A-LVIII, Catalyst A-LIX and Catalyst LX, R₂₆ is particularly chosen from H, alkyl residues with 1 to 4 C atoms, aryl residues with 6 to 12 C atoms, and aralkyl residues with 7 to 14 C atoms.

In Catalyst A-LXI and Catalyst A-LXII, R₂₇ is particularly chosen from H, alkyl residues with 1 to 4 C atoms, aryl residues with 6 to 12 C atoms, and aralkyl residues with 7 to 14 C atoms.

In Catalyst A-LXIII and Catalyst A-LXIV, R₁₀ and R₁₁ are as defined above.

In Catalyst A-LXV and Catalyst A-LXVI, R₁₀ₐ and R₁₁ₐ are the same or different, preferably the same, and are selected from the group consisting of optionally substituted aryl residues with 6 to 12 C atoms, optionally substituted aralkyl residues with 7 to 14 C atoms, and optionally substituted alkylaryl residues with 7 to 14 20 C atoms that can comprise one or more alkyl group attached to the arlyene unit, wherein the optional substituents can be chosen from halogens like F, Cl, Br, I, and pseudohalogen groups, preferably wherein R₁₀ and R₁₁ are the same and are selected from optionally substituted phenyl, tolyl, naphthyl or xylenyl groups.

According to certain embodiments, the catalyst is selected from the group consisting of Catalyst A-I, Catalyst A-II, Catalyst A-III, Catalyst A-IV, Catalyst A-V, Catalyst A-VI, Catalyst A-VII, Catalyst A-VIII, Catalyst A-IX, Catalyst A-X, Catalyst A-XI, Catalyst A-XII, Catalyst A-XIII, and Catalyst A-XIV.

According to certain embodiments, the catalyst is selected from the group consisting of Catalyst A-I, Catalyst A-III, Catalyst A-V, Catalyst A-VII, Catalyst A-IX, Catalyst A-XI, and Catalyst A-XIII.

According to certain embodiments, the catalyst is selected from the group consisting of Catalyst A-II, Catalyst A-IV, Catalyst A-VI, Catalyst A-VIII, Catalyst A-X, Catalyst A-XII, and Catalyst A-XIV.

According to certain embodiments, the catalyst is selected from the group consisting of Catalyst A-I, Catalyst A-III, Catalyst A-V, Catalyst A-VII, Catalyst A-IX, Catalyst A-XI, Catalyst A-XIII, Catalyst A-XV, Catalyst A-XVII, Catalyst A-XIX, Catalyst A-XXI, Catalyst A-XXIII, Catalyst A-XXV, Catalyst A-XXVII, Catalyst A-XXIX, Catalyst A-XXXI, Catalyst A-XXXIII, Catalyst A-XXXV, Catalyst A-XXXVII, Catalyst A-XXXIX, Catalyst A-XLI, Catalyst A-XLIII, Catalyst A-XLV, Catalyst A-XLVII, Catalyst A-XLIX, Catalyst A-LI, Catalyst A-LIII, Catalyst A-LV, Catalyst A-LVII, Catalyst A-LIX, and Catalyst A-LXI. According to certain embodiments, the catalyst is Catalyst A-LXIII, e.g. Catalyst A-XXV.

According to certain embodiments, the catalyst is selected from the group consisting of Catalyst A-II, Catalyst A-IV, Catalyst A-VI, Catalyst A-VIII, Catalyst A-X, Catalyst A-XII, Catalyst A-XIV, Catalyst A-XVI, Catalyst A-XVIII, Catalyst A-XX, Catalyst A-XXII, Catalyst A-XXIV, Catalyst A-XXVI, Catalyst A-XXVIII, Catalyst A-XXX, Catalyst A-XXXII, Catalyst A-XXXIV, Catalyst A-XXXVI, Catalyst A-XXXVIII, Catalyst A-XL, Catalyst A-XLII, Catalyst A-XLIV, Catalyst A-XLVI, Catalyst A-XLVIII, Catalyst A-L, Catalyst A-LII, Catalyst A-LIV, Catalyst A-LVI, Catalyst A-LVIII, Catalyst A-LX, and Catalyst A-LXII. According to certain embodiments, the catalyst is Catalyst A-LXIV, e.g. Catalyst A-XXVI.

According to certain embodiments, the catalyst, particularly the catalyst of Formula Ib, is selected from the following Catalysts B1 to B22.

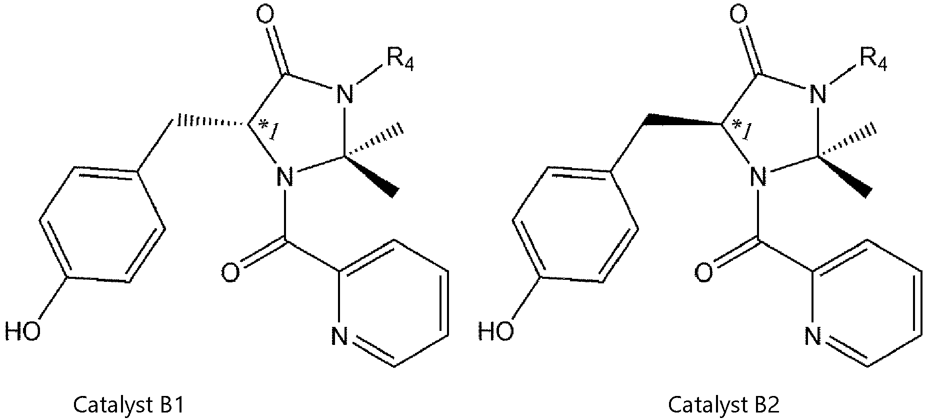

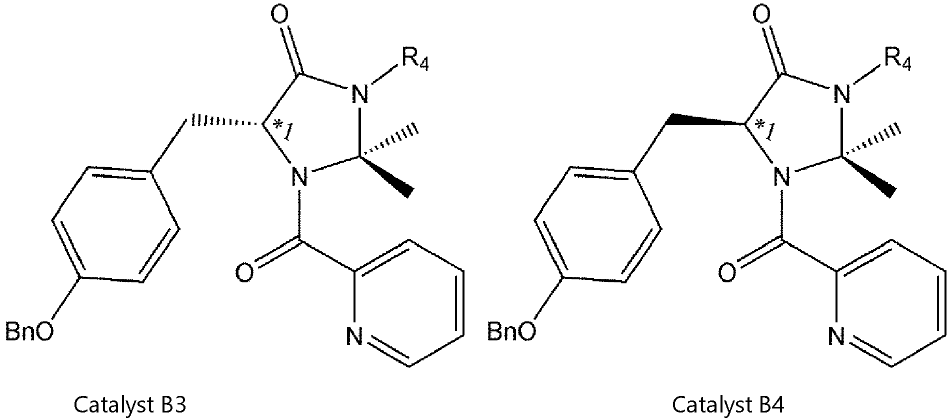

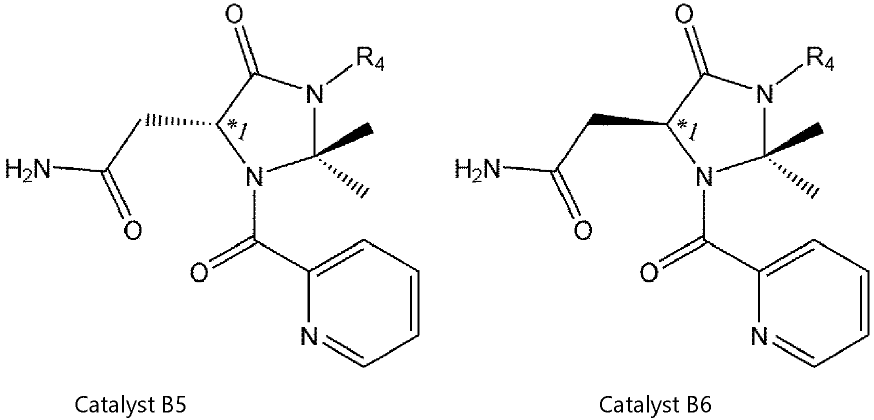

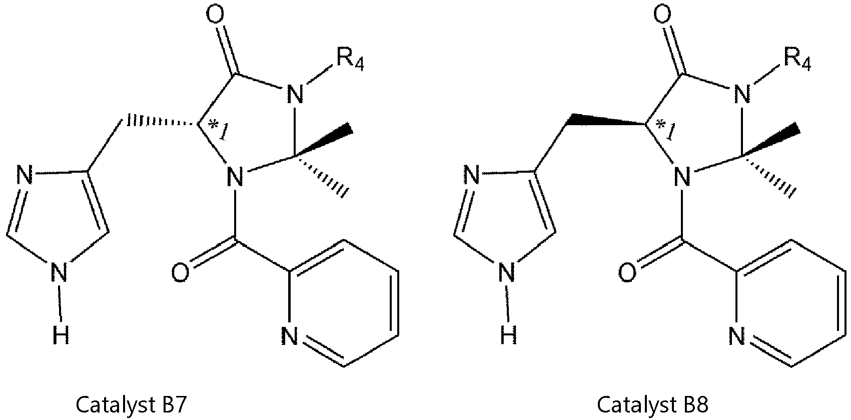

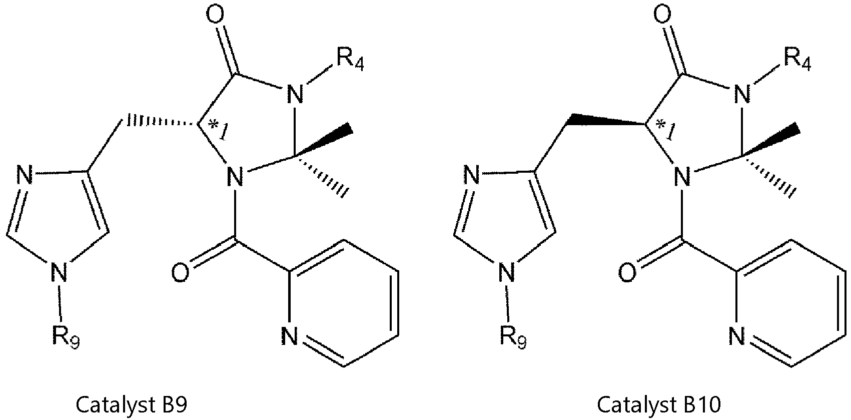

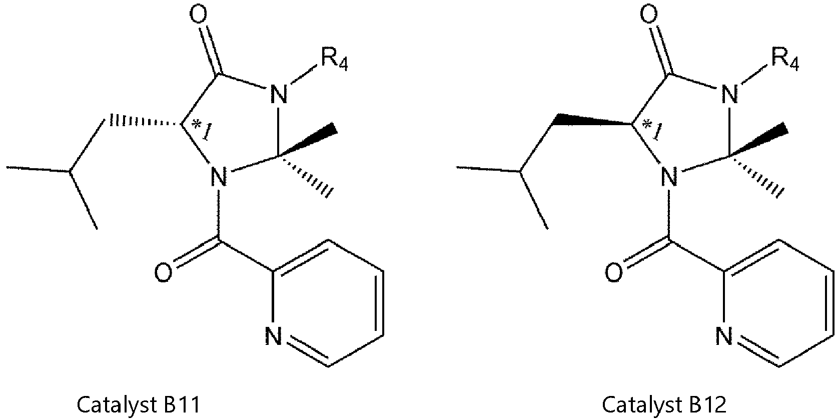

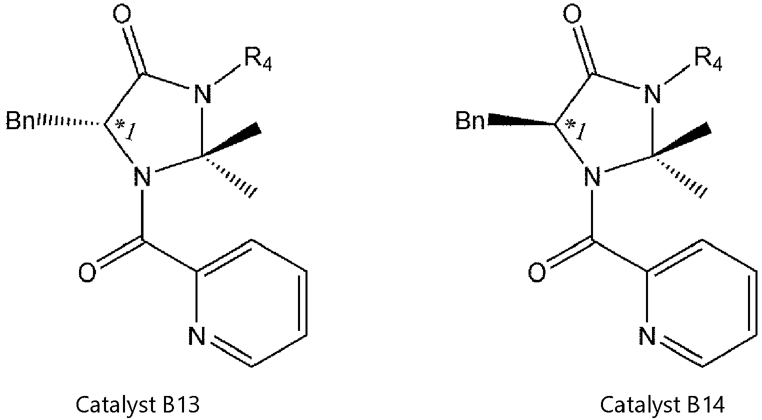

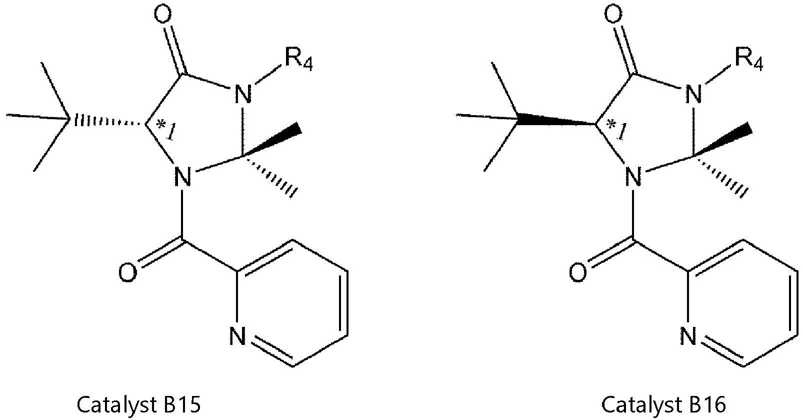

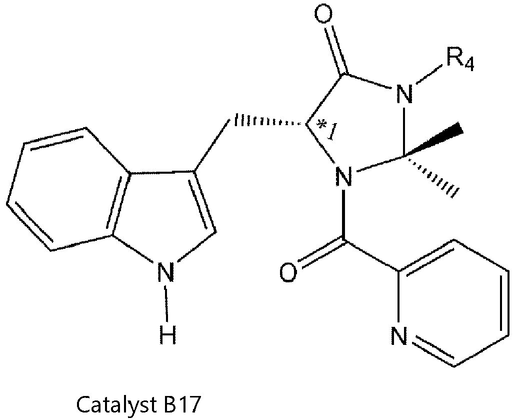

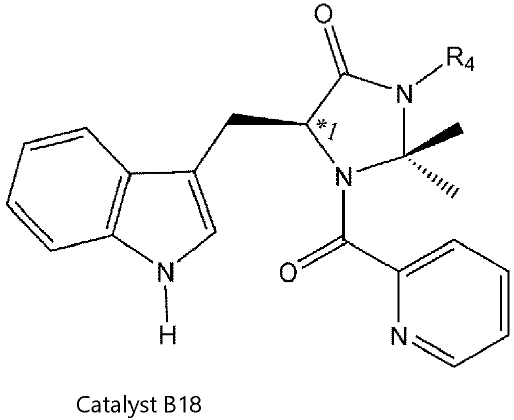

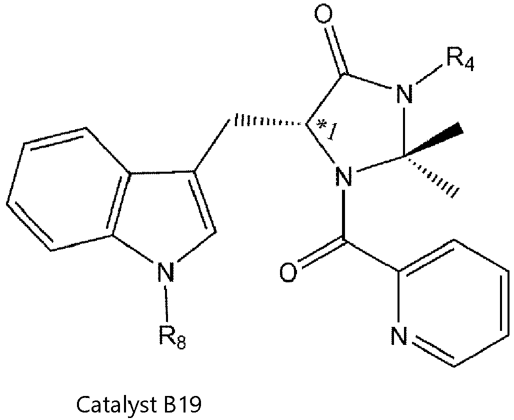

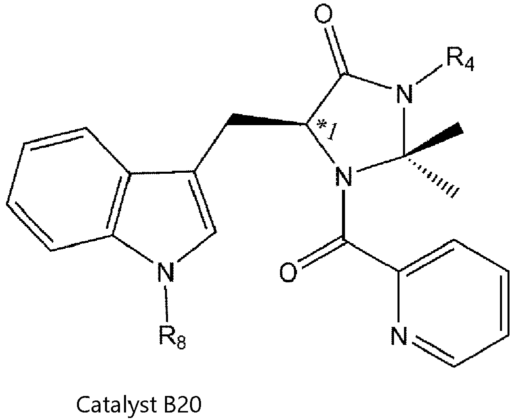

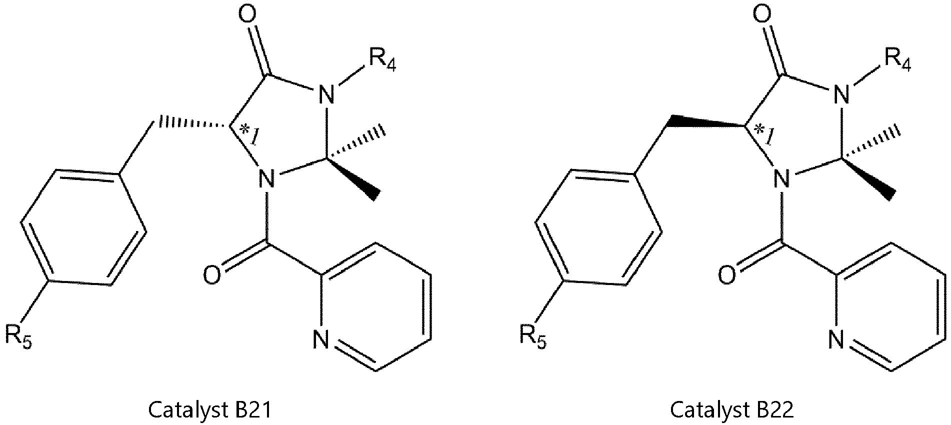

In the structural formulae of Catalysts B1 to B22, R₄ is as defined above, and is particularly a linear alkyl group with 1 to 10 carbon atoms that can be substituted with at least one of F, Cl, Br, I, pseudohalogen groups, alkoxy groups with 1 to 20 C atoms, OH, NO₂ and/or NH₂, preferably an alkyl group with 1 to 6 C atoms, particularly 1 to 4 C atoms, e.g. a butyl group. In the structural formulae of Catalysts B19 and B20, R₈ can be chosen from linear, alkyl groups with 1 to 10 carbon atoms that can be substituted with at least one of F, Cl, Br, I, pseudohalogen groups, alkoxy groups with 1 to 20 C atoms, OH, NO₂ and/or NH₂; F; Cl; Br; I; pseudohalogen groups; alkoxy groups with 1 to 20 C atoms; OH; NO₂; and NH₂.

In the structural formulae of Catalysts B9 and B10, R₉ can be chosen from linear, alkyl groups with 1 to 10 carbon atoms that can be substituted with at least one of F, Cl, Br, I, pseudohalogen groups, alkoxy groups with 1 to 20 C atoms, OH, NO₂ and/or NH₂; F; Cl; Br; I; pseudohalogen groups; alkoxy groups with 1 to 20 C atoms; OH; NO₂; and NH₂.

In the structural formulae of Catalyst B21 and B22, R₅ can be as defined above, and preferably can be selected from H, OH, and an alkoxy group with a linear, branched, cyclic, heteroaromatic and/or aromatic residue having 1 to 20 C atoms, further preferably selected from H, OH, and OBn.

According to certain embodiments, the catalyst is selected from Catalyst B1, Catalyst B3, Catalyst B5, Catalyst B7, Catalyst B9, Catalyst B11, Catalyst B13, Catalyst B15, Catalyst B17, Catalyst B19, and/or B21, preferably B19 and/or B21. According to certain embodiments, the catalyst is selected from Catalyst B2, Catalyst B4, Catalyst B6, Catalyst B8, Catalyst B10, Catalyst B12, Catalyst B14, Catalyst B16, Catalyst B18, Catalyst B20, and/or Catalyst B22, preferably Catalyst B20 and/or Catalyst B22.

According to certain embodiments, the catalyst is selected from the following Catalysts B-I to B-LXXII:

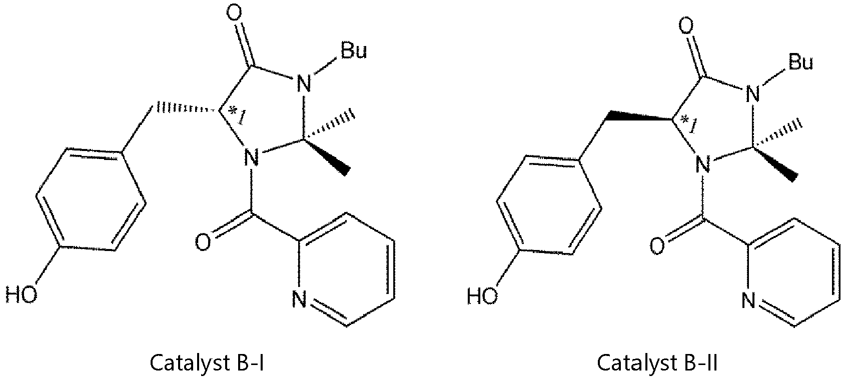

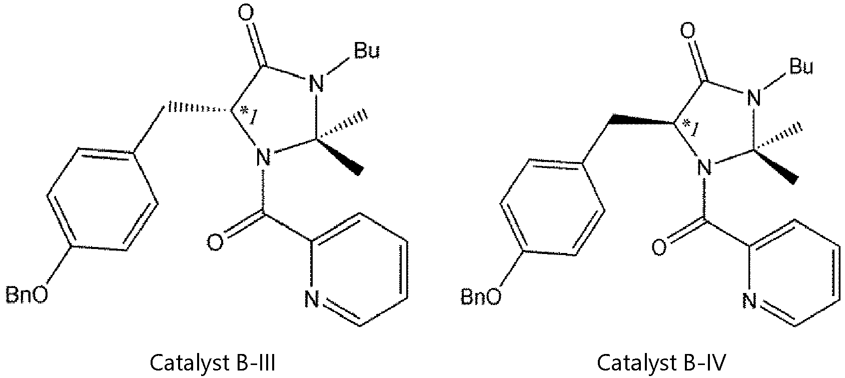

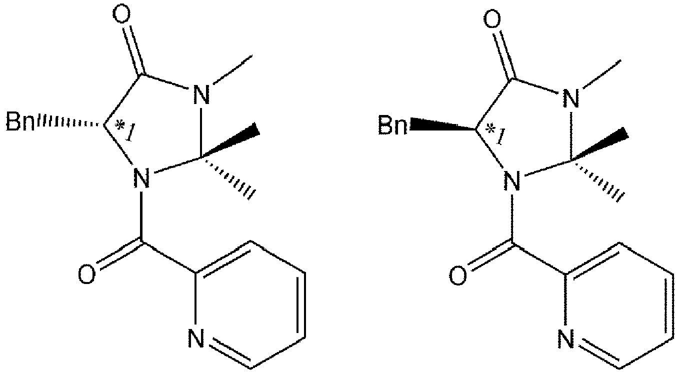

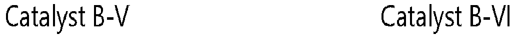

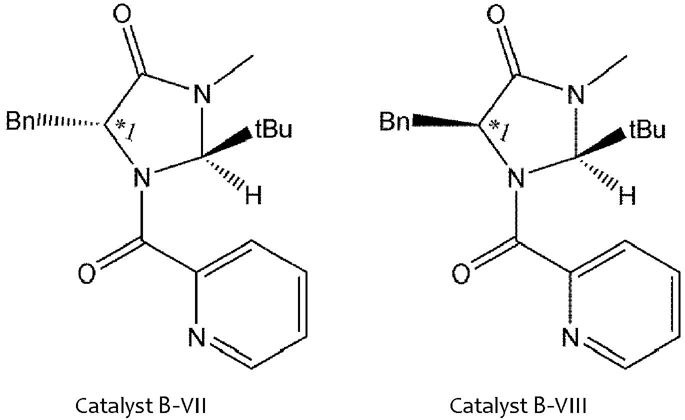

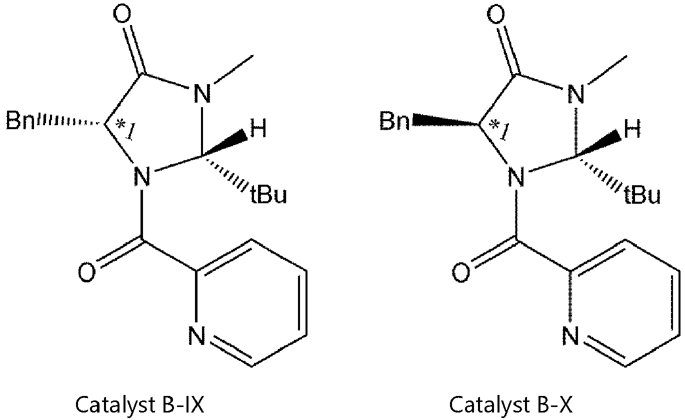

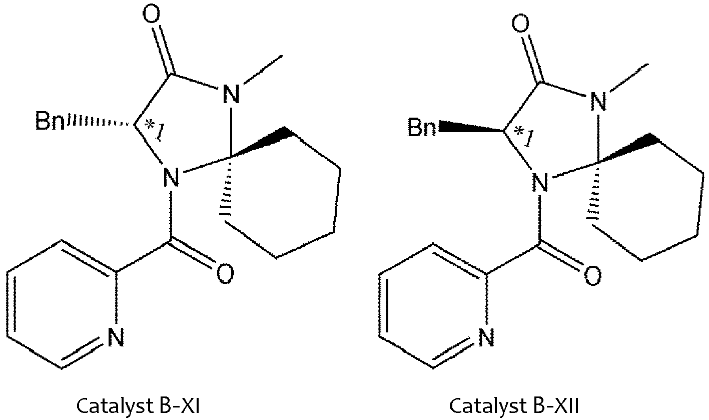

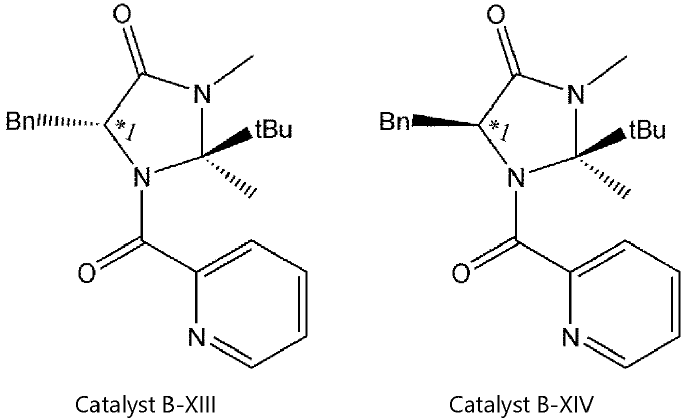

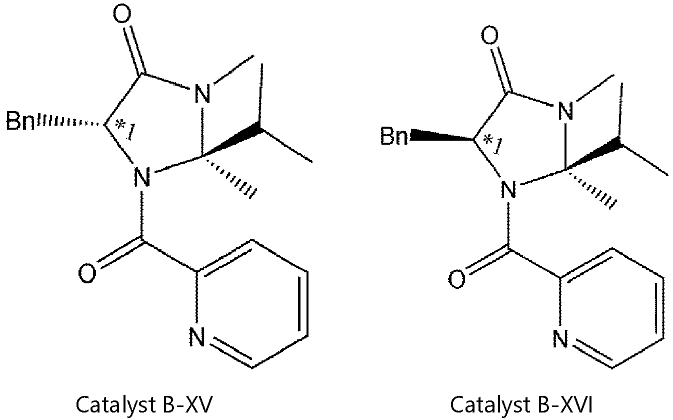

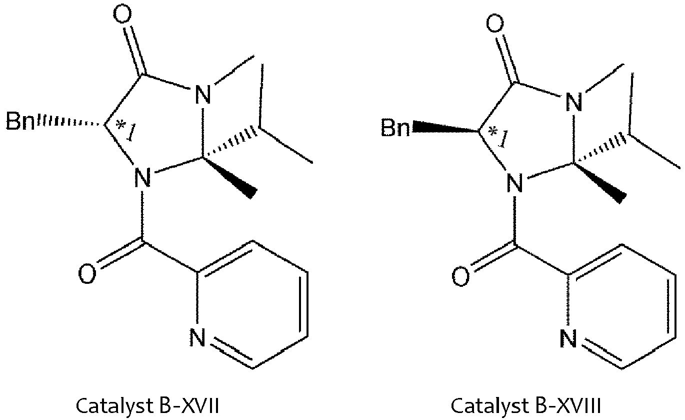

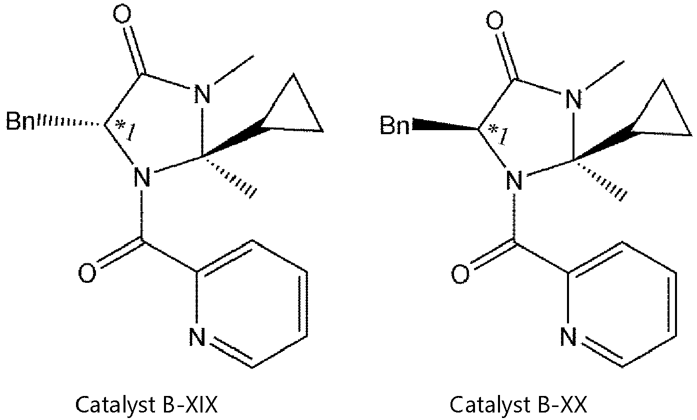

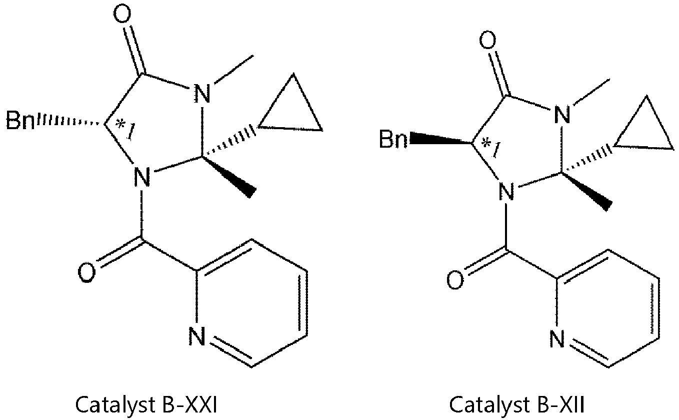

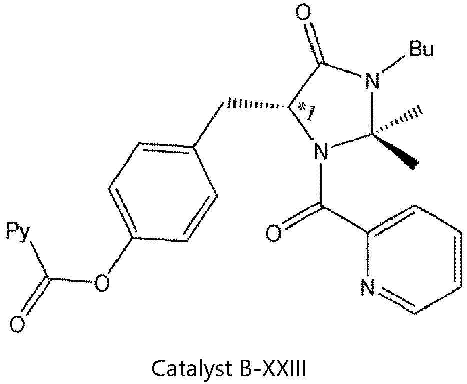

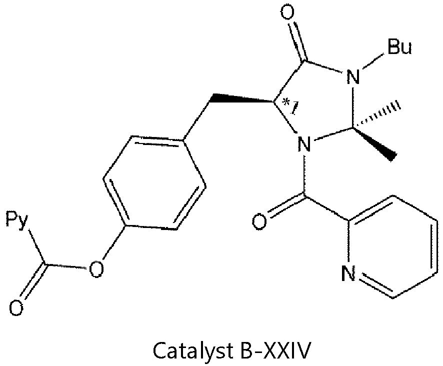

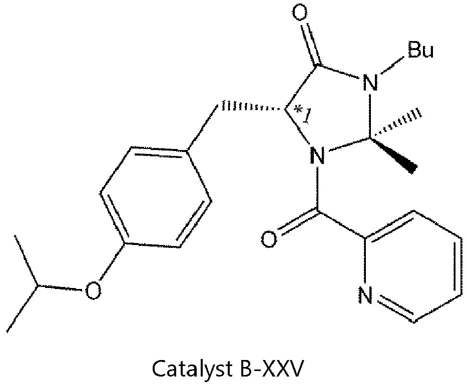

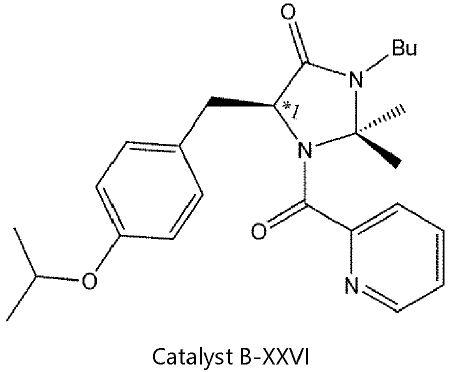

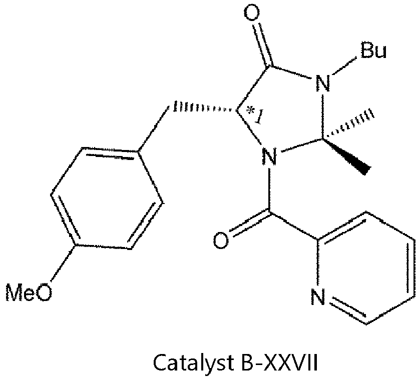

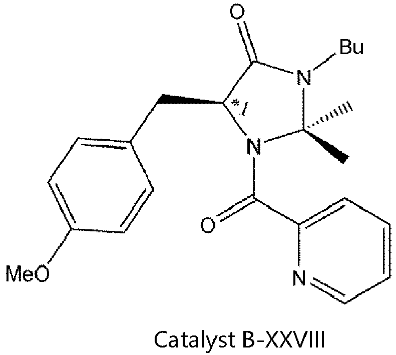

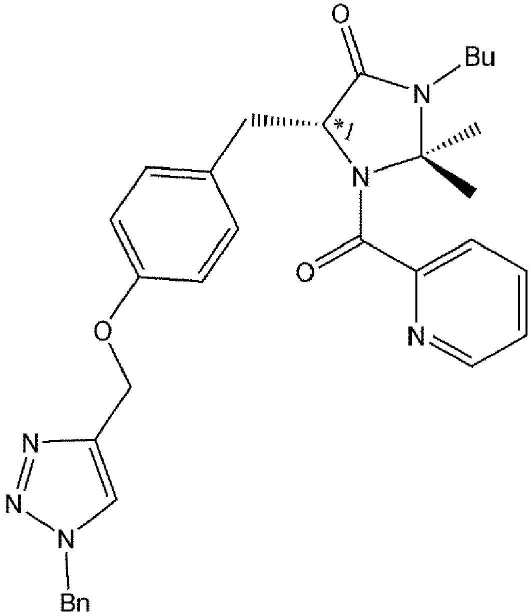

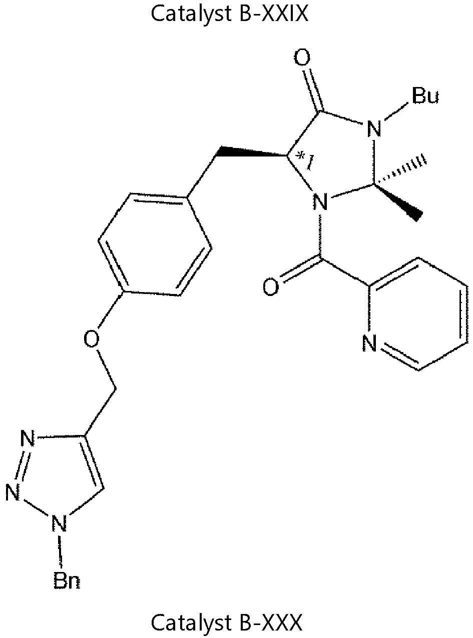

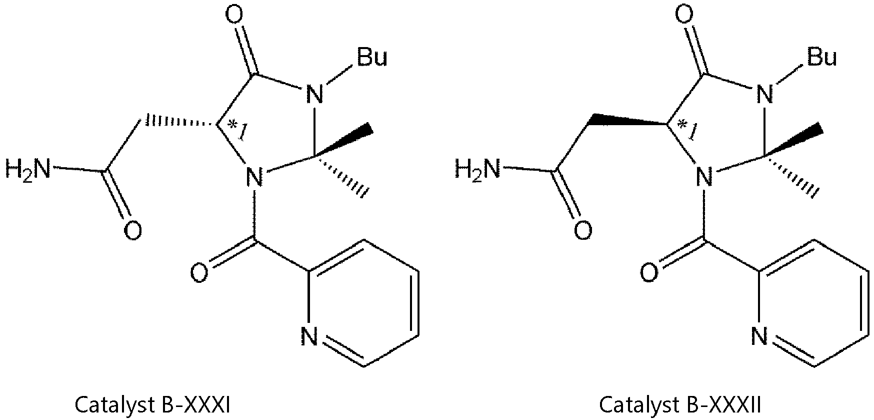

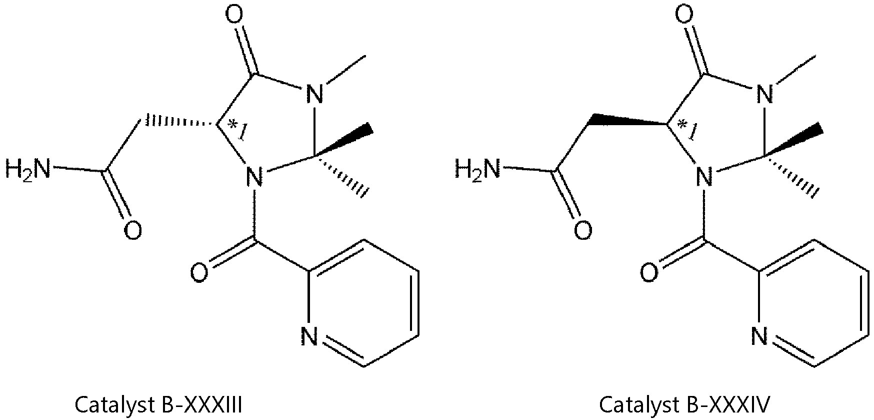

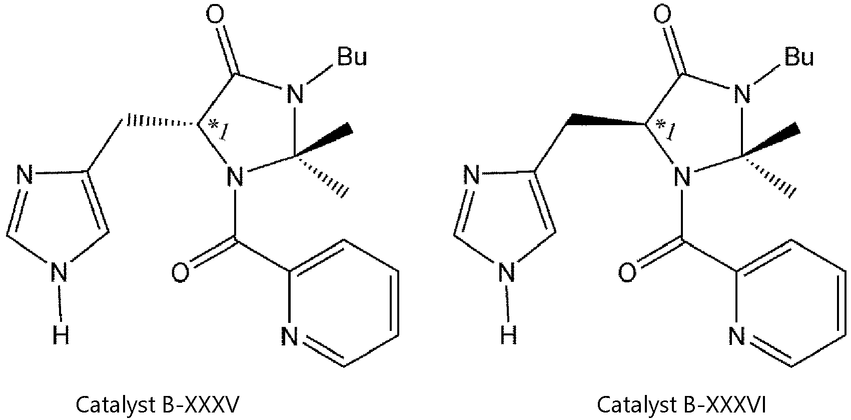

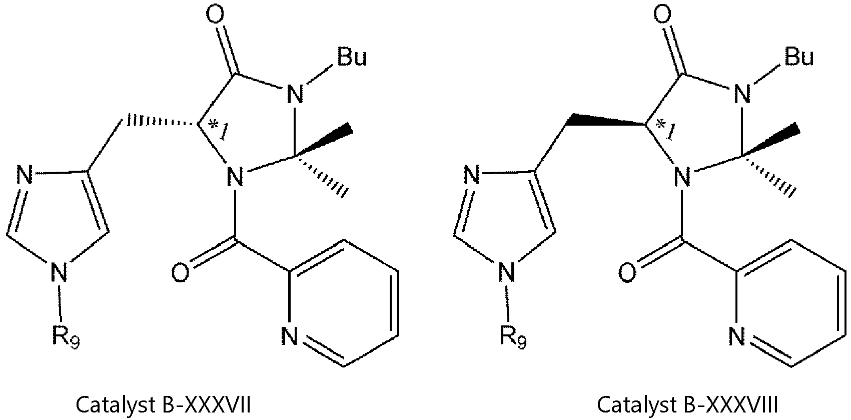

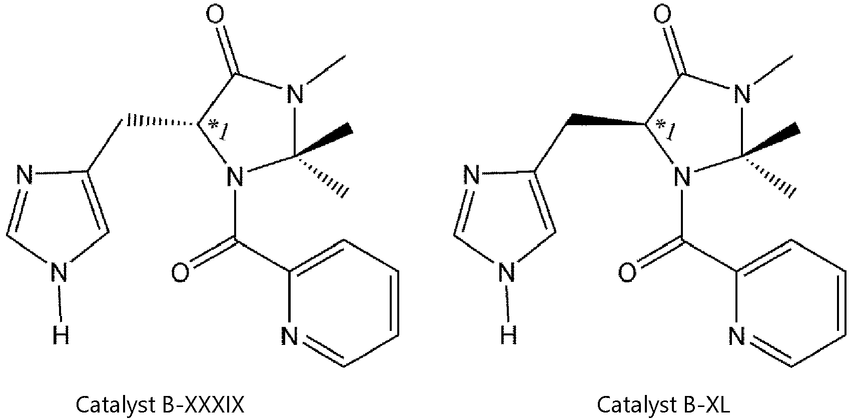

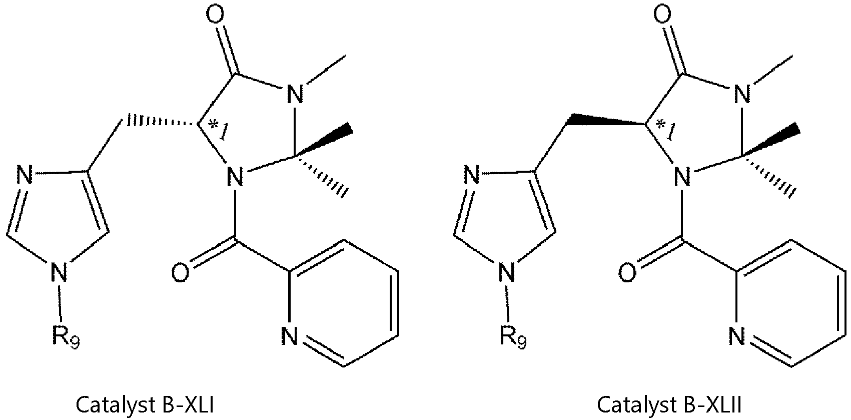

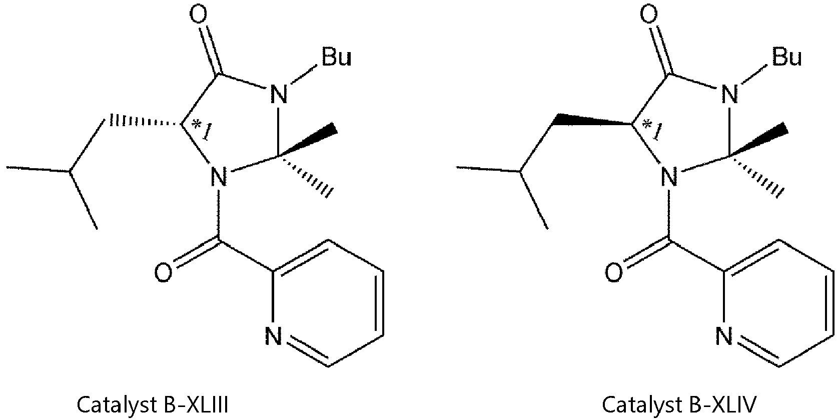

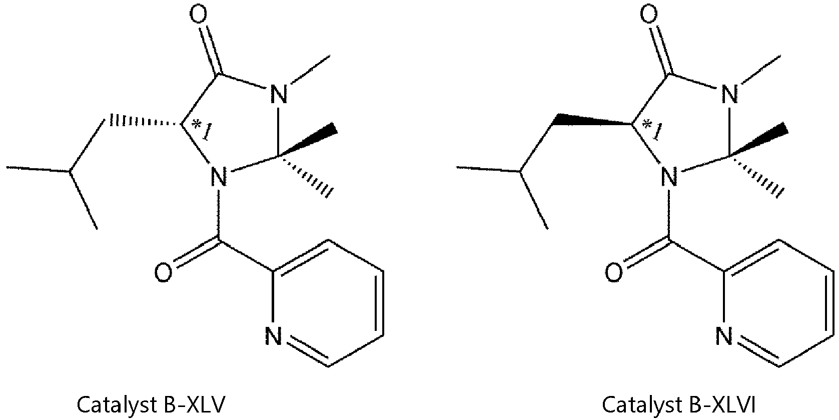

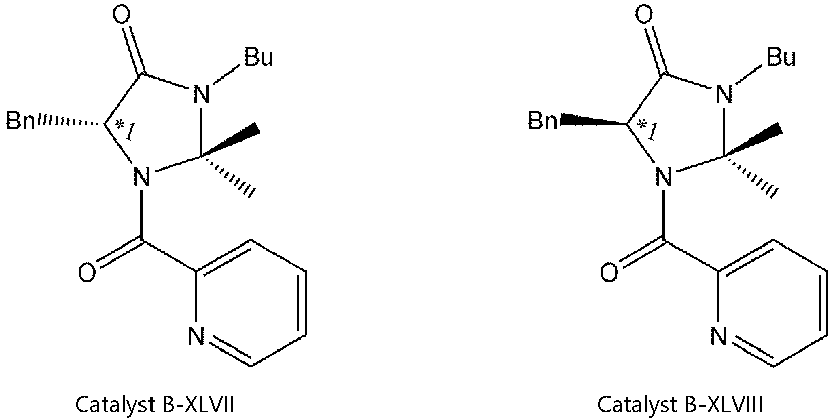

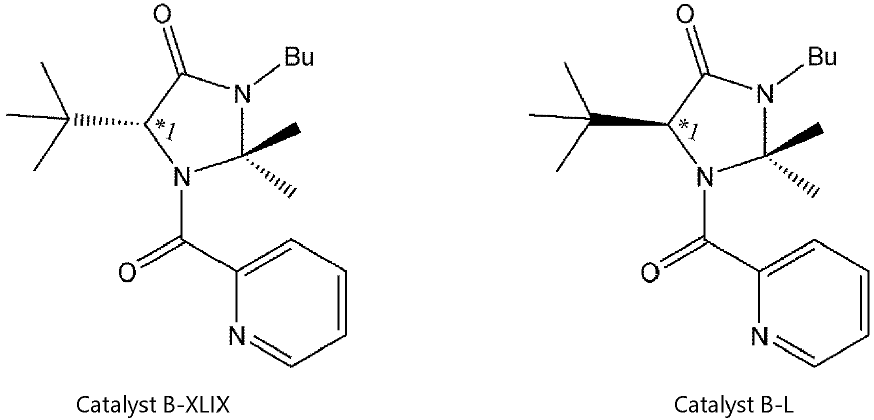

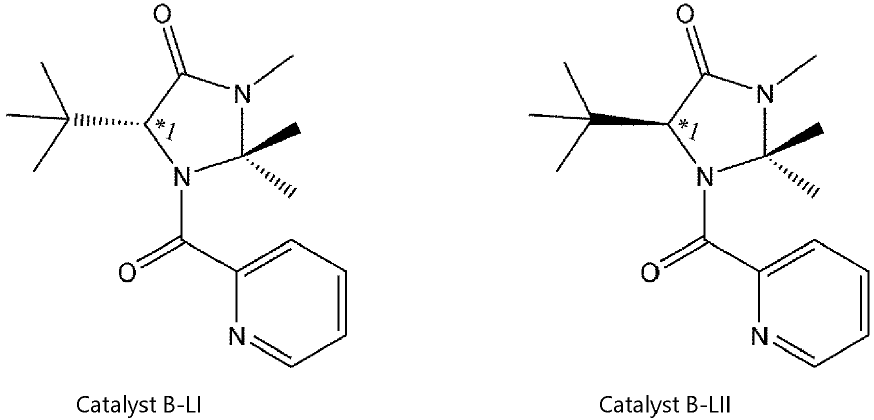

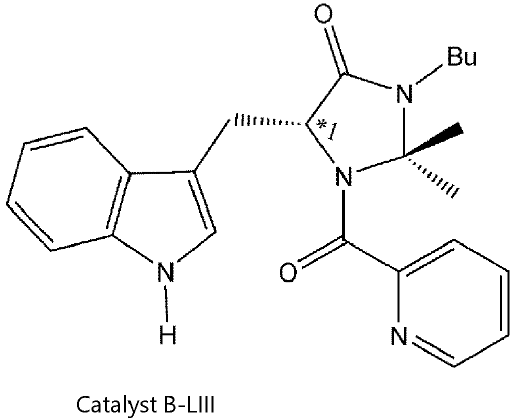

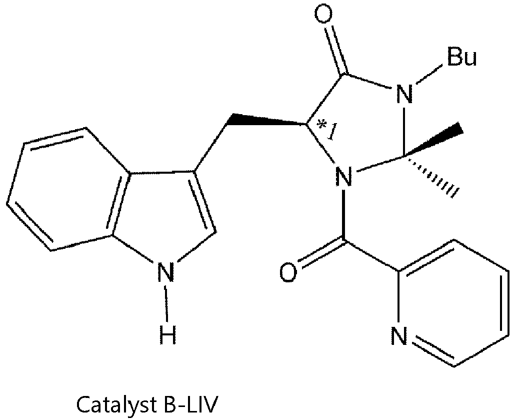

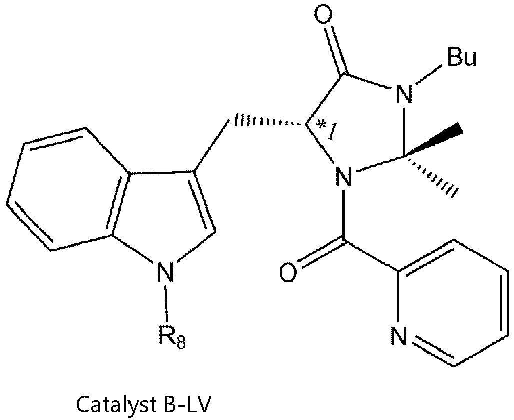

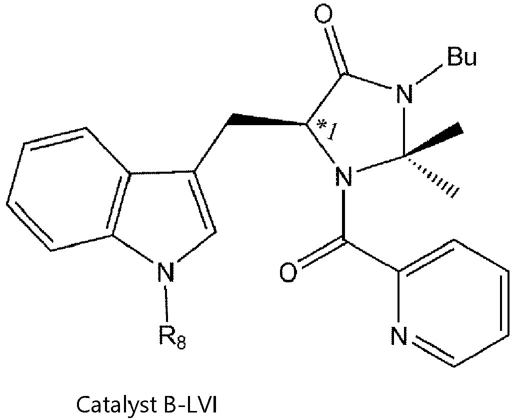

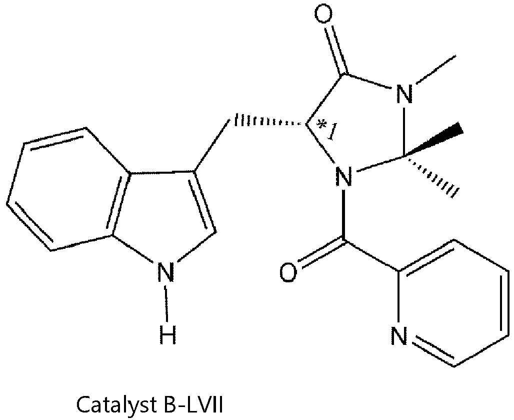

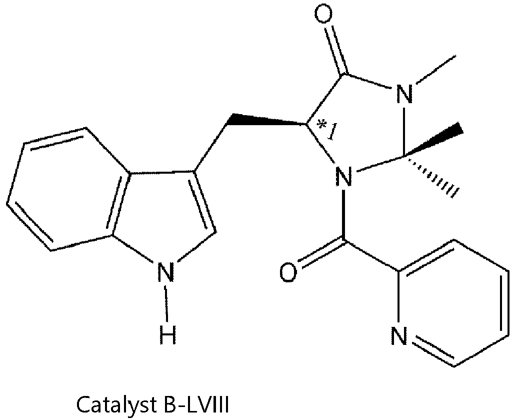

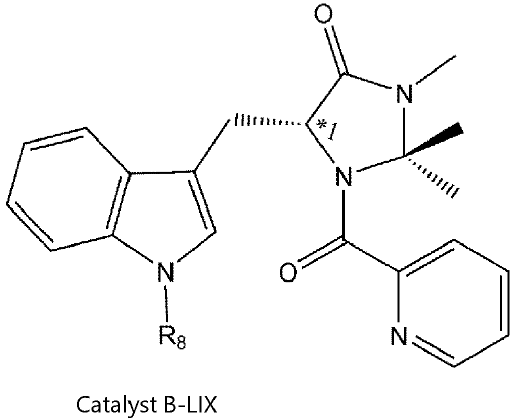

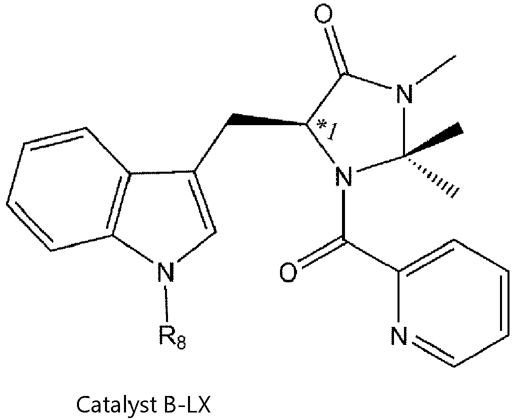

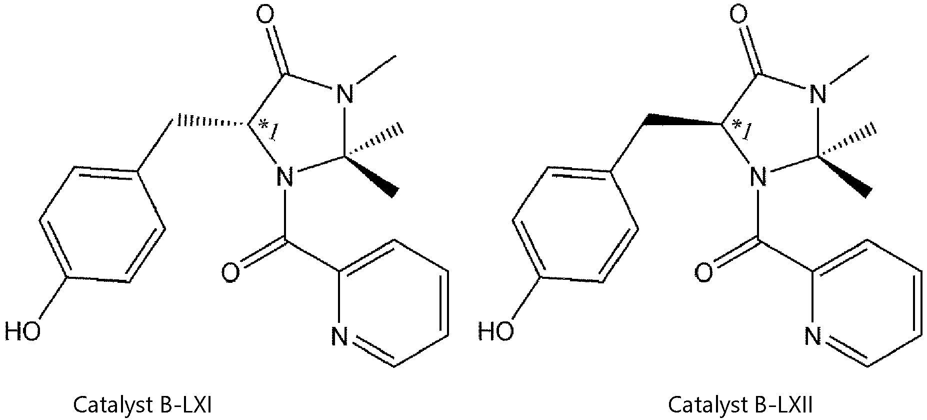

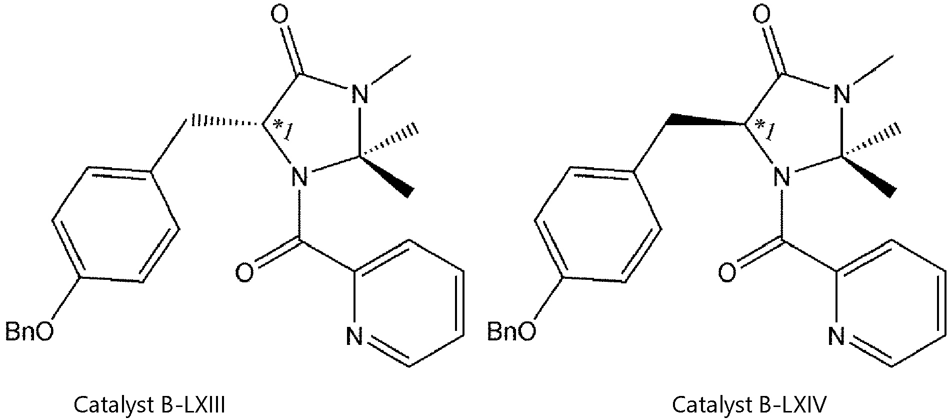

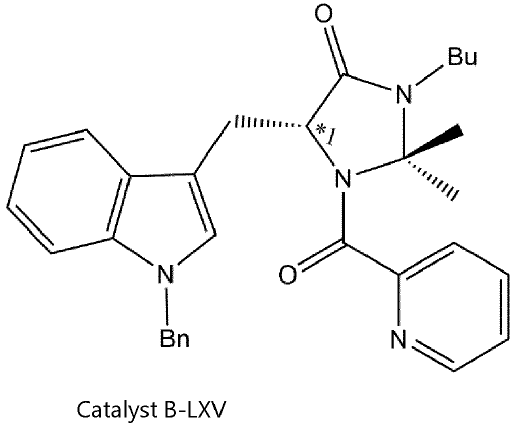

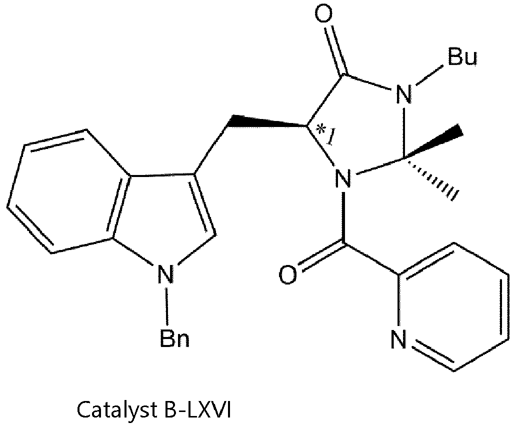

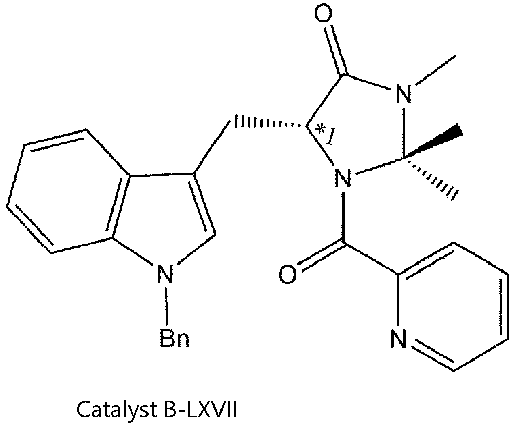

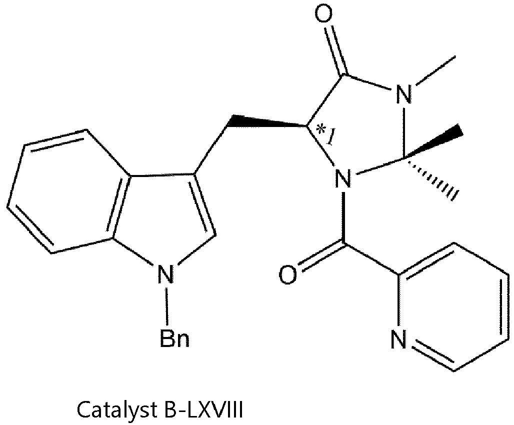

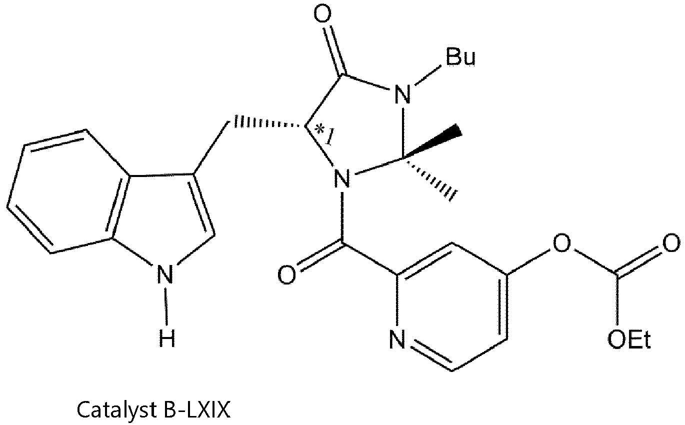

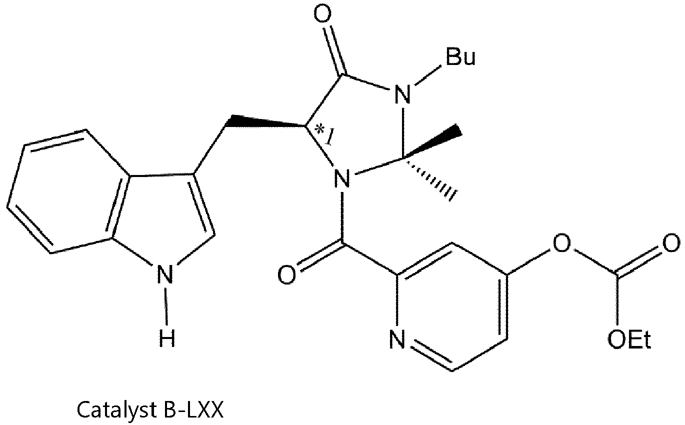

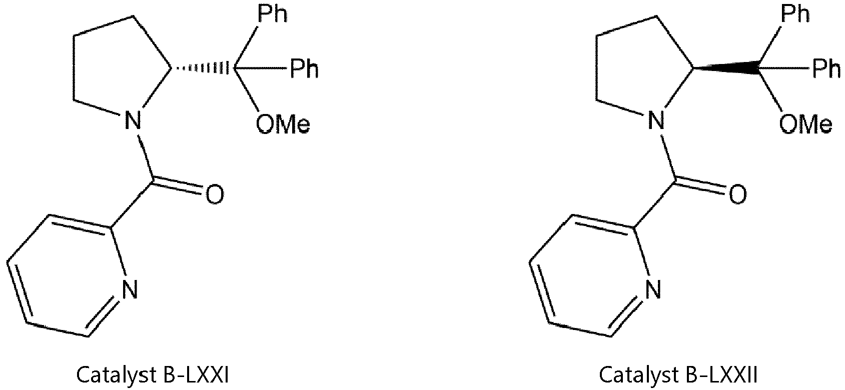

In the structural formulae of Catalysts B-LV, B-LVI, B-LIX and B-LX, R₈ can be chosen from linear, alkyl groups with 1 to 10 carbon atoms that can be substituted with at least one of F, Cl, Br, I, pseudohalogen groups, alkoxy groups with 1 to 20 C atoms, OH, NO₂ and/or NH₂; F; Cl; Br; I; pseudohalogen groups; alkoxy groups with 1 to 20 C atoms; OH; NO₂; and NH₂.

In the structural formulae of Catalysts B-XXXVII, B-XXXVIII, B-XLI and B-XLII, R₉ can be chosen from linear, alkyl groups with 1 to 10 carbon atoms that can be substituted with at least one of F, Cl, Br, I, pseudohalogen groups, alkoxy groups with 1 to 20 C atoms, OH, NO₂ and/or NH₂; F; Cl; Br; I; pseudohalogen groups; alkoxy groups with 1 to 20 C atoms; OH; NO₂; and NH₂.

According to certain embodiments, the catalyst is selected from the group consisting of Catalyst B-I, Catalyst B-II, Catalyst B-III, Catalyst B-IV, Catalyst B-V, Catalyst B-VI, Catalyst B-VII, Catalyst B-VIII, Catalyst B-XI, Catalyst B-XII, Catalyst B-XV, Catalyst B-XIX, Catalyst B-XX, Catalyst B-XXI, Catalyst B-XXII, Catalyst B-XXIII, Catalyst B-XXIV, Catalyst B-XXIX, Catalyst B-XXX, Catalyst B-XXXI, Catalyst B-XXXII, Catalyst B-XXXIII, Catalyst B-XXXIV, Catalyst B-XXXV, Catalyst B-XXXVI, Catalyst B-XXXVII, Catalyst B-XXXVIII, Catalyst B-XXXIX, Catalyst B-XL, Catalyst B-XLI, Catalyst B-XLII, Catalyst B-XLIII, Catalyst B-XLIV, Catalyst B-XLV, Catalyst B-XLVI, Catalyst B-XLVII, Catalyst B-XLVIII, Catalyst B-XLIX, Catalyst B-L, Catalyst B-LI, Catalyst B-LII, Catalyst B-LIII, Catalyst B-LIV, Catalyst B-LV, Catalyst B-LVI, Catalyst B-LVII, Catalyst B-LVIII, Catalyst B-LIX, Catalyst B-LX, Catalyst B-LXI, Catalyst B-LXII, Catalyst B-LXIII, Catalyst B-LXIV, Catalyst B-LXV, Catalyst B-LXVI, Catalyst B-LXVII, Catalyst B-LXVIII, Catalyst B-LXIX, Catalyst B-LXX, Catalyst B-LXXI, and Catalyst B-LXXII, e.g. from the group consisting of Catalyst B-I, Catalyst B-II, Catalyst B-III, Catalyst B-IV, Catalyst B-V, Catalyst B-VI, Catalyst B-VII, Catalyst B-VIII, Catalyst B-XI, Catalyst B-XII, Catalyst B-XV, Catalyst B-XIX, Catalyst B-XX, Catalyst B-XXI, Catalyst B-XXII, Catalyst B-XXIII, Catalyst B-XXIV, Catalyst B-XXIX, Catalyst B-XXX, Catalyst B-XLVII, Catalyst B-XLVIII, Catalyst B-LIII, Catalyst B-LIV, Catalyst B-LXV, Catalyst B-LXVI, Catalyst B-LXVII, Catalyst B-LXVIII, Catalyst B-LXIX, Catalyst B-LXX, Catalyst B-LXXI, and Catalyst B-LXXII, or from the group consisting of Catalyst B-I, Catalyst B-II, Catalyst B-III, Catalyst B-IV, Catalyst B-XXXI, Catalyst B-XXXII, Catalyst B-XXXIII, Catalyst B-XXXIV, Catalyst B-XXXV, Catalyst B-XXXVI, Catalyst B-XXXVII, Catalyst B-XXXVIII, Catalyst B-XXXIX, Catalyst B-XL, Catalyst B-XLI, Catalyst B-XLII, Catalyst B-XLIII, Catalyst B-XLIV, Catalyst B-XLV, Catalyst B-XLVI, Catalyst B-XLVII, Catalyst B-XLVIII, Catalyst B-XLIX, Catalyst B-L, Catalyst B-LI, Catalyst B-LII, Catalyst B-LIII, Catalyst B-LIV, Catalyst B-LV, Catalyst B-LVI, Catalyst B-LVII, Catalyst B-LVIII, Catalyst B-LIX, Catalyst B-LX, Catalyst B-LXI, Catalyst B-LXII, Catalyst B-LXIII, Catalyst B-LXIV, Catalyst B-LXV, Catalyst B-LXVI Catalyst B-LXVII, Catalyst B-LXVIII, Catalyst B-LXIX, Catalyst B-LXX, Catalyst B-LXXI, and Catalyst B-LXXII. In preferred embodiments, the catalyst is selected from the group consisting of Catalyst B-I, Catalyst B-II, Catalyst B-III, Catalyst B-IV, Catalyst B-V, Catalyst B-VI, Catalyst B-XI, Catalyst B-XII Catalyst B-XV, Catalyst B-XVI, Catalyst B-XIX, Catalyst B-XX, Catalyst B-XXV, Catalyst B-XXVI, Catalyst B-XXIX, Catalyst B-XXX, Catalyst B-XXXI, Catalyst B-XXXII, Catalyst B-XXXIII, Catalyst B-XXXIV, Catalyst B-XXXV, Catalyst B-XXXVI, Catalyst B-XXXVII, Catalyst B-XXXVIII, Catalyst B-XXXIX, Catalyst B-XL, Catalyst B-XLI, Catalyst B-XLII, Catalyst B-XLIII, Catalyst B-XLIV, Catalyst B-XLV, Catalyst B-XLVI, Catalyst B-XLVII, Catalyst B-XLVIII, Catalyst B-XLIX, Catalyst B-L, Catalyst B-LI, Catalyst B-LII, Catalyst B-LIII, Catalyst B-LIV, Catalyst B-LV, Catalyst B-LVI, Catalyst B-LVII, Catalyst B-LVIII, Catalyst B-LIX, Catalyst B-LX, Catalyst B-LXI, Catalyst B-LXII, Catalyst B-LXIII, Catalyst B-LXIV, Catalyst B-LXV, Catalyst B-LXVI, Catalyst B-LXVII, Catalyst B-LXVIII, Catalyst B-LXIX, Catalyst B-LXX, Catalyst B-LXXI, and Catalyst B-LXXII, preferably from the group consisting of Catalyst B-I, Catalyst B-II, Catalyst B-III, Catalyst B-IV, Catalyst B-XXXI, Catalyst B-XXXII, Catalyst B-XXXIII, Catalyst B-XXXIV, Catalyst B-XXXV, Catalyst B-XXXVI, Catalyst B-XXXVII, Catalyst B-XXXVIII, Catalyst B-XXXIX, Catalyst B-XL, Catalyst B-XLI, Catalyst B-XLII, Catalyst B-XLIII, Catalyst B-XLIV, Catalyst B-XLV, Catalyst B-XLVI, Catalyst B-XLVII, Catalyst B-XLVIII, Catalyst B-XLIX, Catalyst B-L, Catalyst B-LI, Catalyst B- LII, Catalyst B-LIII, Catalyst B-LIV, Catalyst B-LV, Catalyst B-LVI, Catalyst B-LVII, Catalyst B-LVIII, Catalyst B-LIX, Catalyst B-LX, Catalyst B-LXI, Catalyst B-LXII, Catalyst B-LXIII, Catalyst B-LXIV, Catalyst B-LXV, Catalyst B-LXVI, Catalyst B-LXVII, Catalyst B-LXVIII, Catalyst B-LXIX, Catalyst B-LXX, Catalyst B-LXXI, and Catalyst B-LXXII, in particular from the group consisting of Catalyst B-I, Catalyst B-II, Catalyst B-III, Catalyst B-IV, Catalyst B-XLVII, Catalyst B-XLVIII, Catalyst B-LIII, Catalyst B-LIV, Catalyst B-LXV, Catalyst B-LXVI, Catalyst B-LXXI, and Catalyst B-LXXII,. According to certain embodiments, the catalyst is selected from the group consisting of Catalyst B-I, Catalyst B-III, Catalyst B-V, Catalyst B-VII, Catalyst B-IX, Catalyst B-XI, Catalyst B-XIII, Catalyst B-XV, Catalyst B-XVII, Catalyst B-XIX, Catalyst B-XXI, Catalyst B-XXIII, Catalyst B-XXV, Catalyst B-XXVII, Catalyst B-XXIX, Catalyst B-XXXI, Catalyst B-XXXIII, Catalyst B-XXXV, Catalyst B-XXXVII, Catalyst B-XXXIX, Catalyst B-XLI, Catalyst B-XLIII, Catalyst B-XLV, Catalyst B-XLVII, Catalyst B-XLIX, Catalyst B-LI, Catalyst B-LIII, Catalyst B-LV, Catalyst B-LVII, Catalyst B-LIX, Catalyst B-LXI, Catalyst B-LXIII, Catalyst B-LXV, Catalyst B-LXVII, Catalyst B-LXIX, and Catalyst B-LXXII, preferably Catalyst B-I, Catalyst B-III, Catalyst B-LIII, Catalyst B-XLVII, Catalyst B-LXV, and Catalyst B-LXXII. According to certain embodiments, the catalyst is selected from the group consisting of Catalyst B-II, Catalyst B-IV, Catalyst B-VI, Catalyst B-VIII, Catalyst B-X, Catalyst B-XII, Catalyst B-XIV, Catalyst B-XVI, Catalyst B-XVIII, Catalyst B-XX, Catalyst B-XXII, Catalyst X B-XIV, Catalyst B-XXVI, Catalyst B-XXVIII, Catalyst B-XXX, Catalyst B-XXXII, Catalyst B-XXXIV, Catalyst B-XXXVI, B-Catalyst XXXVIII, Catalyst B-XL, Catalyst B-XLII, Catalyst B-XLIV, Catalyst B-XLVI, Catalyst B-XLVIII, Catalyst B-L, Catalyst B-LII, Catalyst B-LIV, Catalyst B-LVI, Catalyst B-LVIII, Catalyst B-LX, Catalyst B-LXII, Catalyst B-LXIV, Catalyst B-LXVI, Catalyst B-LXVIII, Catalyst B-LXX, and Catalyst B-LXXI, preferably Catalyst B-II, Catalyst B-IV, Catalyst B-XLVIII, Catalyst B-LIV, Catalyst B-LXVI, and Catalyst B-LXXI.

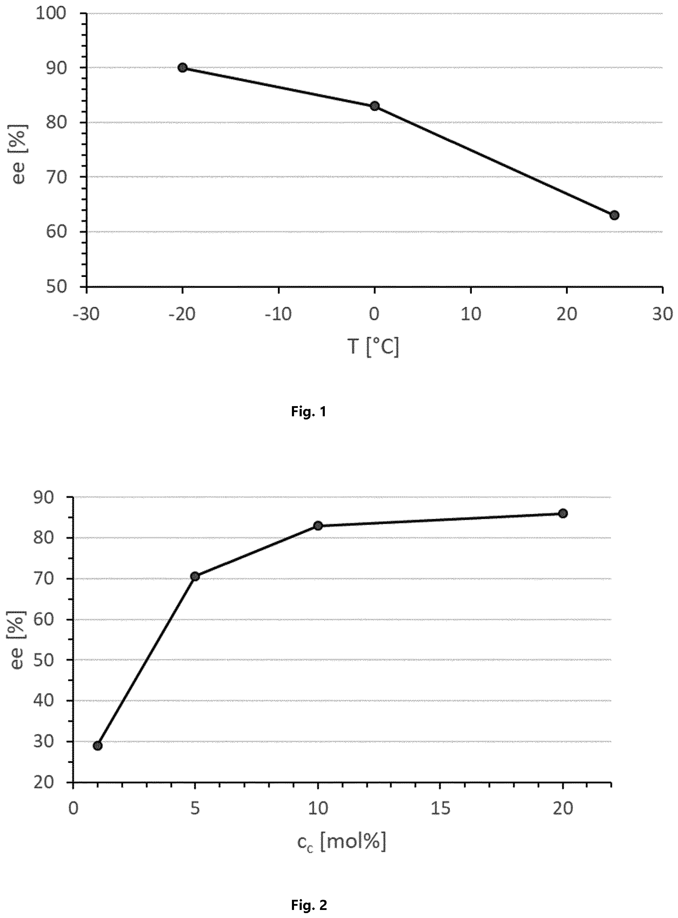

The temperature of the reaction is not particularly limited. According to certain embodiments, the reaction can be conducted at a temperature in the range of -80 °C to 100 °C. In preferred embodiments, the reaction is conducted at a temperature in the range of -30 to 50 °C, in particular -20 °C to 25 °C. Higher temperatures T promote the competing non-catalyzed hydration reaction, which can lead to low enantiomeric excess ee (see e.g. also Figure 1, reaction conditions: 10 mol% Catalyst IV, solvent DCM). Low temperatures can result in very slow reactions with unsatisfactory yields.

The solvent used for the reaction is not particularly limited. The solvent may comprise at least one non-protic solvent or consist essentially of at least one non-protic solvent. In some embodiments, the reaction can be conducted in at least one solvent, selected from the group consisting of dichloromethane (DCM), (trichloromethyl)benzene, (trifluoromethyl)benzene, chloroform, o-xylene, m-xylene, p-xylene, toluene, benzene, chlorobenzene, ethers like anisole and Me-THF, MeCN, acetonitrile, esters like ethyl acetate (EtOAc), acetone and other ketones, e.g. methyl ethyl ketone, methyl isobutyl ketone, methyl isopropyl ketone, cyclopentanone, 2-hexanone, 2- or 3-pentanone, etc., and mixtures of two or more thereof. According to certain embodiments, the solvent is a fluorinated and/or chlorinated solvent and/or aromatic solvents. According to preferred embodiments, the reaction is conducted in DCM as solvent.

The hydrosilane applied in the reaction is not particularly limited. In certain embodiments, the hydrosilane can have a Si-H bonding strength in the range of 84 to 100 kcal/mol. According to certainembodiments, the hydrosilane can be selected from the group of silanes of formulas ABCSiH, ABSiH₂, and ASiH₃, wherein A, B and C can be the same or different. In certain embodiments, A, B and/or C can be F, Cl, Br and/or I. Alternatively or in addition, A, B and/or C can be alkyl groups with 1 to 20 C atoms that can be substituted with at least one of or two or more of F, Cl, Br, I, pseudohalogen groups, and alkoxy groups with 1 to 20 C atoms. Alternatively or in addition, A, B and/or C can in some embodiments be aryl groups with 6 to 20 C atoms that can be substituted with at least one of or two or more of F, Cl, Br, I, pseudohalogen groups, and alkoxy groups with 1 to 20 C atoms. In some embodiments, A, B and/or C can, alternatively or in addition, be aralkyl groups with 7 to 24 C atoms that can be substituted with at least one of or two or more of F, Cl, Br, I, pseudohalogen groups, and alkoxy groups with 1 to 20 C atoms. According to certain embodiments, A, B and/or C can, alternatively or in addition, be alkylaryl groups with 7 to 24 C atoms that can be substituted with at least one of or two or more of F, Cl, Br, I, pseudohalogen groups, and alkoxy groups with 1 to 20 C atoms. In preferred embodiments, the hydrosilane is chosen from trichlorosilane (Cl₃SiH), polymethylhydrosiloxane (PHMS), dichlorosilane, and/or methyldichlorosilane, particularly trichlorosilane and/or dichlorosilane, e.g. is particularly trichlorosilane .

According to certain embodiments, the reaction is carried out in the presence of an acid and/or water and/or another protic solvent, which are all not particularly restricted. Exemplary acids include inorganic acids like HCI and organic acids like acetic acid, tartaric acid, malonic acid, p-toluenesulfonic acid, benzoic acid, trifluoroacetic acid. Examples of other protic solvents include alcohols with e.g. 1 to 20 C atoms, e.g. methanol, ethanol, propanol, 1-butanol, 2-butanol, 2,6-Di-tert-butyl-4-methylphenol, etc. It is assumed that these additives help activate the catalyst and/or myosmine, and they may be involved in the regeneration of the catalyst. The amount of such acid and/or water and/or other other protic solvent is not particularly restricted.

The amount of catalyst is not particularly limited. According to certain embodiments, the catalyst can be present in an amount in the range of 0.001 to 60 mol%, preferably 0.005 to 55 mol%, e.g. 0.1 to 50 mol% with respect to the amount of myosmine. In exemplary embodiments, the catalyst, particularly the catalyst of Formula Ib and any catalysts derived thereof, is present in an amount in the range of 1 to 20 mol%, e.g. in an amount in the range of 3 to 16 mol%, e.g. in an amount in the range of 7 to 13 mol%, e.g. in an amount of about 10 mol% with respect to the amount of myosmine. For the catalysts of Formula Ia and Formula la', it has been surprisingly found that much lower catalyst amounts are already sufficient. Due to the competitive non-catalyzed reaction, low amounts of catalyst, i.e. lower catalyst concentration c_{c}, lead to lower ee's (see e.g. Figure 2, reaction conditions: Catalyst IV, 0°C, solvent DCM). It is assumed that the reaction rate of the catalyzed reaction decreases due to the low concentrated catalyst, while the rate of the uncatalyzed reduction remains the same.

The amount of hydrosilane is not particularly limited. In some embodiments, the hydrosilane can be present in an amount in the range of 1 to 10 equivalents with respect to the amount of myosmine. According to some embodiments, the hydrosilane can be present in an amount in the range of 1.2 to 8 equivalents with respect to the amount of myosmine. Further, the hydrosilane can in certain embodiments be present in an amount in the range of 1.5 to 6 equivalents with respect to the amount of myosmine. According to preferred embodiments, hydrosilane is present in an amount in the range of 2 to 5 equivalents, particularly in an amount of about 3.5 equivalents with respect to the amount of myosmine.

The conduction of the reaction is not particularly limited. According to certain embodiments, the nornicotine is produced as a mixture of (R)- and (S)-nornicotine. However, it may also be possible to produce the nornicotine as essentially pure (R)- or (S)-nornicotine with an ee (enantiomeric excess) of at least 99%, e.g. an ee of at least 99.5%, or even as pure (R)- or (S)-nornicotine. According to some embodiments, the reaction can be conducted in liquid phase, e.g. in a solvent as described above. Further, the reaction can be conducted continuously or discontinuously (batch). The reaction may also be conducted in an inert atmosphere which is not particularly restricted.

According to certain embodiments, the nornicotine is produced as a mixture of (R)- and (S)-nornicotine. According to some embodiments, one of the stereoisomers of nornicotine is produced with enantiomeric excess. According to certain embodiments, the enantiomeric excess (ee) of one stereoisomer of nornicotine can be in the range of 5 to less than 100%, in the range of 10 to 99%, or in the range of 20 to 95%. Furthermore, the enantiomeric excess (ee) of one stereoisomer of nornicotine can in certain embodiments be in the range of 50 to 90%. According to certain embodiments, the enantiomeric excess (ee) of (R)-nornicotine can be in the range of 5 to 100%, in the range of 10 to 99%, or in the range of 20 to 95%. Furthermore, the enantiomeric excess (ee) of (R)-nornicotine can in certain embodiments be in the range of 50 to 90%. According to certain embodiments, the enantiomeric excess (ee) of (S)-nornicotine can be in the range of 5 to 100%, in the range of 10 to 99%, or in the range of 20 to 95%. Furthermore, the enantiomeric excess (ee) of (S)-nornicotine can in certain embodiments be in the range of 50 to 90%.

According to certain embodiments, the method described above can comprise further process steps.

According to certain embodiments, the reaction can be quenched. Quenching is not particularly restricted and allows to break up any complex of the catalyst and product, etc., formed during the reaction to obtain nornicotine, and can be done using a suitable quenching agent like water, a base, an alcohol, etc.

According to certain embodiments, alternatively or in addition to quenching, the obtained reaction mixture can be extracted and/or distilled. Also the extraction and/or distillation is not particularly restricted.

In some embodiments, the method described above further comprises removing the catalyst after reacting myosmine with at least one hydrosilane in the presence of the catalyst. The separation of the catalyst is not particularly limited. The catalyst can easily be separated through various separation methods known in the art. According to some embodiments, the catalyst can be removed by extraction of the reaction mixture, by chromatography, or by filtration upon immobilization, particularly by extraction of the reaction mixture. The extraction is not particularly limited. In certain embodiments, the reaction mixture is extracted with water or an aqueous solution at a specific pH, e.g. pH 7, separating the catalyst into the organic phase and nornicotine into the aqueous phase. According to certain embodiments, the catalyst can be recovered, e.g. through crystallization by concentrating the organic phase, supplementing the organic phase with a suitable solvent like heptane, and cooling the mixture, e.g. to 0°C or below. It is further not excluded to remove unreacted educt, side products, solvent, and /or removing one enantiomeric nornicotine, e.g. the (S)-nornicotine or the (R)-nornicotine, in order to produce enantiomerically pure nornicotine, e.g. the (R)-nornicotine or the (S)-nornicotine. Techniques for such removal are not particularly restricted and are known in the art.

The method of the first aspect can be carried out continuously or discontinuously, e.g. in a flow reactor, e.g. a microfluidic reactor, or a batch reactor.

In a further, second aspect, the invention relates to a composition produced by the method described above, particularly wherein the catalyst is not removed from the reaction mixture. Surprisingly it has been found that the reaction mixture with the catalyst can be kept stable, thus allowing also transfer for work-up in bigger batches, saving solvent and energy in the work-up step, e.g. a quench, distillation and/or extraction. Surprisingly, it has been found that the composition produced can be kept stably overnight, nevertheless allowing normal workup afterwards.

In a third aspect, the invention relates to a composition comprising nornicotine and a catalyst, wherein the catalyst is an organic chiral Lewis base. Particularly, the organic chiral Lewis base is a catalyst as described with regard to the method of the first aspect, and all embodiments described with regard to the catalyst in the first aspect also apply to the third aspect.

According to certain embodiments, the nornicotine can be mixture of (R)- and (S)-nornicotine. According to certain embodiments, one of the stereoisomers of the (R)- and (S)-nornicotine is contained with an enantiomeric excess. According to some embodiments, the enantiomeric excess (ee) of one stereoisomer of nornicotine can be in the range of 5 to 100%, in the range of 10 to 99%, or in the range of 20 to 95%. Furthermore, the enantiomeric excess (ee) of one stereoisomer of nornicotine can in certain embodiments be in the range of 50 to 90%. According to certain embodiments, the enantiomeric excess (ee) of (R)-nornicotine can be in the range of 5 to 100%, in the range of 10 to 99%, or in the range of 20 to 95%. Furthermore, the enantiomeric excess (ee) of (R)-nornicotine can in certain embodiments be in the range of 50 to 90%. According to certain embodiments, the enantiomeric excess (ee) of (S)-nornicotine can be in the range of 5 to 100%, in the range of 10 to 99%, or in the range of 20 to 95%. Furthermore, the enantiomeric excess (ee) of (S)-nornicotine can in certain embodiments be in the range of 50 to 90%.

In a further, fourth aspect, the invention relates to a method of producing nicotine or a salt thereof from myosmine, comprising reacting myosmine with at least one hydrosilane in the presence of a catalyst to produce nornicotine, wherein the catalyst is an organic chiral Lewis base, and reacting the nornicotine to nicotine. Particularly, the organic chiral Lewis base is a catalyst as described with regard to the method of the first aspect, and all embodiments described with regard to the catalyst in the first aspect also apply to the fourth aspect. Also the first step of the method of the fourth aspect corresponds, according to certain embodiments, to the reaction step of the method of the first aspect. Thus, also the different embodiments described with regard to the method of the first aspect also apply, according to certain embodiments, to the method of the fourth aspect, particularly to the first reaction step, and vice versa.

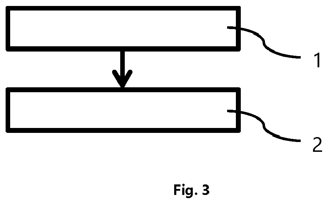

The method steps in the method of the fourth aspect are schematically and exemplary depicted in Figure 3. In step 1 of Figure 3, myosmine is reacted with at least one hydrosilane in the presence of an organic chiral Lewis base as a catalyst to produce nicotine. In a step 2, which according to certain embodiments is a subsequent step, the nornicotine is then reacted to nicotine. In a further, according to certain embodiments subsequent step (not shown), the nicotine can then be reacted to a salt thereof. The inventors particularly found that the two steps of reacting myosmine to nornicotine and then reacting nornicotine to nicotine can be carried out in one pot, i.e. without any cleaning steps in between.

According to some embodiments, the method of the fourth aspect can be conducted according to the following reaction scheme:

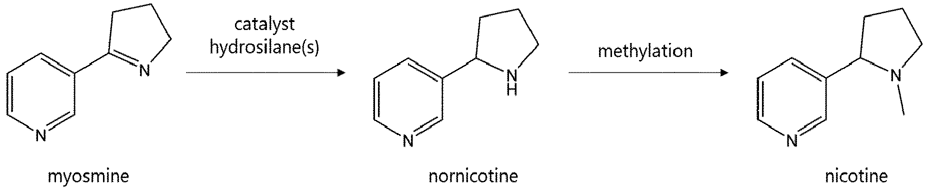

The reaction of myosmine with at least one hydrosilane to produce nornicotine is not particularly limited apart from the presence of an organic chiral Lewis base as catalyst. According to certain embodiments, the reaction of myosmine with at least one hydrosilane to produce nornicotine corresponds to the respective embodiments in the method of producing nornicotine from myosmine of the first aspect, as described above.

The reaction of nornicotine to nicotine is not particularly limited. In some embodiments, the pyrrolidine ring is methylated at the N-atom by a suitable methylating agent, which also is not particularly restricted. According to certain embodiments, the reaction of nornicotine to nicotine is carried out using formic acid and paraformaldehyde, or formic acid and formaldehyde, preferably at a temperature between and including 40 to 95°C, further preferably between and including 60 to 85°C.

According to certain embodiments, the nicotine is further reacted to a salt thereof. The reaction is not particularly restricted, and any suitable salt can be produced, e.g. a tartrate salt or a salicylate salt or a co-crystal compound thereof with e.g. beta-cyclodextrine.

According to certain embodiments, the method can be carried out in a one-pot process. This saves of course further separating steps, solvents, energy and time. It has been surprisingly found that the whole synthesis can be made as a one-pot process without any purification steps of the intermediates in such embodiments. One specific further advantage of the invention is the one-pot synthesis allowing a straight forward reaction sequence, using minimal amounts and types of solvents. According to certain embodiments, no solvent change is necessary in such a one-pot process. It is, however, not excluded that a further solvent is added, e.g. for removing the catalyst and/or for a subsequent step. According to certain embodiments, the catalyst is separated from the mixture after producing nornicotine by extraction, e.g. with an aqueous solvent, e.g. water, and the remaining reaction mixture is directly converted to yield nicotine, without further work-up of the reaction mixture, thus enabling a one-pot synthesis.

Furthermore, since one enantiomer of nornicotine can be produced enantioselectively through the method described above, the corresponding enantiomer of nicotine can also be synthesized with high enantiomeric excess without the application of a resolution step. According to certain embodiments, the nicotine is produced as a mixture of (R)- and (S)-nornicotine. According to some embodiments, one of the stereoisomers of nicotine is produced in enantiomeric excess. According to certain embodiments, the enantiomeric excess (ee) of one stereoisomer of nicotine can be in the range of 5 to 100%, in the range of 10 to 99%, or in the range of 20 to 95%. Furthermore, the enantiomeric excess (ee) of one stereoisomer of nicotine can in certain embodiments be in the range of 50 to 90%. According to certain embodiments, the enantiomeric excess (ee) of (R)-nicotine can be in the range of 5 to 100%, in the range of 10 to 99%, or in the range of 20 to 95%. Furthermore, the enantiomeric excess (ee) of (R)-nicotine can in certain embodiments be in the range of 50 to 90%. According to certain embodiments, the enantiomeric excess (ee) of (S)-nicotine can be in the range of 5 to 100%, in the range of 10 to 99%, or in the range of 20 to 95%. Furthermore, the enantiomeric excess (ee) of (S)-nicotine can in certain embodiments be in the range of 50 to 90%.

In a further, fifth aspect, the invention relates to a composition produced by the method of producing nicotine or a salt thereof from myosmine as described above, wherein the catalyst is not separated from the reaction mixture. As the composition of the fifth aspect directly relates to the fourth aspect, also the corresponding embodiments of the first, and also second, aspect apply. Herein, again, it has been surprisingly found that the mixture can be kept stable, so that a work-up of several batches is possible, thus saving e.g. solvent(s) and energy. Surprisingly, it has been found that the composition produced can be kept stably overnight, nevertheless allowing normal workup afterwards.

In a further, sixth aspect, the invention relates to a composition comprising nicotine or a salt thereof and a catalyst, wherein the catalyst is an organic chiral Lewis base. Particularly, the organic chiral Lewis base is a catalyst as described with regard to the method of the first aspect, and all embodiments described with regard to the catalyst in the first aspect also apply to the sixth aspect.

According to certain embodiments, the nicotine or the salt thereof can be mixture of (R)- and (S)-nicotine or the salts thereof. According to certain embodiments, one of the stereoisomers of the R)- and (S)-nicotine is contained with an enantiomeric excess. According to some embodiments, the enantiomeric excess (ee) of one stereoisomer of nicotine can be in the range of 5 to 100% or in the range of 10 to 99% or in the range of 20 to 95%. Furthermore, the enantiomeric excess (ee) of one stereoisomer of nicotine can in certain embodiments be in the range of 50 to 90%. In some embodiments, the catalyst corresponds to one of the catalysts described above. According to certain embodiments, the enantiomeric excess (ee) of (R)-nicotine can be in the range of 5 to 100%, in the range of 10 to 99%, or in the range of 20 to 95%. Furthermore, the enantiomeric excess (ee) of (R)-nicotine can in certain embodiments be in the range of 50 to 90%. According to certain embodiments, the enantiomeric excess (ee) of (S)-nicotine can be in the range of 5 to 100%, in the range of 10 to 99%, or in the range of 20 to 95%. Furthermore, the enantiomeric excess (ee) of (S)-nicotine can in certain embodiments be in the range of 50 to 90%.

In a further, seventh aspect, the present invention relates to a catalyst of the following formula I 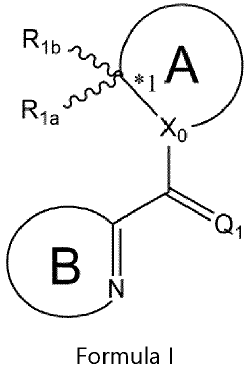 wherein
Ring A comprises, besides X₀ and the chiral C atom with residues R₁ₐ and R_{1b}, a carbonyl group, a thiocarbonyl group, substituted or unsubstituted C atoms and/or substituted or unsubstituted heteroatoms, wherein the substituents can comprise any one of F; Cl; Br; I; pseudohalogen groups; alkoxy groups with 1 to 20 C atoms; OH; NO₂; NH₂; amines of formula NR₁₈R₁₉; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; linear alkenyl groups and/or alkynyl groups with 2 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; branched alkenyl and/or alkynyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂; with the provisio that two or more of the substituents can form one ring or more rings, optionally wherein at least one substituent is part of a polymer;
Ring B is a five- or six-membered ring comprising conjugated double bonds that is particularly aromatic, i.e. is fully conjugated, planar and follows the Hueckel's rule, and that comprises, besides the carbon atom connected to the bridge and the adjacent nitrogen atom, substituted or unsubstituted C atoms and/or substituted or unsubstituted heteroatoms, wherein the substituents can comprise any one of F; Cl; Br; I; pseudohalogen groups; alkoxy groups with 1 to 20 C atoms; OH; NO₂; NH₂; amines of formula NR₁₈R₁₉; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂; with the provisio that two or more of the substituents can form one ring or more rings;
wherein R₁₈ and R₁₉ are the same or different and can be chosen from H, linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂, with the provisio that not both of R₁₈ and R₁₉ are H;
X₀ is selected from N and CR_{A1}, wherein
R_{A1} is selected from the group consisting of H, linear alkyl groups with 1 to 6 C atoms that can optionally be substituted with at least one of F, Cl, Br, I, pseudohalogen groups, alkoxy groups with 1 to 6 C atoms, OH, NO₂ and NH₂; branched alkyl groups with 3 to 6 C atoms that can optionally be substituted with at least one of F, Cl, Br, I, pseudohalogen groups, alkoxy groups with 1 to 6 C atoms, OH, NO₂ and NH₂; cycloalkyl groups with 3 to 7 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 6 C atoms that can optionally be substituted with at least one of F, Cl, Br, I, pseudohalogen groups, alkoxy groups with 1 to 6 C atoms, OH, NO₂ and NH₂; aromatic and/or heteroaromatic groups with 2 to 6 carbon atoms that can optionally be substituted with at least one of F, Cl, Br, I, pseudohalogen groups, alkoxy groups with 1 to 6 C atoms, OH, NO₂ and NH₂; carboxylic acid ester groups with 1 to 6 C atoms that can optionally be substituted with at least one of F, Cl, Br, I, pseudohalogen groups, alkoxy groups with 1 to 6 C atoms, OH, NO₂ and NH₂; alkyl and/or (hetero)aryl oxycarbonyl groups with 1 to 6 C atoms that can optionally be substituted with at least one of F, Cl, Br, I, pseudohalogen groups, alkoxy groups with 1 to 6 C atoms, OH, NO₂ and NH₂; and carboxamide groups with 1 to 6 C atoms that can optionally be substituted with at least one of F, Cl, Br, I, pseudohalogen groups, alkoxy groups with 1 to 6 C atoms, OH, NO₂ and NH₂;
Q₁ is O or S;
R₁ₐ and R_{1b} are different;
R₁ₐ is a saturated and/or unsaturated linear, branched, cyclic and/or aromatic and/or heteroaromatic hydrocarbon residue with 1 to 100 C atoms that can contain heteroatoms selected from the group of O, N, S and P and that can optionally be substituted with at least one of F; Cl; Br; I; pseudohalogen groups; OSiR₂₃R₂₄R₂₅; carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂; carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂; optionally substituted linear, branched and/or cyclic alkoxy, aryloxy, heteroaryloxy, alkenyloxy and/or alkynyloxy groups with 1 to 20 C atoms and/or heteroatoms; OH; NO₂; SH; NH₂; and amines of formula NR₁₈R₁₉;
R₂₃, R₂₄, and R₂₅ are the same or different and are selected from H, linear alkyl groups with 1 to 4 C atoms that can optionally be substituted with at least one of F, Cl, Br, I, pseudohalogen groups, alkoxy groups with 1 to 20 C atoms, OH, NO₂ and NH₂, branched alkyl group with 3 to 6 C atoms that can optionally be substituted with at least one of F, Cl, Br, I, pseudohalogen groups, alkoxy groups with 1 to 20 C atoms, OH, NO₂ and NH₂, aromatic groups with 6 to 12 C atoms that can optionally be substituted with at least one of F, Cl, Br, I, pseudohalogen groups, alkoxy groups with 1 to 20 C atoms, OH, NO₂ and NH₂, and aralkyl groups with 7 to 10 C atoms that can optionally be substituted with at least one of F, Cl, Br, I, pseudohalogen groups, alkoxy groups with 1 to 20 C atoms, OH, NO₂ and NH₂, preferably wherein R₂₃, R₂₄, and R₂₅ are not all hydrogen at the same time and/or wherein R₂₃, R₂₄, and R₂₅ are not all methyl at the same time, further preferably wherein all of R₂₃, R₂₄, and R₂₅ are ethyl;
R_{1b} is selected from the group consisting of H, linear alkyl groups with 1 to 6 C atoms that can optionally be substituted with at least one of F, Cl, Br, I, pseudohalogen groups, alkoxy groups with 1 to 6 C atoms, OH, NO₂ and NH₂; branched alkyl groups with 3 to 6 C atoms that can optionally be substituted with at least one of F, Cl, Br, I, pseudohalogen groups, alkoxy groups with 1 to 6 C atoms, OH, NO₂ and NH₂; heterocycloalkyl groups with 1 to 6 C atoms that can optionally be substituted with at least one of F, Cl, Br, I, pseudohalogen groups, alkoxy groups with 1 to 6 C atoms, OH, NO₂ and NH₂; aromatic and/or heteroaromatic groups with 2 to 6 carbon atoms that can optionally be substituted with at least one of F, Cl, Br, I, pseudohalogen groups, alkoxy groups with 1 to 6 C atoms, OH, NO₂ and NH₂; carboxylic acid ester groups with 1 to 6 C atoms that can optionally be substituted with at least one of F, Cl, Br, I, pseudohalogen groups, alkoxy groups with 1 to 6 C atoms, OH, NO₂ and NH₂; alkyl and/or (hetero)aryl oxycarbonyl groups with 1 to 6 C atoms that can optionally be substituted with at least one of F, Cl, Br, I, pseudohalogen groups, alkoxy groups with 1 to 6 C atoms, OH, NO₂ and NH₂; and carboxamide groups with 1 to 6 C atoms that can optionally be substituted with at least one of F, Cl, Br, I, pseudohalogen groups, alkoxy groups with 1 to 6 C atoms, OH, NO₂ and NH₂, preferably wherein R_{1b} is H or methyl;
wherein the stereocenter *1 of the catalyst according to Formula I is in (R) and/or (S) configuration.

This means that the seventh aspect relates to the catalyst used in the method of the first aspect. Accordingly, the catalyst of the seventh aspect also relates to all the further restricted catalysts disclosed with regard to the first aspect, particularly also the specific catalysts mentioned with regard to the first aspect.

According to certain embodiments, the catalyst is selected from the group consisting of Catalyst A-I, Catalyst A-II, Catalyst A-III, Catalyst A-IV, Catalyst A-V, Catalyst A-VI, Catalyst A-VII, Catalyst A-VIII, Catalyst A-IX, Catalyst A-X, Catalyst A-XI, Catalyst A-XII, Catalyst A-XIII, and Catalyst A-XIV.

According to certain embodiments, the catalyst is selected from the group consisting of Catalyst A-I, Catalyst A-III, Catalyst A-V, Catalyst A-VII, Catalyst A-IX, Catalyst A-XI, and Catalyst A-XIII.

According to certain embodiments, the catalyst is selected from the group consisting of Catalyst A-II, Catalyst A-IV, Catalyst A-VI, Catalyst A-VIII, Catalyst A-X, Catalyst A-XII, and Catalyst A-XIV.

According to certain embodiments, the catalyst is selected from the group consisting of Catalyst A-I, Catalyst A-III, Catalyst A-V, Catalyst A-VII, Catalyst A-IX, Catalyst A-XI, Catalyst A-XIII, Catalyst A-XV, Catalyst A-XVII, Catalyst A-XIX, Catalyst A-XXI, Catalyst A-XXIII, Catalyst A-XXV, Catalyst A-XXVII, Catalyst A-XXIX, Catalyst A-XXXI, Catalyst A-XXXIII, Catalyst A-XXXV, Catalyst A-XXXVII, Catalyst A-XXXIX, Catalyst A-XLI, Catalyst A-XLIII, Catalyst A-XLV, Catalyst A-XLVII, Catalyst A-XLIX, Catalyst A-LI, Catalyst A-LIII, Catalyst A-LV, Catalyst A-LVII, Catalyst A-LIX, and Catalyst A-LXI. According to certain embodiments, the catalyst is Catalyst A-LXIII, e.g. Catalyst A-XXV.

According to certain embodiments, the catalyst is selected from the group consisting of Catalyst A-II, Catalyst A-IV, Catalyst A-VI, Catalyst A-VIII, Catalyst A-X, Catalyst A-XII, Catalyst A-XIV, Catalyst A-XVI, Catalyst A-XVIII, Catalyst A-XX, Catalyst A-XXII, Catalyst A-XXIV, Catalyst A-XXVI, Catalyst A-XXVIII, Catalyst A-XXX, Catalyst A-XXXII, Catalyst A-XXXIV, Catalyst A-XXXVI, Catalyst A-XXXVIII, Catalyst A-XL, Catalyst A-XLII, Catalyst A-XLIV, Catalyst A-XLVI, Catalyst A-XLVIII, Catalyst A-L, Catalyst A-LII, Catalyst A-LIV, Catalyst A-LVI, Catalyst A-LVIII, Catalyst A-LX, and Catalyst A-LXII. According to certain embodiments, the catalyst is Catalyst A-LXIV, e.g. Catalyst A-XXVI.

According to certain embodiments, the catalyst is selected from Catalyst B1, Catalyst B3, Catalyst B5, Catalyst B7, Catalyst B9, Catalyst B11, Catalyst B13, Catalyst B15, Catalyst B17, and/or Catalyst B19. According to certain embodiments, the catalyst is selected from Catalyst B2, Catalyst B4, Catalyst B6, Catalyst B8, Catalyst B10, Catalyst B12, Catalyst B14, Catalyst B16, Catalyst B18, Catalyst B20, and/or Catalyst B22, preferably Catalyst B20 and/or Catalyst B22.

According to certain embodiments, the catalyst is selected from the group consisting of Catalyst B-I, Catalyst B-II, Catalyst B-III, Catalyst B-IV, Catalyst B-V, Catalyst B-VI, Catalyst B-VII, Catalyst B-VIII, Catalyst B-XI, Catalyst B-XII, Catalyst B-XV, Catalyst B-XIX, Catalyst B-XX, Catalyst B-XXI, Catalyst B-XXII, Catalyst B-XXIII, Catalyst B-XXIV, Catalyst B-XXIX, Catalyst B-XXX, Catalyst B-XXXI, Catalyst B-XXXII, Catalyst B-XXXIII, Catalyst B-XXXIV, Catalyst B-XXXV, Catalyst B-XXXVI, Catalyst B-XXXVII, Catalyst B-XXXVIII, Catalyst B-XXXIX, Catalyst B-XL, Catalyst B-XLI, Catalyst B-XLII, Catalyst B-XLIII, Catalyst B-XLIV, Catalyst B-XLV, Catalyst B-XLVI, Catalyst B-XLVII, Catalyst B-XLVIII, Catalyst B-XLIX, Catalyst B-L, Catalyst B-LI, Catalyst B-LII, Catalyst B-LIII, Catalyst B-LIV, Catalyst B-LV, Catalyst B-LVI, Catalyst B-LVII, Catalyst B-LVIII, Catalyst B-LIX, Catalyst B-LX, Catalyst B-LXI, Catalyst B-LXII, Catalyst B-LXIII, Catalyst B-LXIV, Catalyst B-LXV, Catalyst B-LXVI, Catalyst B-LXVII, Catalyst B-LXVIII, Catalyst B-LXIX, Catalyst B-LXX, Catalyst B-LXXI, and Catalyst B-LXXII, e.g. from the group consisting of Catalyst B-I, Catalyst B-II, Catalyst B-III, Catalyst B-IV, Catalyst B-V, Catalyst B-VI, Catalyst B-VII, Catalyst B-VIII, Catalyst B-XI, Catalyst B-XII, Catalyst B-XV, Catalyst B-XIX, Catalyst B-XX, Catalyst B-XXI, Catalyst B-XXII, Catalyst B-XXIII, Catalyst B-XXIV, Catalyst B-XXIX, Catalyst B-XXX, Catalyst B-XLVII, Catalyst B-XLVIII, Catalyst B-LIII, Catalyst B-LIV, Catalyst B-LXV, Catalyst B-LXVI, Catalyst B-LXVII, Catalyst B-LXVIII, Catalyst B-LXIX, Catalyst B-LXX, Catalyst B-LXXI, and Catalyst B-LXXII, or from the group consisting of Catalyst B-I, Catalyst B-II, Catalyst B-III, Catalyst B-IV, Catalyst B-XXXI, Catalyst B-XXXII, Catalyst B-XXXIII, Catalyst B-XXXIV, Catalyst B-XXXV, Catalyst B-XXXVI, Catalyst B-XXXVII, Catalyst B-XXXVIII, Catalyst B-XXXIX, Catalyst B-XL, Catalyst B-XLI, Catalyst B-XLII, Catalyst B-XLIII, Catalyst B-XLIV, Catalyst B-XLV, Catalyst B-XLVI, Catalyst B-XLVII, Catalyst B-XLVIII, Catalyst B-XLIX, Catalyst B-L, Catalyst B-LI, Catalyst B-LII, Catalyst B-LIII, Catalyst B-LIV, Catalyst B-LV, Catalyst B-LVI, Catalyst B-LVII, Catalyst B-LVIII, Catalyst B-LIX, Catalyst B-LX, Catalyst B-LXI, Catalyst B-LXII, Catalyst B-LXIII, Catalyst B-LXIV, Catalyst B-LXV, Catalyst B-LXVI Catalyst B-LXVII, Catalyst B-LXVIII, Catalyst B-LXIX, Catalyst B-LXX, Catalyst B-LXXI, and Catalyst B-LXXII. In preferred embodiments, the catalyst is selected from the group consisting of Catalyst B-I, Catalyst B-II, Catalyst B-III, Catalyst B-IV, Catalyst B-V, Catalyst B-VI, Catalyst B-XI, Catalyst B-XII Catalyst B-XV, Catalyst B-XVI, Catalyst B-XIX, Catalyst B-XX, Catalyst B-XXV, Catalyst B-XXVI, Catalyst B-XXIX, Catalyst B-XXX, Catalyst B-XXXI, Catalyst B-XXXII, Catalyst B-XXXIII, Catalyst B-XXXIV, Catalyst B-XXXV, Catalyst B-XXXVI, Catalyst B-XXXVII, Catalyst B-XXXVIII, Catalyst B-XXXIX, Catalyst B-XL, Catalyst B-XLI, Catalyst B-XLII, Catalyst B-XLIII, Catalyst B-XLIV, Catalyst B-XLV, Catalyst B-XLVI, Catalyst B-XLVII, Catalyst B-XLVIII, Catalyst B-XLIX, Catalyst B-L, Catalyst B-LI, Catalyst B-LII, Catalyst B-LIII, Catalyst B-LIV, Catalyst B-LV, Catalyst B-LVI, Catalyst B-LVII, Catalyst B-LVIII, Catalyst B-LIX, Catalyst B-LX, Catalyst B-LXI, Catalyst B-LXII, Catalyst B-LXIII, Catalyst B-LXIV, Catalyst B-LXV, Catalyst B-LXVI, Catalyst B-LXVII, Catalyst B-LXVIII, Catalyst B-LXIX, Catalyst B-LXX, Catalyst B-LXXI, and Catalyst B-LXXII, preferably from the group consisting of Catalyst B-I, Catalyst B-II, Catalyst B-III, Catalyst B-IV, Catalyst B-XXXI, Catalyst B-XXXII, Catalyst B-XXXIII, Catalyst B-XXXIV, Catalyst B-XXXV, Catalyst B-XXXVI, Catalyst B-XXXVII, Catalyst B-XXXVIII, Catalyst B-XXXIX, Catalyst B-XL, Catalyst B-XLI, Catalyst B-XLII, Catalyst B-XLIII, Catalyst B-XLIV, Catalyst B-XLV, Catalyst B-XLVI, Catalyst B-XLVII, Catalyst B-XLVIII, Catalyst B-XLIX, Catalyst B-L, Catalyst B-LI, Catalyst B- LII, Catalyst B-LIII, Catalyst B-LIV, Catalyst B-LV, Catalyst B-LVI, Catalyst B-LVII, Catalyst B-LVIII, Catalyst B-LIX, Catalyst B-LX, Catalyst B-LXI, Catalyst B-LXII, Catalyst B-LXIII, Catalyst B-LXIV, Catalyst B-LXV, Catalyst B-LXVI, Catalyst B-LXVII, Catalyst B-LXVIII, Catalyst B-LXIX, Catalyst B-LXX, Catalyst B-LXXI, and Catalyst B-LXXII, in particular from the group consisting of Catalyst B-I, Catalyst B-II, Catalyst B-III, Catalyst B-IV, Catalyst B-XLVII, Catalyst B-XLVIII, Catalyst B-LIII, Catalyst B-LIV, Catalyst B-LXV, and Catalyst B-LXVI. According to certain embodiments, the catalyst is selected from the group consisting of Catalyst B-I, Catalyst B-III, Catalyst B-V, Catalyst B-VII, Catalyst B-IX, Catalyst B-XI, Catalyst B-XIII, Catalyst B-XV, Catalyst B-XVII, Catalyst B-XIX, Catalyst B-XXI, Catalyst B-XXIII, Catalyst B-XXV, Catalyst B-XXVII, Catalyst B-XXIX, Catalyst B-XXXI, Catalyst B-XXXIII, Catalyst B-XXXV, Catalyst B-XXXVII, Catalyst B-XXXIX, Catalyst B-XLI, Catalyst B-XLIII, Catalyst B-XLV, Catalyst B-XLVII, Catalyst B-XLIX, Catalyst B-LI, Catalyst B-LIII, Catalyst B-LV, Catalyst B-LVII, Catalyst B-LIX, Catalyst B-LXI, Catalyst B-LXIII, Catalyst B-LXV, Catalyst B-LXVII, Catalyst B-LXIX, and Catalyst B-LXXI, preferably Catalyst B-I, Catalyst B-III, Catalyst B-LIII, Catalyst B-XLVII, and Catalyst B-LXV. According to certain embodiments, the catalyst is selected from the group consisting of Catalyst B-II, Catalyst B-IV, Catalyst B-VI, Catalyst B-VIII, Catalyst B-X, Catalyst B-XII, Catalyst B-XIV, Catalyst B-XVI, Catalyst B-XVIII, Catalyst B-XX, Catalyst B-XXII, Catalyst X B-XIV, Catalyst B-XXVI, Catalyst B-XXVIII, Catalyst B-XXX, Catalyst B-XXXII, Catalyst B-XXXIV, Catalyst B-XXXVI, B-Catalyst XXXVIII, Catalyst B-XL, Catalyst B-XLII, Catalyst B-XLIV, Catalyst B-XLVI, Catalyst B-XLVIII, Catalyst B-L, Catalyst B-LII, Catalyst B-LIV, Catalyst B-LVI, Catalyst B-LVIII, Catalyst B-LX, Catalyst B-LXII, Catalyst B-LXIV, Catalyst B-LXVI, Catalyst B-LXVIII, Catalyst B-LXX, and Catalyst B-LXXII, preferably Catalyst II, Catalyst B-IV, Catalyst B-XLVIII, Catalyst B-LIV, and Catalyst B-LXVI.

In a further, eighth aspect, the present invention relates to the use of the catalyst of the seventh aspect (i.e. the eighth aspect relates to the use of the catalyst of the following formula I 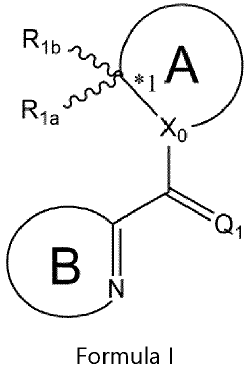 wherein
Ring A comprises, besides X₀ and the chiral C atom with residues R₁ₐ and R_{1b}, a carbonyl group, a thiocarbonyl group, substituted or unsubstituted C atoms and/or substituted or unsubstituted heteroatoms, wherein the substituents can comprise any one of F; Cl; Br; I; pseudohalogen groups; alkoxy groups with 1 to 20 C atoms; OH; NO₂; NH₂; amines of formula NR₁₈R₁₉; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; linear alkenyl groups and/or alkynyl groups with 2 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; branched alkenyl and/or alkynyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂; with the provisio that two or more of the substituents can form one ring or more rings, optionally wherein at least one substituent is part of a polymer;
Ring B is a five- or six-membered ring comprising conjugated double bonds that is particularly aromatic, i.e. is fully conjugated, planar and follows the Hueckel's rule, and that comprises, besides the carbon atom connected to the bridge and the adjacent nitrogen atom, substituted or unsubstituted C atoms and/or substituted or unsubstituted heteroatoms, wherein the substituents can comprise any one of F; Cl; Br; I; pseudohalogen groups; alkoxy groups with 1 to 20 C atoms; OH; NO₂; NH₂; amines of formula NR₁₈R₁₉; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂; with the provisio that two or more of the substituents can form one ring or more rings;
wherein R₁₈ and R₁₉ are the same or different and can be chosen from H, linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂, with the provisio that not both of R₁₈ and R₁₉ are H;
X₀ is selected from N and CR_{A1}, wherein
R_{A1} is selected from the group consisting of H, linear alkyl groups with 1 to 6 C atoms that can optionally be substituted with at least one of F, Cl, Br, I, pseudohalogen groups, alkoxy groups with 1 to 6 C atoms, OH, NO₂ and NH₂; branched alkyl groups with 3 to 6 C atoms that can optionally be substituted with at least one of F, Cl, Br, I, pseudohalogen groups, alkoxy groups with 1 to 6 C atoms, OH, NO₂ and NH₂; cycloalkyl groups with 3 to 7 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 6 C atoms that can optionally be substituted with at least one of F, Cl, Br, I, pseudohalogen groups, alkoxy groups with 1 to 6 C atoms, OH, NO₂ and NH₂; aromatic and/or heteroaromatic groups with 2 to 6 carbon atoms that can optionally be substituted with at least one of F, Cl, Br, I, pseudohalogen groups, alkoxy groups with 1 to 6 C atoms, OH, NO₂ and NH₂; carboxylic acid ester groups with 1 to 6 C atoms that can optionally be substituted with at least one of F, Cl, Br, I, pseudohalogen groups, alkoxy groups with 1 to 6 C atoms, OH, NO₂ and NH₂; alkyl and/or (hetero)aryl oxycarbonyl groups with 1 to 6 C atoms that can optionally be substituted with at least one of F, Cl, Br, I, pseudohalogen groups, alkoxy groups with 1 to 6 C atoms, OH, NO₂ and NH₂; and carboxamide groups with 1 to 6 C atoms that can optionally be substituted with at least one of F, Cl, Br, I, pseudohalogen groups, alkoxy groups with 1 to 6 C atoms, OH, NO₂ and NH₂;
Q₁ is O or S;
R₁ₐ and R_{1b} are different;
R₁ₐ is a saturated and/or unsaturated linear, branched, cyclic and/or aromatic and/or heteroaromatic hydrocarbon residue with 1 to 100 C atoms that can contain heteroatoms selected from the group of O, N, S and P and that can optionally be substituted with at least one of F; Cl; Br; I; pseudohalogen groups; OSiR₂₃R₂₄R₂₅; carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂; carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂; optionally substituted linear, branched and/or cyclic alkoxy, aryloxy, heteroaryloxy, alkenyloxy and/or alkynyloxy groups with 1 to 20 C atoms and/or heteroatoms; OH; NO₂; SH; NH₂; and amines of formula NR₁₈R₁₉;
R₂₃, R₂₄, and R₂₅ are the same or different and are selected from H, linear alkyl groups with 1 to 4 C atoms that can optionally be substituted with at least one of F, Cl, Br, I, pseudohalogen groups, alkoxy groups with 1 to 20 C atoms, OH, NO₂ and NH₂, branched alkyl group with 3 to 6 C atoms that can optionally be substituted with at least one of F, Cl, Br, I, pseudohalogen groups, alkoxy groups with 1 to 20 C atoms, OH, NO₂ and NH₂, aromatic groups with 6 to 12 C atoms that can optionally be substituted with at least one of F, Cl, Br, I, pseudohalogen groups, alkoxy groups with 1 to 20 C atoms, OH, NO₂ and NH₂, and aralkyl groups with 7 to 10 C atoms that can optionally be substituted with at least one of F, Cl, Br, I, pseudohalogen groups, alkoxy groups with 1 to 20 C atoms, OH, NO₂ and NH₂, preferably wherein R₂₃, R₂₄, and R₂₅ are not all hydrogen at the same time and/or wherein R₂₃, R₂₄, and R₂₅ are not all methyl at the same time, further preferably wherein all of R₂₃, R₂₄, and R₂₅ are ethyl;
R_{1b} is selected from the group consisting of H, linear alkyl groups with 1 to 6 C atoms that can optionally be substituted with at least one of F, Cl, Br, I, pseudohalogen groups, alkoxy groups with 1 to 6 C atoms, OH, NO₂ and NH₂; branched alkyl groups with 3 to 6 C atoms that can optionally be substituted with at least one of F, Cl, Br, I, pseudohalogen groups, alkoxy groups with 1 to 6 C atoms, OH, NO₂ and NH₂; heterocycloalkyl groups with 1 to 6 C atoms that can optionally be substituted with at least one of F, Cl, Br, I, pseudohalogen groups, alkoxy groups with 1 to 6 C atoms, OH, NO₂ and NH₂; aromatic and/or heteroaromatic groups with 2 to 6 carbon atoms that can optionally be substituted with at least one of F, Cl, Br, I, pseudohalogen groups, alkoxy groups with 1 to 6 C atoms, OH, NO₂ and NH₂; carboxylic acid ester groups with 1 to 6 C atoms that can optionally be substituted with at least one of F, Cl, Br, I, pseudohalogen groups, alkoxy groups with 1 to 6 C atoms, OH, NO₂ and NH₂; alkyl and/or (hetero)aryl oxycarbonyl groups with 1 to 6 C atoms that can optionally be substituted with at least one of F, Cl, Br, I, pseudohalogen groups, alkoxy groups with 1 to 6 C atoms, OH, NO₂ and NH₂; and carboxamide groups with 1 to 6 C atoms that can optionally be substituted with at least one of F, Cl, Br, I, pseudohalogen groups, alkoxy groups with 1 to 6 C atoms, OH, NO₂ and NH₂, preferably wherein R_{1b} is H or methyl;
wherein the stereocenter *1 of the catalyst according to Formula I is in (R) and/or (S) configuration) in a method of producing nornicotine and/or nicotine and/or a salt of nicotine, particularly from myosmine.

This means that the eighth aspect also relates to the catalyst used in the method of the first aspect. Accordingly, the catalyst used in the eighth aspect also relates to all the further restricted catalysts disclosed with regard to the first aspect, particularly also the specific catalysts mentioned with regard to the first aspect.

A further, ninth aspect relates to method of producing a compound of Formula X-2 from a compound of Formula X-1, comprising:
reacting the compound of Formula X-1 with at least one hydrosilane using a catalyst,
wherein the catalyst is an organic chiral Lewis base, 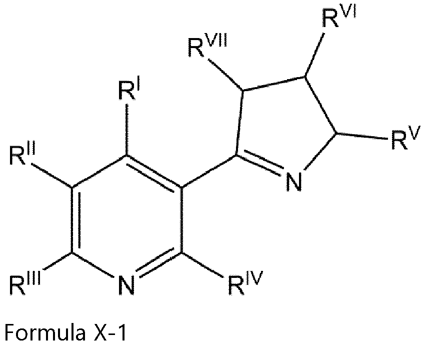 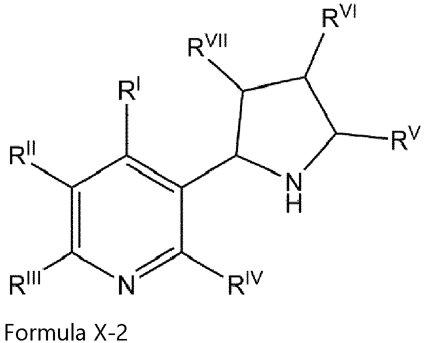
wherein in Formula X-1 and in Formula X-2 R^{I}, R^{II}, Rⁱⁱⁱ, R^{IV}, R^{V}, R^{VI}, and R^{VII} can be the same or different and are not particularly restricted, but preferably can be chosen from H; F; Cl; Br; I; pseudohalogen groups; alkoxy groups with 1 to 20 C atoms; OH; NH₂; NO₂;
linear alkyl groups with 1 to 10 C atoms that can optionally be substituted with at least one of F, Cl, Br, I, pseudohalogen groups, alkoxy groups with 1 to 20 C atoms, OH, NO₂ and NH₂;
branched alkyl groups with 3 to 10 C atoms that can optionally be substituted with at least one of F, Cl, Br, I, pseudohalogen groups, alkoxy groups with 1 to 20 C atoms, OH, NO₂ and NH₂;
aralkyl groups with 7 to 24 C atoms that can optionally be substituted with at least one of F, Cl, Br, I, pseudohalogen groups, alkoxy groups with 1 to 20 C atoms, OH, NO₂ and NH₂;
alkylaryl groups with 7 to 24 C atoms that can optionally be substituted with at least one of F, Cl, Br, I, pseudohalogen groups, alkoxy groups with 1 to 20 C atoms, OH, NO₂ and NH₂;
cycloalkyl groups with 3 to 20 C atoms that can optionally be substituted with at least one of F, Cl, Br, I, pseudohalogen groups, alkoxy groups with 1 to 20 C atoms, OH, NO₂ and NH₂;
heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂;
aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂;
alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂;
heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂;
alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂;
cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂;
alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂;
heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂;
carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂;
alkly and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂;
and carboxamide groups with 1 to 20 C atoms that can optionally be substituted with at least one of F, Cl, Br, I, pseudohalogen groups, alkoxy groups with 1 to 20 C atoms, OH, NO₂ and NH₂;
with the provisio that not all of R^{I}, R^{II}, Rⁱⁱⁱ, R^{IV}, R^{V}, R^{VI}, and R^{VII} are H.

The hydrosilane and the catalyst that is an organic chiral Lewis base are not particularly restricted but are preferably those mentioned with regard to the method of the first aspect. Furthermore, further reaction conditions, like further additives, solvents, temperature, reaction time, etc., are not particularly restricted but can be, according to certain embodiments, be those that are described with regard to the method of the first aspect. The compound of Formula X-2 can be in (R)- and/or (S)-configuration.

In addition, in a tenth aspect, a composition produced by the method according to the ninth aspect is disclosed, particularly wherein the catalyst is not removed from the reaction mixture.

Surprisingly it has been found that the reaction mixture with the catalyst can be kept stable, thus allowing also transfer for work-up in bigger batches, saving solvent and energy in the work-up step, e.g. a quench, distillation and/or extraction. Surprisingly, it has been found that the composition produced can be kept stably overnight, nevertheless allowing normal workup afterwards.

An eleventh aspect of the present invention relates to a composition, comprising a compound of Formula X-2, and a catalyst, wherein the catalyst is an organic chiral Lewis base; 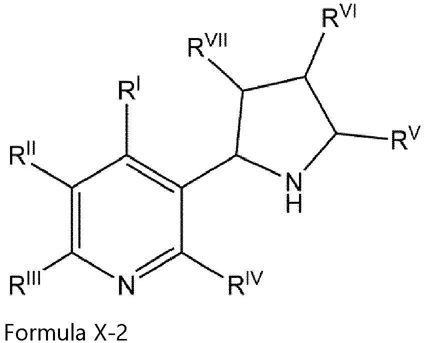
wherein in Formula X-2 R^{I}, R^{II}, Rⁱⁱⁱ, R^{IV}, R^{V}, R^{VI}, and R^{VII} can be the same or different and are not particularly restricted, but preferably can be chosen from H; F; Cl; Br; I; pseudohalogen groups; linear alkyl groups with 1 to 10 C atoms that can optionally be substituted with at least one of F, Cl, Br, I, pseudohalogen groups, alkoxy groups with 1 to 20 C atoms, OH, NO₂ and NH₂;
branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂;
cycloalkyl groups with 3 to 20 C atoms that can optionally be substituted with at least one of F, Cl, Br, I, pseudohalogen groups, alkoxy groups with 1 to 20 C atoms, OH, NO₂ and NH₂;
heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂;
aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂;
alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂;
heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂;
alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂;
cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂;
alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂;
heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂;
carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂;
alkly and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂;
and carboxamide groups with 1 to 20 C atoms that can optionally be substituted with at least one of F, Cl, Br, I, pseudohalogen groups, alkoxy groups with 1 to 20 C atoms, OH, NO₂ and NH₂;
with the provisio that not all of R^{I}, R", Rⁱⁱⁱ, R^{IV}, R^{V}, R^{VI}, and R^{VII} are H.

The catalyst that is an organic chiral Lewis base is not particularly restricted but is preferably the catalyst mentioned with regard to the method of the first aspect.

According to certain embodiments, the enantiomeric excess (ee) of one stereoisomer of the compound of Formula X-2 is in the range of 5 to 100%, optionally in the range of 10 to 99.9%, further optionally in the range of 20 to 99%

A twelfth aspect relates to a method of producing a compound of Formula X-3 or a salt thereof from a compound of Formula X-1, comprising:
reacting the compound of Formula X-1 with at least one hydrosilane in the presence of a catalyst to produce a compound of Formula X-2, wherein the catalyst is an organic chiral Lewis base;
reacting the compound of Formula X-2 to the compound of Formula X-3; and
optionally reacting the nicotine to a salt thereof; 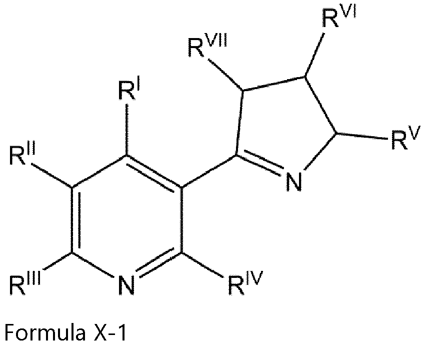 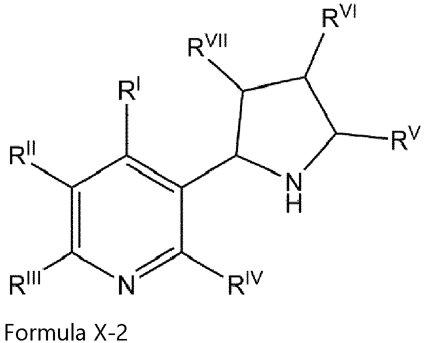 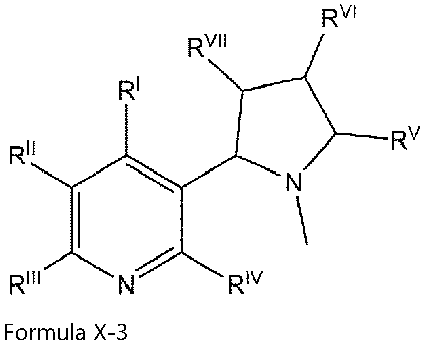
wherein in Formula X-1, in Formula X-2 and in Formula X-3 R^{I}, R", Rⁱⁱⁱ, R^{IV}, R^{V}, R^{VI}, and R^{VII} can be the same or different and are not particularly restricted, but preferably can be chosen from H; F; Cl; Br; I; pseudohalogen groups;
linear alkyl groups with 1 to 10 C atoms that can optionally be substituted with at least one of F, Cl, Br, I, pseudohalogen groups, alkoxy groups with 1 to 20 C atoms, OH, NO₂ and NH₂;
branched alkyl groups with 3 to 10 C atoms that can optionally be substituted with at least one of F, Cl, Br, I, pseudohalogen groups, alkoxy groups with 1 to 20 C atoms, OH, NO₂ and NH₂;
aralkyl groups with 7 to 24 C atoms that can optionally be substituted with at least one of F, Cl, Br, I, pseudohalogen groups, alkoxy groups with 1 to 20 C atoms, OH, NO₂ and NH₂;
alkylaryl groups with 7 to 24 C atoms that can optionally be substituted with at least one of F, Cl, Br, I, pseudohalogen groups, alkoxy groups with 1 to 20 C atoms, OH, NO₂ and NH₂;
cycloalkyl groups with 3 to 20 C atoms that can optionally be substituted with at least one of F, Cl, Br, I, pseudohalogen groups, alkoxy groups with 1 to 20 C atoms, OH, NO₂ and NH₂;
heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂;
aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂;
alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂;
heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂;
alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂;
cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂;
alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂;
heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂;
carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂;
alkly and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂;
and carboxamide groups with 1 to 20 C atoms that can optionally be substituted with at least one of F, Cl, Br, I, pseudohalogen groups, alkoxy groups with 1 to 20 C atoms, OH, NO₂ and NH₂;
with the provisio that not all of R^{I}, R^{II}, Rⁱⁱⁱ, R^{IV}, R^{V}, R^{VI}, and R^{VII} are H.

The hydrosilane and the catalyst that is an organic chiral Lewis base are not particularly restricted but are preferably those mentioned with regard to the method of the first aspect. Furthermore, further reaction conditions, like further additives, solvents, temperature, reaction time, etc., are not particularly restricted but can be, according to certain embodiments, be those that are described with regard to the method of the first aspect and/or the fourth aspect. The compound of Formula X-2 can be in (R)- and/or (S)-configuration.

A thirteenth aspect relates to a composition, produced by the method of the twelvth aspect, wherein the catalyst is not separated from the reaction mixture.

A fourteenth aspect relates to a composition, comprising a compound of Formula X-3, optionally a mixture of the (R)- and (S)-enantiomer thereof, and/or a salt thereof, and a catalyst, wherein the catalyst is an organic chiral Lewis base 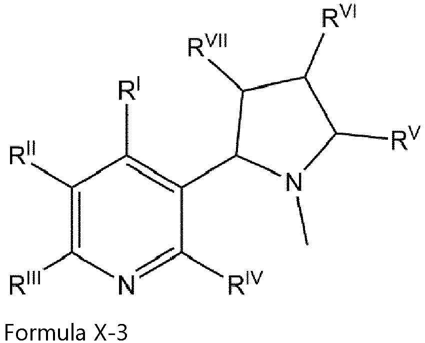
wherein in Formula X-3 R^{I}, R^{II}, Rⁱⁱⁱ, R^{IV}, R^{V}, R^{VI}, and R^{VII} can be the same or different and are not particularly restricted, but preferably can be chosen from H; F; Cl; Br; I; pseudohalogen groups; linear alkyl groups with 1 to 10 C atoms that can optionally be substituted with at least one of F, Cl, Br, I, pseudohalogen groups, alkoxy groups with 1 to 20 C atoms, OH, NO₂ and NH₂;
branched alkyl groups with 3 to 10 C atoms that can optionally be substituted with at least one of F, Cl, Br, I, pseudohalogen groups, alkoxy groups with 1 to 20 C atoms, OH, NO₂ and NH₂;
aralkyl groups with 7 to 24 C atoms that can optionally be substituted with at least one of F, Cl, Br, I, pseudohalogen groups, alkoxy groups with 1 to 20 C atoms, OH, NO₂ and NH₂;
alkylaryl groups with 7 to 24 C atoms that can optionally be substituted with at least one of F, Cl, Br, I, pseudohalogen groups, alkoxy groups with 1 to 20 C atoms, OH, NO₂ and NH₂;
cycloalkyl groups with 3 to 20 C atoms that can optionally be substituted with at least one of F, Cl, Br, I, pseudohalogen groups, alkoxy groups with 1 to 20 C atoms, OH, NO₂ and NH₂;
heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂;
aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂;
alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂;
heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂;
alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂;
cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂;
alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂;
heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂;
carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂;
alkly and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂;
and carboxamide groups with 1 to 20 C atoms that can optionally be substituted with at least one of F, Cl, Br, I, pseudohalogen groups, alkoxy groups with 1 to 20 C atoms, OH, NO₂ and NH₂;
with the provisio that not all of R^{I}, R^{II}, Rⁱⁱⁱ, R^{IV}, R^{V}, R^{VI}, and R^{VII} are H.

The catalyst that is an organic chiral Lewis base is not particularly restricted but is preferably the catalyst mentioned with regard to the method of the first aspect.

According to certain embodiments, the enantiomeric excess (ee) of one stereoisomer of the compound of Formula X-3 is in the range of 5 to 100%, optionally in the range of 10 to 99.9%, further optionally in the range of 20 to 99%

The above embodiments can be combined arbitrarily, if appropriate. Further possible embodiments and implementations of the invention comprise also combinations of features not explicitly mentioned in the foregoing or in the following with regard to the Examples of the invention. Particularly, a person skilled in the art will also add individual aspects as improvements or additions to the respective basic form of the invention.

### Examples

The present invention will now be described in detail with reference to several examples thereof. However, these examples are illustrative and do not limit the scope of the invention.

### Reference Examples A: Catalyst synthesis:

### 1) Catalyst A-I

Catalyst A-I was obtained commercially (Fluorochem Ltd.).

Catalyst A-I was alternatively obtained from A-XLV (described below). A-XLV was dissolved in dry DMF (7 mL) under N₂ atmosphere and precooled to -10°C. NaH (60 % dispersion in mineral oil, 49.8 mg, 1.25 mmol) was added at -10°C and stirred for 0.5 h at rt. Mel (77.5 µL, 1.25 mmol) was added dropwise to the reaction mixture and stirred at RT for 4 h. The reaction mixture was quenched using sat. aq. NH₄Cl (10 mL). The aqueous phase was extracted with ethyl acetate (3x 15 mL) and the combined organic phases were washed with H₂O (2x 15 mL) and dried over Na₂SO₄ and concentrated under reduced pressure. The crude product was purified by column chromatography (gradient: MeCN / H₂O 6:4 in 4 CV to 9:1) to give the catalyst as a colourless solid (0.265 g, 85 %). The ¹H- and ¹³C-NMR spectra are matching the spectra published in Org. Biomol. Chem. 2017, 15, 2422-2435.

### 2) Catalyst A-V (S)-(1-benzyl-1H-imidazol-2-yl)(2-(methoxydiphenylmethyl)pyrrolidin-1-yl)methanone

(S)-diphenyl(pyrrolidin-2-yl)methanol (AB171959 | CAS 112068-01-6 - abcr Gute Chemie) (1.18 g, 4.66 mmol) was dissolved in dry toluene (20 mL) and NaH (60% dispersion in mineral oil, 0.242 g, 6.06 mmol) was added to it at room temperature (rt). After stirring the mixture for 0.5 h at rt, ethyl 1-benzyl-1H-imidazole-2-carboxylate (prepared according to Eur. J. Med. Chem. 2022, 228, 113965 from commercially available bulding blocks (Sigma Aldrich)) (1.20 g, 5.55 mmol) was added. The reaction mixture was heated to 70°C and stirred at the same temperature for another 2.5 h. After cooling the reaction mixture back to rt, the reaction was quenched with saturated aqueous NH₄Cl (30 mL). The phases were separated and the aqueous phase was extracted three times with dichloromethane (3 × 30 mL). The combined organic layers were washed with water (3 × 50 mL), dried over Na₂SO₄ and concentrated under reduced pressure. The resulting oily crude product was dissolved in ethyl acetate/heptane 7:3 (3 mL) and filtered over a short silicagel column using more ethyl acetate/heptane (7:3). The filtrate was concentrated under reduced pressure to give 1.60 g (~ 79 %) of yellowish solid, which was used for the next step without further purification. 0.900 g (2.06 mmol) of the crude product was dissolved in dry DMF (16 mL), the solution was cooled to 0 °C and NaH (60% dispersion in mineral oil, 0.123 g, 3.09 mmol) was added to it. The reaction mixture was stirred for 0.5 h at 0 °C and then let warm to rt. Mel (0.192 mL, 3.09 mmol) was added dropwise and the yellow solution was stirred at rt for another 4 h. The reaction was quenched by the addition of sat. aq. NH₄Cl (15 mL). The aqueous layer was extracted with ethyl acetate (3 × 25 mL). The combined organic layers were washed with water (2 × 25 mL), dried over Na₂SO₄ and concentrated under reduced pressure. The crude product was purified by column chromatography (ethyl acetate/heptane 45:55) to give the product (0.806 g, 69% after two steps) as a white solid. Two rotamers were observed in NMR in CDCl₃ with a ratio of 1.00:0.39.

¹H NMR (400 MHz, CDCl₃, rotamer A) δ 7.52 - 7.28 (m, 14H),7.21 - 7.13 (m, 1H), 6.98 (s, 1H), 6.76 (s, 1H), 6.46 (dd, J = 7.83 Hz, 1H), 5.65 - 5.53 (m, 1H), 5.14 (d, J = 14.87 Hz, 1H), 3.60 - 3.59 (m, 1H), 2.50 - 2.33 (m, 2H), 2.33 (s, 3H), 2.10 - 1.90 (m, 1H), 1.68 - 1.58 (m, 1H), 1.11 - 1.07 (m, 1H). ¹H NMR (400 MHz, CDCl₃, rotamer B) δ 7.52 - 7.28 (m, 15H), 7.09 (s, 1H), 6.92 (s, 1H), 5.65 - 5.53 (m, 2H), 5.37 - 5.30 (m, 1H), 3.50 - 3.49 (m, 1H), 3.03 (s, 3H), 2.99 - 2.84 (m, 1H), 2.10 - 1.90 (m, 2H), 1.43 - 1.19 (m, 1H), 1.11 - 1.07 (m, 1H). ¹³C NMR (101 MHz, CDCl₃, rotamer A) δ 161.0, 142.4, 139.9, 138.9, 136.7, 130.2, 129.5 (multiple signals), 128.8 (multiple signals), 127.7 (multiple signals), 127.1, 121.1, 86.9, 63.4, 51.8, 51.0, 47.6, 27.7, 21.9. ¹³C NMR (101 MHz, CDCl₃, rotamer B) δ 161.5, 141.3, 141.1, 140.0, 137.4, 130.0, 129.7 (multiple signals), 128.8 (multiple signals), 127.7 (multiple signals), 127.5, 122.6, 86.7, 60.6, 52.6, 51.1, 50.6, 26.7, 23.8. LC-MS: [M - OMe]⁺ = 420.2 m/z, [M + H]⁺ = 452.2 m/z, calculated mass 451.2 Da.

### 3) Catalyst A-XIX (S)-(2-(methoxydiphenylmethyl)pyrrolidin-1-yl)(1-methyl-1H-imidazol-2-yl)methanethione

Catalyst A-I (1.00 g, 2.66 mmol) was dissolved in dry 1,4-dioxane (7 mL) and Lawesson's reagent (0.539 g, 1.33 mmol) was added to the solution. The reaction mixtures was heated to 100°C and stirred for 5 h at the same temperature. As conversion was not complete, 0.15 equiv. of Lawesson's reagent (0.162 g, 0.401 mmol) was added to the reaction mixture and the yellow solution was stirred for another 17 h at 100°C. The reaction was cooled down and concentrated under reduced pressure. The crude product was purified by column chromatography (heptane/EtOAc gradient 35-55% EtOAc) to give the product (0.772 g, 74%) as a yellow solid. Only a trace of the opposite rotamer was observed in the NMR spectrum and the minor rotamer was not further characterized.

¹H NMR (400 MHz, CDCl₃, rotamer A) δ 7.36 - 7.26 (m, 10H), 6.97 (s, 1H), 6.82 (s, 1H), 6.50 (d, *J* = 9.39 Hz 1H), 3.97 - 3.92 (m, 1H), 3.90 (s, 3H), 2.53 - 2.44 (m, 2H), 2.36 (s, 3H), 2.06 - 2.00 (m, 1H), 1.65 - 1.61 (m, 1H), 1.04 - 0.99 (m, 1H). ¹³C NMR (101 MHz, CDCl₃) δ 186.5, 147.1, 138.6, 137.9, 130.1 (multiple signals), 129.5 (multiple signals), 128.1, 128.0, 126.0, 122.8, 86.9, 67.2, 54.9, 51.7, 35.2, 27.4, 21.6. LC-MS: [M - OMe]⁺ = 360.1 m/z, [M + H]⁺ = 392.2 m/z, calculated mass 391.2 Da.

### 4) Catalyst A-XLV (S)-(2-(hydroxydiphenylmethyl)pyrrolidin-1-yl)(1-methyl-1H-imidazol-2-yl)methanone

Catalyst A-XLV was prepared according to the procedure described in Org. Biomol. Chem. 2017, 15, 2422-2435 and purified by crystallization from EtOAc/heptane. Catalyst A-XLV was obtained as a colourless solid in 86% yield. LC-MS: [M - OH]⁺ = 344.2 m/z, [M + H]⁺ = 362.2 m/z, calculated M_{w} 361.2

### 5) Catalyst A-VII (S)-(2-(ethoxydiphenylmethyl)pyrrolidin-1-yl)(1-methyl-1H-imidazol-2-yl)methanone

Catalyst A-VII was prepared analogously to catalyst Catalyst A-I. Catalyst A-XLV (1.00 g, 2.77 mmol), NaH (60 % dispersion in mineral oil, 0.166 g, 4.15 mmol) and Etl (0.335 mL, 4.15 mmol) gave Catalyst A-VII as a colourless solid (0.320 g, 30 %). Two rotamers were observed in NMR in CDCl₃ with a ratio of 1.00 : 0.21.

¹H NMR (400 MHz, CDCl₃, rotamer A) δ 7.39 - 7.23 (m, 10H), 6.97 (s, 1H), 6.77 (s, 1H), 6.68 (dd, J = 9.00, 1.39 Hz, 1H), 3.74 (s, 3H), 3.64 (ddd, J = 12.17, 9.63, 5.32 Hz, 1H), 2.90 - 2.83 (m, 1H), 2.57 - 2.43 (m, 2H), 2.36 - 2.29 (m, H), 2.05 - 1.96 (m, 1H), 1.67 - 1.57 (m, 1H), 1.24 - 1.16 (m, 1H), 0.76 (t, J = 6.97 Hz, 3H). ¹H NMR (400 MHz, CDCl₃, rotamer B) δ 7.52 (d, J = 7.10 Hz, 2H), 7.46 (d, *J* = 7.10 Hz, 2H), 7.39 - 7.23 (m, 6H), 7.07 (s, 1H), 6.87 (s, 1H), 5.68 (dd, *J* = 1.00 Hz, 1H), 3.82 (s, 3H), 3.74 - 3.82 (m, 1H), 3.40 - 3.36 (m, 1H), 3.18 - 3.10 (m, 1H), 2.90 - 2.83 (m, 1H), 2.36 - 2.29 (m, 1H), 2.05-1.96 (m, 1H), 1.67 - 1.57 (m, 1H), 1.24 - 1.16 (m, 1H), 1.10 (t, *J* = 6.84. Hz, 3H). ¹³C NMR (101 MHz, CDCl₃, rotamer A) δ 160.7, 142.1, 139.2, 138.8, 129.7 (multiple signals), 127.7 (multiple signals), 127.6, 126.5, 122.4, 86.7, 65.2, 58.9, 47.3, 34.9, 27.5, 21.9, 14.9. ¹³C NMR (101 MHz, CDCl₃, rotamer B) δ 160.9, 141.7, 140.9 140.7, 129.7 (multiple signals), 127.7 (multiple signals), 127.6 (multiple signals), 123.8, 86.3, 61.2, 59.6, 50.7, 35.4, 26.6, 24.1, 15.5. LC-MS: [M - OEt]⁺ = 344.2 m/z, [M + H]⁺ = 390.2 m/z, calculated mass 389.2 Da

### 6) Catalyst A-IX (S)-(2-(bis(3,5-dimethylphenyl)(methoxy)methyl)pyrrolidin-1-yl)(1-methyl-1H-imidazol-2-yl)methanone

Catalyst A-IX was prepared analogously to catalyst Catalyst A-V. (S)-bis(3,5-dimethylphenyl)(pyrrolidin-2-yl)methanol (Sigma Aldrich) (0.500 g, 1.62 mmol), dry toluene (5 mL), NaH (60% dispersion in mineral oil, 0.0840 g, 2.11 mmol) and ethyl-1-methyl-1*H-*imidazole-2-carboxylate (AB245872 | CAS 30148-21-1 - abcr Gute Chemie) (0.296 g, 1.92 mmol) gave (S)-(2-(bis(3,5-dimethylphenyl)(hydro-xy)methyl)pyrrolidin-1-yl)(1-methyl-1H-imidazol-2-yl)methanone as a white solid (0.675 g, ~81%) after purification with column chromatography (heptane/EtOAc, gradient 35-100%). 0.544 g (1.30 mmol) of the purified product, NaH (60% dispersion in mineral oil, 0.0782 g, 1.95 mmol), DMF (8 mL) and Mel (0.122 mL, 1.95 mmol) yielded the catalyst (0.354 g, 59 %) as a white solid after purification with reverse phase column chromatography (MeCN/H₂O 60-90%). Two rotamers were observed in NMR in CDCl₃ with a ratio of 1.00 : 0.27.

¹H NMR (400 MHz, CDCl₃, rotamer A) δ 7.11 (br d, *J* = 7.83 Hz, 1H) 7.06 - 6.86 (m, 6H), 6.80 (s, 1H), 6.18 (d, *J* = 7.83 Hz, 1H), 3.84 (s, 3H), 3.58 - 3.51 (m, 1H), 2.37 - 2.31 (m, 2H), 2.33 (s, 3H), 2.30 (s, 6H), 2.26 (s, 6H), 2.01 - 1.99 (m, 1H), 1.60 - 1.56 (m, 1H), 1.08 - 1.06 (m, 1H). ¹H NMR (400 MHz, CDCl₃, rotamer B) δ 7.06 - 6.86 (m, 8H), 5.60 (br dd, *J* = 9.00, 3.00 Hz 1H), 3.90 (s, 3H), 3.78 - 3.65 (m, 1H), 2.98 (s, 3H), 2.78 - 2.88 (m, 1H), 2.43 - 2.33 (m, 1H), 2.30 (s, 6H), 2.26 (s, 6H), 2.01 - 1.99 (m, 1H), 1.60 - 1.56 (m, 1H), 1.08 - 1.06 (m, 1H). ¹³C NMR (101 MHz, CDCl₃, rotamer A) δ 161.0, 142.9, 140.1, 139.1, 137.1, 136.9, 136.7, 129.4 (multiple signals), 129.1 (multiple signals), 128.0, 127.4, 126.7, 122.4, 86.9, 63.4, 51.7, 47.3, 34.2, 28.0, 21.9, 21.7, 21.6.13C NMR (101 MHz, CDCl₃, rotamer B) δ 161.0, 141.1, 140.9, 140.9, 136.8 (multiple signals), 129.4 (multiple signals), 129.1 (multiple signals), 127.9, 127.4, 127.1, 124.0, 87.3, 60.6, 52.5, 50.9, 35.5, 26.8, 24.2, 21.7, 21.8. LC-MS: [M -OMe]⁺ = 400.2 m/z, [M + H]⁺ = 432.3 m/z, calculated mass 431.3 Da.

### 7) Catalyst LXLVII (R₂₃, R₂₄ = methyl, R₂₅ = t-butyl) (S)-(2-((tert-butyldimethylsilyloxy)diphenylmethyl)pyrrolidin-1-yl)(1-methyl-1H-imidazol-2-yl)methanone

Catalyst A-XLV (0.200 g, 0.553 mmol) was dissolved in dry DCM (1.0 mL) under N₂ atmosphere and precooled to 0°C. 2,6-Lutidine (0.446 mL, 3.83 mmol) and tert-butyldimethylsilyl triflate (TBSOTf) (0.625 mL, 2.72 mmol) were added to it at 0°C and the reaction was let warm up to rt. After stirring the reaction mixture for 5 hours at the same temperature, it was quenched with sat. aq. NH₄Cl (1 mL). The layers were separated and the aqueous layer was extracted with EtOAc (3 × 3 mL). The combined organic layers were washed with 9% aq. NaHCO₃ (3 × 3 mL), then with H₂O (2 × 3 mL) and finally dried over Na₂SO₄ and concentrated under reduced pressure. The crude product was purified by column chromatography (EtOAc/Heptane: 30 % EtOAc isocratic) to give the product as a colourless oil (0.222 g, 85 %). Two rotamers were observed in NMR in CDCl₃ with a ratio of 1.00 : 0.87.

¹H NMR (400 MHz, CDCl₃, rotamer A) δ 7.32 - 7.14 (m, 10H), 6.91 (br s, 1H), 6.69 (br s, 2H), 3.82 - 3.70 (m, 1H), 3.25 (s, 3H), 3.20 - 3.13 (m, 1H), 2.31 - 2.25 (m, 1H), 2.11 - 2.07 (m, 1H), 1.91 - 1.77 (m, 1H), 1.67 - 1.61 (m, 1H), 0.88 (s, 9H), -0.23 (s, 3H), -0.51 (s, 3H). ¹H NMR (400 MHz, CDCl₃, rotamer B) δ 7.64 - 7.55 (m, 10H), 7.02 (br s, 1H), 6.86 (br s, 1H), 5.81 (d, *J* = 6.87 Hz, 1H), 4.00 - 3.92 (m, 1H), 3.82 (s, 3H), 3.01 - 2.93 (m, 1H), 2.58 - 2.51 (m, 2H), 1.67 - 1.61 (m, 1H), 1.13 - 1.10 (m, 1H), 0.93 (s, 9H), -0.34 (s, 3H), -0.40 (s, 3H).¹³C NMR (400 MHz, CDCl₃, rotamer A) δ 159.7, 144.1, 140.9 (multiple signals), 130.5 (multiple signals), 130.2 (multiple signals), 128.7 (multiple signals), 127.8 (multiple signals), 127.7 (multiple signals), 126.5, 123.2, 86.0, 65.4, 47.6, 34.9, 26.4, 26.4, 22.9, 19.2, -2.23, -3.1 (multiple signals). ¹³C NMR (400 MHz, CDCl₃, rotamer B) δ 161.7, 143.6, 140.9 (multiple signals), 130.5 (multiple signals), 130.2 (multiple signals), 128.7 (multiple signals),127.8 (multiple signals), 127.7 (multiple signals), 126.5, 124.0, 84.6, 62.4, 51.2, 35.5, 28.2, 26.4, 24.2, 14.3, -2.2, -3.1 (multiple signals). LC-MS: [M - OTBMS]⁺ = 344.2 m/z, calculated mass 475.3 Da

### 8) Catalyst A-XI (S)-(2-(diphenyl(prop-2-ynyloxy)methyl)pyrrolidin-1-yl)(1-methyl-1H-imidazol-2-yl)methanone

Catalyst A-XI was synthesized analogously to Catalyst A-I. Catalyst A-XLV (0.500 g, 1.39 mmol) in DMF (9 mL), NaH (60 % dispersion in mineral oil, 0.0830 g, 2.08 mmol) and propagyl bromide (80% in toluene, 0.231 mL, 2.08 mmol) gave the product as a yellow oil (0.161 g, 29 %) after the purification with reverse phase column chromatography (MeCN/H₂O 60-90%). Two rotamers were observed in NMR in CDCl₃ with a ratio of 1.00 : 0.21.

¹H NMR (400 MHz, CDCl₃, rotamer A) δ 7.56 - 7.50 (m, 1H), 7.47 - 7.29 (m, 9H), 6.99 (s, 1H), 6.80 (s, 1H), 6.61 (d, *J* = 9.06 Hz, 1H), 3.81 (s, 3H), 3.70 - 3.53 (m, 1H), 3.41 (dd, *J* = 17.32, 2.63 Hz, 1H), 3.12 (d, *J* = 17.15, 2.10 Hz, 1H), 2.53 - 2.43 (m, 1H), 2.42 - 2.30 (m, 1H), 2.29 - 2.19 (m, 1H), 2.12 - 1.90 (m, 1H), 1.75 - 1.53 (m, 1H), 1.35 - 1.12 (m, 1H). ¹H NMR (400 MHz, CDCl₃, rotamer B) δ 7.47 - 7.29 (m, 10H), 7.08 (s, 1H), 6.91 (s, 1H), 5.68 - 5.61 (m, 1H), 4.16 (d, *J* = 15.40 Hz, 1H), 3.91 - 3.87 (m, 1H), 3.87 (s, 3H), 3.57 - 3.63 (m, 1H), 2.29-3.18 (m, 1H), 2.42 - 2.30 (m, 1H) 2.29 - 2.19 (m, 1H), 2.12 - 1.90 (m, 1H), 1.75 - 1.53 (m, 1H), 1.35 - 1.12 (m, 1H). ¹³C NMR (101 MHz, CDCl₃, rotamer A) δ 160.9, 142.0, 138.2, 138.0, 130.0, 129.6 (multiple signals), 128.1 (multiple signals), 127.9 multiple signals), 126.5, 123.0, 87.8, 80.5, 72.8, 64.7, 52.6, 47.4, 35.2, 27.7, 21.9. ¹³C NMR (101 MHz, CDCl₃, rotamer B) δ 161.1, 141.0, 139.5 (signals), 130.1 (multiple signals), 129.9 (multiple signals), 128.4, 128.2, 125.4, 124.0, 87.3, 81.3, 72.9, 61.2, 53.2, 50.8, 35.6, 26.8, 24.1. LC-MS: [M - O-propargyl]⁺ = 344.2 m/z, [M + H]⁺ = 400.2 m/z, calculated mass 399.2 Da.

### 9) Catalyst A-XIII (S)-(2-(benzyloxydiphenylmethyl)pyrrolidin-1-yl)(1-methyl-1H-imidazol-2-yl)methanone

Catalyst A-XIII was synthesized analogously to Catalyst A-V. Catalyst A-XLV (0.500 g, 1.39 mmol) in DMF (9 mL), NaH (60 % dispersion in mineral oil, 0.0830 g, 2.08 mmol) and benzyl bromide (0.246 mL, 2.08 mmol) gave the product as a yellow oil (0.385 g, 62 %) after the purification with reverse phase column chromatography (MeCN/H₂O 60-90%). Two rotamers were observed in NMR in CDCl₃ with a ratio of 1.00 : 0.16.

¹H NMR (400 MHz, CDCl₃, rotamer A) δ 7.71 - 7.23 (m, 15H), 6.98 (s, 1H), 6.71 (d, *J* = 9.10 Hz, 1H), 6.51 (s, 1H), 3.84 (d, *J* = 10.85 Hz, 1H), 3.65 - 3.55 (m, 1H), 3.53 (d, *J* = 11.20 Hz, 1H), 3.19 (s, 3H), 2.48 - 2.32 (m, 2H), 2.20- 1.95 (m, 1H), 1.74 - 1.55 (m, 1H), 1.27 - 1.08 (m, 1H). ¹H NMR (400 MHz, CDCl₃, rotamer B) δ 7.71 - 7.23 (m, 11H), 7.08 (s, 1H), 6.95 - 6.90 (m, 4H), 6.89 (s, 1H), 5.82 (d, *J* =7.70 Hz, 1H), 4.53 (d, *J* = 12.25 Hz, 1H), 4.02 (d, *J* = 10.85 Hz, 1H), 3.90 - 3.87 (m, 1H), 3.78 (s, 3H), 2.20 - 1.95 (m, 4H), 1.35-1.27 (m, 1H). ¹³C NMR (101 MHz, CDCl₃, rotamer A) δ 160.9, 142.0, 138.8, 138.8, 138.6, 130.0 (multiple signals), 128.1 (multiple signals), 127.9 (multiple signals), 127.8, 127.3, 126.1, 123.2, 87.4, 66.1, 64.9, 47.5, 34.1, 27.6, 21.9. ¹³C NMR (101 MHz, CDCl₃, rotamer B) δ 161.0, 141.4, 140.9, 140.4, 139.7, 130.0 (multiple signals), 128.1 (multiple signals), 127.9 (multiple signals), 127.8, 127.3, 127.1, 124.0, 87.0, 66.1, 61.6, 50.7, 35.5, 26.7, 24.2. LC-MS: [M - O-benzyl]⁺ = 344.2 m/z, [M + H]⁺ = 452.3 m/z, calculated mass 451.2 Da.

### 10) Catalyst A-III (S)-(1-methyl-1H-imidazol-2-yl)(2-((perfluorobenzyloxy)diphenylmethyl)-pyrrolidin-1-yl)methanone

Catalyst A-III was produced analogously to Catalyst A-V. Catalyst A-XLV (0.800 g, 2.21 mmol, 1.00 equiv.), NaH (60 % dispersion in mineral oil, 0.133 g, 3.32 mmol, 1.50 equiv.), 2,3,4,5,6-pentafluorobenzyl bromide solution (0.501 mL, 3.32 mmol, 1.50 equiv.) and DMF (14 mL) gave Catalyst A-III as a white solid (0.366 g, 36 %). Two rotamers were observed in NMR in CDCl₃ with a ratio of 1.00 : 0.21.

¹H NMR (400 MHz, CDCl₃, rotamer A) δ 7.66 - 7.23 (m, 10H), 6.93 (s, 1H), 6.73 (d, *J* = 9.22 Hz, 1H), 6.46 (s, 1H), 4.03 (d, *J* = 9.98 Hz, 1H), 3.67 - 3.42 (m, 2H), 3.53 (s, 3H), 2.45 - 2.29 (m, 1H), 2.29 - 2.15 (m, 1H), 2.01 - 1.89 (m, 1H), 1.71 - 1.46 (m, 1H), 1.21 - 1.05 (m, 1H). ¹H NMR (400 MHz, CDCl₃rotamer B) δ 7.66 - 7.23 (m, 10H), 7.06 (s, 1H), 6.89 (s, 1H), 6.73 (m, 1H), 4.70 (d, *J* = 11.01 Hz, 1H), 4.12 (m, 1H), 3.88 - 3.75 (m, 1H), 3.83 (s, 3H), 3.11 - 3.00 (m, 1H), 2.45 - 2.29 (m, 1H), 2.15 - 2.06 (m, 1H), 1.71 - 1.46 (m, 1H), 1.21 - 1.05 (m, 1H). ¹³C NMR (400 MHz, CDCl₃, due to the complexity of the spectrum, it was not possible to assign the specific peaks to rotamer A or B and the peaks are reported altogether) δ 161.1, 160.0 - 159.3 (multiplet), 147.1 - 146.7 (multiplet), 144.6 - 144.0 (multiplet), 142.6 - 141.9 (multiplet), 141.5 - 141.0 (multiplet), 140.7, 140.0, 139.2 - 139.8 (multiplet), 138.4 - 138.5 (multiplet), 137.6, 136.8, 136.3 - 135.7 (multiplet), 130.1 (multiple signals), 129.7 (multiple signals), 128.5, 128.2 (multiple signals), 127.8, 127.7, 127.6, 125.2 - 126.1, 124.2, 122.2, 111.7 (td, J = 18.2, 3.6 Hz), 88.4, 87.3, 65.7, 60.8, 54.4, 53.3, 50.8, 47.5, 35.5, 34.8, 27.7, 26.9, 24.0, 21.9.

### 11) Further catalysts

For further studies, also some further catalysts were synthesized without actually being tested afterwards. Catalysts A-XVII, A-XXVII and A-XXIX were prepared analogously to Catalyst A-V. Catalyst A-XLIX was prepared analogously to Catalyst A-VII. Catalyst A-LVII was prepared from Catalyst A-XI by CuAAC (copper (I)-catalyzed alkyne-azide cycloaddition). Catalysts A-XXIII and A-XXV were prepared as follows: 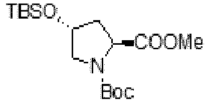 was reacted with PhMgBr in THF to give 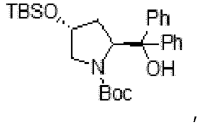 which was further reacted with Mel and NaH to 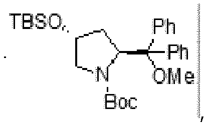 which was then reacted with HCI to give 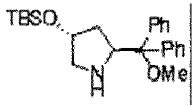 *(*Org. Lett. 2005, 19, 4253-4256). 1-methyl-1*H*-imidazole-2-carboxylic acidwas reacted with (COCl)₂ (described in WO 2006010264 A1) to give 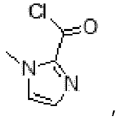 to which 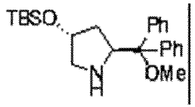 was added together with a base. The resulting 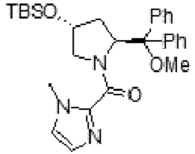 was reacted with TBAF (tetra-n-butylammonium fluoride) to obtain 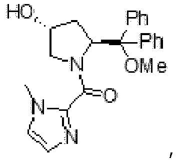 which subsequently was reacted with t-Bu(CO)CI and base to Catalyst A-XXV.

Alternatively, 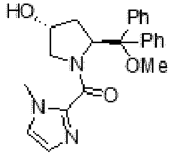 was reacted with PhCOOH, DIAD (diisopropyl azodicarboxylate) and PPh₃, then further with base, and finally with t-Bu(CO)CI to obtain Catalyst A-XXIII.

### Reference Examples B: Catalyst synthesis:

### 1) Catalyst B-II

### Step 1: Synthesis of (S)-tyrosine methyl ester hydrochloride

1 eq (S)-tyrosine (0.5 g) are dissolved in 10 mL MeOH in inert atmosphere. The reaction mixture is cooled with an ice bath. 2.5 eq SOCl2 (0.5 mL) are added, followed by stirring at rt (21 - 23°C, e.g. 22°C) for 10 h. The solvent was evaporated, resulting in (S)-tyrosine methyl ester hydrochloride, which is washed with acetone and filtered.

### Step 2: Synthesis of (S)-3-butyl-5-(4-hydroxybenzyl)-2,2-dimethylimidazolidin-4-one

8 eq n-butylamine (2 mL) are added to 1 eq (S)-tyrosine methyl ester hydrochloride (1 g). The mixture is stirred for 24h. After the reaction time, the mixture is dried in vacuo and the resulting solid is washed with THF. 10 mL dry MeOH and 30 eq dry acetone (0.5 ml) were added to the obtained solid and the reaction mixture is stirred at 60 °C for 24 h. The reaction mixture is dried in vacuo to give the crude product, which is further purified over a silica gel column, resulting in (5S)-3-butyl-5-[(4-hydroxyphenyl)methyl]-2,2-dimethylimidazolidine-4-one.

### Step 3: Synthesis of Catalyst B-II

1 eq picolinic acid (20 mg) is dissolved in 10 mL THF under nitrogen atmosphere. 1.5 eq N-methylmorpholine (0.03 mL) are added and then cooled to 0 °C. 1 eq ethyl chloroformate (20 mg) is added dropwise and stirring was continued at the same temperature for 15 min. 1 eq (S)-3-butyl-5-(4-hydroxybenzyl)-2,2-dimethylimidazolidin-4-one (60 mg) is dissolved in 2 mL DMF and added slowly. Then stirring is carried out at rt overnight, followed by drying the mixture in vacuo, dissolving the residue in 25 mL EtOAc and washing with 20 mL H₂O. The organic phase is collected, dried, filtered and dried in vacuo. The obtained solid is purified over a silica gel column, resulting in Catalyst B-II.

### 2) Catalyst B-IV (R)-5-(4-(benzyloxy)benzyl)-3-butyl-2,2-dimethyl-1-picolinoylimidazolidin-4-one

1 eq Catalyst BII (1 g) is dissolved in 15ml dry acetonitrile under nitrogen atmosphere, and 2 eq K₂CO₃ (700mg) are added. After stirring for 10 min, 1.3 eq benzyl bromide (400 mg) are added slowly, and the mixture is stirred for 18 h at rt. The reaction mixture is filtered and dried in vacuo. The obtained crude product is purified over a silica gel column, resulting in Catalyst B-IV.

### 3) Catalyst B-I (S)-3-butyl-5-(4-hydroxybenzyl)-2,2-dimethyl-1-picolinoylimidazolidin-4-one

Catalyst B-I is synthesized as Catalyst II (see above), except that (R)-tyrosine is used instead of (S)-tyrosine.

¹H NMR (400 MHz, CDCl₃) δ 8.68 - 8.56 (m, 1H), 7.97 - 7.81 (m, 2H), 7.44 (ddd, *J* = 6.9, 5.0, 1.8 Hz, 1H), 6.91 - 6.77 (m, 2H), 6.77 - 6.64 (m, 2H), 5.69 - 5.60 (m, 1H), 3.24 - 3.12 (m, 1H), 3.10 - 2.95 (m, 2H), 2.10 (dd, *J* = 14.5, 5.5 Hz, 1H), 1.73 (s, 3H), 1.67 - 1.57 (m, 1H), 1.55 - 1.42 (m, 1H), 1.39 - 1.26 (m, 2H), 0.97 (s, 3H), 0.94 (t, *J* = 7.4 Hz, 3H). ¹³C NMR (101 MHz, CDCl₃) δ 168.7, 165.6, 156.0, 154.2, 148.4, 137.7, 131.4, 126.8, 125.7, 124.1, 115.5, 81.1, 61.2, 40.1, 36.4, 30.9, 24.7, 24.2, 20.6, 13.8.

### 4) Catalyst B-III (S)-5-(4-(benzyloxy)benzyl)-3-butyl-2,2-dimethyl-1-picolinoyl-imidazolidin-4-one

Catalyst B-III is synthesized as Catalyst B-IV (see above), except that Catalyst B-I is used instead of Catalyst B-II.

### 5) Catalyst B-XLVII (R)-5-benzyl-3-butyl-2,2-dimethyl-1-picolinoylimidazolidin-4-one (R)-2-amino-N-butyl-3-phenylpropanamide

*N*-butylamine (18.5 mL, 0.187 mol) was added to (*R*)-phenylalanine methyl ester hydrochloride (5.07 g, 0.0235 mol). After stirring the mixture 24 h at rt, volatiles were removed in vacuo to give a white slurry. The crude product was placed on a Buechner funnel and washed with THF (80 mL). The filtrate was collected and concentrated under reduced pressure and dichloromethane (40 mL) and water (40 mL) were added to it. The layers were separated and the aqueous phase was extracted with dichloromethane (40 mL). The combined organic layers were dried over Na₂SO₄ and concentrated under reduced pressure to give the crude (*R*)-2-amino-*N*-butyl-3-phenylpropanamide as a colourless oil (4.16 g, ~80%).

### (R)-5-benzyl-3-butyl-2,2-dimethylimidazolidin-4-one

To the crude (*R*)-2-amino-*N*-butyl-3-phenylpropanamide (4.16 g, 0.0189 mol), p-toluenesulfonic acid monohydrate (217 mg, 1.14 mmol), dry MeOH (74 mL) and dry acetone (42 mL, 0.56 mol) were added. The reaction mixture was stirred at 60 °C for 20 h and then concentrated under reduced pressure. The crude product was purified by column chromatography (SiO₂, DCM/EtOAC 5-40% gradient) to give (*R*)-5-benzyl-3-butyl-2,2-dimethylimidazolidin-4-one as a colorless oil (3.45 g, ~70%) that crystallizes upon prolonged standing.

### (R)-5-benzyl-3-butyl-2,2-dimethyl-1-picolinoylimidazolidin-4-one

Picolinic acid (470 mg, 3.82 mmol) was dissolved in dry THF (25 mL) under nitrogen atmosphere and *N*-methylmorpholine (554 µL, 5.76 mmol) was added to it. The solution was cooled to 0 °C and ethyl chloroformate (366 µL, 3.84 mmol) was added dropwise. The reaction mixture was stirred at 0 °C for 1 h. (*R*)-5-Benzyl-3-butyl-2,2-dimethylimidazolidin-4-one (1.00 g, 3.84 mmol) was dissolved in dry DMF (5 mL) and the solution was added dropwise to the reaction mixture at 0 °C. The reaction was let warm to room temperature and stirred overnight. The volatiles were removed in vacuo, EtOAc (40 mL) and water (30 mL) were added and the layers were separated. The aqueous layer was extracted two more times with EtOAc (2 × 40 mL) and the combined organic layers were concentrated under reduced pressure. The crude product was purified by column chromatography (SiO₂, Hexane/EtOAC 30-60% gradient) to give Catalyst B-XLVII as a colorless oil (850 mg, 61 %) that crystallizes upon prolonged standing.

¹H NMR (400 MHz, CDCl₃) δ 8.59 (dt, *J* = 4.8, 1.3 Hz, 1 H), 7.92 - 7.84 (m, 2 H), 7.52 - 7.31 (m, 1 H), 7.25 - 7.09 (m, 3 H), 7.00 - 6.89 (m, 2 H), 5.68 (dd, *J* = 5.7, 2.5 Hz, 1 H), 3.21 - 3.12 (m, 1 H), 3.11 - 3.05 (m, 1 H), 3.05 - 2.96 (m, 1 H), 2.13 (dd, *J* = 14.1, 5.9 Hz, 1 H), 1.70 (s, 3 H), 1.69 - 1.56 (m, 1 H), 1.48 - 1.36 (m, 1 H), 1.35 - 1.21 (m, 2 H), 0.95 - 0.89 (m, 3 H), 0.85 (s, 3 H). ¹³C NMR (101 MHz, CDCl₃):168.2, 165.2, 154.4, 148.1, 137.3, 135.6, 130.2, 128.2, 127.0, 125.3, 124.0, 80.6, 60.6, 39.8, 37.0, 30.7, 24.5, 23.8, 20.4, 13.6.

### 6) Catalyst B-LIII (R)-5-((1H-indol-3-yl)methyl)-3-butyl-2,2-dimethyl-1-picolinoylimidazolidin-4-one

### (R)-2-amino-N-butyl-3-(1H-indol-3-yl)propanamide

*N*-butylamine (15.5 mL, 0.157 mol) was added to (*R*)-tryptophane methyl ester hydrochloride (5.06 g, 0.0199 mol). After stirring the mixture 24 h at rt, volatiles were removed in vacuo to give a brown oil. Dichloromethane (40 mL) and water (40 mL) were added to it. The layers were separated and the aqueous phase was extracted with dichloromethane (40 mL). The combined organic layers were dried over Na₂SO₄ and concentrated under reduced pressure to give the crude (*R*)-2-amino-*N*-butyl-3-(1*H*-indol-3-yl)propanamide as a yellow solid (4.85 g, ~94%).

### (R)-2-amino-N-butyl-3-(1H-indol-3-yl)propanamide

To the crude (*R*)-2-amino-*N*-butyl-3-(1*H*-indol-3-yl)propanamide (2.02 g, ~7.79 mmol), p-toluenesulfonic acid (88 mg, 0.46 mmol), dry MeOH (30 mL) and dry acetone (17 mL, 0.23 mol) were added. The reaction mixture was stirred at 60 °C for 20 h and then concentrated under reduced pressure. The crude product was redissolved in EtOAC (~ 500 mL) and filtered through silica. The first ~ 100 mL of the filtrate were disposed and the rest of it was concentrated under reduced pressure yielding (*R*)-2-amino-*N*-butyl-3-(1*H*-indol-3-yl)propanamide as a yellowish solid (1.95 g, ~84%).

### (R)-5-((1H-indol-3-yl)methyl)-3-butyl-2,2-dimethyl-1-picolinoylimidazolidin-4-one

Catalyst B-LIII was prepared analogously to Catalyst B-XLVII. Picolinic acid (410 mg, 3.33 mmol) in dry THF (25 mL), *N*-methylmorpholine (482 µL, 5.01 mmol), ethyl chloroformate (319 µL, 3.35 mmol) and (*R*)-2-amino-*N*-butyl-3-(1*H*-indol-3-yl)propanamide **(17)** (1.00 g, 3.34 mmol) in dry DMF (5 mL) gave the catalyst Catalyst B-LIII as a light yellow solidified foam (1.10 g, 82%) after purification by column chromatography (SiO₂, Hexane/EtOAC 20-100% gradient) and several redissolving of the product followed by its re-evaporation/drying from chloroform and toluene to remove the residual DMF.

¹H NMR (400 MHz, CDCl₃) δ 8.53 (dd, *J* = 4.9, 1.0 Hz, 1H), 7.81 - 7.74 (m, 2H), 7.46 (d, *J* = 7.8 Hz, 1H), 7.30 (ddt, *J* = 6.6, 4.5, 2.1 Hz, 1H), 7.25 (d, *J* = 8.6 Hz, 1H), 7.14 - 7.07 (m, 1H), 7.07 - 6.99 (m, 2H), 6.88 - 6.85 (m, 1H), 5.73 (t, *J* = 4.5 Hz, 1H), 3.23 (dd, *J* = 15.1, 3.7 Hz, 1H), 3.12 - 2.97 (m, 2H), 2.54 (dd, *J* = 15.1, 5.3 Hz, 1H), 1.72 (s, 3H), 1.50 - 1.39 (m, 1H), 1.37 - 1.14 (m, 3H), 0.98 (s, 3H), 0.87 (t, *J* = 7.2 Hz, 3H). ¹³C NMR (101 MHz, CDCl₃) δ 169.4, 165.7, 154.2, 148.2, 137.2, 136.0, 127.7, 125.1, 124.0, 123.8, 122.1, 119.7, 119.6, 110.9, 110.0, 80.8, 60.6, 39.9, 30.9, 28.3, 24.8, 24.5, 20.6, 13.8.

### 7) Catalyst B-LXV (R)-5-((1-benzyl-1H-indol-3-yl)methyl)-3-butyl-2,2-dimethyl-1-picolinoylimidazolidin-4-one

To dry THF (2.0 mL) at -5 °C, NaH (25 mg, 0.625 mmol, 60% in mineral oil) followed by (*R*)-5-((1*H*-indol-3-yl)methyl)-3-butyl-2,2-dimethyl-1-picolinoylimidazolidin-4-one (Catalyst B-LIII) (250 mg, 0.618 mmol) were added under nitrogen atmosphere. To the dark yellow solution, benzyl bromide (88 µL, 0.74 mmol) was added and the reaction was let warm to room temperature and further stir for 3 h. The reaction mixture was cooled with an ice bath to 0 °C and carefully quenched with sat. aq. NH₄Cl. EtOAc (6 mL) was added, the layers were separated and the aqeuoues phase was extracted twice with EtOAc (2 × 5 mL). The combined organic layers were dried over Na₂SO₄ and concentrated under reduced pressure. The crude product was purified by column chromatography (SiO₂, Hexane/EtOAC 20-60% gradient) to give the catalyst Catalyst B-LXV as a colorless oil (277 mg, 91%) that gets more viscous upon prolonged standing.

¹H NMR (400 MHz, CDCl₃) δ 8.55 - 8.50 (m, 1H), 7.81 - 7.72 (m, 1H), 7.72 - 7.66 (m, 1H), 7.44 (d, *J* = 7.8 Hz, 1H), 7.35 - 7.24 (m, 4H), 7.22 - 7.16 (m, 1H), 7.15 - 7.09 (m, 3H), 7.08 - 6.99 (m, 1H), 6.75 (s, 1H), 5.74 (dd, *J* = 5.5, 3.5 Hz, 1H), 5.21 (s, 2H), 3.23 (dd, *J* = 15.1, 3.3 Hz, 1H), 2.99 (t, *J* = 8.0 Hz, 2H), 2.50 (dd, *J* = 15.1, 5.3 Hz, 1H), 1.71 (s, 3H), 1.50 - 1.32 (m, 2H), 1.31 - 1.18 (m, 2H), 0.91 (s, 3H), 0.88 (t, *J* = 7.2 Hz, 3H). ¹³C NMR (101 MHz, CDCl₃) δ 169.4, 165.5, 154.4, 148.1, 137.5, 137.2, 136.3, 128.9, 128.5, 127.9, 127.8, 127.2, 125.1, 124.1, 122.0, 120.0, 119.5, 109.4, 109.3, 80.7, 60.6, 50.1, 39.9, 31.0, 28.1, 24.8, 24.5, 20.6, 13.9.

### 8) Catalyst B-LXIX (R)-2-(5-((1H-indol-3-yl)methyl)-3-butyl-2,2-dimethyl-4-oxo-imidazolidine-1-carbonyl)pyridin-4-yl ethyl carbonate

Catalyst B-LXIX was prepared analogously to Catalyst B-XLVII. The reaction was started with 4-hydroxypicolinic acid which caught the ethyl carbonate moiety during the activation step with ethyl chloroformate. 4-Hydroxypicolinic acid (140 mg, 1.0 equiv) in dry THF (7.5 mL), *N-*methylmorpholine (145 µL, 1.51 mmol), ethyl chloroformate (96 µL, 1.01 mmol) and (*R*)-2-amino-*N*-butyl-3-(1*H*-indol-3-yl)propanamide (300 mg, 1.00 mmol) in dry DMF (2.0 mL) gave the Catalyst B-LXX as a colorless oil (78 mg, 16%) after purification by column chromatography (SiO₂, Hexane/EtOAC 20-50% gradient).

¹H NMR (400 MHz, CDCl₃) δ 8.37 (br s, 1H), 8.33 - 8.23 (m, 1H), 7.46 - 7.41 (m, 2H), 7.20 - 7.12 (m, 1H), 7.08 (td, *J* = 7.4, 1.2 Hz, 1H), 7.04 - 6.93 (m, 2H), 6.80 (d, *J* = 2.3 Hz, 1H), 5.77 (dd, *J* = 5.9, 4.7 Hz, 1H), 4.38 (q, *J* = 7.0 Hz, 2H), 3.17 - 3.06 (m, 3H), 2.81 (dd, *J* = 14.9, 4.7 Hz, 1H), 1.73 (s, 3H), 1.43 (t, *J* = 7.2 Hz, 3H), 1.57 - 1.39 (m, 2H), 1.31 - 1.25 (m, 2H), 1.22 (s, 3H), 0.89 (t, *J* = 7.2 Hz, 3H). ¹³C NMR (101 MHz, CDCl₃) δ 169.4, 164.9, 158.2, 155.5, 151.9, 149.2, 135.9, 127.4, 124.0, 122.1, 119.5, 119.4, 116.5, 116.0, 110.9, 110.0, 80.8, 65.7, 60.4, 40.0, 31.0, 29.3, 24.9, 24.6, 20.6, 14.3, 13.8.

### 9) Catalyst B-LXXII (S)-(2-(methoxydiphenylmethyl)pyrrolidin-1-yl)(pyridin-2-yl)-methanone

Catalyst B-LXXII was prepared according to the literature (Org. Biomol. Chem. 2017, 15, 2422-2435). The obtained ¹H and 1³C NMR spectra are matching the reported spectra.

### Reference examples C: reference catalysts

For Comparative Examples, Catalyst C and Catalyst D were provided.

Catalyst C was obtained commercially.

Catalyst D was synthesized according to its published procedure (Adv. Synth. Catal. 2008, 350, 619-623) and ¹H NMR is matching the published spectra.

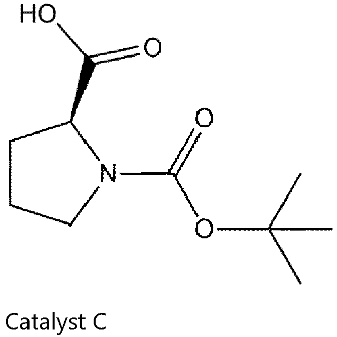

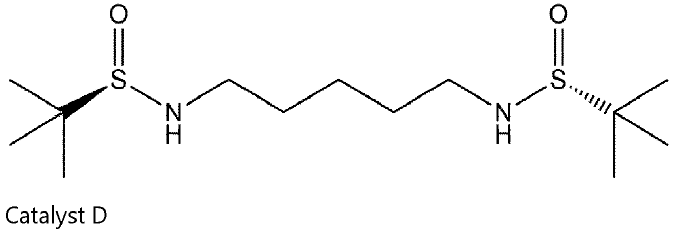

### Example A-1A: Synthesis of (R/S)-nornicotine

The synthesis is carried out according to the following general reaction:

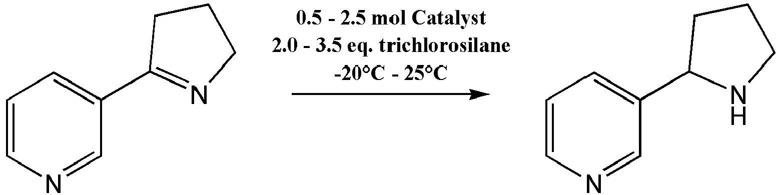

1 eq myosmine (100 mg; 305 mg in entry A4 - A-9) and the respective catalyst with varying amounts, as given in Table 1 below, are dissolved in DCM (dried) as solvent, as given in Table 1. In some experiments, also an additive (benzoic acid) was given, as some additives showed a further improvement in results, as discussed above and also shown below. Different equivalents (eq.) of trichlorosilane, as given in Table 1 below, are added at different reaction temperatures as given in Table 1 below, and the solution is stirred for 18-22h. After the reaction time, 8-30% NaOH in water is added until all solids solubilize and the mixture is extracted twice with 40 mL DCM each. The organic phases are collected, dried, filtered and reduced *in vacuo,* resulting in a mixture of (R/S)-nornicotine. The enantiomeric excess is determined by HPLC (mobile phase hexane:isopropanol = 95:5 1 mL/min, stationary phase Daicel Chiralcel OD-H 25 cm × 0.45 cm, detection wavelength 220 nm). The results of different Examples are given in Table 1.

**Table 1: Results of a series of examples with varying catalysts, amounts of catalyst, temperature, silane loading equivalents, and additive, following the procedure described above. The enantiomeric excess refers to (S)-nornicotine.**

| Entry | Catalyst | Catalyst concentration [mol%] | Myosmine conc. [mol%] | Temp [°C] | Silane loading [eq.] | Additive ([mol%]) | Conversion [%] | ee [%] |
|---|---|---|---|---|---|---|---|---|
| A-1 | A-I | 0.5 | 0.18 | 0 | 3.5 | none | 99+ | 90 |
| A-2 | A-I | 1.0 | 0.10 | -20 | 3.5 | none | 99+ | ~93-94 |
| A-3 | A-I | 0.5 | 0.18 | 0 | 2.0 | none | 99+ | 88 |
| A-4 | A-I | 1.0 | 0.18 | 0 | 3.5 | benzoic acid (5) | 99+ | 89 |
| A-5 | A-XV | 1.0 | 0.18 | 0 | 3.5 | benzoic acid (5) | 99+ | 78 |
| A-6 | A-XIX | 1.0 | 0.18 | 0 | 3.5 | benzoic acid (5) | 11 | 19 |
| A-7 | A-VII | 1.0 | 0.18 | 0 | 3.5 | benzoic acid (5) | 99+ | 90 |
| A-8 | A-IX | 1.0 | 0.18 | 0 | 3.5 | benzoic acid (5) | 99+ | 89 |
| A-9 | A-III | 0.5 | 0.18 | 0 | 2.0 | benzoic acid (5) | 99+ | 87 |

In further samples, 300 mg myosmine and different catalysts at 0.5 mol% are dissolved with 5 mol% benzoic acid in DCM as above, as specified in Table 2. The mixture is brought to a reaction temperature of 0°C. Trichlorosilane is added at 2.0 eq., and the mixture is reacted for the times given in Table 2. The reaction is quenched by adding an 8-30% sodium hydroxide solution (in water). The organic phase is separated and analyzed for enantiopurity by HPLC (mobile phase hexane:isopropanol = 99:1 0.8 mL/min, stationary phase Daicel Chiralcel OD-H 25 cm × 0.45 cm, detection wavelength 254 nm).

**Table 2: Results of a series of examples with varying catalysts and reaction times, following the procedure described above. The enantiomeric excess refers to (S)-nornicotine.**

| Entry | Catalyst | Reaction time [hours] | Conversion [%] | ee [%] |
|---|---|---|---|---|
| A-10 | A-I | 2 | 86 | 87 |
| A-11 | A-I | 22 | 99+ | 87 |
| A-12 | A-VII | 2 | 80 | 89 |
| A-13 | A-VII | 22 | 99+ | 89 |
| A-14 | A-IX | 2 | 89 | 86 |
| A-15 | A-IX | 22 | 99 | 86 |

To test the influence of reaction time, loading amounts of myosmine and catalyst, solvent, additive, and silane load, varying amounts of myosmine and catalyst A-I at varying amounts are dissolved with the respective additive in the respective solvent, as above, as specified in Table 3. The mixture is brought to a reaction temperature of 0°C. Trichlorosilane is added at the amount given, and the mixture is reacted for the times given in Table 3. The reaction is quenched by adding an 8-30% sodium hydroxide solution (in water). The organic phase is separated and analyzed for enantiopurity by HPLC (mobile phase hexane:isopropanol = 99:1 0.8 mL/min, stationary phase Daicel Chiralcel OD-H 25 cm × 0.45 cm, detection wavelength 254 nm).

**Table 3: Results of a series of examples with varying catalysts, amounts of catalyst, temperature, silane loading equivalents, and additive, following the procedure described above. The enantiomeric excess refers to (S)-nornicotine.**

| Entry | Myosmine load [mg] | Myosmine concentration (M) | Catalyst concentration [mol%] | Solvent | Silane loading [eq.] | Additive ([mol%]) | Reaction time [hours] | Conversion [%] | ee [%] |
|---|---|---|---|---|---|---|---|---|---|
| A-16 | 300 | 0.18 | 0.5 | DCM | 2.0 | PTSA*H₂O (5) | 18 | 99 | 79 |
| A-17 | 300 | 0.18 | 0.5 | DCM | 2.0 | Malonic acid (5) | 18 | 99 | 74 |
| A-18 | 300 | 0.19 | 0.5 | DCM | 2.0 | Benzoic acid (10) | 18 | 99+ | 83 |
| A-19 | 500 | 0.50 | 0.5 | chlorobenzene | 3.5 | BHT (5) | 5 | 99 | 88 |
| A-20 | 100 | 0.18 | 0,5 | chloroform | 3.5 | none | 18 | 99 | 76 |
| A-21 | 100 | 0.18 | 0.5 | MeCN | 3.5 | none | 18 | 92 | 31 |
| A-22 | 500 | 0.5 | 0.5 | dichloromethane | 3.5 | Benzoic acid (2) | 3 | 95.5 | 89 |
| A-23 | 500 | 0.5 | 0.5 | chlorobenzene | 3.5 | Benzoic acid (2) | 3 | 99 | 89 |
| A-24 | 1000 | 1.0 | 1.0 | chlorobenzene | 3.5 | Benzoic acid (2) | 4 | 99 | 87 |
| A-25 | 1000 | 1.0 | 1.0 | anisole | 3.5 | Benzoic acid (2) | 4 | 99+ | 87 |
| A-26 | 1000 | 1.0 | 1.0 | toluene | 3.5 | Benzoic acid (2) | 4 | 59 | 86 |
| A-27 | 1000 | 1.0 | 2.5 | toluene | 3.5 | Benzoic acid (2) | 4 | 98 | 89 |
| A-28 | 1000 | 1.0 | 2.5 | p-xylene | 3.5 | Benzoic acid (2) | 4 | 33 | 81 |
| A-29 | 1000 | 1.0 | 2.5 | m-xylene | 3.5 | Benzoic acid (2) | 4 | 46 | 84 |
| A-30 | 1000 | 1.0 | 1.0 | toluene/chloro benzene (5/1) | 3.5 | Benzoic acid (2) | 4 | 77 | 87 |
| A-31 | 1000 | 1.0 | 1.0 | toluene/anisole (5/1) | 3.5 | Benzoic acid (2) | 4 | 92 | 86 |
| A-32 | 1000 | 1.0 | 1.0 | toluene/DCM (5/1) | 3.5 | Benzoic acid (2) | 4 | 99+ | 88 |
| A-33 | 20000 | 1.0 | 1.0 | chlorobenzene | 3.5 | Benzoic acid (2) | 3 | 99+ | 88 |
| A-34 | 5000 | 1.0 | 1.0 | anisole | 3.5 | Benzoic acid (2) | 3 | 99+ | 86 |
| A-35 | 300 | 0.18 | 2.5 | DCM | 3.5 | none | 18 | 99+ | 89 |
| A-36 | 1000 | 0.49 | 0.5 | DCM | 3.5 | Benzoic acid (2) | 3 | 87 | 89 |
| A-37 | 1000 | 0.49 | 0.5 | DCM | 3.5 | Benzoic acid (3) | 3 | 92 | 87 |
| A-38 | 1000 | 0.49 | 0.5 | DCM | 2.1 | Benzoic acid (2) | 3 | 56 | 88 |
| A-39 | 1000 | 0.49 | 0.5 | DCM | 2.1 | Benzoic acid (4) | 3 | 66 | 86 |
| A-40 | 500 | 0.5 | 0.5 | chlorobenzene | 3.5 | Benzoic acid (2) | 3 | 99 | 88 |
| A-41 | 500 | 0.5 | 0.5 | chlorobenzene | 2.1 | Benzoic acid (2) | 3 | 99+ | 88 |
| A-42 | 500 | 0.5 | 1.0 | chlorobenzene | 2.1 | Benzoic acid (2) | 3 | 99+ | 86 |

| | | | | | | | | | |
|---|---|---|---|---|---|---|---|---|---|
| PTSA = p-toluenesulfonic acid BHT = 2,6-Di-tert-butyl-4-methylphenol | | | | | | | | | |

### Example B-1A: Synthesis of (R/S)-nornicotine

The synthesis is carried out according to the following general reaction:

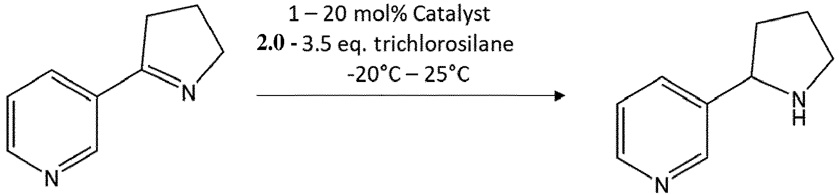

1 eq myosmine (100 mg) (pre-dried in B-18 and B-19) and the respective catalyst with varying amounts, as given in Table 4 below, are dissolved in 6.8 mL DCM as solvent, as given in Table 4. Different equivalents (eq.) of trichlorosilane (in B-1 to B-18, B-20 to B-23), respectively H₂Cl₂Si (B-19), as given in Table 4 below, are added and the solution is stirred for 18h, except for entries B-20 and B-21, where stirring was for 2 hours, and entries B-22 and B-23, where stirring was for 22 hours, respectively. After the reaction time, 10% wt.% NaOH in water is added until all solids dissolved and the mixture is extracted twice with 40 mL DCM each. The organic phases are collected, dried, filtered and reduced *in vacuo,* resulting in a mixture of (R/S)-nornicotine. The enantiomeric excess is determined by HPLC (mobile phase hexane:isopropanol = 95:5 1 mL/min, stationary phase Daicel Chiralcel OD-H 25 cm × 0.45 cm, detection wavelength 220 nm). The results of different Examples are given in Table 1.

**Table 4: Results of a series of examples with varying catalysts, amounts of catalyst, temperature and silane load following the procedure described above. The enantiomeric excess refers to (S)-nornicotine.**

| Entry | Catalyst | Catalyst concentration [mol%] | Temp [°C] | Silane loading [eq.] | Conversion [%] | Enantiomer produced | ee [%] |
|---|---|---|---|---|---|---|---|
| B-1 | B-II | 10 | 0 | 3.5 | 99+ | R | 82 |
| B-2 | B-IV | 10 | 0 | 3.5 | 99+ | R | 84 |
| B-3 | B-XLVII | 10 | 0 | 3.5 | 96 | S | 79 |
| B-4 | B-LIII | 10 | 0 | 3.5 | 99+ | S | 82 |
| B-5 | B-LXV | 10 | 0 | 3.5 | 99+ | S | 86 |
| B-6 | B-IV | 10 | 0 | 2.8 | 98 | R | 81 |
| B-7 | B-IV | 10 | 0 | 2.0 | 97 | R | 79 |
| B-8 | B-LIII | 5 | 0 | 3.5 | 56 | S | 70 |
| B-9 | B-LIII | 2.5 | 0 | 3.5 | 41 | S | 54 |
| B-12 | B-XLVII | 5 | rt (25°C) | 3.5 | 78 | S | 30 |
| B-13 | B-LIII | 5 | rt | 3.5 | 84 | S | 30 |
| B-14 | B-LXV | 5 | rt | 3.5 | 86 | S | 43 |
| B-15 | B-LXV | 10 | rt | 3.5 | 95 | S | 85 |
| B-16 | B-LXIX | 5 | -20 | 3.5 | 6 | S | 84 |
| B-17 | B-LIII | 5 | -20 | 3.5 | 44 | S | 93 |
| B-18 | B-IV | 5 | 0 | 3.5 | 57 | R | 64 |
| B-19 | B-IV | 5 | 0 | 3.5 | 10 | R | 34 |
| B-20 | B-LXXII | 0.5 | 0 | 2.0 | 8 | S | 58 |
| B-21 | B-IV | 0.5 | 0 | 2.0 | 6 | R | 32-33 |
| B-22 | B-LXXII | 0.5 | 0 | 2.0 | 15 | S | 51 |
| B-23 | B-IV | 0.5 | 0 | 2.0 | 40 | R | 18 |

In further samples, myosmine and different catalyst are dissolved with the same ratio as above, as specified in Table 5. The mixture is brought to the reaction temperature given. Trichlorosilane is added at 3.5 eq., and the mixture is aged until reaction control indicates complete consumption of myosmine. The reaction is quenched by adding a 10 wt.% sodium hydroxide solution (in water). The organic phase is separated and analyzed for enantiopurity by HPLC (mobile phase hexane:isopropanol = 99:1 0.8 mL/min, stationary phase Daicel Chiralcel OD-H 25 cm × 0.45 cm, detection wavelength 254 nm).

**Table 5: Results of a series of examples with varying catalyst, amounts of catalyst, temperature and solvent following the procedure described above. The enantiomeric excess refers to (S)-nornicotine.**

| Entry | Catalyst | Catalyst concentration mol% | Temp °C | Solvent | ee % |
|---|---|---|---|---|---|
| B-1a | B-III | 10 | 0 | DCM | 83 |
| B-2a | B-III | 10 | -20 | DCM | 90 |
| B-3a | B-III | 5 | 0 | DCM | 71 |
| B-4a | B-III | 20 | -20 | DCM | 95 |
| B-5a | B-III | 10 | 0 | toluene | 79 |
| B-6a | B-III | 20 | 0 | DCM | 86 |
| B-7a | B-I | 10 | 0 | DCM | 75 |

### Example B-1B: Upscaling and separation of nornicotine from the reaction mixture

### Step 1:

Myosmine (100 g) and Catalyst B-III (35 g) are dissolved in dichloromethane (2000 g) and cooled to -20°C. Trichlorosilane (200 g) is dosed slowly within 24 h to the mixture. Sodium hydroxide (1 L, 10%) is added, thoroughly stirred and the aq. phase is separated. The organic phase is polish filtered and washed with water. The organic phase is extracted at pH 7, separating the catalyst into an organic phase II and nornicotine into an aqueous phase II.

### Step 2:

The aqueous phase II is extracted at pH > 10 (by further addition of 10 wt.% NaOH in water, as in step 1) into dichloromethane and purified by distillation.

### Example B-1C: Influence of acid addition

A conversion from myosmine to (S)-nornicotine was carried out similar to the experiments in Example 1B-1A using 0.18 M myosmine, 5 mol% of catalyst B-LXV, and 2 equivalents of HCl₃Si (TCS) in DCM for reaction at 0°C for 6 hours. To the mixture, either no additive, benzoic acid or proton sponge were added prior to the addition of TCS. While addition of benzoic acid led to higher conversion (59%) and slightly higher ee (77) than the reference experiment (48% and 76 ee), presence of proton sponge resulted in exactly the opposite (23% and 74 ee).

### Example B-2A: Synthesis of (R)-nicotine

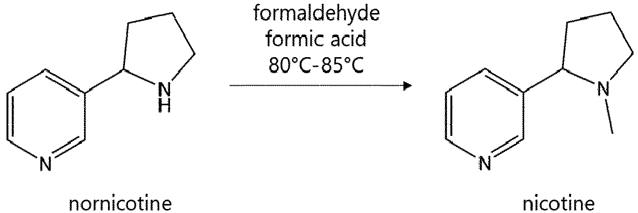

1 eq. (R)-nornicotine (100 mg) with 82% ee (produced according to entry B-1 of Table 4) is dissolved in 4 mL formic acid, and 36 eq. formaldehyde solution in water (37 wt.%, 2 mL) are added. The reaction mixture is stirred for 3 h at 80°C. After the reaction time, 2 mol/L NaOH is added until pH 10 and the mixture is extracted twice with DCM. The organic phases are collected, dried over MgSO₄, filtered, and the solvent is evaporated. This results in 100 mg (R)-nicotine as a yellow oil, which is a yield of 91%. The enantiomeric excess is determined by HPLC (mobile phase hexane:isopropanol = 95:5 1 mL/min, stationary phase Daicel Chiralcel OD-H 25 cm × 0.45 cm, detection wavelength 220 nm), which is 82%.

### Example B-2B: Synthesis of (S)-nicotine

The aqueous solution of nornicotine from step 1 of Example B-1B is supplemented without further cleaning steps (in a one-pot synthesis) with paraformaldehyde (76 g) and formic acid (60 g). The mixture is gradually heated to 80-85°C and is further treated as described in WO2020/098978 Example 7b, yielding 80 g of the (S)-nicotine with >99% purity and 95% enantiopurity.

If desired this material can be further crystallized from aq. ethanol as hydrate bitartrate salt, further upgrading the enantiopurity of (S)-nicotine to 100%.

### Example B-3: Catalyst recovery

The organic phase II from Example 1B is concentrated to a low volume, supplemented with heptane and cooled to 0°C. The resulting solid is isolated and dried, yielding 30 g of Catalyst B-III, which is suited for use in a subsequent experiment.

### Comparative Example 1: Myosmine reduction without catalyst

Myosmine (114 mg) is dissolved in 6.8 mL DCM. Trichlorosilane (370 mg) is added at 0°C and the solution is stirred for 18h. After the reaction time, 10% NaOH is added until all solids dissolve and the mixture is extracted twice with 40 mL DCM each. The organic phases are collected, dried, filtered, and reduced *in vacuo,* yielding racemic nornicotine (0% ee).

### Comparative Example 2: Myosmine reduction with Catalyst C

The experiment was carried out similar to entry B-1, but with catalyst C and 2.0 eq. of TCS. While conversion was at 95%, no ee was obtained.

### Comparative Example 3: Myosmine reduction with Catalyst D

The experiment was carried out similar to entry B-1, but with catalyst D in an amount of 33 mol%. While conversion was at 65%, no ee was obtained.

## Claims

1. A method of producing nornicotine from myosmine, comprising:
reacting myosmine with at least one hydrosilane using a catalyst,
wherein the catalyst is an organic chiral Lewis base.

2. The method according to claim 1, wherein the catalyst is a catalyst of the following formula I 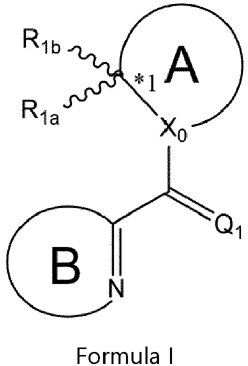 wherein
Ring A comprises, besides X₀ and the chiral C atom with residues R₁ₐ and R_{1b}, a carbonyl group, a thiocarbonyl group, substituted or unsubstituted C atoms and/or substituted or unsubstituted heteroatoms, wherein the substituents can comprise any one of F; Cl; Br; I; pseudohalogen groups; alkoxy groups with 1 to 20 C atoms; OH; NO₂; NH₂; amines of formula NR₁₈R₁₉; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; linear alkenyl groups and/or alkynyl groups with 2 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; branched alkenyl and/or alkynyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂; with the provisio that two or more of the substituents can form one ring or more rings, optionally wherein at least one substituent is part of a polymer;
Ring B is a five- or six-membered ring comprising conjugated double bonds that is particularly aromatic, i.e. is fully conjugated, planar and follows the Hueckel's rule, and that comprises, besides the carbon atom connected to the bridge and the adjacent nitrogen atom, substituted or unsubstituted C atoms and/or substituted or unsubstituted heteroatoms, wherein the substituents can comprise any one of F; Cl; Br; I; pseudohalogen groups; alkoxy groups with 1 to 20 C atoms; OH; NO₂; NH₂; amines of formula NR₁₈R₁₉; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂; with the provisio that two or more of the substituents can form one ring or more rings;
wherein R₁₈ and R₁₉ are the same or different and can be chosen from H, linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂, with the provisio that not both of R₁₈ and R₁₉ are H;
X₀ is selected from N and CR_{A1}, wherein
R_{A1} is selected from the group consisting of H, linear alkyl groups with 1 to 6 C atoms that can optionally be substituted with at least one of F, Cl, Br, I, pseudohalogen groups, alkoxy groups with 1 to 6 C atoms, OH, NO₂ and NH₂; branched alkyl groups with 3 to 6 C atoms that can optionally be substituted with at least one of F, Cl, Br, I, pseudohalogen groups, alkoxy groups with 1 to 6 C atoms, OH, NO₂ and NH₂; cycloalkyl groups with 3 to 7 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 6 C atoms that can optionally be substituted with at least one of F, Cl, Br, I, pseudohalogen groups, alkoxy groups with 1 to 6 C atoms, OH, NO₂ and NH₂; aromatic and/or heteroaromatic groups with 2 to 6 carbon atoms that can optionally be substituted with at least one of F, Cl, Br, I, pseudohalogen groups, alkoxy groups with 1 to 6 C atoms, OH, NO₂ and NH₂; carboxylic acid ester groups with 1 to 6 C atoms that can optionally be substituted with at least one of F, Cl, Br, I, pseudohalogen groups, alkoxy groups with 1 to 6 C atoms, OH, NO₂ and NH₂; alkyl and/or (hetero)aryl oxycarbonyl groups with 1 to 6 C atoms that can optionally be substituted with at least one of F, Cl, Br, I, pseudohalogen groups, alkoxy groups with 1 to 6 C atoms, OH, NO₂ and NH₂; and carboxamide groups with 1 to 6 C atoms that can optionally be substituted with at least one of F, Cl, Br, I, pseudohalogen groups, alkoxy groups with 1 to 6 C atoms, OH, NO₂ and NH₂;
Q₁ is O or S;
R₁ₐ and R_{1b} are different;
R₁ₐ is a saturated and/or unsaturated linear, branched, cyclic and/or aromatic and/or heteroaromatic hydrocarbon residue with 1 to 100 C atoms that can contain heteroatoms selected from the group of O, N, S and P and that can optionally be substituted with at least one of F; Cl; Br; I; pseudohalogen groups; OSiR₂₃R₂₄R₂₅; carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂; carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂; optionally substituted linear, branched and/or cyclic alkoxy, aryloxy, heteroaryloxy, alkenyloxy and/or alkynyloxy groups with 1 to 20 C atoms and/or heteroatoms; OH; N SH; O₂; NH₂; and amines of formula NR₁₈R₁₉;
R₁₈ and R₁₉ are the same or different and can be chosen from H, linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂, with the provisio that not both of R₁₈ and R₁₉ are H;
R₂₃, R₂₄, and R₂₅ are the same or different and are selected from H, linear alkyl groups with 1 to 4 C atoms that can optionally be substituted with at least one of F, Cl, Br, I, pseudohalogen groups, alkoxy groups with 1 to 20 C atoms, OH, NO₂ and NH₂, branched alkyl group with 3 to 6 C atoms that can optionally be substituted with at least one of F, Cl, Br, I, pseudohalogen groups, alkoxy groups with 1 to 20 C atoms, OH, NO₂ and NH₂, aromatic groups with 6 to 12 C atoms that can optionally be substituted with at least one of F, Cl, Br, I, pseudohalogen groups, alkoxy groups with 1 to 20 C atoms, OH, NO₂ and NH₂, and aralkyl groups with 7 to 10 C atoms that can optionally be substituted with at least one of F, Cl, Br, I, pseudohalogen groups, alkoxy groups with 1 to 20 C atoms, OH, NO₂ and NH₂, preferably wherein R₂₃, R₂₄, and R₂₅ are not all hydrogen at the same time and/or wherein R₂₃, R₂₄, and R₂₅ are not all methyl at the same time, further preferably wherein all of R₂₃, R₂₄, and R₂₅ are ethyl;
R_{1b} is selected from the group consisting of H, linear alkyl groups with 1 to 6 C atoms that can optionally be substituted with at least one of F, Cl, Br, I, pseudohalogen groups, alkoxy groups with 1 to 6 C atoms, OH, NO₂ and NH₂; branched alkyl groups with 3 to 6 C atoms that can optionally be substituted with at least one of F, Cl, Br, I, pseudohalogen groups, alkoxy groups with 1 to 6 C atoms, OH, NO₂ and NH₂; heterocycloalkyl groups with 1 to 6 C atoms that can optionally be substituted with at least one of F, Cl, Br, I, pseudohalogen groups, alkoxy groups with 1 to 6 C atoms, OH, NO₂ and NH₂; aromatic and/or heteroaromatic groups with 2 to 6 carbon atoms that can optionally be substituted with at least one of F, Cl, Br, I, pseudohalogen groups, alkoxy groups with 1 to 6 C atoms, OH, NO₂ and NH₂; carboxylic acid ester groups with 1 to 6 C atoms that can optionally be substituted with at least one of F, Cl, Br, I, pseudohalogen groups, alkoxy groups with 1 to 6 C atoms, OH, NO₂ and NH₂; alkyl and/or (hetero)aryl oxycarbonyl groups with 1 to 6 C atoms that can optionally be substituted with at least one of F, Cl, Br, I, pseudohalogen groups, alkoxy groups with 1 to 6 C atoms, OH, NO₂ and NH₂; and carboxamide groups with 1 to 6 C atoms that can optionally be substituted with at least one of F, Cl, Br, I, pseudohalogen groups, alkoxy groups with 1 to 6 C atoms, OH, NO₂ and NH₂, preferably wherein R_{1b} is H or methyl;
wherein the stereocenter *1 of the catalyst according to Formula I is in (R) and/or (S) configuration.

3. The method according to claim 1 or claim 2, wherein the catalyst, particularly the catalyst of Formula I is a catalyst of Formula Ia, Formula la', Formula Ib, Formula Ic, or Formula Id: 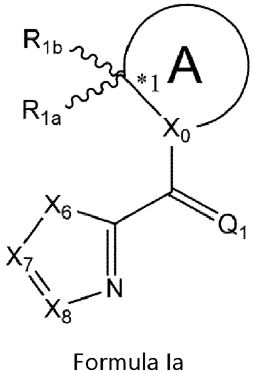 wherein in Formula Ia
Ring A, R₁ₐ and R_{1b}, Q₁ and X₀ are as defined in claim 2;
R₁ₐ and R_{1b} are different;
X₆ is selected from O, S, NR₁₃ and CR₁₃ₐR_{13b};
R₁₃ is chosen from H, linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂;
R₁₃ₐ and R_{13b} are the same or different and can be chosen from H, linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂;
X₇ and X₈ are independently selected from N and CR₆; with the provisio that when both X₇ and X₈ are represented by CR₆, the two R₆ can be the same or different and/or the two R₆ can form a ring, and with the provisio that if at least one of X₇ and X₈ is represented by CR₆, the at least one R₆ can form a ring with R₁₃, R₁₃ₐ and/or R_{13b}, preferably with the provisio that at least one of X₇ and X₈ is CR₆ and/or X₆ is CR₁₃ₐR_{13b}. According to preferred embodiments, X₇ and X₈ are independently selected from N and CR₆; with the provisio that when both X₇ and X₈ are represented by CR₆, the two R₆ can be the same or different, preferably with the provisio that at least one of X₇ and X₈ is CR₆ and/or X₆ is CR₁₃ₐR_{13b}. A formation of a ring is not preferable.
R₆ is chosen from H; F; Cl; Br; I; alkoxy groups with 1 to 20 C atoms; pseudohalogen groups; OH; NO₂; NH₂; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂;
wherein the stereocenter *1 of the catalyst according to Formula la is in (R) and/or (S) configuration; 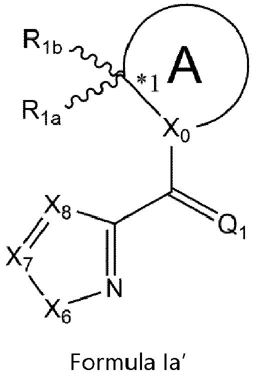 wherein in Formula la'
Ring A, R₁ₐ and R_{1b}, Q₁, X₀, X₆, X₇ and X₈ are as defined above;
R₁ₐ and R_{1b} are different; and
wherein the stereocenter *1 of the catalyst according to Formula la' is in (R) and/or (S) configuration; 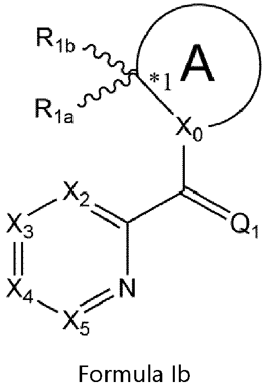 wherein in Formula Ib
Ring A, R₁ₐ and R_{1b}, Q₁ and X₀ are as defined above;
R₁ₐ and R_{1b} are different; and
X₂, X₃, X₄, and X₅ are independently selected from N and CR₆, with the provisio that when more than one of X₂, X₃, X₄, and X₅ is represented by CR₆, the more than one R₆ can be the same or different and/or more than one R₆ can form at least one ring, and with the provisio that at least one of X₂, X₃, X₄, and X₅ is CR₆;
wherein R₆ is as defined above;
wherein the stereocenter *1 of the catalyst according to Formula Ib is in (R) and/or (S) configuration; 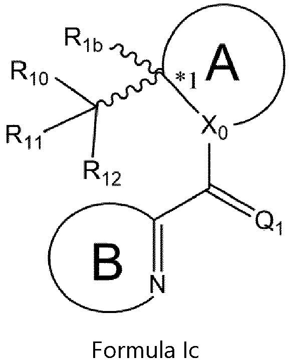 wherein in Formula Ic
Ring A, ring B, R_{1b}, Q₁ and X₀ are as defined above;
R₁₀ and R₁₁ are the same or different and are chosen from H; F; Cl; Br; I; pseudohalogen groups; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂;
R₁₂ is chosen from H, F; Cl; Br; I; pseudohalogen groups; OH; OR₁₂ₐ; SH; SR₁₂ₐ; COOH; COOR₁₂ₐ; NO₂; NH₂; (NR₁₈'R₁₉'R₂₀')⁺; and amine groups NR₁₄R₁₅, with the provisio that not both of R₁₄ and R₁₅ are H, wherein R₁₈', R₁₉', and R₂₀' are the same or different and can be chosen from H; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂;
R₁₂ₐ is chosen from H,
linear alkyl groups with 1 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups (including (CO)NH₂) with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
linear alkenyl and/or alkynyl groups with 2 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
branched alkyl, alkenyl and/or alkynyl groups with 3 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
aralkyl groups with 7 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkylaryl groups with 7 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
cycloalkyl groups with 3 to 20 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heterocycloalkyl groups with 1 to 15 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
aromatic and/or heteroaromatic groups with 2 to 40 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl heteroaromatic groups with 2 to 50 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heteroaromatic groups with at least one alkyl residue with 3 to 50 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl cycloalkyl groups with 4 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
cycloalkyl alkyl groups with 4 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl heterocycloalkyl groups with 2 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heterocycloalkyl alkyl groups with 2 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
SiR₂₃R₂₄R₂s;
R₂₃, R₂₄, and R₂₅ are the same or different and are selected from H, linear alkyl groups with 1 to 4 C atoms that can optionally be substituted with at least one of F, Cl, Br, I, pseudohalogen groups, alkoxy groups with 1 to 20 C atoms, OH, NO₂ and NH₂, branched alkyl group with 3 to 6 C atoms that can optionally be substituted with at least one of F, Cl, Br, I, pseudohalogen groups, alkoxy groups with 1 to 20 C atoms, OH, NO₂ and NH₂, aromatic groups with 6 to 12 C atoms that can optionally be substituted with at least one of F, Cl, Br, I, pseudohalogen groups, alkoxy groups with 1 to 20 C atoms, OH, NO₂ and NH₂, and aralkyl groups with 7 to 10 C atoms that can optionally be substituted with at least one of F, Cl, Br, I, pseudohalogen groups, alkoxy groups with 1 to 20 C atoms, OH, NO₂ and NH₂, preferably wherein R₂₃, R₂₄, and R₂₅ are not all hydrogen at the same time and/or wherein R₂₃, R₂₄, and R₂₅ are not all methyl at the same time, further preferably wherein all of R₂₃, R₂₄, and R₂₅ are ethyl;
R₁₄ and R₁₅ are the same or different and can be chosen from H,
linear alkyl groups with 1 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups (including (CO)NH₂) with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
linear alkenyl and/or alkynyl groups with 2 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
branched alkyl, alkenyl and/or alkynyl groups with 3 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
aralkyl groups with 7 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkylaryl groups with 7 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
cycloalkyl groups with 3 to 20 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heterocycloalkyl groups with 1 to 15 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
aromatic and/or heteroaromatic groups with 2 to 40 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl heteroaromatic groups with 2 to 50 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heteroaromatic groups with at least one alkyl residue with 3 to 50 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl cycloalkyl groups with 4 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
cycloalkyl alkyl groups with 4 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl heterocycloalkyl groups with 2 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heterocycloalkyl alkyl groups with 2 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂.
wherein the stereocenter *1 of the catalyst according to Formula Ic is in (R) and/or (S) configuration; 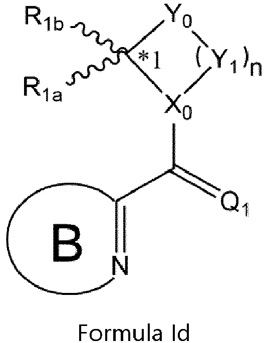 wherein in Formula Id
Ring B, R₁ₐ and R_{1b}, Q₁ and X₀ are as defined above;
R₁ₐ and R_{1b} are different;
Y₁ is chosen from substituted or unsubstituted C atoms and/or substituted or unsubstituted heteroatoms, wherein the substituents can comprise any one of F; Cl; Br; I; pseudohalogen groups; alkoxy groups with 1 to 20 C atoms; OH; SR₂₁; S(CO)R₂₂; NO₂; NH₂; amines of formula NR₁₈R₁₉; (NR₁₈'R₁₉'R₂₀')⁺; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂; with the provisio that two or more of the substituents can form a ring or more rings, optionally wherein at least one substituent is part of a polymer, and with the provisio that if more than one Y₁ is present, these can be the same or different;
n is an integer from 0 to 8; and
Y₀ is chosen from a carbonyl group, a thiocarbonyl group, and CR₁₆R₁₇;
R₁₆ is chosen from H; F; Cl; Br; I; pseudohalogen groups; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂;
R₁₇ is chosen from alkoxy groups with 1 to 20 C atoms; OH; NO₂; NH₂; and amine groups NR₁₈R₁₉,
R₁₈, R₁₉, R₁₈', R₁₉', R₂₀', R₂₁, and R₂₂ are the same or different and can be chosen from H; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂, with the provisio that not both of R₁₈ and R₁₉ are H;
wherein the stereocenter *1 of the catalyst according to Formula Id is in (R) and/or (S) configuration.

4. The method according to claim 3, wherein the catalyst of Formula Ia is used and is a catalyst of the following Formula II: 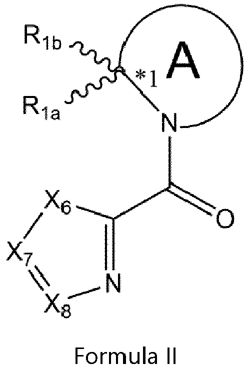 wherein
Ring A, R₁ₐ, R_{1b}, X₆, X₇ and Xs are as defined in claim 3;
R₁ₐ and R_{1b} are different;
wherein the stereocenter *1 of the catalyst according to Formula II is in (R) and/or (S) configuration.

5. The method according to claim 4, wherein the catalyst of Formula II is a catalyst of the following Formula IIa 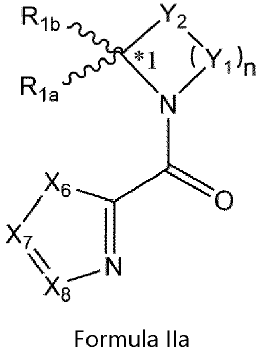 wherein
R₁ₐ, R_{1b}, X₆, X₇ and X₈ are as defined in claim 4;
R₁ₐ and R_{1b} are different;
Y₁ is chosen from substituted or unsubstituted C atoms and/or substituted or unsubstituted heteroatoms, wherein the substituents can comprise any one of F; Cl; Br; I; pseudohalogen groups; alkoxy groups with 1 to 20 C atoms; OH; SR₂₁; S(CO)R₂₂; NO₂; NH₂; amines of formula NR₁₈R₁₉; (NR₁₈'R₁₉'R₂₀')⁺; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂; with the provisio that two or more of the substituents can form a ring or more rings, optionally wherein at least one substituent is part of a polymer, and with the provisio that if more than one Y₁ is present, these can be the same or different;
Y₂ is chosen from a carbonyl group, a thiocarbonyl group, and substituted or unsubstituted C atoms and/or substituted or unsubstituted heteroatoms, wherein the substituents can comprise any one of F; Cl; Br; I; pseudohalogen groups; alkoxy groups with 1 to 20 C atoms; OH; NO₂; NH₂; amines of formula NR₁₈R₁₉; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂;
R₁₈, R₁₉, R₁₈', R₁₉', R₂₀', R₂₁, and R₂₂ are the same or different and can be chosen from H; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂, with the provisio that not both of R₁₈ and R₁₉ are H;
n is an integer from 0 to 8;
wherein the stereocenter *1 of the catalyst according to Formula IIa is in (R) and/or (S) configuration;
preferably wherein the catalyst of Formula IIa is a catalyst of the following Formula IIa-1 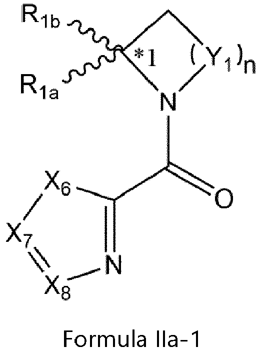 wherein
R₁ₐ, R_{1b}, n, Y₁, X₆, X₇ and X₈ are as defined above;
R₁ₐ and R_{1b} are different;
wherein the stereocenter *1 of the catalyst according to Formula IIa-1 is in (R) and/or (S) configuration;
or wherein the catalyst of Formula II is a catalyst of the following Formula IIb 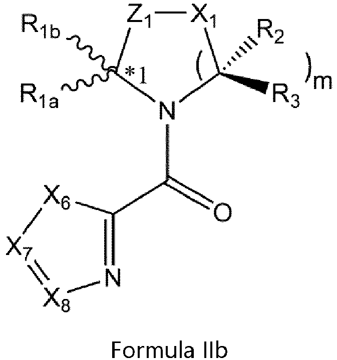 wherein
R₁ₐ, R_{1b}, X₆, X₇ and X₈ are as defined above;
R₁ₐ and R_{1b} are different;
m is an integer from 0 to 7;
R₂ and R₃ are the same or different and are independently selected from H; F; Cl; Br; I; pseudohalogen groups; alkoxy groups with 1 to 20 C atoms; OH; NO₂; NH₂; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂;
X₁ is selected from the group of O, NR₄, and CR₄ₐR_{4b}, wherein R₄ₐ and R_{4b} can be the same or different;
R₄, R₄ₐ, and R_{4b} are independently selected from H; F; Cl; Br; I; pseudohalogen groups; alkoxy groups with 1 to 20 C atoms; OH; SR₂₁; S(CO)R₂₂; NO₂; NH₂; amines of formula NR₁₈R₁₉; (NR₁₈'R₁₉'R₂₀')⁺; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester (carboxylate) groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂; wherein R₄ₐ and R_{4b} can be the same or different; with the provisio that two or more of R₂, R₃, R₄, R₄ₐ, and R_{4b} can form a ring or more rings, optionally wherein at least one of R₂, R₃, R₄, R₄ₐ, and R_{4b} is part of a polymer, wherein R₁₈, R₁₉, R₁₈', R₁₉', R₂₀', R₂₁, and R₂₂ are the same or different and can be chosen from H; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂, with the provisio that not both of R₁₈ and R₁₉ are H;
Z₁ is chosen from a carbonyl group, a thiocarbonyl group, and CR₂₀ₐR_{20b};
R₂₀ₐ and R_{20b} are the same or different and can be chosen from H, linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂;
wherein the stereocenter *1 of the catalyst according to Formula IIb is in (R) and/or (S) configuration;
preferably wherein the catalyst of Formula IIb is a catalyst of the following Formula IIb1 or a catalyst of the following Formula IIb2 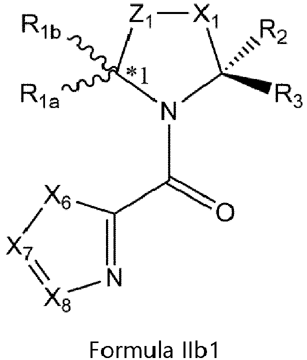 wherein
R₁ₐ, R_{1b}, R₂, R₃, X₁, X₆, X₇, X₈ and Z₁ are as defined above;
R₁ₐ and R_{1b} are different;
wherein the stereocenter *1 of the catalyst according to Formula IIb1 is in (R) and/or (S) configuration; 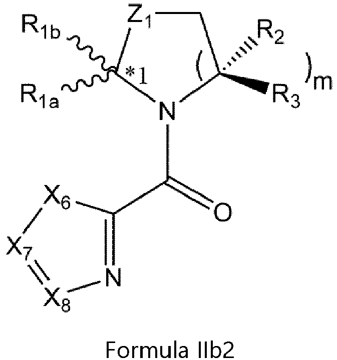 wherein
m, R₁ₐ, R_{1b}, R₂, R₃, X₆, X₇, X₈ and Z₁ are as defined above;
R₁ₐ and R_{1b} are different;
wherein the stereocenter *1 of the catalyst according to Formula IIb2 is in (R) and/or (S) configuration.

6. The method according to claim 4 or 5, wherein in the catalyst of Formula II, respectively the catalyst of Formula IIa or the catalyst of Formula IIb, preferably the catalyst of Formula IIa-1, the catalyst of Formula IIb1, or the catalyst of Formula IIb2, R₁ₐ is represented by a tertiary residue CR₁₀R₁₁R₁₂, wherein
R₁₀ and R₁₁ are the same or different and are chosen from H; F; Cl; Br; I; pseudohalogen groups; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkly and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂; and
R₁₂ is chosen from H, F; Cl; Br; I; pseudohalogen groups; OH; OR₁₂ₐ; SH; SR₁₂ₐ; COOH; COOR₁₂ₐ; NO₂; NH₂; (NR₁₈'R₁₉'R₂₀')⁺; and amine groups NR₁₄R₁₅, with the provisio that not both of R₁₄ and R₁₅ are H, wherein R₁₈', R₁₉', and R₂₀' are the same or different and can be chosen from H; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂;
R₁₂ₐ is chosen from H,
linear alkyl groups with 1 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups (including (CO)NH₂) with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
linear alkenyl and/or alkynyl groups with 2 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
branched alkyl, alkenyl and/or alkynyl groups with 3 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
aralkyl groups with 7 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkylaryl groups with 7 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
cycloalkyl groups with 3 to 20 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heterocycloalkyl groups with 1 to 15 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
aromatic and/or heteroaromatic groups with 2 to 40 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl heteroaromatic groups with 2 to 50 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heteroaromatic groups with at least one alkyl residue with 3 to 50 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl cycloalkyl groups with 4 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
cycloalkyl alkyl groups with 4 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl heterocycloalkyl groups with 2 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heterocycloalkyl alkyl groups with 2 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
SiR₂₃R₂₄R₂₅;
R₂₃, R₂₄, and R₂₅ are the same or different and are selected from H, linear alkyl groups with 1 to 4 C atoms that can optionally be substituted with at least one of F, Cl, Br, I, pseudohalogen groups, alkoxy groups with 1 to 20 C atoms, OH, NO₂ and NH₂, branched alkyl group with 3 to 6 C atoms that can optionally be substituted with at least one of F, Cl, Br, I, pseudohalogen groups, alkoxy groups with 1 to 20 C atoms, OH, NO₂ and NH₂, aromatic groups with 6 to 12 C atoms that can optionally be substituted with at least one of F, Cl, Br, I, pseudohalogen groups, alkoxy groups with 1 to 20 C atoms, OH, NO₂ and NH₂, and aralkyl groups with 7 to 10 C atoms that can optionally be substituted with at least one of F, Cl, Br, I, pseudohalogen groups, alkoxy groups with 1 to 20 C atoms, OH, NO₂ and NH₂, preferably wherein R₂₃, R₂₄, and R₂₅ are not all hydrogen at the same time and/or wherein R₂₃, R₂₄, and R₂₅ are not all methyl at the same time, further preferably wherein all of R₂₃, R₂₄, and R₂₅ are ethyl;
R₁₄ and R₁₅ are the same or different and can be chosen from H;
linear alkyl groups with 1 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups (including (CO)NH₂) with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
linear alkenyl and/or alkynyl groups with 2 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
branched alkyl, alkenyl and/or alkynyl groups with 3 to 10 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
aralkyl groups with 7 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkylaryl groups with 7 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
cycloalkyl groups with 3 to 20 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heterocycloalkyl groups with 1 to 15 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
aromatic and/or heteroaromatic groups with 2 to 40 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl heteroaromatic groups with 2 to 50 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heteroaromatic groups with at least one alkyl residue with 3 to 50 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl cycloalkyl groups with 4 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
cycloalkyl alkyl groups with 4 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
alkyl heterocycloalkyl groups with 2 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
heterocycloalkyl alkyl groups with 2 to 24 C atoms that can optionally be substituted with at least one of F, Cl, Br, I, pseudohalogen groups, carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂, alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂, carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂, alkoxy groups with 1 to 20 C atoms, OH, SH, NO₂ and NH₂;
and/or
wherein X₆ is NR₁₃, wherein
R₁₃ is chosen from H, linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂;heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂.

7. The method according to claim 3, wherein the catalyst of Formula lb is used and is a catalyst of the following Formula III 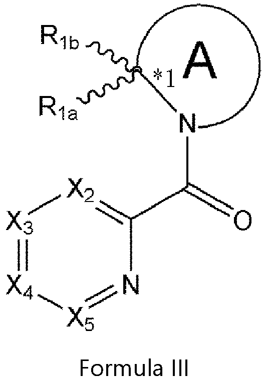 wherein
Ring A, R₁ₐ, R_{1b}, X₂, X₃, X₄ and X₅ are as defined in claim 3;
R₁ₐ and R_{1b} are different;
wherein the stereocenter *1 of the catalyst according to Formula III is in (R) and/or (S) configuration.

8. The method according to claim 7, wherein the catalyst of Formula III is represented by the following Formula IIIa 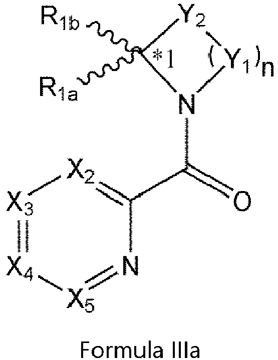 wherein
R₁ₐ, R_{1b}, X₂, X₃, X₄ and X₅ are as defined in claim 6;
R₁ₐ and R_{1b} are different;
n is an integer from 0 to 8;
Y₁ is chosen from substituted or unsubstituted C atoms and/or substituted or unsubstituted heteroatoms, wherein the substituents can comprise any one of F; Cl; Br; I; pseudohalogen groups; alkoxy groups with 1 to 20 C atoms; OH; SR₂₁; S(CO)R₂₂; NO₂; NH₂; amines of formula NR₁₈R₁₉; (NR₁₈'R₁₉'R₂₀')⁺; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂; with the provisio that two or more of the substituents can form a ring or more rings, optionally wherein at least one substituent is part of a polymer, and with the provisio that if more than one Y₁ is present, these can be the same or different, wherein
R₁₈, R₁₉, R₁₈', R₁₉', R₂₀', R₂₁, and R₂₂ are the same or different and can be chosen from H; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂, with the provisio that not both of R₁₈ and R₁₉ are H;
Y₂ is chosen from a carbonyl group, a thiocarbonyl group, and substituted or unsubstituted C atoms and/or substituted or unsubstituted heteroatoms, wherein the substituents can comprise any one of F; Cl; Br; I; pseudohalogen groups; alkoxy groups with 1 to 20 C atoms; OH; NO₂; NH₂; amines of formula NR₁₈R₁₉; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂;
wherein the stereocenter *1 of the catalyst according to Formula IIIa is in (R) and/or (S) configuration.

9. The method according to claim 8, wherein the catalyst of Formula IIIa is represented by the following Formula IIIa2-a1a 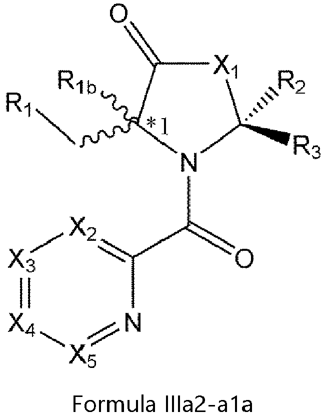 wherein
R_{1b}, X₂, X₃, X₄ and X₅ are as defined in claim 7;
R₁ is selected from the group consisting of linear alkyl groups with 1 to 9 C atoms that can optionally be substituted with at least one of F, Cl, Br, I, pseudohalogen groups, alkoxy groups with 1 to 20 C atoms, OH, SH, NO₂ and NH₂; branched alkyl groups with 3 to 9 C atoms that can optionally be substituted with at least one of F, Cl, Br, I, pseudohalogen groups, alkoxy groups with 1 to 20 C atoms, OH, SH, NO₂ and NH₂; aralkyl groups with 7 to 23 C atoms that can optionally be substituted with at least one of F, Cl, Br, I, pseudohalogen groups, alkoxy groups with 1 to 20 C atoms, OH, SH, NO₂ and NH₂; alkylaryl groups with 7 to 23 C atoms that can optionally be substituted with at least one of F, Cl, Br, I, pseudohalogen groups, alkoxy groups with 1 to 20 C atoms, OH, SH, NO₂ and NH₂; cycloalkyl groups with 3 to 19 C atoms that can optionally be substituted with at least one of F, Cl, Br, I, pseudohalogen groups, alkoxy groups with 1 to 20 C atoms, OH, SH, NO₂ and NH₂; heterocycloalkyl groups with 1 to 14 C atoms that can optionally be substituted with at least one of F, Cl, Br, I, pseudohalogen groups, alkoxy groups with 1 to 20 C atoms, OH, SH, NO₂ and NH₂; aromatic groups with 6 to 19 C atoms that can optionally be substituted with at least one of F, Cl, Br, I, pseudohalogen groups, alkoxy groups with a linear, branched, cyclic and/or aromatic residue having 1 to 20 C atoms that can comprise one or more heteroatoms selected from O, N, S and P, carboxylic ester groups with a linear, branched, cyclic and/or aromatic residue having 1 to 20 C atoms that can comprise one or more heteroatoms selected from O, N, S and P, oxycarbonyl groups with a linear, branched, cyclic and/or aromatic residue having 1 to 20 C atoms that can comprise one or more heteroatoms selected from O, N, S and P, carboxamide groups with a linear, branched, cyclic and/or aromatic residue having 1 to 20 C atoms that can comprise one or more heteroatoms selected from O, N, S and P, OH, SH, NO₂ and NH₂; heteroaromatic groups with 2 to 39 C atoms that can optionally be substituted with at least one of F, Cl, Br, I, pseudohalogen groups, alkoxy groups with 1 to 20 C atoms, OH, SH, NO₂ and NH₂; alkyl heteroaromatic groups with 3 to 49 C atoms that can optionally be substituted with at least one of F, Cl, Br, I, pseudohalogen groups, alkoxy groups with 1 to 20 C atoms, OH, SH, NO₂ and NH₂, heteroaromatic groups with at least one alkyl and/or aromatic residue (i.e. wherein an alkyl or aromatic group is bonded to a heteroaromatic group) with 3 to 49 C atoms that can optionally be substituted with at least one of F, Cl, Br, I, pseudohalogen groups, alkoxy groups with 1 to 20 C atoms, OH, SH, NO₂ and NH₂; alkyl cycloalkyl groups with 4 to 23 C atoms that can optionally be substituted with at least one of F, Cl, Br, I, pseudohalogen groups, alkoxy groups with 1 to 20 C atoms, OH, SH, NO₂ and NH₂; cycloalkyl alkyl groups with 4 to 23 C atoms that can optionally be substituted with at least one of F, Cl, Br, I, pseudohalogen groups, alkoxy groups with 1 to 20 C atoms, OH, SH, NO₂ and NH₂; carboxylic acid ester groups with 1 to 19 C atoms that can optionally be substituted with at least one of F, Cl, Br, I, pseudohalogen groups, alkoxy groups with 1 to 20 C atoms, OH, SH, NO₂ and NH₂; alkyl and/or (hetero)aryl oxycarbonyl groups with 1 to 19 C atoms that can optionally be substituted with at least one of F, Cl, Br, I, pseudohalogen groups, alkoxy groups with 1 to 20 C atoms, OH, SH, NO₂ and NH₂; and carboxamide groups with 1 to 19 C atoms that can optionally be substituted with at least one of F, Cl, Br, I, pseudohalogen groups, alkoxy groups with 1 to 20 C atoms, OH, SH, NO₂ and NH₂; preferably wherein R₁ is selected from aromatic groups with 6 to 39 C atoms, particularly phenyl groups and naphtyl groups, that can optionally be substituted with at least one of F, Cl, Br, I, pseudohalogen groups, alkoxy groups with 1 to 20 C atoms, carboxylic ester groups with 1 to 20 C atoms, alkly and/or (hetero)aryl oxycarbonyl groups with 1 to 20 C atoms, carboxamide groups with 1 to 20 C atoms, OH, NO₂ and NH₂;
R₂ and R₃ are the same or different and are independently selected from H; F; Cl; Br; I; pseudohalogen groups; alkoxy groups with 1 to 20 C atoms; OH; NO₂; NH₂; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂;
wherein the stereocenter *1 of the catalyst according to Formula IIIa2-a1a is in (R) and/or (S) configuration.

10. The method according to claim 3, wherein the catalyst of Formula Ic is used and is a catalyst of the following Formula IV 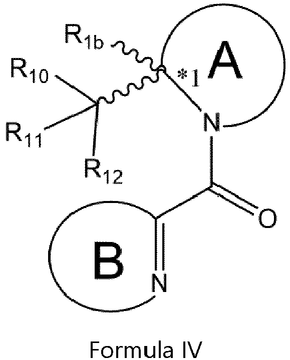 wherein
Ring A, ring B, R_{1b}, R₁₀, R₁₁ and R₁₂ are as defined in claim 3;
R₁ₐ and R_{1b} are different;
wherein the stereocenter *1 of the catalyst according to Formula IV is in (R) and/or (S) configuration.

11. The method according to claim 3, wherein the catalyst of Formula la' is used and is a catalyst of the following Formula II' 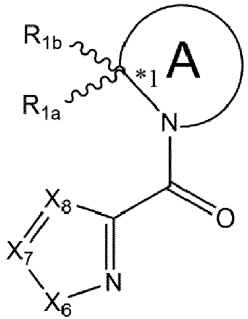 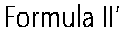 wherein
Ring A, R₁ₐ, R_{1b}, X₆, X₇ and X₈ are as defined in claim 3;
R₁ₐ and R_{1b} are different;
wherein the stereocenter *1 of the catalyst according to Formula II' is in (R) and/or (S) configuration.

12. The method according to claim 3, wherein the catalyst of Formula Id is used and is a catalyst of the following Formula V 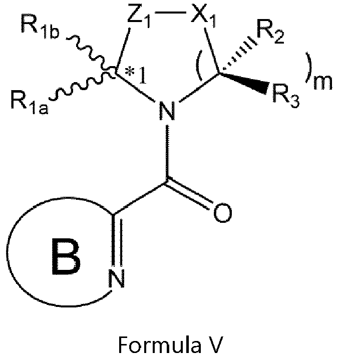 wherein
Ring B, R₁ₐ, and R_{1b} are as defined in claim 3;
R₁ₐ and R_{1b} are different;
m is an integer from 0 to 7;
R₂ and R₃ are the same or different and are independently selected from H; F; Cl; Br; I; pseudohalogen groups; alkoxy groups with 1 to 20 C atoms; OH; NO₂; NH₂; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂;
X₁ is selected from the group of O, NR₄, and CR₄ₐR_{4b}, wherein R₄ₐ and R_{4b} can be the same or different;
R₄, R₄ₐ, and R_{4b} are independently selected from H; F; Cl; Br; I; pseudohalogen groups; alkoxy groups with 1 to 20 C atoms; OH; SR₂₁; S(CO)R₂₂; NO₂; NH₂; amines of formula NR₁₈R₁₉; (NR₁₈'R₁₉'R₂₀')⁺; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester (carboxylate) groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂; wherein R₄ₐ and R_{4b} can be the same or different; with the provisio that two or more of R₂, R₃, R₄, R₄ₐ, and R_{4b} can form a ring or more rings, optionally wherein at least one of R₂, R₃, R₄, R₄ₐ, and R_{4b} is part of a polymer, wherein R₁₈, R₁₉, R₁₈', R₁₉', R₂₀', R₂₁, and R₂₂ are the same or different and can be chosen from H; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂, with the provisio that not both of R₁₈ and R₁₉ are H;
Z₁ is chosen from a carbonyl group, a thiocarbonyl group, and CR₂₀ₐR_{20b};
R₂₀ₐ and R_{20b} are the same or different and can be chosen from H, linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂;
wherein the stereocenter *1 of the catalyst according to Formula V is in (R) and/or (S) configuration.

13. The method according to any one of the preceding claims, wherein
the reaction is conducted at a temperature in the range of -80 °C to 100 °C, preferably -30 to 50 °C, further preferably -20 °C to 25 °C, and/or wherein
the reaction is conducted in a solvent comprising at least one non-protic solvent, preferably wherein the at least one solvent in selected from the group consisting of dichloromethane (DCM), (trichloromethyl)benzene, (trifluoromethyl)benzene, chloroform, o-xylene, m-xylene, p-xylene, toluene, benzene, chlorobenzene, ethers like anisole and Me-THF, acetonitrile, ethyl acetate (EtOAc), acetone and other ketones, and mixtures of two or more thereof.

14. The method according to any one of the preceding claims, wherein the at least one hydrosilane has a Si-H bonding strength in the range of 84 to 100 kcal/mol, and/or wherein the at least one hydrosilane is selected from the group of silanes of formulas ABCSiH, ABSiH₂, and ASiH₃, wherein A, B and C can be the same or different and are selected from the group consisting of F; Cl; Br; I; alkyl groups with 1 to 20 C atoms that can optionally be substituted with at least one of F, Cl, Br, I, pseudohalogen groups, and alkoxy groups with 1 to 20 C atoms; aryl groups with 6 to 20 C atoms that can optionally be substituted with at least one of F, Cl, Br, I, pseudohalogen groups, and alkoxy groups with 1 to 20 C atoms; aralkyl groups with 7 to 24 C atoms that can optionally be substituted with at least one of F, Cl, Br, I, pseudohalogen groups, and alkoxy groups with 1 to 20 C atoms; and alkylaryl groups with 7 to 24 C atoms that can optionally be substituted with at least one of F, Cl, Br, I, pseudohalogen groups, and alkoxy groups with 1 to 20 C atoms; optionally wherein the at least one hydrosilane is Cl₃SiH.

15. The method according to any one of the preceding claims, wherein the reaction is carried out in the presence of an acid and/or water and/or another protic solvent.

16. The method according to any one of the preceding claims, wherein
the catalyst is present in an amount in the range of 0.001 to 60 mol%, preferably 0.005 to 55 mol%, e.g. 0.1 to 50 mol%, with respect to the amount of myosmine, optionally in the range of 0.3 to 20 mol% with respect to the amount of myosmine, and/or wherein
the hydrosilane is present in an amount in the range of 1 to 10 equivalents with respect to the amount of myosmine, optionally in the range of 1.2 to 8 equivalents, further optionally in the range of 1.5 to 6 equivalents, further optionally in the range of 2 to 5 equivalents with respect to the amount of myosmine.

17. The method according to any one of the preceding claims, wherein the nornicotine is produced as a mixture of (R)- and (S)-nornicotine.

18. The method according to any one of the preceding claims, further comprising removing the catalyst after reacting myosmine with at least one hydrosilane in the presence of the catalyst.

19. A composition, produced by the method according to any one of claims 1-17, particularly wherein the catalyst is not removed from the reaction mixture.

20. A composition, comprising nornicotine, optionally a mixture of (R)- and (S)-nornicotine, and a catalyst, wherein the catalyst is an organic chiral Lewis base.

21. The composition according to claim 19, wherein the enantiomeric excess (ee) of one stereoisomer of nornicotine is in the range of 5 to 100%, optionally in the range of 10 to 99.9%, further optionally in the range of 20 to 99%

22. A method of producing nicotine or a salt thereof from myosmine, comprising:
reacting myosmine with at least one hydrosilane in the presence of a catalyst to produce nornicotine, wherein the catalyst is an organic chiral Lewis base;
reacting the nornicotine to nicotine; and
optionally reacting the nicotine to a salt thereof.

23. A composition, produced by the method according to claim 21, wherein the catalyst is not separated from the reaction mixture.

24. A composition, comprising nicotine, optionally a mixture of (R)- and (S)-nicotine, or a salt thereof, and a catalyst, wherein the catalyst is an organic chiral Lewis base.

25. A catalyst of the following formula I 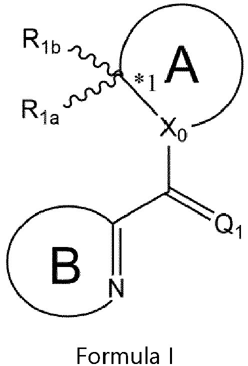 wherein
Ring A comprises, besides X₀ and the chiral C atom with residues R₁ₐ and R_{1b}, a carbonyl group, a thiocarbonyl group, substituted or unsubstituted C atoms and/or substituted or unsubstituted heteroatoms, wherein the substituents can comprise any one of F; Cl; Br; I; pseudohalogen groups; alkoxy groups with 1 to 20 C atoms; OH; NO₂; NH₂; amines of formula NR₁₈R₁₉; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; linear alkenyl groups and/or alkynyl groups with 2 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; branched alkenyl and/or alkynyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂; with the provisio that two or more of the substituents can form one ring or more rings, optionally wherein at least one substituent is part of a polymer;
Ring B is a five- or six-membered ring comprising conjugated double bonds that is particularly aromatic, i.e. is fully conjugated, planar and follows the Hueckel's rule, and that comprises, besides the carbon atom connected to the bridge and the adjacent nitrogen atom, substituted or unsubstituted C atoms and/or substituted or unsubstituted heteroatoms, wherein the substituents can comprise any one of F; Cl; Br; I; pseudohalogen groups; alkoxy groups with 1 to 20 C atoms; OH; NO₂; NH₂; amines of formula NR₁₈R₁₉; linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂; alkyl heterocycloalkyl groups with 2 to 24 C atoms that can optionally be substituted with at least one of F, Cl, Br, I, pseudohalogen groups, alkoxy groups with 1 to 20 C atoms, OH, NO₂ and NH₂; heterocycloalkyl alkyl groups with 2 to 24 C atoms that can optionally be substituted with at least one of F, Cl, Br, I, pseudohalogen groups, alkoxy groups with 1 to 20 C atoms, OH, NO₂ and NH₂; carboxylic acid ester groups with 1 to 20 C atoms that can optionally be substituted with at least one of F, Cl, Br, I, pseudohalogen groups, alkoxy groups with 1 to 20 C atoms, OH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, NO₂ and NH₂; and carboxamide groups with 1 to 20 C atoms that can optionally be substituted with at least one of F, Cl, Br, I, pseudohalogen groups, alkoxy groups with 1 to 20 C atoms, OH, NO₂ and NH₂; with the provisio that two or more of the substituents can form one ring or more rings;
wherein R₁₈ and R₁₉ are the same or different and can be chosen from H, linear alkyl groups with 1 to 10 C atoms that can optionally be substituted with at least one of F, Cl, Br, I, pseudohalogen groups, alkoxy groups with 1 to 20 C atoms, OH, NO₂ and NH₂; branched alkyl groups with 3 to 10 C atoms that can optionally be substituted with at least one of F, Cl, Br, I, pseudohalogen groups, alkoxy groups with 1 to 20 C atoms, OH, NO₂ and NH₂; aralkyl groups with 7 to 24 C atoms that can optionally be substituted with at least one of F, Cl, Br, I, pseudohalogen groups, alkoxy groups with 1 to 20 C atoms, OH, NO₂ and NH₂; alkylaryl groups with 7 to 24 C atoms that can optionally be substituted with at least one of F, Cl, Br, I, pseudohalogen groups, alkoxy groups with 1 to 20 C atoms, OH, NO₂ and NH₂; cycloalkyl groups with 3 to 20 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 15 C atoms that can optionally be substituted with at least one of F, Cl, Br, I, pseudohalogen groups, alkoxy groups with 1 to 20 C atoms, OH, NO₂ and NH₂; aromatic and/or heteroaromatic groups with 2 to 40 C atoms that can optionally be substituted with at least one of F, Cl, Br, I, pseudohalogen groups, alkoxy groups with 1 to 20 C atoms, OH, NO₂ and NH₂; alkyl heteroaromatic groups with 3 to 50 C atoms that can optionally be substituted with at least one of F, Cl, Br, I, pseudohalogen groups, alkoxy groups with 1 to 20 C atoms, OH, NO₂ and NH₂; heteroaromatic groups with at least one alkyl residue with 3 to 50 C atoms that can optionally be substituted with at least one of F, Cl, Br, I, pseudohalogen groups, alkoxy groups with 1 to 20 C atoms, OH, NO₂ and NH₂; alkyl cycloalkyl groups with 4 to 24 C atoms that can optionally be substituted with at least one of F, Cl, Br, I, pseudohalogen groups, alkoxy groups with 1 to 20 C atoms, OH, NO₂ and NH₂; cycloalkyl alkyl groups with 4 to 24 C atoms that can optionally be substituted with at least one of F, Cl, Br, I, pseudohalogen groups, alkoxy groups with 1 to 20 C atoms, OH, NO₂ and NH₂, with the provisio that not both of R₁₈ and R₁₉ are H;
X₀ is selected from N and CR_{A1}, wherein
R_{A1} is selected from the group consisting of H, linear alkyl groups with 1 to 6 C atoms that can optionally be substituted with at least one of F, Cl, Br, I, pseudohalogen groups, alkoxy groups with 1 to 6 C atoms, OH, NO₂ and NH₂; branched alkyl groups with 3 to 6 C atoms that can optionally be substituted with at least one of F, Cl, Br, I, pseudohalogen groups, alkoxy groups with 1 to 6 C atoms, OH, NO₂ and NH₂; cycloalkyl groups with 3 to 7 C atoms that can optionally be substituted with at least one of F, Cl, Br, I, pseudohalogen groups, alkoxy groups with 1 to 20 C atoms, OH, NO₂ and NH₂; heterocycloalkyl groups with 1 to 6 C atoms that can optionally be substituted with at least one of F, Cl, Br, I, pseudohalogen groups, alkoxy groups with 1 to 6 C atoms, OH, NO₂ and NH₂; aromatic and/or heteroaromatic groups with 2 to 6 carbon atoms that can optionally be substituted with at least one of F, Cl, Br, I, pseudohalogen groups, alkoxy groups with 1 to 6 C atoms, OH, NO₂ and NH₂; carboxylic acid ester groups with 1 to 6 C atoms that can optionally be substituted with at least one of F, Cl, Br, I, pseudohalogen groups, alkoxy groups with 1 to 6 C atoms, OH, NO₂ and NH₂; alkyl and/or (hetero)aryl oxycarbonyl groups with 1 to 6 C atoms that can optionally be substituted with at least one of F, Cl, Br, I, pseudohalogen groups, alkoxy groups with 1 to 6 C atoms, OH, NO₂ and NH₂; and carboxamide groups with 1 to 6 C atoms that can optionally be substituted with at least one of F, Cl, Br, I, pseudohalogen groups, alkoxy groups with 1 to 6 C atoms, OH, NO₂ and NH₂;
Q₁ is O or S;
R₁ₐ and R_{1b} are different;
R₁ₐ is a saturated and/or unsaturated linear, branched, cyclic and/or aromatic and/or heteroaromatic hydrocarbon residue with 1 to 100 C atoms that can contain heteroatoms selected from the group of O, N, S and P and that can optionally be substituted with at least one of F; Cl; Br; I; pseudohalogen groups; OSiR₂₃R₂₄R₂₅; carboxylic acid ester groups with 1 to 20 C atoms that can optionally be substituted with at least one of F, Cl, Br, I, pseudohalogen groups, alkoxy groups with 1 to 20 C atoms, OH, SH, NO₂ and NH₂; alkyl and/or (hetero)aryl oxycarbonyl groups with 1 to 20 C atoms that can optionally be substituted with at least one of F, Cl, Br, I, pseudohalogen groups, alkoxy groups with 1 to 20 C atoms, OH, SH, NO₂ and NH₂; carboxamide groups with 1 to 20 C atoms that can optionally be substituted with at least one of F, Cl, Br, I, pseudohalogen groups, alkoxy groups with 1 to 20 C atoms, OH, SH, NO₂ and NH₂; optionally substituted linear, branched and/or cyclic alkoxy, aryloxy, heteroaryloxy, alkenyloxy and/or alkynyloxy groups with 1 to 20 C atoms and/or heteroatoms; OH; NO₂; SH; NH₂; and amines of formula NR₁₈R₁₉;
R₂₃, R₂₄, and R₂₅ are the same or different and are selected from H, linear alkyl groups with 1 to 4 C atoms that can optionally be substituted with at least one of F, Cl, Br, I, pseudohalogen groups, alkoxy groups with 1 to 20 C atoms, OH, NO₂ and NH₂, branched alkyl group with 3 to 6 C atoms that can optionally be substituted with at least one of F, Cl, Br, I, pseudohalogen groups, alkoxy groups with 1 to 20 C atoms, OH, NO₂ and NH₂, aromatic groups with 6 to 12 C atoms that can optionally be substituted with at least one of F, Cl, Br, I, pseudohalogen groups, alkoxy groups with 1 to 20 C atoms, OH, NO₂ and NH₂, and aralkyl groups with 7 to 10 C atoms that can optionally be substituted with at least one of F, Cl, Br, I, pseudohalogen groups, alkoxy groups with 1 to 20 C atoms, OH, NO₂ and NH₂, preferably wherein R₂₃, R₂₄, and R₂₅ are not all hydrogen at the same time and/or wherein R₂₃, R₂₄, and R₂₅ are not all methyl at the same time, further preferably wherein all of R₂₃, R₂₄, and R₂₅ are ethyl;
R_{1b} is selected from the group consisting of H, linear alkyl groups with 1 to 6 C atoms that can optionally be substituted with at least one of F, Cl, Br, I, pseudohalogen groups, alkoxy groups with 1 to 6 C atoms, OH, NO₂ and NH₂; branched alkyl groups with 3 to 6 C atoms that can optionally be substituted with at least one of F, Cl, Br, I, pseudohalogen groups, alkoxy groups with 1 to 6 C atoms, OH, NO₂ and NH₂; heterocycloalkyl groups with 1 to 6 C atoms that can optionally be substituted with at least one of F, Cl, Br, I, pseudohalogen groups, alkoxy groups with 1 to 6 C atoms, OH, NO₂ and NH₂; aromatic and/or heteroaromatic groups with 2 to 6 carbon atoms that can optionally be substituted with at least one of F, Cl, Br, I, pseudohalogen groups, alkoxy groups with 1 to 6 C atoms, OH, NO₂ and NH₂; carboxylic acid ester groups with 1 to 6 C atoms that can optionally be substituted with at least one of F, Cl, Br, I, pseudohalogen groups, alkoxy groups with 1 to 6 C atoms, OH, NO₂ and NH₂; alkyl and/or (hetero)aryl oxycarbonyl groups with 1 to 6 C atoms that can optionally be substituted with at least one of F, Cl, Br, I, pseudohalogen groups, alkoxy groups with 1 to 6 C atoms, OH, NO₂ and NH₂; and carboxamide groups with 1 to 6 C atoms that can optionally be substituted with at least one of F, Cl, Br, I, pseudohalogen groups, alkoxy groups with 1 to 6 C atoms, OH, NO₂ and NH₂, preferably wherein R_{1b} is H or methyl;
wherein the stereocenter *1 of the catalyst according to Formula I is in (R) and/or (S) configuration.

26. Use of the catalyst of claim 25 in a method of producing nornicotine and/or nicotine and/or a salt of nicotine, particularly from myosmine.
